# EUROPEAN PATENT SPECIFICATION

(11) **EP 2 112 166 B1**
(45) Date of publication and mention of the grant of the patent: **21.11.2018**
(21) Application number: 09007161.4
(22) Date of filing: 23.12.1999
(51) Int. Cl.: C07K 16/28, A61K 39/395, A61P 35/00

(54) **HUMAN MONOCLONAL ANTIBODIES TO CTLA-4**
HUMANE MONOKLONALE ANTIKÖRPER GEGEN CTLA-4
ANTICORPS MONOCLONAUX HUMAINES POUR CTLA-4

(30) Priority: 23.12.1998 US 113647 P
(43) Date of publication of application: 28.10.2009
(62) Divisional of application: 99966644.9
(73) Proprietor: Pfizer Inc., New York, NY 10017 (US); Amgen Fremont Inc., Thousand Oaks, CA 91320-1799 (US)
(72) Inventor: Hanson, Douglas Charles, Niantic, CT 06357 (US); Neveu, Mark Joseph, Mystic, CT 06355 (US); Mueller, Eileen Elliot, Old Lyme, CT 06371 (US); Hanke, Jeffrey Herbert, Reading, MA 01867 (US); Gilman, Steven Christopher, Old Lyme, CT 06371 (US); Davis, C. Geoffrey, Auburn, CA 95604 (US); Corvalan, José Ramon, Foster City, CA 94404 (US)
(74) Representative: Vossius & Partner Patentanwälte Rechtsanwälte mbB

(56) References cited:
- WO-A1-95/33770
- WO-A1-96/33735
- WO-A1-98/42752
- WO-A2-01/14424
- M. PISTILLO ET AL.: "Recombinant human anti-CTLA-4 antibodies selected from a phage display library.", HUMAN IMMUNOLOGY, vol. 55, no. suppl.1, 1997, page 45, XP008159492,
- A. JAKOBOVITS: "The long-awaited magic bullets: therapeutic human monoclonal antibodies from transgenic mice.", EXPERT OPINION ON INVESTIGATIONAL DRUGS, vol. 7, no. 4, April 1998 (1998-04), pages 607-614, XP002084509, London, GB
- CAMACHO LUIS H: "Novel therapies targeting the immune system: CTLA4 blockade with tremelimumab (CP-675,206), a fully human monoclonal antibody.", EXPERT OPINION ON INVESTIGATIONAL DRUGS MAR 2008, vol. 17, no. 3, March 2008 (2008-03), pages 371-385, XP2556463, ISSN: 1744-7658
- O'DAY STEVEN J ET AL: "Targeting cytotoxic T-lymphocyte antigen-4 (CTLA-4): a novel strategy for the treatment of melanoma and other malignancies.", CANCER 15 DEC 2007, vol. 110, no. 12, 15 December 2007 (2007-12-15), pages 2614-2627, XP55097404, ISSN: 0008-543X
- CRANMER LEE D ET AL: "The role of the CTLA4 blockade in the treatment of malignant melanoma.", CANCER INVESTIGATION OCT 2007, vol. 25, no. 7, October 2007 (2007-10), pages 613-631, ISSN: 1532-4192
- GERSHONI JONATHAN M ET AL: "Epitope mapping - The first step in developing epitope-based vaccines", BIODRUGS,, vol. 21, no. 3, 1 January 2007 (2007-01-01), pages 145-156, XP009103541, ISSN: 1173-8804, DOI: 10.2165/00063030-200721030-00002
- METZLER W J ET AL: "Solution structure of human CTLA-4 and delineation of a CD80/CD86 binding site conserved in CD28.", NATURE STRUCTURAL BIOLOGY JUL 1997, vol. 4, no. 7, July 1997 (1997-07), pages 527-531, ISSN: 1072-8368

### SEQUENCE LISTING

<110> ABGENIX, INC. PFIZER INC.
<120> HUMAN MONOCLONAL ANTIBODIES TO CTLA-4
<130> ABX-PF1 PCT
<140> PCT/US99/30895
   <141> 1999-12-23
<150> 60/113,647
   <151> 1998-12-23
<160> 147
<170> PatentIn Ver. 2.1
<210> 1
   <211> 463
   <212> PRT
   <213> Homo sapiens
<400> 1
   Met Glu Phe Gly Leu Ser Trp Val Phe Leu Val Ala Leu Leu Arg Gly
     1               5                  10                  15
   Val Gln Cys Gln Val Gln Leu Val Glu Ser Gly Gly Gly Val Val Gln
                20                  25                  30
   Pro Gly Arg Ser Leu Arg Leu Ser Cys Val Ala Ser Gly Phe Thr Phe
            35                  40                  45
   Ser Ser His Gly Met His Trp Val Arg Gln Ala Pro Gly Lys Gly Leu
        50                  55                  60
   Glu Trp Val Ala Val Ile Trp Tyr Asp Gly Arg Asn Lys Tyr Tyr Ala
    65                  70                  75                  80
   Asp Ser Val Lys Gly Arg Phe Thr Ile Ser Arg Asp Asn Ser Lys Asn
                    85                  90                  95
   Thr Leu Phe Leu Gln Met Asn Ser Leu Arg Ala Glu Asp Thr Ala Val
               100                 105                 110
   Tyr Tyr Cys Ala Arg Gly Gly His Phe Gly Pro Phe Asp Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Cys Ser Arg Ser Thr Ser Glu Ser Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Asn Phe Gly Thr Gln Thr Tyr Thr Cys Asn Val Asp His
       210                 215                 220
   Lys Pro Ser Asn Thr Lys Val Asp Lys Thr Val Glu Arg Lys Cys Cys
   225                 230                 235                 240
   Val Glu Cys Pro Pro Cys Pro Ala Pro Pro Val Ala Gly Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Gln Phe Asn Trp Tyr Val Asp Gly Val Glu Val His Asn Ala Lys
       290                 295                 300
   Thr Lys Pro Arg Glu Glu Gln Phe Asn Ser Thr Phe Arg Val Val Ser
   305                 310                 315                 320
   Val Leu Thr Val Val His Gln Asp Trp Leu Asn Gly Lys Glu Tyr Lys
                   325                 330                 335
   Cys Lys Val Ser Asn Lys Gly Leu Pro Ala Pro Ile Glu Lys Thr Ile
               340                 345                 350
   Ser Lys Thr Lys Gly Gln Pro Arg Glu Pro Gln Val Tyr Thr Leu Pro
           355                 360                 365
   Pro Ser Arg Glu Glu Met Thr Lys Asn Gln Val Ser Leu Thr Cys Leu
       370                 375                 380
   Val Lys Gly Phe Tyr Pro Ser Asp Ile Ala Val Glu Trp Glu Ser Asn
   385                 390                 395                 400
   Gly Gln Pro Glu Asn Asn Tyr Lys Thr Thr Pro Pro Met Leu Asp Ser
                   405                 410                 415
   Asp Gly Ser Phe Phe Leu Tyr Ser Lys Leu Thr Val Asp Lys Ser Arg
               420                 425                 430
   Trp Gln Gln Gly Asn Val Phe Ser Cys Ser Val Met His Glu Ala Leu
           435                 440                 445
   His Asn His Tyr Thr Gln Lys Ser Leu Ser Leu Ser Pro Gly Lys
       450                 455                 460
<210> 2
   <211> 464
   <212> PRT
   <213> Homo sapiens
<400> 2
   Met Glu Phe Gly Leu Ser Trp Val Phe Leu Val Ala Leu Leu Arg Gly
    1               5                  10                  15
   Val Gln Cys Gln Val Gln Leu Val Glu Ser Gly Gly Gly Val Val Gln
               20                  25                  30
   Pro Gly Arg Ser Leu Arg Leu Ser Cys Thr Ala Ser Gly Phe Thr Phe
           35                  40                  45
   Ser Asn Tyr Gly Met His Trp Val Arg Gln Ala Pro Gly Lys Gly Leu
       50                  55                  60
   Glu Trp Val Ala Val Ile Trp Tyr Asp Gly Ser Asn Lys His Tyr Gly
   65                  70                  75                  80
   Asp Ser Val Lys Gly Arg Phe Thr Ile Ser Ser Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Gly Glu Arg Leu Gly Ser Tyr Phe Asp Tyr Trp
           115                 120                 125
   Gly Gln Gly Thr Leu Val Thr Val Ser Ser Ala Ser Thr Lys Gly Pro
       130                 135                 140
   Ser Val Phe Pro Leu Ala Pro Cys Ser Arg Ser Thr Ser Glu Ser Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Asn Phe Gly Thr Gln Thr Tyr Thr Cys Asn Val Asp
       210                 215                 220
   His Lys Pro Ser Asn Thr Lys Val Asp Lys Thr Val Glu Arg Lys Cys
   225                 230                 235                 240
   Cys Val Glu Cys Pro Pro Cys Pro Ala Pro Pro Val Ala Gly Pro Ser
                   245                 250                 255
   Val Phe Leu Phe Pro Pro Lys Pro Lys Asp Thr Leu Met Ile Ser Arg
               260                 265                 270
   Thr Pro Glu Val Thr Cys Val Val Val Asp Val Ser His Glu Asp Pro
           275                 280                 285
   Glu Val Gln Phe Asn Trp Tyr Val Asp Gly Val Glu Val His Asn Ala
           290                 295                 300
   Lys Thr Lys Pro Arg Glu Glu Gln Phe Asn Ser Thr Phe Arg Val Val
   305                 310                 315                 320
   Ser Val Leu Thr Val Val His Gln Asp Trp Leu Asn Gly Lys Glu Tyr
                   325                 330                 335
   Lys Cys Lys Val Ser Asn Lys Gly Leu Pro Ala Pro Ile Glu Lys Thr
               340                 345                 350
   Ile Ser Lys Thr Lys Gly Gln Pro Arg Glu Pro Gln Val Tyr Thr Leu
           355                 360                 365
   Pro Pro Ser Arg Glu Glu Met Thr Lys Asn Gln Val Ser Leu Thr Cys
       370                 375                 380
   Leu Val Lys Gly Phe Tyr Pro Ser Asp Ile Ala Val Glu Trp Glu Ser
   385                 390                 395                 400
   Asn Gly Gln Pro Glu Asn Asn Tyr Lys Thr Thr Pro Pro Met Leu Asp
                   405                 410                 415
   Ser Asp Gly Ser Phe Phe Leu Tyr Ser Lys Leu Thr Val Asp Lys Ser
               420                 425                 430
   Arg Trp Gln Gln Gly Asn Val Phe Ser Cys Ser Val Met His Glu Ala
           435                 440                 445
   Leu His Asn His Tyr Thr Gln Lys Ser Leu Ser Leu Ser Pro Gly Lys
       450                 455                 460
<210> 3
   <211> 163
   <212> PRT
   <213> Homo sapiens
<400> 3
   Pro Gly Arg Ser Leu Arg Leu Ser Cys Ala Ala Ser Gly Phe Thr Phe
   1               5                   10                  15
   Ser Ser His Gly Ile His Trp Val Arg Gln Ala Pro Gly Lys Gly Leu
               20                  25                  30
   Glu Trp Val Ala Val Ile Trp Tyr Asp Gly Arg Asn Lys Asp Tyr Ala
           35                  40                  45
   Asp Ser Val Lys Gly Arg Phe Thr Ile Ser Arg Asp Asn Ser Lys Lys
       50                  55                  60
   Thr Leu Tyr Leu Gln Met Asn Ser Leu Arg Ala Glu Asp Thr Ala Val
   65                  70                  75                  80
   Tyr Tyr Cys Ala Arg Val Ala Pro Leu Gly Pro Leu Asp Tyr Trp Gly
                   85                  90                  95
   Gln Gly Thr Leu Val Thr Val Ser Ser Ala Ser Thr Lys Gly Pro Ser
   100 105 110
   Val Phe Pro Leu Ala Pro Cys Ser Arg Ser Thr Ser Glu Ser Thr Ala
   115 120 125
   Ala Leu Gly Cys Leu Val Lys Asp Tyr Phe Pro Glu Pro Val Thr Val
   130 135 140
   Ser Trp Asn Ser Gly Ala Leu Thr Ser Gly Val His Thr Phe Pro Ala
   145 150 155 160
   Val Leu Gln
<210> 4
   <211> 463
   <212> PRT
   <213> Homo sapiens
<400> 4
   Met Glu Phe Gly Leu Ser Trp Val Phe Leu Val Ala Leu Leu Arg Gly
   1 5 10 15
   Val Gln Cys Gln Val Gln Leu Val Glu Ser Gly Gly Gly Val Val Glu
   20 25 30
   Pro Gly Arg Ser Leu Arg Leu Ser Cys Thr Ala Ser Gly Phe Thr Phe
   35 40 45
   Ser Ser Tyr Gly Met His Trp Val Arg Gln Ala Pro Gly Lys Gly Leu
   50 55 60
   Glu Trp Val Ala Val Ile Trp Tyr Asp Gly Ser Asn Lys His Tyr Ala
   65 70 75 80
   Asp Ser Ala Lys Gly Arg Phe Thr Ile Ser Arg Asp Asn Ser Lys Asn
   85 90 95
   Thr Leu Tyr Leu Gln Met Asn Ser Leu Arg Ala Glu Asp Thr Ala Val
   100 105 110
   Tyr Tyr Cys Ala Arg Ala Gly Leu Leu Gly Tyr Phe Asp Tyr Trp Gly
   115 120 125
   Gln Gly Thr Leu Val Thr Val Ser Ser Ala Ser Thr Lys Gly Pro Ser
   130 135 140
   Val Phe Pro Leu Ala Pro Cys Ser Arg Ser Thr Ser Glu Ser Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Asn Phe Gly Thr Gln Thr Tyr Thr Cys Asn Val Asp His
       210                 215                 220
   Lys Pro Ser Asn Thr Lys Val Asp Lys Thr Val Glu Arg Lys Cys Cys
   225                 230                 235                 240
   Val Glu Cys Pro Pro Cys Pro Ala Pro Pro Val Ala Gly Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Gln Phe Asn Trp Tyr Val Asp Gly Val Glu Val His Asn Ala Lys
       290                 295                 300
   Thr Lys Pro Arg Glu Glu Gln Phe Asn Ser Thr Phe Arg Val Val Ser
   305                 310                 315                 320
   Val Leu Thr Val Val His Gln Asp Trp Leu Asn Gly Lys Glu Tyr Lys
                   325                 330                 335
   Cys Lys Val Ser Asn Lys Gly Leu Pro Ala Pro Ile Glu Lys Thr Ile
               340                 345                 350
   Ser Lys Thr Lys Gly Gln Pro Arg Glu Pro Gln Val Tyr Thr Leu Pro
           355                 360                 365
   Pro Ser Arg Glu Glu Met Thr Lys Asn Gln Val Ser Leu Thr Cys Leu
       370                 375                 380
   Val Lys Gly Phe Tyr Pro Ser Asp Ile Ala Val Glu Trp Glu Ser Asn
   385                 390                 395                 400
   Gly Gln Pro Glu Asn Asn Tyr Lys Thr Thr Pro Pro Met Leu Asp Ser
                   405                 410                 415
   Asp Gly Ser Phe Phe Leu Tyr Ser Lys Leu Thr Val Asp Lys Ser Arg
               420                 425                 430
   Trp Gln Gln Gly Asn Val Phe Ser Cys Ser Val Met His Glu Ala Leu
           435                 440                 445
   His Asn His Tyr Thr Gln Lys Ser Leu Ser Leu Ser Pro Gly Lys
       450                 455                 460
<210> 5
   <211> 169
   <212> PRT
   <213> Homo sapiens
<400> 5
   Gly Val Val Gln Pro Gly Arg Ser Leu Arg Leu Ser Cys Ala Ala Ser
   1               5                   10                  15
   Gly Phe Thr Phe Ser Ser Tyr Gly Met His Trp Val Arg Gln Ala Pro
               20                  25                  30
   Gly Lys Gly Leu Glu Trp Val Ala Val Ile Trp Tyr Asp Gly Ser Asn
           35                  40                  45
   Lys Tyr Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
   65                  70                  75                  80
   Asp Thr Ala Val Tyr Tyr Cys Ala Arg Gly Ala Arg Ile Ile Thr Pro
                   85                  90                  95
   Cys Met Asp Val Trp Gly Gln Gly Thr Thr Val Thr Val Ser Ser Ala
               100                 105                 110
   Ser Thr Lys Gly Pro Ser Val Phe Pro Leu Ala Pro Cys Ser Arg Ser
           115                 120                 125
   Thr Ser Glu Ser Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln
                   165
<210> 6
   <211> 167
   <212> PRT
   <213> Homo sapiens
<400> 6
   Gly Val Val Gln Pro Gly Arg Ser Leu Arg Leu Ser Cys Val Ala Ser
   1               5                   10                  15
   Gly Phe Ile Phe Ser Ser His Gly Ile His Trp Val Arg Gln Ala Pro
               20                  25                  30
   Gly Lys Gly Leu Glu Trp Val Ala Val Ile Trp Tyr Asp Gly Arg Asn
           35                  40                  45
   Lys Asp Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
   65                  70                  75                  80
   Asp Thr Ala Val Tyr Tyr Cys Ala Arg Val Ala Pro Leu Gly Pro Leu
                   85                  90                  95
   Asp Tyr Trp Gly Gln Gly Thr Leu Val Thr Val Ser Ser Ala Ser Thr
               100                 105                 110
   Lys Gly Pro Ser Val Phe Pro Leu Ala Pro Cys Ser Arg Ser Thr Ser
           115                 120                 125
   Glu Ser Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln
                   165
<210> 7
   <211> 172
   <212> PRT
   <213> Homo sapiens
<400> 7
   Ser Gly Pro Gly Leu Val Lys Pro Ser Gln Ile Leu Ser Leu Thr Cys
   1               5                   10                  15
   Thr Val Ser Gly Gly Ser Ile Ser Ser Gly Gly His Tyr Trp Ser Trp
               20                  25                  30
   Ile Arg Gln His Pro Gly Lys Gly Leu Glu Trp Ile Gly Tyr Ile Tyr
           35                  40                  45
   Tyr Ile Gly Asn Thr Tyr Tyr Asn Pro Ser Leu Lys Ser Arg Val Thr
       50                  55                  60
   Ile Ser Val Asp Thr Ser Lys Asn Gln Phe Ser Leu Lys Leu Ser Ser
   65                  70                  75                  80
   Val Thr Ala Ala Asp Thr Ala Val Tyr Tyr Cys Ala Arg Asp Ser Gly
                   85                  90                  95
   Asp Tyr Tyr Gly Ile Asp Val Trp Gly Gln Gly Thr Thr Val Thr Val
               100                 105                 110
   Ser Ser Ala Ser Thr Lys Gly Pro Ser Val Phe Pro Leu Ala Pro Cys
           115                 120                 125
   Ser Arg Ser Thr Ser Glu Ser Thr Ala Ala Leu Gly Cys Leu Val Lys
       130                 135                 140
   Asp Tyr Phe Pro Glu Pro Val Thr Val Ser Trp Asn Ser Gly Ala Leu
   145                 150                 155                 160
   Thr Ser Gly Val His Thr Phe Pro Ala Val Leu Gln
                   165                 170
<210> 8
   <211> 153
   <212> PRT
   <213> Homo sapiens
<400> 8
   Pro Gly Arg Ser Leu Arg Leu Ser Cys Ala Ala Ser Gly Phe Thr Phe
    1               5                  10                  15
   Ser Ser His Gly Ile His Trp Val Arg Gln Ala Pro Gly Lys Gly Leu
               20                  25                  30
   Glu Trp Val Ala Val Ile Trp Tyr Asp Gly Arg Asn Lys Asp Tyr Ala
           35                  40                  45
   Asp Ser Val Lys Gly Arg Phe Thr Ile Ser Arg Asp Asn Ser Lys Asn
       50                  55                  60
   Thr Leu Tyr Leu Gln Met Asn Ser Leu Arg Ala Glu Asp Thr Ala Val
    65                  70                  75                  80
   Tyr Tyr Cys Ala Arg Val Ala Pro Leu Gly Pro Leu Asp Tyr Trp Gly
                   85                  90                  95
   Gln Gly Thr Leu Val Thr Val Ser Ser Ala Ser Thr Lys Gly Pro Ser
               100                 105                 110
   Val Phe Pro Leu Ala Pro Cys Ser Arg Ser Thr Ser Glu Ser Thr Ala
           115                 120                 125
   Ala Leu Gly Cys Leu Val Lys Asp Tyr Phe Pro Glu Pro Val Thr Val
       130                 135                 140
   Ser Trp Asn Ser Gly Ala Leu Thr Ser
   145                 150
<210> 9
   <211> 167
   <212> PRT
   <213> Homo sapiens
<220>
   <221> MOD_RES
   <222> (103)
   <223> Any amino acid
<400> 9
   Gly Val Val Gln Pro Gly Arg Ser Leu Arg Leu Ser Cys Ala Ala Ser
    1               5                  10                  15
   Gly Phe Thr Phe Ser Ser Tyr Gly Met His Trp Val Arg Gln Ala Pro
               20                  25                  30
   Gly Lys Gly Leu Glu Trp Val Ala Val Ile Trp Tyr Asp Gly Ser Asn
           35                  40                  45
   Lys Tyr Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
    65                  70                  75                  80
   Asp Thr Ala Val Tyr Tyr Cys Ala Arg Asp Pro Arg Gly Ala Thr Leu
                   85                  90                  95
   Tyr Tyr Tyr Tyr Tyr Arg Xaa Asp Val Trp Gly Gln Gly Thr Thr Val
               100                 105                 110
   Thr Val Ser Ser Ala Ser Thr Lys Gly Pro Ser Val Phe Pro Leu Ala
           115                 120                 125
   Pro Cys Ser Arg Ser Thr Ser Glu Ser Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His
                   165
<210> 10
   <211> 151
   <212> PRT
   <213> Homo sapiens
<400> 10
   Gly Val Val Gln Pro Gly Arg Ser Leu Arg Leu Ser Cys Ala Ala Ser
     1               5                  10                  15
   Gly Phe Thr Phe Ser Ser Tyr Gly Met His Trp Val Arg Gln Ala Pro
                20                  25                  30
   Gly Lys Gly Leu Glu Trp Val Ala Val Ile Trp Tyr Asp Gly Ser His
           35                  40                  45
   Lys Tyr Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
    65                  70                  75                  80
   Asp Thr Ala Val Tyr Tyr Cys Ala Arg Gly Ala Val Val Val Pro Ala
                   85                  90                  95
   Ala Met Asp Val Trp Gly Gln Gly Thr Thr Val Thr Val Ser Ser Ala
               100                 105                 110
   Ser Thr Lys Gly Pro Ser Val Phe Pro Leu Ala Pro Cys Ser Arg Ser
           115                 120                 125
   Thr Ser Glu Ser Thr Ala Ala Leu Gly Cys Leu Val Lys Asp Tyr Phe
       130                 135                 140
   Pro Glu Pro Val Thr Val Ser
   145                 150
<210> 11
   <211> 146
   <212> PRT
   <213> Homo sapiens
<220>
   <221> MOD_RES
   <222> (22)
   <223> Any amino acid
<400> 11
   Val Val Gln Pro Gly Arg Ser Leu Arg Leu Ser Cys Ala Ala Ser Gly
    1               5                  10                  15
   Phe Thr Phe Ser Ser Xaa Gly Met His Trp Val Arg Gln Ala Pro Gly
               20                  25                  30
   Lys Gly Leu Glu Trp Val Ala Val Ile Trp Ser Asp Gly Ser His Lys
           35                  40                  45
   Tyr Tyr Ala Asp Ser Val Lys Gly Arg Phe Thr Ile Ser Arg Asp Asn
       50                  55                  60
   Ser Lys Asn Thr Leu Tyr Leu Gln Met Asn Ser Leu Arg Ala Glu Asp
   65                  70                  75                  80
   Thr Ala Val Tyr Tyr Cys Ala Arg Gly Thr Met Ile Val Val Gly Thr
                   85                  90                  95
   Leu Asp Tyr Trp Gly Gln Gly Thr Leu Val Thr Val Ser Ser Ala Ser
               100                 105                 110
   Thr Lys Gly Pro Ser Val Phe Pro Leu Ala Pro Cys Ser Arg Ser Thr
           115                 120                 125
   Ser Glu Ser Thr Ala Ala Leu Gly Cys Leu Val Lys Asp Tyr Phe Pro
       130                 135                 140
   Glu Pro
   145
<210> 12
   <211> 174
   <212> PRT
   <213> Homo sapiens
<400> 12
   Ser Gly Gly Gly Val Val Gln Pro Gly Arg Ser Leu Arg Leu Ser Cys
    1               5                  10                  15
   Ala Ala Ser Gly Phe Thr Phe Ser Ser Tyr Gly Val His Trp Val Arg
               20                  25                  30
   Gln Ala Pro Gly Lys Gly Leu Glu Trp Val Ala Val Ile Trp Tyr Asp
           35                  40                  45
   Gly Ser Asn Lys Tyr Tyr Ala Asp Ser Val Lys Gly Arg Phe Thr Ile
       50                  55                  60
   Ser Arg Asp Asn Ser Lys Ser Thr Leu Tyr Leu Gln Met Asn Ser Leu
    65                  70                  75                  80
   Arg Ala Glu Asp Thr Ala Val Tyr Tyr Cys Ala Arg Asp Ser Tyr Tyr
                    85                  90                  95
   Asp Phe Trp Ser Gly Arg Gly Gly Met Asp Val Trp Gly Gln Gly Thr
               100                 105                 110
   Thr Val Thr Val Ser Ser Ala Ser Thr Lys Gly Pro Ser Val Phe Pro
           115                 120                 125
   Leu Ala Pro Cys Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val
                   165                 170
<210> 13
   <211> 163
   <212> PRT
   <213> Homo sapiens
<400> 13
   Val Gln Pro Gly Arg Ser Leu Arg Leu Ser Cys Ala Ala Ser Gly Phe
     1               5                  10                  15
   Thr Phe Ser Asn Tyr Ala Met His Trp Val Arg Gln Ala Pro Gly Lys
                20                  25                  30
   Gly Leu Glu Trp Val Val Val Ile Trp His Asp Gly Asn Asn Lys Tyr
            35                  40                  45
   Tyr Ala Glu Ser Val Lys Gly Arg Phe Thr Ile Ser Arg Asp Asn Ser
        50                  55                  60
   Lys Asn Thr Leu Tyr Leu Gln Met Asn Ser Leu Arg Ala Glu Asp Thr
    65                  70                  75                  80
   Ala Val Tyr Tyr Cys Ala Arg Asp Gln Gly Thr Gly Trp Tyr Gly Gly
                    85                  90                  95
   Phe Asp Phe Trp Gly Gln Gly Thr Leu Val Thr Val Ser Ser Ala Ser
               100                 105                 110
   Thr Lys Gly Pro Ser Val Phe Pro Leu Ala Pro Cys Ser Arg Ser Thr
           115                 120                 125
   Ser Glu Ser Thr Ala Ala Leu Gly Cys Leu Val Lys Asp Tyr Phe Pro
       130                 135                 140
   Glu Pro Val Thr Val Ser Trp Asn Ser Gly Ala Leu Thr Ser Gly Val
   145                 150                 155                 160
   His Thr Phe
<210> 14
   <211> 235
   <212> PRT
   <213> Homo sapiens
<400> 14
   Met Glu Thr Pro Ala Gln Leu Leu Phe Leu Leu Leu Leu Trp Leu Pro
   1               5                   10                  15
   Asp Thr Thr Gly Glu Ile Val Leu Thr Gln Ser Pro Gly Thr Leu Ser
               20                  25                  30
   Leu Ser Pro Gly Glu Arg Ala Thr Leu Ser Cys Arg Ala Ser Gln Ser
           35                  40                  45
   Ile Ser Ser Ser Phe Leu Ala Trp Tyr Gln Gln Arg Pro Gly Gln Ala
       50                  55                  60
   Pro Arg Leu Leu Ile Tyr Gly Ala Ser Ser Arg Ala Thr Gly Ile Pro
   65                  70                  75                  80
   Asp Arg Phe Ser Gly Ser Gly Ser Gly Thr Asp Phe Thr Leu Thr Ile
                   85                  90                  95
   Ser Arg Leu Glu Pro Glu Asp Phe Ala Val Tyr Tyr Cys Gln Gln Tyr
               100                 105                 110
   Gly Thr Ser Pro Trp Thr Phe Gly Gln Gly Thr Lys Val Glu Ile Lys
           115                 120                 125
   Arg Thr Val Ala Ala Pro Ser Val Phe Ile Phe Pro Pro Ser Asp Glu
       130                 135                 140
   Gln Leu Lys Ser Gly Thr Ala Ser Val Val Cys Leu Leu Asn Asn Phe
   145                 150                 155                 160
   Tyr Pro Arg Glu Ala Lys Val Gln Trp Lys Val Asp Asn Ala Leu Gln
                   165                 170                 175
   Ser Gly Asn Ser Gln Glu Ser Val Thr Glu Gln Asp Ser Lys Asp Ser
               180                 185                 190
   Thr Tyr Ser Leu Ser Ser Thr Leu Thr Leu Ser Lys Ala Asp Tyr Glu
           195                 200                 205
   Lys His Lys Val Tyr Ala Cys Glu Val Thr His Gln Gly Leu Ser Ser
       210                 215                 220
   Pro Val Thr Lys Ser Phe Asn Arg Gly Glu Cys
   225                 230                 235
<210> 15
   <211> 233
   <212> PRT
   <213> Homo sapiens
<400> 15
   Met Glu Thr Pro Ala Gln Leu Leu Phe Leu Leu Leu Leu Trp Leu Pro
    1               5                  10                  15
   Asp Thr Thr Gly Glu Ile Val Leu Thr Gln Ser Pro Gly Thr Leu Ser
               20                  25                  30
   Leu Ser Pro Gly Glu Arg Ala Thr Leu Ser Cys Arg Thr Ser Val Ser
           35                  40                  45
   Ser Ser Tyr Leu Ala Trp Tyr Gln Gln Lys Pro Gly Gln Ala Pro Arg
       50                  55                  60
   Leu Leu Ile Tyr Gly Ala Ser Ser Arg Ala Thr Gly Ile Pro Asp Arg
   65                  70                  75                  80
   Phe Ser Gly Ser Gly Ser Gly Thr Asp Phe Thr Leu Thr Ile Ser Arg
                   85                  90                  95
   Leu Glu Pro Glu Asp Phe Ala Val Tyr Tyr Cys Gln Gln Tyr Gly Ile
               100                 105                 110
   Ser Pro Phe Thr Phe Gly Gly Gly Thr Lys Val Glu Ile Lys Arg Thr
           115                 120                 125
   Val Ala Ala Pro Ser Val Phe Ile Phe Pro Pro Ser Asp Glu Gln Leu
       130                 135                 140
   Lys Ser Gly Thr Ala Ser Val Val Cys Leu Leu Asn Asn Phe Tyr Pro
   145                 150                 155                 160
   Arg Glu Ala Lys Val Gln Trp Lys Val Asp Asn Ala Leu Gln Ser Gly
                   165                 170                 175
   Asn Ser Gln Glu Ser Val Thr Glu Gln Asp Ser Lys Asp Ser Thr Tyr
               180                 185                 190
   Ser Leu Ser Ser Thr Leu Thr Leu Ser Lys Ala Asp Tyr Glu Lys His
           195                 200                 205
   Lys Val Tyr Ala Cys Glu Val Thr His Gln Gly Leu Ser Ser Pro Val
       210                 215                 220
   Thr Lys Ser Phe Asn Arg Gly Glu Cys
   225                 230
<210> 16
   <211> 139
   <212> PRT
   <213> Homo sapiens
<400> 16
   Gly Thr Leu Ser Leu Ser Pro Gly Glu Arg Ala Thr Leu Ser Cys Arg
    1               5                  10                  15
   Ala Ser Gln Ser Val Ser Ser Tyr Leu Ala Trp Tyr Gln Gln Lys Pro
               20                  25                  30
   Gly Gln Ala Pro Arg Leu Leu Ile Tyr Gly Ala Ser Ser Arg Ala Thr
           35                  40                  45
   Gly Ile Pro Asp Arg Phe Ser Gly Ser Gly Ser Gly Thr Asp Phe Thr
       50                  55                  60
   Leu Thr Ile Ser Arg Leu Glu Pro Glu Asp Phe Ala Val Tyr Tyr Cys
   65                  70                  75                  80
   Gln Gln Tyr Gly Arg Ser Pro Phe Thr Phe Gly Pro Gly Thr Lys Val
                   85                  90                  95
   Asp Ile Lys Arg Thr Val Ala Ala Pro Ser Val Phe Ile Phe Pro Pro
               100                 105                 110
   Ser Asp Glu Gln Leu Lys Ser Gly Thr Ala Ser Val Val Cys Leu Leu
           115                 120                 125
   Asn Asn Phe Tyr Pro Arg Glu Ala Lys Val Gln
       130                 135
<210> 17
   <211> 234
   <212> PRT
   <213> Homo sapiens
<400> 17
   Met Glu Thr Pro Ala Gln Leu Leu Phe Leu Leu Leu Leu Trp Leu Pro
   1               5                   10                  15
   Asp Thr Thr Gly Glu Ile Val Leu Thr Gln Ser Pro Gly Thr Leu Ser
               20                  25                  30
   Leu Ser Pro Gly Glu Arg Ala Thr Leu Ser Cys Arg Ala Ser Gln Ser
           35                  40                  45
   Val Ser Ser Tyr Leu Ala Trp Tyr Gln Gln Lys Pro Gly Gln Ala Pro
       50                  55                  60
   Arg Pro Leu Ile Tyr Gly Val Ser Ser Arg Ala Thr Gly Ile Pro Asp
   65                  70                  75                  80
   Arg Phe Ser Gly Ser Gly Ser Gly Thr Asp Phe Thr Leu Thr Ile Ser
                   85                  90                  95
   Arg Leu Glu Pro Glu Asp Phe Ala Val Tyr Tyr Cys Gln Gln Tyr Gly
               100                 105                 110
   Ile Ser Pro Phe Thr Phe Gly Pro Gly Thr Lys Val Asp Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 18
   <211> 152
   <212> PRT
   <213> Homo sapiens
<400> 18
   Gln Ser Pro Ser Ser Leu Ser Ala Ser Val Gly Asp Arg Val Thr Ile
     1               5                  10                  15
   Thr Cys Arg Ala Ser Gln Ser Ile Asn Thr Tyr Leu Ile Trp Tyr Gln
               20                  25                  30
   Gln Lys Pro Gly Lys Ala Pro Asn Phe Leu Ile Ser Ala Thr Ser Ile
           35                  40                  45
   Leu Gln Ser Gly Val Pro Ser Arg Phe Arg Gly Ser Gly Ser Gly Thr
       50                  55                  60
   Asn Phe Thr Leu Thr Ile Asn Ser Leu His Pro Glu Asp Phe Ala Thr
    65                  70                  75                  80
   Tyr Tyr Cys Gln Gln Ser Tyr Ser Thr Pro Phe Thr Phe Gly Pro Gly
                   85                  90                  95
   Thr Lys Val Asp Ile Lys Arg Thr Val Ala Ala Pro Ser Val Phe Ile
               100                 105                 110
   Phe Pro Pro Ser Asp Glu Gln Leu Lys Ser Gly Thr Ala Ser Val Val
           115                 120                 125
   Cys Leu Leu Asn Asn Phe Tyr Pro Arg Glu Ala Lys Val Gln Trp Lys
       130                 135                 140
   Val Asp Asn Ala Leu Gln Ser Gly
   145                 150
<210> 19
   <211> 142
   <212> PRT
   <213> Homo sapiens
<400> 19
   Ser Pro Gly Thr Leu Ser Leu Ser Pro Gly Glu Arg Ala Thr Leu Ser
    1               5                  10                  15
   Cys Arg Ala Ser Gln Ser Ile Ser Ser Asn Phe Leu Ala Trp Tyr Gln
               20                  25                  30
   Gln Lys Pro Gly Gln Ala Pro Arg Leu Leu Ile Tyr Arg Pro Ser Ser
           35                  40                  45
   Arg Ala Thr Gly Ile Pro Asp Ser Phe Ser Gly Ser Gly Ser Gly Thr
       50                  55                  60
   Asp Phe Thr Leu Thr Ile Ser Arg Leu Glu Pro Glu Asp Phe Ala Leu
   65                  70                  75                  80
   Tyr Tyr Cys Gln Gln Tyr Gly Thr Ser Pro Phe Thr Phe Gly Pro Gly
                   85                  90                  95
   Thr Lys Val Asp Ile Lys Arg Thr Val Ala Ala Pro Ser Val Phe Ile
               100                 105                 110
   Phe Pro Pro Ser Asp Glu Gln Leu Lys Ser Gly Thr Ala Ser Val Val
           115                 120                 125
   Cys Leu Leu Asn Asn Phe Tyr Pro Arg Glu Ala Lys Val Gln
       130                 135                 140
<210> 20
   <211> 155
   <212> PRT
   <213> Homo sapiens
<400> 20
   Ser Pro Asp Phe Gln Ser Val Thr Pro Lys Glu Lys Val Thr Ile Thr
    1               5                  10                  15
   Cys Arg Ala Ser Gln Ser Ile Gly Ser Ser Leu His Trp Tyr Gln Gln
               20                  25                  30
   Lys Pro Asp Gln Ser Pro Lys Leu Leu Ile Lys Tyr Ala Ser Gln Ser
           35                  40                  45
   Phe Ser Gly Val Pro Ser Arg Phe Ser Gly Ser Gly Ser Gly Thr Asp
       50                  55                  60
   Phe Thr Leu Thr Ile Asn Ser Leu Glu Ala Glu Asp Ala Ala Thr Tyr
   65                  70                  75                  80
   Tyr Cys His Gln Ser Ser Ser Leu Pro Leu Thr Phe Gly Gly Gly Thr
                   85                  90                  95
   Lys Val Glu Ile Lys Arg Thr Val Ala Ala Pro Ser Val Phe Ile Phe
                   100                 105                 110
   Pro Pro Ser Asp Glu Gln Leu Lys Ser Gly Thr Ala Ser Val Val Cys
           115                 120                 125
   Leu Leu Asn Asn Phe Tyr Pro Arg Glu Ala Lys Val Gln Trp Lys Val
       130                 135                 140
   Asp Asn Ala Leu Gln Ser Gly Asn Ser Gln Glu
   145                 150                 155
<210> 21
   <211> 146
   <212> PRT
   <213> Homo sapiens
<400> 21
   Gln Ser Pro Gly Thr Leu Ser Leu Ser Pro Gly Glu Arg Ala Thr Leu
   1               5                   10                  15
   Ser Cys Arg Ala Ser Gln Ser Val Ser Ser Tyr Leu Ala Trp Tyr Gln
               20                  25                  30
   Gln Lys Pro Gly Gln Ala Pro Arg Leu Leu Ile Tyr Gly Ala Ser Ser
           35                  40                  45
   Arg Ala Thr Gly Ile Pro Asp Arg Phe Ser Gly Ser Gly Ser Gly Thr
       50                  55                  60
   Asp Phe Thr Leu Thr Ile Ser Arg Leu Glu Pro Glu Asp Phe Ala Val
   65                  70                  75                  80
   Tyr Tyr Cys Gln Gln Tyr Gly Arg Ser Pro Phe Thr Phe Gly Pro Gly
                   85                  90                  95
   Thr Lys Val Asp Ile Lys Arg Thr Val Ala Ala Pro Ser Val Phe Ile
               100                 105                 110
   Phe Pro Pro Ser Asp Glu Gln Leu Lys Ser Gly Thr Ala Ser Val Val
           115                 120                 125
   Cys Leu Leu Asn Asn Phe Tyr Pro Arg Glu Ala Lys Val Gln Trp Lys
       130                 135                 140
   Gly Gly
   145
<210> 22
   <211> 139
   <212> PRT
   <213> Homo sapiens
<400> 22
   Pro Ser Ser Leu Ser Ala Ser Val Gly Asp Arg Val Thr Ile Thr Cys
   1               5                   10                  15
   Arg Ala Ser Gln Ser Ile Asn Ser Tyr Leu Asp Trp Tyr Gln Gln Lys
               20                  25                  30
   Pro Gly Lys Ala Pro Lys Leu Leu Ile Tyr Ala Ala Ser Ser Leu Gln
           35                  40                  45
   Ser Gly Val Pro Ser Arg Phe Ser Gly Ser Gly Ser Gly Thr Asp Phe
       50                  55                  60
   Thr Leu Thr Ile Ser Ser Leu Gln Pro Glu Asp Phe Ala Thr Tyr Tyr
   65                  70                  75                  80
   Cys Gln Gln Tyr Tyr Ser Thr Pro Phe Thr Phe Gly Pro Gly Thr Lys
                   85                  90                  95
   Val Glu Ile Lys Arg Thr Val Ala Ala Pro Ser Val Phe Ile Phe Pro
               100                 105                 110
   Pro Ser Asp Glu Gln Leu Lys Ser Gly Thr Ala Ser Val Val Cys Leu
           115                 120                 125
   Leu Asn Asn Phe Tyr Pro Arg Glu Ala Lys Val
       130                 135
<210> 23
   <211> 134
   <212> PRT
   <213> Homo sapiens
<400> 23
   Thr Gln Ser Pro Ser Ser Leu Ser Ala Ser Val Gly Asp Arg Val Thr
   1               5                   10                  15
   Ile Thr Cys Arg Ala Ser Gln Asn Ile Ser Arg Tyr Leu Asn Trp Tyr
               20                  25                  30
   Gln Gln Lys Pro Gly Lys Ala Pro Lys Phe Leu Ile Tyr Val Ala Ser
           35                  40                  45
   Ile Leu Gln Ser Gly Val Pro Ser Gly Phe Ser Ala Ser Gly Ser Gly
       50                  55                  60
   Pro Asp Phe Thr Leu Thr Ile Ser Ser Leu Gln Pro Glu Asp Phe Ala
   65                  70                  75                  80
   Thr Tyr Tyr Cys Gln Gln Ser Tyr Ser Thr Pro Phe Thr Phe Gly Pro
                   85                  90                  95
   Gly Thr Lys Val Asp Ile Lys Arg Thr Val Ala Ala Pro Ser Val Phe
               100                 105                 110
   Ile Phe Pro Pro Ser Asp Glu Gln Leu Lys Ser Gly Thr Ala Ser Val
           115                 120                 125
   Val Cys Leu Leu Asn Asn
       130
<210> 24
   <211> 150
   <212> PRT
   <213> Homo sapiens
<400> 24
   Thr Gln Ser Pro Ser Ser Leu Ser Ala Ser Val Gly Asp Arg Val Thr
    1               5                  10                  15
   Ile Thr Cys Arg Ala Ser Gln Ser Ile Cys Asn Tyr Leu Asn Trp Tyr
               20                  25                  30
   Gln Gln Lys Pro Gly Lys Ala Pro Arg Val Leu Ile Tyr Ala Ala Ser
           35                  40                  45
   Ser Leu Gln Gly Gly Val Pro Ser Arg Phe Ser Gly Ser Gly Ser Gly
       50                  55                  60
   Ile Asp Cys Thr Leu Thr Ile Ser Ser Leu Gln Pro Glu Asp Phe Ala
   65                  70                  75                  80
   Thr Tyr Tyr Cys Gln Gln Ser Tyr Ile Thr Pro Phe Thr Phe Gly Pro
                   85                  90                  95
   Gly Thr Arg Val Asp Ile Glu Arg Thr Val Ala Ala Pro Ser Val Phe
               100                 105                 110
   Ile Phe Pro Pro Ser Asp Glu Gln Leu Lys Ser Gly Thr Ala Ser Val
           115                 120                 125
   Val Cys Leu Leu Asn Asn Phe Tyr Pro Arg Glu Ala Lys Val Gln Trp
       130                 135                 140
   Lys Val Asp Asn Ala Tyr
   145                 150
<210> 25
   <211> 139
   <212> PRT
   <213> Homo sapiens
<400> 25
   Pro Leu Ser Leu Pro Val Thr Leu Gly Gln Pro Ala Ser Ile Ser Cys
    1               5                  10                  15
   Arg Ser Ser Gln Ser Leu Val Tyr Ser Asp Gly Asn Thr Tyr Leu Asn
               20                  25                  30
   Trp Phe Gln Gln Arg Pro Gly Gln Ser Pro Arg Arg Leu Ile Tyr Lys
           35                  40                  45
   Val Ser Asn Trp Asp Ser Gly Val Pro Asp Arg Phe Ser Gly Ser Gly
       50                  55                  60
   Ser Gly Thr Asp Phe Thr Leu Lys Ile Ser Arg Val Glu Ala Glu Asp
   65                  70                  75                  80
   Val Gly Val Tyr Tyr Cys Met Gln Gly Ser His Trp Pro Pro Thr Phe
                   85                  90                  95
   Gly Gln Gly Thr Lys Val Glu Ile Lys Arg Thr Val Ala Ala Pro Ser
               100                 105                 110
   Val Phe Ile Phe Pro Pro Ser Asp Glu Gln Leu Lys Ser Gly Thr Ala
           115                 120                 125
   Ser Val Val Cys Leu Leu Asn Asn Phe Tyr Pro
       130                 135
<210> 26
   <211> 133
   <212> PRT
   <213> Homo sapiens
<400> 26
   Pro Gly Glu Pro Ala Ser Ile Ser Cys Arg Ser Ser Gln Ser Leu Leu
   1               5                   10                  15
   His Ser Asn Gly Tyr Asn Tyr Leu Asp Trp Tyr Leu Gln Lys Pro Gly
               20                  25                  30
   Gln Ser Pro Gln Leu Leu Ile Tyr Leu Gly Ser Asn Arg Ala Ser Gly
           35                  40                  45
   Val Pro Asp Arg Phe Ser Gly Ser Gly Ser Gly Thr Asp Phe Thr Leu
       50                  55                  60
   Lys Leu Ser Arg Val Glu Ala Glu Asp Val Gly Val Tyr Tyr Cys Met
   65                  70                  75                  80
   Gln Ala Leu Gln Thr Pro Leu Thr Phe Gly Gly Gly Thr Lys Val Glu
                   85                  90                  95
   Ile Lys Arg Thr Val Ala Ala Pro Ser Val Phe Ile Phe Pro Pro Ser
               100                 105                 110
   Asp Glu Gln Leu Lys Ser Gly Thr Ala Ser Val Val Cys Leu Leu Asn
           115                 120                 125
   Asn Phe Tyr Pro Arg
       130
<210> 27
   <211> 1392
   <212> DNA
   <213> Homo sapiens
<400> 27
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtgtagcgt ctggattcac cttcagtagc catggcatgc actgggtccg ccaggctcca 180
   ggcaaggggc tggagtgggt ggcagttata tggtatgatg gaagaaataa atactatgca 240
   gactccgtga agggccgatt caccatctcc agagacaatt ccaagaacac gctgtttctg 300
   caaatgaaca gcctgagagc cgaggacacg gctgtgtatt actgtgcgag aggaggtcac 360
   ttcggtcctt ttgactactg gggccaggga accctggtca ccgtctcctc agcctccacc 420
   aagggcccat cggtcttccc cctggcgccc tgctccagga gcacctccga gagcacagcg 480
   gccctgggct gcctggtcaa ggactacttc cccgaaccgg tgacggtgtc gtggaactca 540
   ggcgctctga ccagcggcgt gcacaccttc ccagctgtcc tacagtcctc aggactctac 600
   tccctcagca gcgtggtgac cgtgccctcc agcaacttcg gcacccagac ctacacctgc 660
   aacgtagatc acaagcccag caacaccaag gtggacaaga cagttgagcg caaatgttgt 720
   gtcgagtgcc caccgtgccc agcaccacct gtggcaggac cgtcagtctt cctcttcccc 780
   ccaaaaccca aggacaccct catgatctcc cggacccctg aggtcacgtg cgtggtggtg 840
   gacgtgagcc acgaagaccc cgaggtccag ttcaactggt acgtggacgg cgtggaggtg 900
   cataatgcca agacaaagcc acgggaggag cagttcaaca gcacgttccg tgtggtcagc 960
   gtcctcaccg ttgtgcacca ggactggctg aacggcaagg agtacaagtg caaggtctcc 1020
   aacaaaggcc tcccagcccc catcgagaaa accatctcca aaaccaaagg gcagccccga 1080
   gaaccacagg tgtacaccct gcccccatcc cgggaggaga tgaccaagaa ccaggtcagc 1140
   ctgacctgcc tggtcaaagg cttctacccc agcgacatcg ccgtggagtg ggagagcaat 1200
   gggcagccgg agaacaacta caagaccaca cctcccatgc tggactccga cggctccttc 1260
   ttcctctaca gcaagctcac cgtggacaag agcaggtggc agcaggggaa cgtcttctca 1320
   tgctccgtga tgcatgaggc tctgcacaac cactacacgc agaagagcct ctccctgtct 1380
   ccgggtaaat ga                                                     1392
<210> 28
   <211> 1395
   <212> DNA
   <213> Homo sapiens
<400> 28
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtacagcgt ctggattcac cttcagtaac tatggcatgc actgggtccg ccaggctcca 180
   ggcaaggggc tggagtgggt ggcagttata tggtatgatg gaagtaataa acactatgga 240
   gactccgtga agggccgatt caccatctcc agtgacaatt ccaagaacac gctgtatctg 300
   caaatgaaca gcctgagagc cgaggacacg gctgtgtatt actgtgcgag aggagagaga 360
   ctggggtcct actttgacta ctggggccag ggaaccctgg tcaccgtctc ctcagcctcc 420
   accaagggcc catcggtctt ccccctggcg ccctgctcca ggagcacctc cgagagcaca 480
   gcggccctgg gctgcctggt caaggactac ttccccgaac cggtgacggt gtcgtggaac 540
   tcaggcgctc tgaccagcgg cgtgcacacc ttcccagctg tcctacagtc ctcaggactc 600
   tactccctca gcagcgtggt gaccgtgccc tccagcaact tcggcaccca gacctacacc 660
   tgcaacgtag atcacaagcc cagcaacacc aaggtggaca agacagttga gcgcaaatgt 720
   tgtgtcgagt gcccaccgtg cccagcacca cctgtggcag gaccgtcagt cttcctcttc 780
   cccccaaaac ccaaggacac cctcatgatc tcccggaccc ctgaggtcac gtgcgtggtg 840
   gtggacgtga gccacgaaga ccccgaggtc cagttcaact ggtacgtgga cggcgtggag 900
   gtgcataatg ccaagacaaa gccacgggag gagcagttca acagcacgtt ccgtgtggtc 960
   agcgtcctca ccgttgtgca ccaggactgg ctgaacggca aggagtacaa gtgcaaggtc 1020
   tccaacaaag gcctcccagc ccccatcgag aaaaccatct ccaaaaccaa agggcagccc 1080
   cgagaaccac aggtgtacac cctgccccca tcccgggagg agatgaccaa gaaccaggtc 1140
   agcctgacct gcctggtcaa aggcttctac cccagcgaca tcgccgtgga gtgggagagc 1200
   aatgggcagc cggagaacaa ctacaagacc acacctccca tgctggactc cgacggctcc 1260
   ttcttcctct acagcaagct caccgtggac aagagcaggt ggcagcaggg gaacgtcttc 1320
   tcatgctccg tgatgcatga ggctctgcac aaccactaca cgcagaagag cctctccctg 1380
   tctccgggta aatga                                                  1395
<210> 29
   <211> 489
   <212> DNA
   <213> Homo sapiens
<400> 29
   cctgggaggt ccctgagact ctcctgtgca gcgtctggat tcaccttcag tagtcatggc 60
   atccactggg tccgccaggc tccaggcaag gggctggagt gggtggcagt tatatggtat 120
   gatggaagaa ataaagacta tgcagactcc gtgaagggcc gattcaccat ctccagagac 180
   aattccaaga agacgctgta tttgcaaatg aacagcctga gagccgagga cacggctgtg 240
   tattactgtg cgagagtggc cccactgggg ccacttgact actggggcca gggaaccctg 300
   gtcaccgtct cctcagcctc caccaagggc ccatcggtct tccccctggc gccctgctcc 360
   aggagcacct ccgagagcac agcggccctg ggctgcctgg tcaaggacta cttccccgaa 420
   ccggtgacgg tgtcgtggaa ctcaggcgct ctgaccagcg gcgtgcacac cttcccagct 480
   gtcctacag                                                         489
<210> 30
   <211> 1392
   <212> DNA
   <213> Homo sapiens
<400> 30
   atggagtttg ggctgagctg ggttttcctc gttgctcttt taagaggtgt ccagtgtcag 60
   gtgcagctgg tggagtctgg gggaggcgtg gtcgagcctg ggaggtccct gagactctcc 120
   tgtacagcgt ctggattcac cttcagtagt tatggcatgc actgggtccg ccaggctcca 180
   ggcaaggggc tggagtgggt ggcagttata tggtatgatg gaagcaataa acactatgca 240
   gactccgcga agggccgatt caccatctcc agagacaatt ccaagaacac gctgtatctg 300
   caaatgaaca gcctgagagc cgaggacacg gctgtgtatt actgtgcgag agccggactg 360
   ctgggttact ttgactactg gggccaggga accctggtca ccgtctcctc agcctccacc 420
   aagggcccat cggtcttccc cctggcgccc tgctccagga gcacctccga gagcacagcg 480
   gccctgggct gcctggtcaa ggactacttc cccgaaccgg tgacggtgtc gtggaactca 540
   ggcgctctga ccagcggcgt gcacaccttc ccagctgtcc tacagtcctc aggactctac 600
   tccctcagca gcgtggtgac cgtgccctcc agcaacttcg gcacccagac ctacacctgc 660
   aacgtagatc acaagcccag caacaccaag gtggacaaga cagttgagcg caaatgttgt 720
   gtcgagtgcc caccgtgccc agcaccacct gtggcaggac cgtcagtctt cctcttcccc 780
   ccaaaaccca aggacaccct catgatctcc cggacccctg aggtcacgtg cgtggtggtg 840
   gacgtgagcc acgaagaccc cgaggtccag ttcaactggt acgtggacgg cgtggaggtg 900
   cataatgcca agacaaagcc acgggaggag cagttcaaca gcacgttccg tgtggtcagc 960
   gtcctcaccg ttgtgcacca ggactggctg aacggcaagg agtacaagtg caaggtctcc 1020
   aacaaaggcc tcccagcccc catcgagaaa accatctcca aaaccaaagg gcagccccga 1080
   gaaccacagg tgtacaccct gcccccatcc cgggaggaga tgaccaagaa ccaggtcagc 1140
   ctgacctgcc tggtcaaagg cttctacccc agcgacatcg ccgtggagtg ggagagcaat 1200
   gggcagccgg agaacaacta caagaccaca cctcccatgc tggactccga cggctccttc 1260
   ttcctctaca gcaagctcac cgtggacaag agcaggtggc agcaggggaa cgtcttctca 1320
   tgctccgtga tgcatgaggc tctgcacaac cactacacgc agaagagcct ctccctgtct 1380
   ccgggtaaat ga                                                      1392
<210> 31
   <211> 507
   <212> DNA
   <213> Homo sapiens
<400> 31
   ggcgtggtcc agcctgggag gtccctgaga ctctcctgtg cagcgtctgg attcaccttc 60
   agtagctatg gcatgcactg ggtccgccag gctccaggca aggggctgga gtgggtggca 120
   gttatatggt atgatggaag taataaatac tatgcagact ccgtgaaggg ccgattcacc 180
   atctccagag acaattccaa gaacacgctg tatctgcaaa tgaacagcct gagagccgag 240
   gacacggctg tgtattactg tgcgagaggg gcccgtataa taaccccttg tatggacgtc 300
   tggggccaag ggaccacggt caccgtctcc tcagcctcca ccaagggccc atcggtcttc 360
   cccctggcgc cctgctccag gagcacctcc gagagcacag cggccctggg ctgcctggtc 420
   aaggactact tccccgaacc ggtgacggtg tcgtggaact caggcgctct gaccagcggc 480
   gtgcacacct tcccagctgt cctacag                                     507
<210> 32
   <211> 501
   <212> DNA
   <213> Homo sapiens
<400> 32
   ggcgtggtcc agcctgggag gtccctgaga ctctcctgtg tagcgtctgg attcatcttc 60
   agtagtcatg gcatccactg ggtccgccag gctccaggca aggggctgga gtgggtggca 120
   gttatatggt atgatggaag aaataaagac tatgcagact ccgtgaaggg ccgattcacc 180
   atctccagag acaattccaa gaacacgctg tatttgcaaa tgaacagcct gagagccgag 240
   gacacggctg tgtattactg tgcgagagtg gccccactgg ggccacttga ctactggggc 300
   cagggaaccc tggtcaccgt ctcctcagcc tccaccaagg gcccatcggt cttccccctg 360
   gcgccctgct ccaggagcac ctccgagagc acagcggccc tgggctgcct ggtcaaggac 420
   tacttccccg aaccggtgac ggtgtcgtgg aactcaggcg ctctgaccag cggcgtgcac 480
   accttcccag ctgtcctaca g                                           501
<210> 33
   <211> 516
   <212> DNA
   <213> Homo sapiens
<400> 33
   tcgggcccag gactggtgaa gccttcacag atcctgtccc tcacctgcac tgtctctggt 60
   ggctccatca gcagtggtgg tcactactgg agctggatcc gccagcaccc agggaagggc 120
   ctggagtgga ttgggtacat ctattacatt gggaacacct actacaaccc gtccctcaag 180
   agtcgagtta ccatatcagt agacacgtct aagaaccagt tctccctgaa gctgagctct 240
   gtgactgccg cggacacggc cgtgtattat tgtgcgagag atagtgggga ctactacggt 300
   atagacgtct ggggccaagg gaccacggtc accgtctcct cagcttccac caagggccca 360
   tccgtcttcc ccctggcgcc ctgctccagg agcacctccg agagcacagc cgccctgggc 420
   tgcctggtca aggactactt ccccgaaccg gtgacggtgt cgtggaactc aggcgccctg 480
   accagcggcg tgcacacctt cccggctgtc ctacaa                           516
<210> 34
   <211> 459
   <212> DNA
   <213> Homo sapiens
<400> 34
   cctgggaggt ccctgagact ctcctgtgca gcgtctggat tcaccttcag tagtcatggc 60
   atccactggg tccgccaggc tccaggcaag gggctggagt gggtggcagt tatatggtat 120
   gatggaagaa ataaagacta tgcagactcc gtgaagggcc gattcaccat ctccagagac 180
   aattccaaga acacgctgta tttgcaaatg aacagcctga gagccgagga cacggctgtg 240
   tattactgtg cgagagtggc cccactgggg ccacttgact actggggcca gggaaccctg 300
   gtcaccgtct cctcagcctc caccaagggc ccatcggtct tccccctggc gccctgctcc 360
   aggagcacct ccgagagcac agcggccctg ggctgcctgg tcaaggacta cttccccgaa 420
   ccggtgacgg tgtcgtggaa ctcaggcgct ctgaccagc 459
<210> 35
   <211> 503
   <212> DNA
   <213> Homo sapiens
<400> 35
   ggcgtggtcc agcctgggag gtccctgaga ctctcctgtg cagcgtctgg attcaccttc 60
   agtagctatg gcatgcactg ggtccgccag gctccaggca aggggctgga gtgggtggca 120
   gttatatggt atgatggaag taataaatac tatgcagact ccgtgaaggg ccgattcacc 180
   atctccagag acaattccaa gaacacgctg tatctgcaaa tgaacagcct gagagccgag 240
   gacacggctg tgtattactg tgcgagagat ccgaggggag ctacccttta ctactactac 300
   taccggtkgg acgtctgggg ccaagggacc acggtcaccg tctcctcagc ctccaccaag 360
   ggcccatcgg tcttccccct ggcgccctgc tccaggagca cctccgagag cacagcggcc 420
   ctgggctgcc tggtcaagga ctacttcccc gaaccggtga cggtgtcgtg gaactcaggc 480
   gctctgacca gcggcgtgca cac 503
<210> 36
   <211> 451
   <212> DNA
   <213> Homo sapiens
<400> 36
   ggcgtggtcc agcctgggag gtccctgaga ctctcctgtg cagcgtctgg attcaccttc 60
   agtagctatg gcatgcactg ggtccgccag gctccaggca aggggctgga gtgggtggca 120
   gttatatggt atgatggaag tcataaatac tatgcagact ccgtgaaggg ccgattcacc 180
   atctccagag acaattccaa gaacacgctg tatctgcaaa tgaacagcct gagagccgag 240
   gacacggctg tgtattactg tgcgagaggc gctgtagtag taccagctgc tatggacgtc 300
   tggggccaag ggaccacggt caccgtctcc tcagcctcca ccaagggccc atcggtcttc 360
   cccctggcgc cctgctccag gagcacctcc gagagcacag cggccctggg ctgcctggtc 420
   aaggactact tccccgaacc ggtgacggtg t 451
<210> 37
   <211> 438
   <212> DNA
   <213> Homo sapiens
<220>
   <221> modified_base
   <222> (64)
   <223> a, c, t, g, other or unknown
<400> 37
   gtggtccagc ctgggaggtc cctgagactc tcctgtgcag cgtctggatt caccttcagt 60
   agcngtggca tgcactgggt ccgccaggct ccaggcaagg ggctggagtg ggtggcagtt 120
   atatggtctg atggaagtca taaatactat gcagactccg tgaagggccg attcaccatc 180
   tccagagaca attccaagaa cacgctgtat ctgcaaatga acagcctgag agccgaggac 240
   acggctgtgt attactgtgc gagaggaact atgatagtag tgggtaccct tgactactgg 300
   ggccagggaa ccctggtcac cgtctcctca gcctccacca agggcccatc ggtcttcccc 360
   ctggcgccct gctccaggag cacctccgag agcacagcgg ccctgggctg cctggtcaag 420
   gactacttcc ccgaaccg 438
<210> 38
   <211> 562
   <212> DNA
   <213> Homo sapiens
<400> 38
   tcctgtgcag cgtctggatt caccttcagt tactatggcg tctgggggag gcgtggtcca 60
   gcctgggagg tccctgagac tctcctgtgc agcgtctgga ttcaccttca gtagctatgg 120
   cgtgcactgg gtccgccagg ctccaggcaa ggggctggag tgggtggcag ttatatggta 180
   tgatggaagt aataaatact atgcagactc cgtgaagggc cgattcacca tctccagaga 240
   caattccaag agcacgctgt atctgcaaat gaacagcctg agagccgagg acacggctgt 300
   gtattattgt gcgagagact cgtattacga tttttggagt ggtcggggcg gtatggacgt 360
   ctggggccaa gggaccacgg tcaccgtctc ctcagcctcc accaagggcc catcggtctt 420
   ccccctggcg ccctgctcca ggagcacctc cgagagcaca gcggccctgg gctgcctggt 480
   caaggactac ttccccgaac cggtgacggt gtcgtggaac tcaggcgctc tgaccagcgg 540
   cgtgcacacc ttcccagctg tc 562
<210> 39
   <211> 490
   <212> DNA
   <213> Homo sapiens
<400> 39
   gtccagcctg ggaggtccct gagactctcc tgtgcagcgt ctggattcac cttcagtaac 60
   tatgccatgc actgggtccg ccaggctcca ggcaaggggc tggagtgggt ggtagttatt 120
   tggcatgatg gaaataataa atactatgca gagtccgtga agggccgatt caccatctcc 180
   agagacaatt ccaagaacac gctgtatctg caaatgaaca gcctgagagc cgaggacacg 240
   gctgtatatt actgtgcgag agatcagggc actggctggt acggaggctt tgacttctgg 300
   ggccagggaa ccctggtcac cgtctcctca gcctccacca agggcccatc ggtcttcccc 360
   ctggcgccct gctccaggag cacctccgag agcacagcgg ccctgggctg cctggtcaag 420
   gactacttcc ccgaaccggt gacggtgtcg tggaactcag gcgctctgac cagcggcgtg 480
   cacaccttcc 490
<210> 40
   <211> 708
   <212> DNA
   <213> Homo sapiens
<400> 40
   atggaaaccc cagcgcagct tctcttcctc ctgctactct ggctcccaga taccaccgga 60
   gaaattgtgt tgacgcagtc tccaggcacc ctgtctttgt ctccagggga aagagccacc 120
   ctctcctgca gggccagtca gagtattagc agcagcttct tagcctggta ccagcagaga 180
   cctggccagg ctcccaggct cctcatctat ggtgcatcca gcagggccac tggcatccca 240
   gacaggttca gtggcagtgg gtctgggaca gacttcactc tcaccatcag cagactggag 300
   cctgaagatt ttgcagtgta ttactgtcag cagtatggta cctcaccctg gacgttcggc 360
   caagggacca aggtggaaat caaacgaact gtggctgcac catctgtctt catcttcccg 420
   ccatctgatg agcagttgaa atctggaact gcctctgttg tgtgcctgct gaataacttc 480
   tatcccagag aggccaaagt acagtggaag gtggataacg ccctccaatc gggtaactcc 540
   caggagagtg tcacagagca ggacagcaag gacagcacct acagcctcag cagcaccctg 600
   acgctgagca aagcagacta cgagaaacac aaagtctacg cctgcgaagt cacccatcag 660
   ggcctgagct cgcccgtcac aaagagcttc aacaggggag agtgttag 708
<210> 41
   <211> 702
   <212> DNA
   <213> Homo sapiens
<400> 41
   atggaaaccc cagcgcagct tctcttcctc ctgctactct ggctcccaga taccaccgga 60
   gaaattgtgt tgacgcagtc tccaggcacc ctgtctttgt ctccagggga aagagccacc 120
   ctctcctgca ggaccagtgt tagcagcagt tacttagcct ggtaccagca gaaacctggc 180
   caggctccca ggctcctcat ctatggtgca tccagcaggg ccactggcat cccagacagg 240
   ttcagtggca gtgggtctgg gacagacttc actctcacca tcagcagact ggagcctgaa 300
   gattttgcag tctattactg tcagcagtat ggcatctcac ccttcacttt cggcggaggg 360
   accaaggtgg agatcaagcg aactgtggct gcaccatctg tcttcatctt cccgccatct 420
   gatgagcagt tgaaatctgg aactgcctct gttgtgtgcc tgctgaataa cttctatccc 480
   agagaggcca aagtacagtg gaaggtggat aacgccctcc aatcgggtaa ctcccaggag 540
   agtgtcacag agcaggacag caaggacagc acctacagcc tcagcagcac cctgacgctg 600
   agcaaagcag actacgagaa acacaaagtc tacgcctgcg aagtcaccca tcagggcctg 660
   agctcgcccg tcacaaagag cttcaacagg ggagagtgtt ag 702
<210> 42
   <211> 417
   <212> DNA
   <213> Homo sapiens
<400> 42
   ggcaccctgt ctttgtctcc aggggaaaga gccaccctct cctgcagggc cagtcagagt 60
   gtcagcagct acttagcctg gtaccagcag aaacctggcc aggctcccag actcctcatc 120
   tatggtgcat ccagcagggc cactggcatc ccagacaggt tcagtggcag tgggtctggg 180
   acagacttca ctctcaccat cagcagactg gagcctgagg attttgcagt gtattactgt 240
   cagcagtatg gtaggtcacc attcactttc ggccctggga ccaaagtgga tatcaagcga 300
   actgtggctg caccatctgt cttcatcttc ccgccatctg atgagcagtt gaaatctgga 360
   actgcctctg ttgtgtgcct gctgaataac ttctatccca gagaggccaa agtacag 417
<210> 43
   <211> 705
   <212> DNA
   <213> Homo sapiens
<400> 43
   atggaaaccc cagcgcagct tctcttcctc ctgctactct ggctcccaga taccaccgga 60
   gaaattgtgt tgacgcagtc tccaggcacc ctgtctttgt ctccagggga aagagccacc 120
   ctctcctgta gggccagtca aagtgttagc agctacttag cctggtacca acagaaacct 180
   ggccaggctc ccaggcccct catctatggt gtatccagca gggccactgg catcccagac 240
   aggttcagtg gcagtgggtc tgggacagac ttcactctca ccatcagcag actggagcct 300
   gaagattttg cagtgtatta ctgtcagcag tatggtatct caccattcac tttcggccct 360
   gggaccaaag tggatatcaa acgaactgtg gctgcaccat ctgtcttcat cttcccgcca 420
   tctgatgagc agttgaaatc tggaactgcc tctgttgtgt gcctgctgaa taacttctat 480
   cccagagagg ccaaagtaca gtggaaggtg gataacgccc tccaatcggg taactcccag 540
   gagagtgtca cagagcagga cagcaaggac agcacctaca gcctcagcag caccctgacg 600
   ctgagcaaag cagactacga gaaacacaaa gtctacgcct gcgaagtcac ccatcagggc 660
   ctgagctcgc ccgtcacaaa gagcttcaac aggggagagt gttag                 705
<210> 44
   <211> 458
   <212> DNA
   <213> Homo sapiens
<400> 44
   cagtctccat cctccctgtc tgcatctgta ggagacagag tcaccatcac ttgccgggca 60
   agtcagagca ttaacaccta tttaatttgg tatcagcaga aaccagggaa agcccctaac 120
   ttcctgatct ctgctacatc cattttgcaa agtggggtcc catcaaggtt ccgtggcagt 180
   ggctctggga caaatttcac tctcaccatc aacagtcttc atcctgaaga ttttgcaact 240
   tactactgtc aacagagtta cagtacccca ttcactttcg gccctgggac caaagtggat 300
   atcaaacgaa ctgtggctgc accatctgtc ttcatcttcc cgccatctga tgagcagttg 360
   aaatctggaa ctgcctctgt tgtgtgcctg ctgaataact tctatcccag agaggccaaa 420
   gtacagtgga aggtggataa cgccctccaa tcgggtaa                         458
<210> 45
   <211> 426
   <212> DNA
   <213> Homo sapiens
<400> 45
   tctccaggca ccctgtcttt gtctccaggg gaaagagcca ccctctcctg cagggccagt 60
   cagagtatta gcagcaattt cttagcctgg taccagcaga aacctggcca ggctcccagg 120
   ctcctcatct atcgtccatc cagcagggcc actggcatcc cagacagttt cagtggcagt 180
   gggtctggga cagacttcac tctcaccatc agcagactgg agcctgagga ttttgcatta 240
   tattactgtc agcagtatgg tacgtcacca ttcactttcg gccctgggac caaagtggat 300
   atcaagcgaa ctgtggctgc accatctgtc ttcatcttcc cgccatctga tgagcagttg 360
   aaatctggaa ctgcctctgt tgtgtgcctg ctgaataact tctatcccag agaggccaaa 420
   gtacag                                                            426
<210> 46
   <211> 465
   <212> DNA
   <213> Homo sapiens
<400> 46
   tctccagact ttcagtctgt gactccaaag gagaaagtca ccatcacctg ccgggccagt 60
   cagagcattg gtagtagctt acattggtat cagcagaaac cagatcagtc tccaaagctc 120
   ctcatcaagt atgcttccca gtccttctct ggggtcccct cgaggttcag tggcagtgga 180
   tctgggacag atttcaccct caccatcaat agcctggaag ctgaagatgc tgcaacgtat 240
   tactgtcatc agagtagtag tttaccgctc actttcggcg gagggaccaa ggtggagatc 300
   aaacgaactg tggctgcacc atctgtcttc atcttcccgc catctgatga gcagttgaaa 360
   tctggaactg cctctgttgt gtgcctgctg aataacttct atcccagaga ggccaaagta 420
   cagtggaagg tggataacgc cctccaatcg ggtaactccc aggag                 465
<210> 47
   <211> 440
   <212> DNA
   <213> Homo sapiens
<400> 47
   cagtctccag gcaccctgtc tttgtctcca ggggaaagag ccaccctctc ctgcagggcc 60
   agtcagagtg tcagcagcta cttagcctgg taccagcaga aacctggcca ggctcccagg 120
   ctcctcatct atggtgcatc cagcagggcc actggcatcc cagacaggtt cagtggcagt 180
   gggtctggga cagacttcac tctcaccatc agcagactgg agcctgagga ttttgcagtg 240
   tattactgtc aacagtatgg taggtcacca ttcactttcg gccctgggac caaagtagat 300
   atcaagcgaa ctgtggctgc accatctgtc ttcatcttcc cgccatctga tgagcagttg 360
   aaatctggaa ctgcctctgt tgtgtgcctg ctgaataact tctatcccag agaggccaaa 420
   gtacagtgga aaggtggata                                             440
<210> 48
   <211> 417
   <212> DNA
   <213> Homo sapiens
<400> 48
   ccatcctccc tgtctgcatc tgtaggagac agagtcacca tcacttgccg ggcaagtcag 60
   agcattaaca gctatttaga ttggtatcag cagaaaccag ggaaagcccc taaactcctg 120
   atctatgctg catccagttt gcaaagtggg gtcccatcaa ggttcagtgg cagtggatct 180
   gggacagatt tcactctcac catcagcagt ctgcaacctg aagattttgc aacttactac 240
   tgtcaacagt attacagtac tccattcact ttcggccctg ggaccaaagt ggaaatcaaa 300
   cgaactgtgg ctgcaccatc tgtcttcatc ttcccgccat ctgatgagca gttgaaatct 360
   ggaactgcct ctgttgtgtg cctgctgaat aacttctatc ccagagaggc caaagta    417
<210> 49
   <211> 402
   <212> DNA
   <213> Homo sapiens
<220>
   <221> modified_base
   <222> (207)
   <223> a, c, t, g, other or unknown
<400> 49
   acccagtctc catcctccct gtctgcatct gtaggagaca gagtcaccat cacttgccgg 60
   gcaagtcaga acattagcag gtatttaaat tggtatcaac agaaaccagg gaaagcccct 120
   aagttcctga tctatgttgc atctattttg caaagtgggg tcccatcagg gttcagtgcc 180
   agtggatctg ggccagattt cactctnacc atcagcagtc tgcaacctga agattttgca 240
   acttactact gtcaacagag ttacagtacc ccattcactt tcggccctgg gaccaaagtg 300
   gatatcaaac gaactgtggc tgcaccatct gtcttcatct tcccgccatc tgatgagcag 360
   ttgaaatctg gaactgcctc tgttgtgtgc ctgctgaata ac 402
<210> 50
   <211> 451
   <212> DNA
   <213> Homo sapiens
<400> 50
   acccagtctc catcctccct gtctgcatct gtaggagaca gagtcaccat cacttgccgg 60
   gcaagtcaga gcatttgcaa ctatttaaat tggtatcagc agaaaccagg aaaagcccct 120
   agggtcctga tctatgctgc atccagtttg caaggtgggg tcccgtcaag gttcagtggc 180
   agtggatctg ggacagattg cactctcacc atcagcagtc tgcaacctga agattttgca 240
   acttactact gtcaacagag ttacactacc ccattcactt tcggccctgg gaccagagtg 300
   gatatcgaac gaactgtggc tgcaccatct gtcttcatct tcccgccatc tgatgagcag 360
   ttgaaatctg gaactgcctc tgttgtgtgc ctgctgaata acttctatcc cagagaggcc 420
   aaagtacagt ggaaggtgga taacgcctat t 451
<210> 51
   <211> 419
   <212> DNA
   <213> Homo sapiens
<400> 51
   ccactctccc tgcccgtcac ccttggacag ccggcctcca tctcctgcag gtctagtcaa 60
   agcctcgtat acagtgatgg aaacacctac ttgaattggt ttcagcagag gccaggccaa 120
   tctccaaggc gcctaattta taaggtttct aactgggact ctggggtccc agacagattc 180
   agcggcagtg ggtcaggcac tgatttcaca ctgaaaatca gcagggtgga ggctgaggat 240
   gttggggttt attactgcat gcaaggttca cactggcctc cgacgttcgg ccaagggacc 300
   aaggtggaaa tcaaacgaac tgtggctgca ccatctgtct tcatcttccc gccatctgat 360
   gagcagttga aatctggaac tgcctctgtt gtgtgcctgc tgaataactt ctatcccac 419
<210> 52
   <211> 419
   <212> DNA
   <213> Homo sapiens
<400> 52
   cctggagagc cggcttccat ctcttgcagg tctagtcaga gcctcctgca tagtaatgga 60
   tacaactatt tggattggta cctgcagaag ccaggacagt ctccacagct cctgatctat 120
   ttgggttcta atcgggcctc cggggtccct gacaggttca gtggcagtgg atcaggcaca 180
   gattttacac tgaaactcag cagagtggag gctgaggatg ttggggttta ttactgcatg 240
   caagctctac aaactcctct cactttcggc ggagggacca aggtggagat caaacgaact 300
   gtggctgcac catctgtctt catcttcccg ccatctgatg agcagttgaa atctggaact 360
   gcctctgttg tgtgcctgct gaataacttc tatcccagar aggccaaagt acattccat 419
<210> 53
   <211> 1392
   <212> DNA
   <213> Homo sapiens
<400> 53
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtgtagcgt ctggattcac cttcagtagc catggcatgc actgggtccg ccaggctcca 180
   ggcaaggggc tggagtgggt ggcagttata tggtatgatg gaagaaataa atactatgca 240
   gactccgtga agggccgatt caccatctcc agagacaatt ccaagaacac gctgtttctg 300
   caaatgaaca gcctgagagc cgaggacacg gctgtgtatt actgtgcgag aggaggtcac 360
   ttcggtcctt ttgactactg gggccaggga accctggtca ccgtctcctc agcctccacc 420
   aagggcccat cggtcttccc cctggcgccc tgctccagga gcacctccga gagcacagcg 480
   gccctgggct gcctggtcaa ggactacttc cccgaaccgg tgacggtgtc gtggaactca 540
   ggcgctctga ccagcggcgt gcacaccttc ccagctgtcc tacagtcctc aggactctac 600
   tccctcagca gcgtggtgac cgtgccctcc agcaacttcg gcacccagac ctacacctgc 660
   aacgtagatc acaagcccag caacaccaag gtggacaaga cagttgagcg caaatgttgt 720
   gtcgagtgcc caccgtgccc agcaccacct gtggcaggac cgtcagtctt cctcttcccc 780
   ccaaaaccca aggacaccct catgatctcc cggacccctg aggtcacgtg cgtggtggtg 840
   gacgtgagcc acgaagaccc cgaggtccag ttcaactggt acgtggacgg cgtggaggtg 900
   cataatgcca agacaaagcc acgggaggag cagttcaaca gcacgttccg tgtggtcagc 960
   gtcctcaccg ttgtgcacca ggactggctg aacggcaagg agtacaagtg caaggtctcc 1020
   aacaaaggcc tcccagcccc catcgagaaa accatctcca aaaccaaagg gcagccccga 1080
   gaaccacagg tgtacaccct gcccccatcc cgggaggaga tgaccaagaa ccaggtcagc 1140
   ctgacctgcc tggtcaaagg cttctacccc agcgacatcg ccgtggagtg ggagagcaat 1200
   gggcagccgg agaacaacta caagaccaca cctcccatgc tggactccga cggctccttc 1260
   ttcctctaca gcaagctcac cgtggacaag agcaggtggc agcaggggaa cgtcttctca 1320
   tgctccgtga tgcatgaggc tctgcacaac cactacacgc agaagagcct ctccctgtct 1380
   ccgggtaaat ga 1392
<210> 54
   <211> 1999
   <212> DNA
   <213> Homo sapiens
<400> 54
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtgtagcgt ctggattcac cttcagtagc catggcatgc actgggtccg ccaggctcca 180
   ggcaaggggc tggagtgggt ggcagttata tggtatgatg gaagaaataa atactatgca 240
   gactccgtga agggccgatt caccatctcc agagacaatt ccaagaacac gctgtttctg 300
   caaatgaaca gcctgagagc cgaggacacg gctgtgtatt actgtgcgag aggaggtcac 360
   ttcggtcctt ttgactactg gggccaggga accctggtca ccgtctcctc agctagcacc 420
   aagggcccat cggtcttccc cctggcgccc tgctccagga gcacctccga gagcacagcg 480
   gccctgggct gcctggtcaa ggactacttc cccgaaccgg tgacggtgtc gtggaactca 540
   ggcgctctga ccagcggcgt gcacaccttc ccagctgtcc tacagtcctc aggactctac 600
   tccctcagca gcgtggtgac cgtgccctcc agcaacttcg gcacccagac ctacacctgc 660
   aacgtagatc acaagcccag caacaccaag gtggacaaga cagttggtga gaggccagct 720
   cagggaggga gggtgtctgc tggaagccag gctcagccct cctgcctgga cgcaccccgg 780
   ctgtgcagcc ccagcccagg gcagcaaggc aggccccatc tgtctcctca cccggaggcc 840
   tctgcccgcc ccactcatgc tcagggagag ggtcttctgg ctttttccac caggctccag 900
   gcaggcacag gctgggtgcc cctaccccag gcccttcaca cacaggggca ggtgcttggc 960
   tcagacctgc caaaagccat atccgggagg accctgcccc tgacctaagc cgaccccaaa 1020
   ggccaaactg tccactccct cagctcggac accttctctc ctcccagatc cgagtaactc 1080
   ccaatcttct ctctgcagag cgcaaatgtt gtgtcgagtg cccaccgtgc ccaggtaagc 1140
   cagcccaggc ctcgccctcc agctcaaggc gggacaggtg ccctagagta gcctgcatcc 1200
   agggacaggc cccagctggg tgctgacacg tccacctcca tctcttcctc agcaccacct 1260
   gtggcaggac cgtcagtctt cctcttcccc ccaaaaccca aggacaccct catgatctcc 1320
   cggacccctg aggtcacgtg cgtggtggtg gacgtgagcc acgaagaccc cgaggtccag 1380
   ttcaactggt acgtggacgg cgtggaggtg cataatgcca agacaaagcc acgggaggag 1440
   cagttcaaca gcacgttccg tgtggtcagc gtcctcaccg ttgtgcacca ggactggctg 1500
   aacggcaagg agtacaagtg caaggtctcc aacaaaggcc tcccagcccc catcgagaaa 1560
   accatctcca aaaccaaagg tgggacccgc ggggtatgag ggccacatgg acagaggccg 1620
   gctcggccca ccctctgccc tgggagtgac cgctgtgcca acctctgtcc ctacagggca 1680
   gccccgagaa ccacaggtgt acaccctgcc cccatcccgg gaggagatga ccaagaacca 1740
   ggtcagcctg acctgcctgg tcaaaggctt ctaccccagc gacatcgccg tggagtggga 1800
   gagcaatggg cagccggaga acaactacaa gaccacacct cccatgctgg actccgacgg 1860
   ctccttcttc ctctacagca agctcaccgt ggacaagagc aggtggcagc aggggaacgt 1920
   cttctcatgc tccgtgatgc atgaggctct gcacaaccac tacacgcaga agagcctctc 1980
   cctgtctccg ggtaaatga                                              1999
<210> 55
   <211> 1392
   <212> DNA
   <213> Homo sapiens
<400> 55
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtgtagcgt ctggattcac cttcagtagc catggcatgc actgggtccg ccaggctcca 180
   ggcaaggggc tggagtgggt ggcagttata tggtatgatg gaagaaataa atactatgca 240
   gactccgtga agggccgatt caccatctcc agagacaatt ccaagaacac gctgtttctg 300
   caaatgaaca gcctgagagc cgaggacacg gctgtgtatt actgtgcgag aggaggtcac 360
   ttcggtcctt ttgactactg gggccaggga accctggtca ccgtctcctc agcctccacc 420
   aagggcccat cggtcttccc cctggcgccc tgctccagga gcacctccga gagcacagcg 480
   gccctgggct gcctggtcaa ggactacttc cccgaaccgg tgacggtgtc gtggaactca 540
   ggcgctctga ccagcggcgt gcacaccttc ccagctgtcc tacagtcctc aggactctac 600
   tccctcagca gcgtggtgac cgtgccctcc agcaacttcg gcacccagac ctacacctgc 660
   aacgtagatc acaagcccag caacaccaag gtggacaaga cagttgagcg caaatgttgt 720
   gtcgagtgcc caccgtgccc agcaccacct gtggcaggac cgtcagtctt cctcttcccc 780
   ccaaaaccca aggacaccct catgatctcc cggacccctg aggtcacgtg cgtggtggtg 840
   gacgtgagcc acgaagaccc cgaggtccag ttcaactggt acgtggacgg cgtggaggtg 900
   cataatgcca agacaaagcc acgggaggag cagttccaaa gcacgttccg tgtggtcagc 960
   gtcctcaccg ttgtgcacca ggactggctg aacggcaagg agtacaagtg caaggtctcc 1020
   aacaaaggcc tcccagcccc catcgagaaa accatctcca aaaccaaagg gcagccccga 1080
   gaaccacagg tgtacaccct gcccccatcc cgggaggaga tgaccaagaa ccaggtcagc 1140
   ctgacctgcc tggtcaaagg cttctacccc agcgacatcg ccgtggagtg ggagagcaat 1200
   gggcagccgg agaacaacta caagaccaca cctcccatgc tggactccga cggctccttc 1260
   ttcctctaca gcaagctcac cgtggacaag agcaggtggc agcaggggaa cgtcttctca 1320
   tgctccgtga tgcatgaggc tctgcacaac cactacacgc agaagagcct ctccctgtct 1380
   ccgggtaaat ga                                                     1392
<210> 56
   <211> 708
   <212> DNA
   <213> Homo sapiens
<400> 56
   atggaaaccc cagcgcagct tctcttcctc ctgctactct ggctcccaga taccaccgga 60
   gaaattgtgt tgacgcagtc tccaggcacc ctgtctttgt ctccagggga aagagccacc 120
   ctctcctgca gggccagtca gagtattagc agcagcttct tagcctggta ccagcagaga 180
   cctggccagg ctcccaggct cctcatctat ggtgcatcca gcagggccac tggcatccca 240
   gacaggttca gtggcagtgg gtctgggaca gacttcactc tcaccatcag cagactggag 300
   cctgaagatt ttgcagtgta ttactgtcag cagtatggta cctcaccctg gacgttcggc 360
   caagggacca aggtggaaat caaacgaact gtggctgcac catctgtctt catcttcccg 420
   ccatctgatg agcagttgaa atctggaact gcctctgttg tgtgcctgct gaataacttc 480
   tatcccagag aggccaaagt acagtggaag gtggataacg ccctccaatc gggtaactcc 540
   caggagagtg tcacagagca ggacagcaag gacagcacct acagcctcag cagcaccctg 600
   acgctgagca aagcagacta cgagaaacac aaagtctacg cctgcgaagt cacccatcag 660
   ggcctgagct cgcccgtcac aaagagcttc aacaggggag agtgttag             708
<210> 57
   <211> 1395
   <212> DNA
   <213> Homo sapiens
<400> 57
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtacagcgt ctggattcac cttcagtaac tatggcatgc actgggtccg ccaggctcca 180
   ggcaaggggc tggagtgggt ggcagttata tggtatgatg gaagtaataa acactatgga 240
   gactccgtga agggccgatt caccatctcc agtgacaatt ccaagaacac gctgtatctg 300
   caaatgaaca gcctgagagc cgaggacacg gctgtgtatt actgtgcgag aggagagaga 360
   ctggggtcct actttgacta ctggggccag ggaaccctgg tcaccgtctc ctcagcctcc 420
   accaagggcc catcggtctt ccccctggcg ccctgctcca ggagcacctc cgagagcaca 480
   gcggccctgg gctgcctggt caaggactac ttccccgaac cggtgacggt gtcgtggaac 540
   tcaggcgctc tgaccagcgg cgtgcacacc ttcccagctg tcctacagtc ctcaggactc 600
   tactccctca gcagcgtggt gaccgtgccc tccagcaact tcggcaccca gacctacacc 660
   tgcaacgtag atcacaagcc cagcaacacc aaggtggaca agacagttga gcgcaaatgt 720
   tgtgtcgagt gcccaccgtg cccagcacca cctgtggcag gaccgtcagt cttcctcttc 780
   cccccaaaac ccaaggacac cctcatgatc tcccggaccc ctgaggtcac gtgcgtggtg 840
   gtggacgtga gccacgaaga ccccgaggtc cagttcaact ggtacgtgga cggcgtggag 900
   gtgcataatg ccaagacaaa gccacgggag gagcagttca acagcacgtt ccgtgtggtc 960
   agcgtcctca ccgttgtgca ccaggactgg ctgaacggca aggagtacaa gtgcaaggtc 1020
   tccaacaaag gcctcccagc ccccatcgag aaaaccatct ccaaaaccaa agggcagccc 1080
   cgagaaccac aggtgtacac cctgccccca tcccgggagg agatgaccaa gaaccaggtc 1140
   agcctgacct gcctggtcaa aggcttctac cccagcgaca tcgccgtgga gtgggagagc 1200
   aatgggcagc cggagaacaa ctacaagacc acacctccca tgctggactc cgacggctcc 1260
   ttcttcctct acagcaagct caccgtggac aagagcaggt ggcagcaggg gaacgtcttc 1320
   tcatgctccg tgatgcatga ggctctgcac aaccactaca cgcagaagag cctctccctg 1380
   tctccgggta aatga                                                  1395
<210> 58
   <211> 702
   <212> DNA
   <213> Homo sapiens
<400> 58
   atggaaaccc cagcgcagct tctcttcctc ctgctactct ggctcccaga taccaccgga 60
   gaaattgtgt tgacgcagtc tccaggcacc ctgtctttgt ctccagggga aagagccacc 120
   ctctcctgca ggaccagtgt tagcagcagt tacttagcct ggtaccagca gaaacctggc 180
   caggctccca ggctcctcat ctatggtgca tccagcaggg ccactggcat cccagacagg 240
   ttcagtggca gtgggtctgg gacagacttc actctcacca tcagcagact ggagcctgaa 300
   gattttgcag tctattactg tcagcagtat ggcatctcac ccttcacttt cggcggaggg 360
   accaaggtgg agatcaagcg aactgtggct gcaccatctg tcttcatctt cccgccatct 420
   gatgagcagt tgaaatctgg aactgcctct gttgtgtgcc tgctgaataa cttctatccc 480
   agagaggcca aagtacagtg gaaggtggat aacgccctcc aatcgggtaa ctcccaggag 540
   agtgtcacag agcaggacag caaggacagc acctacagcc tcagcagcac cctgacgctg 600
   agcaaagcag actacgagaa acacaaagtc tacgcctgcg aagtcaccca tcagggcctg 660
   agctcgcccg tcacaaagag cttcaacagg ggagagtgtt ag                    702
<210> 59
   <211> 1392
   <212> DNA
   <213> Homo sapiens
<400> 59
   atggagtttg ggctgagctg ggttttcctc gttgctcttt taagaggtgt ccagtgtcag 60
   gtgcagctgg tggagtctgg gggaggcgtg gtcgagcctg ggaggtccct gagactctcc 120
   tgtacagcgt ctggattcac cttcagtagt tatggcatgc actgggtccg ccaggctcca 180
   ggcaaggggc tggagtgggt ggcagttata tggtatgatg gaagcaataa acactatgca 240
   gactccgcga agggccgatt caccatctcc agagacaatt ccaagaacac gctgtatctg 300
   caaatgaaca gcctgagagc cgaggacacg gctgtgtatt actgtgcgag agccggactg 360
   ctgggttact ttgactactg gggccaggga accctggtca ccgtctcctc agcctccacc 420
   aagggcccat cggtcttccc cctggcgccc tgctccagga gcacctccga gagcacagcg 480
   gccctgggct gcctggtcaa ggactacttc cccgaaccgg tgacggtgtc gtggaactca 540
   ggcgctctga ccagcggcgt gcacaccttc ccagctgtcc tacagtcctc aggactctac 600
   tccctcagca gcgtggtgac cgtgccctcc agcaacttcg gcacccagac ctacacctgc 660
   aacgtagatc acaagcccag caacaccaag gtggacaaga cagttgagcg caaatgttgt 720
   gtcgagtgcc caccgtgccc agcaccacct gtggcaggac cgtcagtctt cctcttcccc 780
   ccaaaaccca aggacaccct catgatctcc cggacccctg aggtcacgtg cgtggtggtg 840
   gacgtgagcc acgaagaccc cgaggtccag ttcaactggt acgtggacgg cgtggaggtg 900
   cataatgcca agacaaagcc acgggaggag cagttcaaca gcacgttccg tgtggtcagc 960
   gtcctcaccg ttgtgcacca ggactggctg aacggcaagg agtacaagtg caaggtctcc 1020
   aacaaaggcc tcccagcccc catcgagaaa accatctcca aaaccaaagg gcagccccga 1080
   gaaccacagg tgtacaccct gcccccatcc cgggaggaga tgaccaagaa ccaggtcagc 1140
   ctgacctgcc tggtcaaagg cttctacccc agcgacatcg ccgtggagtg ggagagcaat 1200
   gggcagccgg agaacaacta caagaccaca cctcccatgc tggactccga cggctccttc 1260
   ttcctctaca gcaagctcac cgtggacaag agcaggtggc agcaggggaa cgtcttctca 1320
   tgctccgtga tgcatgaggc tctgcacaac cactacacgc agaagagcct ctccctgtct 1380
   ccgggtaaat ga                                                     1392
<210> 60
   <211> 705
   <212> DNA
   <213> Homo sapiens
<400> 60
   atggaaaccc cagcgcagct tctcttcctc ctgctactct ggctcccaga taccaccgga 60
   gaaattgtgt tgacgcagtc tccaggcacc ctgtctttgt ctccagggga aagagccacc 120
   ctctcctgta gggccagtca aagtgttagc agctacttag cctggtacca acagaaacct 180
   ggccaggctc ccaggcccct catctatggt gtatccagca gggccactgg catcccagac 240
   aggttcagtg gcagtgggtc tgggacagac ttcactctca ccatcagcag actggagcct 300
   gaagattttg cagtgtatta ctgtcagcag tatggtatct caccattcac tttcggccct 360
   gggaccaaag tggatatcaa acgaactgtg gctgcaccat ctgtcttcat cttcccgcca 420
   tctgatgagc agttgaaatc tggaactgcc tctgttgtgt gcctgctgaa taacttctat 480
   cccagagagg ccaaagtaca gtggaaggtg gataacgccc tccaatcggg taactcccag 540
   gagagtgtca cagagcagga cagcaaggac agcacctaca gcctcagcag caccctgacg 600
   ctgagcaaag cagactacga gaaacacaaa gtctacgcct gcgaagtcac ccatcagggc 660
   ctgagctcgc ccgtcacaaa gagcttcaac aggggagagt gttag                 705
<210> 61
   <211> 1413
   <212> DNA
   <213> Homo sapiens
<400> 61
   atggagtttg ggctgagctg ggttttcctc gttgctcttt taagaggtgt ccagtgtcag 60
   gtgcagctgg tggagtctgg gggaggcgtg gtccagcctg ggaggtccct gagactctcc 120
   tgtgcagcgt ctggattcac cttcagtagc tatggcatgc actgggtccg ccaggctcca 180
   ggcaaggggc tggagtgggt ggcagttata tggtatgatg gaagtaataa atactatgca 240
   gactccgtga agggccgatt caccatctcc agagacaatt ccaagaacac gctgtatctg 300
   caaatgaaca gcctgagagc cgaggacacg gctgtgtatt actgtgcgag agatccgagg 360
   ggagctaccc tttactacta ctactacggt atggacgtct ggggccaagg gaccacggtc 420
   accgtctcct cagcctccac caagggccca tcggtcttcc ccctggcgcc ctgctccagg 480
   agcacctccg agagcacagc ggccctgggc tgcctggtca aggactactt ccccgaaccg 540
   gtgacggtgt cgtggaactc aggcgctctg accagcggcg tgcacacctt cccagctgtc 600
   ctacagtcct caggactcta ctccctcagc agcgtggtga ccgtgccctc cagcaacttc 660
   ggcacccaga cctacacctg caacgtagat cacaagccca gcaacaccaa ggtggacaag 720
   acagttgagc gcaaatgttg tgtcgagtgc ccaccgtgcc cagcaccacc tgtggcagga 780
   ccgtcagtct tcctcttccc cccaaaaccc aaggacaccc tcatgatctc ccggacccct 840
   gaggtcacgt gcgtggtggt ggacgtgagc cacgaagacc ccgaggtcca gttcaactgg 900
   tacgtggacg gcgtggaggt gcataatgcc aagacaaagc cacgggagga gcagttcaac 960
   agcacgttcc gtgtggtcag cgtcctcacc gttgtgcacc aggactggct gaacggcaag 1020
   gagtacaagt gcaaggtctc caacaaaggc ctcccagccc ccatcgagaa aaccatctcc 1080
   aaaaccaaag ggcagccccg agaaccacag gtgtacaccc tgcccccatc ccgggaggag 1140
   atgaccaaga accaggtcag cctgacctgc ctggtcaaag gcttctaccc cagcgacatc 1200
   gccgtggagt gggagagcaa tgggcagccg gagaacaact acaagaccac acctcccatg 1260
   ctggactccg acggctcctt cttcctctac agcaagctca ccgtggacaa gagcaggtgg 1320
   cagcagggga acgtcttctc atgctccgtg atgcatgagg ctctgcacaa ccactacacg 1380
   cagaagagcc tctccctgtc tccgggtaaa tga                              1413
<210> 62
   <211> 714
   <212> DNA
   <213> Homo sapiens
<400> 62
   atggacatga gggtccccgc tcagctcctg gggctcctgc tactctggct ccgaggtgcc 60
   agatgtgaca tccagatgac ccagtctcca tcctccctgt ctgcatctgt aggagacaga 120
   gtcaccatca cttgccgggc aagtcagagc attaacagct atttagattg gtatcagcag 180
   aaaccaggga aagcccctaa actcctgatc tatgctgcat ccagtttgca aagtggggtc 240
   ccatcaaggt tcagtggcag tggatctggg acagatttca ctctcaccat cagcagtctg 300
   caacctgaag attttgcaac ttactactgt caacagtatt acagtactcc attcactttc 360
   ggccctggga ccaaagtgga aatcaaacga actgtggctg caccatctgt cttcatcttc 420
   ccgccatctg atgagcagtt gaaatctgga actgcctctg ttgtgtgcct gctgaataac 480
   ttctatccca gagaggccaa agtacagtgg aaggtggata acgccctcca atcgggtaac 540
   tcccaggaga gtgtcacaga gcaggacagc aaggacagca cctacagcct cagcagcacc 600
   ctgacgctga gcaaagcaga ctacgagaaa cacaaagtct acgcctgcga agtcacccat 660
   cagggcctga gctcgcccgt cacaaagagc ttcaacaggg gagagtgtta gtga       714
<210> 63
   <211> 463
   <212> PRT
   <213> Homo sapiens
<400> 63
   Met Glu Phe Gly Leu Ser Trp Val Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Val Ala Ser Gly Phe Thr Phe
           35                  40                  45
   Ser Ser His Gly Met His Trp Val Arg Gln Ala Pro Gly Lys Gly Leu
       50                  55                  60
   Glu Trp Val Ala Val Ile Trp Tyr Asp Gly Arg Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Phe Leu Gln Met Asn Ser Leu Arg Ala Glu Asp Thr Ala Val
               100                 105                 110
   Tyr Tyr Cys Ala Arg Gly Gly His Phe Gly Pro Phe Asp Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Cys Ser Arg Ser Thr Ser Glu Ser Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Asn Phe Gly Thr Gln Thr Tyr Thr Cys Asn Val Asp His
       210                 215                 220
   Lys Pro Ser Asn Thr Lys Val Asp Lys Thr Val Glu Arg Lys Cys Cys
   225                 230                 235                 240
   Val Glu Cys Pro Pro Cys Pro Ala Pro Pro Val Ala Gly Pro Ser Val
   245 250 255
   Phe Leu Phe Pro Pro Lys Pro Lys Asp Thr Leu Met Ile Ser Arg Thr
   260 265 270
   Pro Glu Val Thr Cys Val Val Val Asp Val Ser His Glu Asp Pro Glu
   275 280 285
   Val Gln Phe Asn Trp Tyr Val Asp Gly Val Glu Val His Asn Ala Lys
   290 295 300
   Thr Lys Pro Arg Glu Glu Gln Phe Asn Ser Thr Phe Arg Val Val Ser
   305 310 315 320
   Val Leu Thr Val Val His Gln Asp Trp Leu Asn Gly Lys Glu Tyr Lys
   325 330 335
   Cys Lys Val Ser Asn Lys Gly Leu Pro Ala Pro Ile Glu Lys Thr Ile
   340 345 350
   Ser Lys Thr Lys Gly Gln Pro Arg Glu Pro Gln Val Tyr Thr Leu Pro
   355 360 365
   Pro Ser Arg Glu Glu Met Thr Lys Asn Gln Val Ser Leu Thr Cys Leu
   370 375 380
   Val Lys Gly Phe Tyr Pro Ser Asp Ile Ala Val Glu Trp Glu Ser Asn
   385 390 395 400
   Gly Gln Pro Glu Asn Asn Tyr Lys Thr Thr Pro Pro Met Leu Asp Ser
   405 410 415
   Asp Gly Ser Phe Phe Leu Tyr Ser Lys Leu Thr Val Asp Lys Ser Arg
   420 425 430
   Trp Gln Gln Gly Asn Val Phe Ser Cys Ser Val Met His Glu Ala Leu
   435 440 445
   His Asn His Tyr Thr Gln Lys Ser Leu Ser Leu Ser Pro Gly Lys
   450 455 460
<210> 64
   <211> 463
   <212> PRT
   <213> Homo sapiens
<400> 64
   Met Glu Phe Gly Leu Ser Trp Val Phe Leu Val Ala Leu Leu Arg Gly
   1 5 10 15
   Val Gln Cys Gln Val Gln Leu Val Glu Ser Gly Gly Gly Val Val Gln
   20 25 30
   Pro Gly Arg Ser Leu Arg Leu Ser Cys Val Ala Ser Gly Phe Thr Phe
   35 40 45
   Ser Ser His Gly Met His Trp Val Arg Gln Ala Pro Gly Lys Gly Leu
       50                  55                  60
   Glu Trp Val Ala Val Ile Trp Tyr Asp Gly Arg Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Phe Leu Gln Met Asn Ser Leu Arg Ala Glu Asp Thr Ala Val
               100                 105                 110
   Tyr Tyr Cys Ala Arg Gly Gly His Phe Gly Pro Phe Asp Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Cys Ser Arg Ser Thr Ser Glu Ser Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Asn Phe Gly Thr Gln Thr Tyr Thr Cys Asn Val Asp His
       210                 215                 220
   Lys Pro Ser Asn Thr Lys Val Asp Lys Thr Val Glu Arg Lys Cys Cys
   225                 230                 235                 240
   Val Glu Cys Pro Pro Cys Pro Ala Pro Pro Val Ala Gly Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Gln Phe Asn Trp Tyr Val Asp Gly Val Glu Val His Asn Ala Lys
       290                 295                 300
   Thr Lys Pro Arg Glu Glu Gln Phe Gln Ser Thr Phe Arg Val Val Ser
   305                 310                 315                 320
   Val Leu Thr Val Val His Gln Asp Trp Leu Asn Gly Lys Glu Tyr Lys
                   325                 330                 335
   Cys Lys Val Ser Asn Lys Gly Leu Pro Ala Pro Ile Glu Lys Thr Ile
               340                 345                 350
   Ser Lys Thr Lys Gly Gln Pro Arg Glu Pro Gln Val Tyr Thr Leu Pro
   355 360 365
   Pro Ser Arg Glu Glu Met Thr Lys Asn Gln Val Ser Leu Thr Cys Leu
   370 375 380
   Val Lys Gly Phe Tyr Pro Ser Asp Ile Ala Val Glu Trp Glu Ser Asn
   385 390 395 400
   Gly Gln Pro Glu Asn Asn Tyr Lys Thr Thr Pro Pro Met Leu Asp Ser
   405 410 415
   Asp Gly Ser Phe Phe Leu Tyr Ser Lys Leu Thr Val Asp Lys Ser Arg
   420 425 430
   Trp Gln Gln Gly Asn Val Phe Ser Cys Ser Val Met His Glu Ala Leu
   435 440 445
   His Asn His Tyr Thr Gln Lys Ser Leu Ser Leu Ser Pro Gly Lys
   450 455 460
<210> 65
   <211> 235
   <212> PRT
   <213> Homo sapiens
<400> 65
   Met Glu Thr Pro Ala Gln Leu Leu Phe Leu Leu Leu Leu Trp Leu Pro
   1 5 10 15
   Asp Thr Thr Gly Glu Ile Val Leu Thr Gln Ser Pro Gly Thr Leu Ser
   20 25 30
   Leu Ser Pro Gly Glu Arg Ala Thr Leu Ser Cys Arg Ala Ser Gln Ser
   35 40 45
   Ile Ser Ser Ser Phe Leu Ala Trp Tyr Gln Gln Arg Pro Gly Gln Ala
   50 55 60
   Pro Arg Leu Leu Ile Tyr Gly Ala Ser Ser Arg Ala Thr Gly Ile Pro
   65 70 75 80
   Asp Arg Phe Ser Gly Ser Gly Ser Gly Thr Asp Phe Thr Leu Thr Ile
   85 90 95
   Ser Arg Leu Glu Pro Glu Asp Phe Ala Val Tyr Tyr Cys Gln Gln Tyr
   100 105 110
   Gly Thr Ser Pro Trp Thr Phe Gly Gln Gly Thr Lys Val Glu Ile Lys
   115 120 125
   Arg Thr Val Ala Ala Pro Ser Val Phe Ile Phe Pro Pro Ser Asp Glu
   130 135 140
   Gln Leu Lys Ser Gly Thr Ala Ser Val Val Cys Leu Leu Asn Asn Phe
   145 150 155 160
   Tyr Pro Arg Glu Ala Lys Val Gln Trp Lys Val Asp Asn Ala Leu Gln
   165 170 175
   Ser Gly Asn Ser Gln Glu Ser Val Thr Glu Gln Asp Ser Lys Asp Ser
   180 185 190
   Thr Tyr Ser Leu Ser Ser Thr Leu Thr Leu Ser Lys Ala Asp Tyr Glu
   195 200 205
   Lys His Lys Val Tyr Ala Cys Glu Val Thr His Gln Gly Leu Ser Ser
   210 215 220
   Pro Val Thr Lys Ser Phe Asn Arg Gly Glu Cys
   225 230 235
<210> 66
   <211> 464
   <212> PRT
   <213> Homo sapiens
<400> 66
   Met Glu Phe Gly Leu Ser Trp Val Phe Leu Val Ala Leu Leu Arg Gly
   1 5 10 15
   Val Gln Cys Gln Val Gln Leu Val Glu Ser Gly Gly Gly Val Val Gln
   20 25 30
   Pro Gly Arg Ser Leu Arg Leu Ser Cys Thr Ala Ser Gly Phe Thr Phe
   35 40 45
   Ser Asn Tyr Gly Met His Trp Val Arg Gln Ala Pro Gly Lys Gly Leu
   50 55 60
   Glu Trp Val Ala Val Ile Trp Tyr Asp Gly Ser Asn Lys His Tyr Gly
   65 70 75 80
   Asp Ser Val Lys Gly Arg Phe Thr Ile Ser Ser Asp Asn Ser Lys Asn
   85 90 95
   Thr Leu Tyr Leu Gln Met Asn Ser Leu Arg Ala Glu Asp Thr Ala Val
   100 105 110
   Tyr Tyr Cys Ala Arg Gly Glu Arg Leu Gly Ser Tyr Phe Asp Tyr Trp
   115 120 125
   Gly Gln Gly Thr Leu Val Thr Val Ser Ser Ala Ser Thr Lys Gly Pro
   130 135 140
   Ser Val Phe Pro Leu Ala Pro Cys Ser Arg Ser Thr Ser Glu Ser Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
           195                 200                 205
   Val Pro Ser Ser Asn Phe Gly Thr Gln Thr Tyr Thr Cys Asn Val Asp
       210                 215                 220
   His Lys Pro Ser Asn Thr Lys Val Asp Lys Thr Val Glu Arg Lys Cys
   225                 230                 235                 240
   Cys Val Glu Cys Pro Pro Cys Pro Ala Pro Pro Val Ala Gly Pro Ser
                   245                 250                 255
   Val Phe Leu Phe Pro Pro Lys Pro Lys Asp Thr Leu Met Ile Ser Arg
               260                 265                 270
   Thr Pro Glu Val Thr Cys Val Val Val Asp Val Ser His Glu Asp Pro
           275                 280                 285
   Glu Val Gln Phe Asn Trp Tyr Val Asp Gly Val Glu Val His Asn Ala
       290                 295                 300
   Lys Thr Lys Pro Arg Glu Glu Gln Phe Asn Ser Thr Phe Arg Val Val
   305                 310                 315                 320
   Ser Val Leu Thr Val Val His Gln Asp Trp Leu Asn Gly Lys Glu Tyr
                   325                 330                 335
   Lys Cys Lys Val Ser Asn Lys Gly Leu Pro Ala Pro Ile Glu Lys Thr
               340                 345                 350
   Ile Ser Lys Thr Lys Gly Gln Pro Arg Glu Pro Gln Val Tyr Thr Leu
           355                 360                 365
   Pro Pro Ser Arg Glu Glu Met Thr Lys Asn Gln Val Ser Leu Thr Cys
       370                 375                 380
   Leu Val Lys Gly Phe Tyr Pro Ser Asp Ile Ala Val Glu Trp Glu Ser
   385                 390                 395                 400
   Asn Gly Gln Pro Glu Asn Asn Tyr Lys Thr Thr Pro Pro Met Leu Asp
                   405                 410                 415
   Ser Asp Gly Ser Phe Phe Leu Tyr Ser Lys Leu Thr Val Asp Lys Ser
               420                 425                 430
   Arg Trp Gln Gln Gly Asn Val Phe Ser Cys Ser Val Met His Glu Ala
           435                 440                 445
   Leu His Asn His Tyr Thr Gln Lys Ser Leu Ser Leu Ser Pro Gly Lys
       450                 455                 460
<210> 67
   <211> 233
   <212> PRT
   <213> Homo sapiens
<400> 67
   Met Glu Thr Pro Ala Gln Leu Leu Phe Leu Leu Leu Leu Trp Leu Pro
   1               5                   10                  15
   Asp Thr Thr Gly Glu Ile Val Leu Thr Gln Ser Pro Gly Thr Leu Ser
               20                  25                  30
   Leu Ser Pro Gly Glu Arg Ala Thr Leu Ser Cys Arg Thr Ser Val Ser
           35                  40                  45
   Ser Ser Tyr Leu Ala Trp Tyr Gln Gln Lys Pro Gly Gln Ala Pro Arg
       50                  55                  60
   Leu Leu Ile Tyr Gly Ala Ser Ser Arg Ala Thr Gly Ile Pro Asp Arg
   65                  70                  75                  80
   Phe Ser Gly Ser Gly Ser Gly Thr Asp Phe Thr Leu Thr Ile Ser Arg
                   85                  90                  95
   Leu Glu Pro Glu Asp Phe Ala Val Tyr Tyr Cys Gln Gln Tyr Gly Ile
               100                 105                 110
   Ser Pro Phe Thr Phe Gly Gly Gly Thr Lys Val Glu Ile Lys Arg Thr
           115                 120                 125
   Val Ala Ala Pro Ser Val Phe Ile Phe Pro Pro Ser Asp Glu Gln Leu
       130                 135                 140
   Lys Ser Gly Thr Ala Ser Val Val Cys Leu Leu Asn Asn Phe Tyr Pro
   145                 150                 155                 160
   Arg Glu Ala Lys Val Gln Trp Lys Val Asp Asn Ala Leu Gln Ser Gly
                   165                 170                 175
   Asn Ser Gln Glu Ser Val Thr Glu Gln Asp Ser Lys Asp Ser Thr Tyr
               180                 185                 190
   Ser Leu Ser Ser Thr Leu Thr Leu Ser Lys Ala Asp Tyr Glu Lys His
           195                 200                 205
   Lys Val Tyr Ala Cys Glu Val Thr His Gln Gly Leu Ser Ser Pro Val
       210                 215                 220
   Thr Lys Ser Phe Asn Arg Gly Glu Cys
   225                 230
<210> 68
   <211> 463
   <212> PRT
   <213> Homo sapiens
<400> 68
   Met Glu Phe Gly Leu Ser Trp Val Phe Leu Val Ala Leu Leu Arg Gly
   1               5                   10                  15
   Val Gln Cys Gln Val Gln Leu Val Glu Ser Gly Gly Gly Val Val Glu
               20                  25                  30
   Pro Gly Arg Ser Leu Arg Leu Ser Cys Thr Ala Ser Gly Phe Thr Phe
               35                  40                  45
   Ser Ser Tyr Gly Met His Trp Val Arg Gln Ala Pro Gly Lys Gly Leu
       50                  55                  60
   Glu Trp Val Ala Val Ile Trp Tyr Asp Gly Ser Asn Lys His Tyr Ala
   65                  70                  75                  80
   Asp Ser Ala Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Ala Gly Leu Leu Gly Tyr Phe Asp Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Cys Ser Arg Ser Thr Ser Glu Ser Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Asn Phe Gly Thr Gln Thr Tyr Thr Cys Asn Val Asp His
       210                 215                 220
   Lys Pro Ser Asn Thr Lys Val Asp Lys Thr Val Glu Arg Lys Cys Cys
   225                 230                 235                 240
   Val Glu Cys Pro Pro Cys Pro Ala Pro Pro Val Ala Gly Pro Ser Val
                   245                 250                 255
   Phe Leu Phe Pro Pro Lys Pro Lys Asp Thr Leu Met Ile Ser Arg Thr
               260                 265                 270
   Pro Glu Val Thr Cys Val Val Val Asp Val Ser His Glu Asp Pro Glu
           275                 280                 285
   Val Gln Phe Asn Trp Tyr Val Asp Gly Val Glu Val His Asn Ala Lys
       290                 295                 300
   Thr Lys Pro Arg Glu Glu Gln Phe Asn Ser Thr Phe Arg Val Val Ser
   305                 310                 315                 320
   Val Leu Thr Val Val His Gln Asp Trp Leu Asn Gly Lys Glu Tyr Lys
                   325                 330                 335
   Cys Lys Val Ser Asn Lys Gly Leu Pro Ala Pro Ile Glu Lys Thr Ile
               340                 345                 350
   Ser Lys Thr Lys Gly Gln Pro Arg Glu Pro Gln Val Tyr Thr Leu Pro
           355                 360                 365
   Pro Ser Arg Glu Glu Met Thr Lys Asn Gln Val Ser Leu Thr Cys Leu
       370                 375                 380
   Val Lys Gly Phe Tyr Pro Ser Asp Ile Ala Val Glu Trp Glu Ser Asn
   385                 390                 395                 400
   Gly Gln Pro Glu Asn Asn Tyr Lys Thr Thr Pro Pro Met Leu Asp Ser
                   405                 410                 415
   Asp Gly Ser Phe Phe Leu Tyr Ser Lys Leu Thr Val Asp Lys Ser Arg
               420                 425                 430
   Trp Gln Gln Gly Asn Val Phe Ser Cys Ser Val Met His Glu Ala Leu
           435                 440                 445
   His Asn His Tyr Thr Gln Lys Ser Leu Ser Leu Ser Pro Gly Lys
       450                 455                 460
<210> 69
   <211> 234
   <212> PRT
   <213> Homo sapiens
<400> 69
   Met Glu Thr Pro Ala Gln Leu Leu Phe Leu Leu Leu Leu Trp Leu Pro
   1               5                   10                  15
   Asp Thr Thr Gly Glu Ile Val Leu Thr Gln Ser Pro Gly Thr Leu Ser
               20                  25                  30
   Leu Ser Pro Gly Glu Arg Ala Thr Leu Ser Cys Arg Ala Ser Gln Ser
           35                  40                  45
   Val Ser Ser Tyr Leu Ala Trp Tyr Gln Gln Lys Pro Gly Gln Ala Pro
       50                  55                  60
   Arg Pro Leu Ile Tyr Gly Val Ser Ser Arg Ala Thr Gly Ile Pro Asp
   65                  70                  75                  80
   Arg Phe Ser Gly Ser Gly Ser Gly Thr Asp Phe Thr Leu Thr Ile Ser
                   85                  90                  95
   Arg Leu Glu Pro Glu Asp Phe Ala Val Tyr Tyr Cys Gln Gln Tyr Gly
               100                 105                 110
   Ile Ser Pro Phe Thr Phe Gly Pro Gly Thr Lys Val Asp Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 70
   <211> 451
   <212> PRT
   <213> Homo sapiens
<400> 70
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Pro Arg Gly Ala Thr Leu Tyr Tyr Tyr Tyr Tyr Gly Met
               100                 105                 110
   Asp Val Trp Gly Gln Gly Thr Thr Val Thr Val Ser Ser Ala Ser Thr
           115                 120                 125
   Lys Gly Pro Ser Val Phe Pro Leu Ala Pro Cys Ser Arg Ser Thr Ser
       130                 135                 140
   Glu Ser Thr Ala Ala Leu Gly Cys Leu Val Lys Asp Tyr Phe Pro Glu
   145                 150                 155                 160
   Pro Val Thr Val Ser Trp Asn Ser Gly Ala Leu Thr Ser Gly Val His
                   165                 170                 175
   Thr Phe Pro Ala Val Leu Gln Ser Ser Gly Leu Tyr Ser Leu Ser Ser
               180                 185                 190
   Val Val Thr Val Pro Ser Ser Asn Phe Gly Thr Gln Thr Tyr Thr Cys
           195                 200                 205
   Asn Val Asp His Lys Pro Ser Asn Thr Lys Val Asp Lys Thr Val Glu
       210                 215                 220
   Arg Lys Cys Cys Val Glu Cys Pro Pro Cys Pro Ala Pro Pro Val Ala
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Gln Phe Asn Trp Tyr Val Asp Gly Val Glu Val
           275                 280                 285
   His Asn Ala Lys Thr Lys Pro Arg Glu Glu Gln Phe Asn Ser Thr Phe
       290                 295                 300
   Arg Val Val Ser Val Leu Thr Val Val His Gln Asp Trp Leu Asn Gly
   305                 310                 315                 320
   Lys Glu Tyr Lys Cys Lys Val Ser Asn Lys Gly Leu Pro Ala Pro Ile
                   325                 330                 335
   Glu Lys Thr Ile Ser Lys Thr Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Met Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 71
   <211> 214
   <212> PRT
   <213> Homo sapiens
<400> 71
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Asn Ser Tyr
   20 25 30
   Leu Asp Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Tyr Ser Thr Pro Phe
   85 90 95
   Thr Phe Gly Pro Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 72
   <211> 89
   <212> PRT
   <213> Homo sapiens
<400> 72
   Gly Val Val Gln Pro Gly Arg Ser Leu Arg Leu Ser Cys Ala Ala Ser
   1 5 10 15
   Gly Phe Thr Phe Ser Ser Tyr Gly Met His Trp Val Arg Gln Ala Pro
   20 25 30
   Gly Lys Gly Leu Glu Trp Val Ala Val Ile Trp Tyr Asp Gly Ser Asn
   35 40 45
   Lys Tyr Tyr Ala Asp Ser Val Lys Gly Arg Phe Thr Ile Ser Arg Asp
   50 55 60
   Asn Ser Lys Asn Thr Leu Tyr Leu Gln Met Asn Ser Leu Arg Ala Glu
   65 70 75 80
   Asp Thr Ala Val Tyr Tyr Cys Ala Arg
   85
<210> 73
   <211> 169
   <212> PRT
   <213> Homo sapiens
<400> 73
   Gly Val Val Gln Pro Gly Arg Ser Leu Arg Leu Ser Cys Ala Ala Ser
   1 5 10 15
   Gly Phe Thr Phe Ser Ser Tyr Gly Met His Trp Val Arg Gln Ala Pro
   20 25 30
   Gly Lys Gly Leu Glu Trp Val Ala Val Ile Trp Tyr Asp Gly Ser Asn
   35 40 45
   Lys Tyr Tyr Ala Asp Ser Val Lys Gly Arg Phe Thr Ile Ser Arg Asp
   50 55 60
   Asn Ser Lys Asn Thr Leu Tyr Leu Gln Met Asn Ser Leu Arg Ala Glu
   65 70 75 80
   Asp Thr Ala Val Tyr Tyr Cys Ala Arg Gly Ala Arg Ile Ile Thr Pro
   85 90 95
   Cys Met Asp Val Trp Gly Gln Gly Thr Thr Val Thr Val Ser Ser Ala
   100 105 110
   Ser Thr Lys Gly Pro Ser Val Phe Pro Leu Ala Pro Cys Ser Arg Ser
   115 120 125
   Thr Ser Glu Ser Thr Ala Ala Leu Gly Cys Leu Val Lys Asp Tyr Phe
   130 135 140
   Pro Glu Pro Val Thr Val Ser Trp Asn Ser Gly Ala Leu Thr Ser Gly
   145 150 155 160
   Val His Thr Phe Pro Ala Val Leu Gln
   165
<210> 74
   <211> 167
   <212> PRT
   <213> Homo sapiens
<400> 74
   Gly Val Val Gln Pro Gly Arg Ser Leu Arg Leu Ser Cys Val Ala Ser
   1               5                   10                  15
   Gly Phe Thr Phe Ser Ser His Gly Met His Trp Val Arg Gln Ala Pro
               20                  25                  30
   Gly Lys Gly Leu Glu Trp Val Ala Val Ile Trp Tyr Asp Gly Arg Asn
           35                  40                  45
   Lys Tyr Tyr Ala Asp Ser Val Lys Gly Arg Phe Thr Ile Ser Arg Asp
       50                  55                  60
   Asn Ser Lys Asn Thr Leu Phe Leu Gln Met Asn Ser Leu Arg Ala Glu
   65                  70                  75                  80
   Asp Thr Ala Val Tyr Tyr Cys Ala Arg Gly Gly His Phe Gly Pro Phe
                   85                  90                  95
   Asp Tyr Trp Gly Gln Gly Thr Leu Val Thr Val Ser Ser Ala Ser Thr
               100                 105                 110
   Lys Gly Pro Ser Val Phe Pro Leu Ala Pro Cys Ser Arg Ser Thr Ser
           115                 120                 125
   Glu Ser Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln
                   165
<210> 75
   <211> 166
   <212> PRT
   <213> Homo sapiens
<400> 75
   Gly Val Val Gln Pro Gly Arg Ser Leu Arg Leu Ser Cys Thr Ala Ser
   1               5                   10                  15
   Gly Phe Thr Phe Ser Asn Tyr Gly Met His Trp Val Arg Gln Ala Pro
               20                  25                  30
   Gly Lys Gly Leu Glu Trp Val Ala Val Ile Trp Tyr Asp Gly Ser Asn
           35                  40                  45
   Lys His Tyr Gly Asp Ser Val Lys Gly Arg Phe Thr Ile Ser Ser Asp
       50                  55                  60
   Asn Ser Lys Asn Thr Leu Tyr Leu Gln Met Asn Ser Leu Arg Ala Glu
   65                  70                  75                  80
   Asp Thr Ala Val Tyr Tyr Cys Ala Arg Gly Glu Arg Leu Gly Ser Tyr
                   85                  90                  95
   Phe Asp Tyr Trp Gly Gln Gly Thr Leu Val Thr Val Ser Ser Ala Ser
                   100                 105                 110
   Thr Lys Gly Pro Ser Val Phe Pro Leu Ala Pro Cys Ser Arg Ser Thr
           115                 120                 125
   Ser Glu Ser Thr Ala Ala Leu Gly Cys Leu Val Lys Asp Tyr Phe Pro
       130                 135                 140
   Glu Pro Val Thr Val Ser Trp Asn Ser Gly Ala Leu Thr Ser Gly Val
   145                 150                 155                 160
   His Thr Phe Pro Ala Val
                   165
<210> 76
   <211> 167
   <212> PRT
   <213> Homo sapiens
<400> 76
   Gly Val Val Gln Pro Gly Arg Ser Leu Arg Leu Ser Cys Val Ala Ser
   1               5                   10                  15
   Gly Phe Ile Phe Ser Ser His Gly Ile His Trp Val Arg Gln Ala Pro
               20                  25                  30
   Gly Lys Gly Leu Glu Trp Val Ala Val Ile Trp Tyr Asp Gly Arg Asn
           35                  40                  45
   Lys Asp Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
   65                  70                  75                  80
   Asp Thr Ala Val Tyr Tyr Cys Ala Arg Val Ala Pro Leu Gly Pro Leu
                   85                  90                  95
   Asp Tyr Trp Gly Gln Gly Thr Leu Val Thr Val Ser Ser Ala Ser Thr
               100                 105                 110
   Lys Gly Pro Ser Val Phe Pro Leu Ala Pro Cys Ser Arg Ser Thr Ser
           115                 120                 125
   Glu Ser Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln
                   165
<210> 77
   <211> 153
   <212> PRT
   <213> Homo sapiens
<400> 77
   Pro Gly Arg Ser Leu Arg Leu Ser Cys Ala Ala Ser Gly Phe Thr Phe
   1               5                   10                  15
   Ser Ser His Gly Ile His Trp Val Arg Gln Ala Pro Gly Lys Gly Leu
               20                  25                  30
   Glu Trp Val Ala Val Ile Trp Tyr Asp Gly Arg Asn Lys Asp Tyr Ala
           35                  40                  45
   Asp Ser Val Lys Gly Arg Phe Thr Ile Ser Arg Asp Asn Ser Lys Asn
       50                  55                  60
   Thr Leu Tyr Leu Gln Met Asn Ser Leu Arg Ala Glu Asp Thr Ala Val
   65                  70                  75                  80
   Tyr Tyr Cys Ala Arg Val Ala Pro Leu Gly Pro Leu Asp Tyr Trp Gly
                   85                  90                  95
   Gln Gly Thr Leu Val Thr Val Ser Ser Ala Ser Thr Lys Gly Pro Ser
               100                 105                 110
   Val Phe Pro Leu Ala Pro Cys Ser Arg Ser Thr Ser Glu Ser Thr Ala
           115                 120                 125
   Ala Leu Gly Cys Leu Val Lys Asp Tyr Phe Pro Glu Pro Val Thr Val
       130                 135                 140
   Ser Trp Asn Ser Gly Ala Leu Thr Ser
   145                 150
<210> 78
   <211> 163
   <212> PRT
   <213> Homo sapiens
<400> 78
   Pro Gly Arg Ser Leu Arg Leu Ser Cys Ala Ala Ser Gly Phe Thr Phe
   1               5                   10                  15
   Ser Ser His Gly Ile His Trp Val Arg Gln Ala Pro Gly Lys Gly Leu
               20                  25                  30
   Glu Trp Val Ala Val Ile Trp Tyr Asp Gly Arg Asn Lys Asp Tyr Ala
           35                  40                  45
   Asp Ser Val Lys Gly Arg Phe Thr Ile Ser Arg Asp Asn Ser Lys Lys
       50                  55                  60
   Thr Leu Tyr Leu Gln Met Asn Ser Leu Arg Ala Glu Asp Thr Ala Val
   65                  70                  75                  80
   Tyr Tyr Cys Ala Arg Val Ala Pro Leu Gly Pro Leu Asp Tyr Trp Gly
                   85                  90                  95
   Gln Gly Thr Leu Val Thr Val Ser Ser Ala Ser Thr Lys Gly Pro Ser
   100 105 110
   Val Phe Pro Leu Ala Pro Cys Ser Arg Ser Thr Ser Glu Ser Thr Ala
   115 120 125
   Ala Leu Gly Cys Leu Val Lys Asp Tyr Phe Pro Glu Pro Val Thr Val
   130 135 140
   Ser Trp Asn Ser Gly Ala Leu Thr Ser Gly Val His Thr Phe Pro Ala
   145 150 155 160
   Val Leu Gln
<210> 79
   <211> 138
   <212> PRT
   <213> Homo sapiens
<400> 79
   Gly Gly Val Val Glu Pro Gly Arg Ser Leu Arg Leu Ser Cys Thr Ala
   1 5 10 15
   Ser Gly Phe Thr Phe Ser Ser Tyr Gly Met His Trp Val Arg Gln Ala
   20 25 30
   Pro Gly Lys Gly Leu Glu Trp Val Ala Val Ile Trp Tyr Asp Gly Ser
   35 40 45
   Asn Lys His Tyr Ala Asp Ser Ala Lys Gly Arg Phe Thr Ile Ser Arg
   50 55 60
   Asp Asn Ser Lys Asn Thr Leu Tyr Leu Gln Met Asn Ser Leu Arg Ala
   65 70 75 80
   Glu Asp Thr Ala Val Tyr Tyr Cys Ala Arg Ala Gly Leu Leu Gly Tyr
   85 90 95
   Phe Asp Tyr Trp Gly Gln Gly Thr Leu Val Thr Val Ser Ser Ala Ser
   100 105 110
   Thr Lys Gly Pro Ser Val Phe Pro Leu Ala Pro Cys Ser Arg Ser Thr
   115 120 125
   Ser Glu Ser Thr Ala Ala Leu Gly Cys Leu
   130 135
<210> 80
   <211> 167
   <212> PRT
   <213> Homo sapiens
<400> 80
   Gly Val Val Gln Pro Gly Arg Ser Leu Arg Leu Ser Cys Ala Ala Ser
   1 5 10 15
   Gly Phe Thr Phe Ser Ser Tyr Gly Met His Trp Val Arg Gln Ala Pro
               20                  25                  30
   Gly Lys Gly Leu Glu Trp Val Ala Val Ile Trp Tyr Asp Gly Ser Asn
           35                  40                  45
   Lys Tyr Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
   65                  70                  75                  80
   Asp Thr Ala Val Tyr Tyr Cys Ala Arg Asp Pro Arg Gly Ala Thr Leu
                   85                  90                  95
   Tyr Tyr Tyr Tyr Tyr Gly Met Asp Val Trp Gly Gln Gly Thr Thr Val
               100                 105                 110
   Thr Val Ser Ser Ala Ser Thr Lys Gly Pro Ser Val Phe Pro Leu Ala
           115                 120                 125
   Pro Cys Ser Arg Ser Thr Ser Glu Ser Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His
                   165
<210> 81
   <211> 150
   <212> PRT
   <213> Homo sapiens
<400> 81
   Gly Val Val Gln Pro Gly Arg Ser Leu Arg Leu Ser Cys Ala Ala Ser
    1              5                   10                  15
   Gly Phe Thr Phe Ser Ser Tyr Gly Met His Trp Val Arg Gln Ala Pro
               20                  25                  30
   Gly Lys Gly Leu Glu Trp Val Ala Val Ile Trp Tyr Asp Gly Ser His
           35                  40                  45
   Lys Tyr Tyr Ala Asp Ser Val Lys Gly Arg Phe Thr Ile Ser Arg Asp
       50                  55                  60
   Asn Ser Lys Asn Thr Leu Tyr Leu Gln Met Asn Ser Leu Arg Ala Glu
   65                  70                  75                  80
   Asp Thr Ala Val Tyr Tyr Cys Ala Arg Gly Ala Val Val Val Pro Ala
                   85                  90                  95
   Ala Met Asp Val Trp Gly Gln Gly Thr Thr Val Thr Val Ser Ser Ala
               100                 105                 110
   Ser Thr Lys Gly Pro Ser Val Phe Pro Leu Ala Pro Cys Ser Arg Ser
           115                 120                 125
   Thr Ser Glu Ser Thr Ala Ala Leu Gly Cys Leu Val Lys Asp Tyr Phe
       130                 135                 140
   Pro Glu Pro Val Thr Val
   145                 150
<210> 82
   <211> 146
   <212> PRT
   <213> Homo sapiens
<400> 82
   Val Val Gln Pro Gly Arg Ser Leu Arg Leu Ser Cys Ala Ala Ser Gly
   1               5                   10                  15
   Phe Thr Phe Ser Ser Cys Gly Met His Trp Val Arg Gln Ala Pro Gly
               20                  25                  30
   Lys Gly Leu Glu Trp Val Ala Val Ile Trp Ser Asp Gly Ser His Lys
           35                  40                  45
   Tyr Tyr Ala Asp Ser Val Lys Gly Arg Phe Thr Ile Ser Arg Asp Asn
       50                  55                  60
   Ser Lys Asn Thr Leu Tyr Leu Gln Met Asn Ser Leu Arg Ala Glu Asp
   65                  70                  75                  80
   Thr Ala Val Tyr Tyr Cys Ala Arg Gly Thr Met Ile Val Val Gly Thr
                   85                  90                  95
   Leu Asp Tyr Trp Gly Gln Gly Thr Leu Val Thr Val Ser Ser Ala Ser
               100                 105                 110
   Thr Lys Gly Pro Ser Val Phe Pro Leu Ala Pro Cys Ser Arg Ser Thr
           115                 120                 125
   Ser Glu Ser Thr Ala Ala Leu Gly Cys Leu Val Lys Asp Tyr Phe Pro
       130                 135                 140
   Glu Pro
   145
<210> 83
   <211> 171
   <212> PRT
   <213> Homo sapiens
<400> 83
   Gly Val Val Gln Pro Gly Arg Ser Leu Arg Leu Ser Cys Ala Ala Ser
   1               5                   10                  15
   Gly Phe Thr Phe Ser Ser Tyr Gly Val His Trp Val Arg Gln Ala Pro
               20                  25                  30
   Gly Lys Gly Leu Glu Trp Val Ala Val Ile Trp Tyr Asp Gly Ser Asn
           35                  40                  45
   Lys Tyr Tyr Ala Asp Ser Val Lys Gly Arg Phe Thr Ile Ser Arg Asp
       50                  55                  60
   Asn Ser Lys Ser Thr Leu Tyr Leu Gln Met Asn Ser Leu Arg Ala Glu
   65                  70                  75                  80
   Asp Thr Ala Val Tyr Tyr Cys Ala Arg Asp Ser Tyr Tyr Asp Phe Trp
                   85                  90                  95
   Ser Gly Arg Gly Gly Met Asp Val Trp Gly Gln Gly Thr Thr Val Thr
               100                 105                 110
   Val Ser Ser Ala Ser Thr Lys Gly Pro Ser Val Phe Pro Leu Ala Pro
           115                 120                 125
   Cys Ser Arg Ser Thr Ser Glu Ser Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val
                   165                 170
<210> 84
   <211> 163
   <212> PRT
   <213> Homo sapiens
<400> 84
   Val Gln Pro Gly Arg Ser Leu Arg Leu Ser Cys Ala Ala Ser Gly Phe
   1               5                   10                  15
   Thr Phe Ser Asn Tyr Ala Met His Trp Val Arg Gln Ala Pro Gly Lys
               20                  25                  30
   Gly Leu Glu Trp Val Val Val Ile Trp His Asp Gly Asn Asn Lys Tyr
           35                  40                  45
   Tyr Ala Glu Ser Val Lys Gly Arg Phe Thr Ile Ser Arg Asp Asn Ser
       50                  55                  60
   Lys Asn Thr Leu Tyr Leu Gln Met Asn Ser Leu Arg Ala Glu Asp Thr
   65                  70                  75                  80
   Ala Val Tyr Tyr Cys Ala Arg Asp Gln Gly Thr Gly Trp Tyr Gly Gly
                   85                  90                  95
   Phe Asp Phe Trp Gly Gln Gly Thr Leu Val Thr Val Ser Ser Ala Ser
               100                 105                 110
   Thr Lys Gly Pro Ser Val Phe Pro Leu Ala Pro Cys Ser Arg Ser Thr
           115                 120                 125
   Ser Glu Ser Thr Ala Ala Leu Gly Cys Leu Val Lys Asp Tyr Phe Pro
   130 135 140
   Glu Pro Val Thr Val Ser Trp Asn Ser Gly Ala Leu Thr Ser Gly Val
   145 150 155 160
   His Thr Phe
<210> 85
   <211> 76
   <212> PRT
   <213> Homo sapiens
<400> 85
   Val Ser Gly Gly Ser Ile Ser Ser Gly Gly Tyr Tyr Trp Ser Trp Ile
   1 5 10 15
   Arg Gln His Pro Gly Lys Gly Leu Glu Trp Ile Gly Tyr Ile Tyr Tyr
   20 25 30
   Ser Gly Ser Thr Tyr Tyr Asn Pro Ser Leu Lys Ser Arg Val Thr Ile
   35 40 45
   Ser Val Asp Thr Ser Lys Asn Gln Phe Ser Leu Lys Leu Ser Ser Val
   50 55 60
   Thr Ala Ala Asp Thr Ala Val Tyr Tyr Cys Ala Arg
   65 70 75
<210> 86
   <211> 172
   <212> PRT
   <213> Homo sapiens
<400> 86
   Ser Gly Pro Gly Leu Val Lys Pro Ser Gln Ile Leu Ser Leu Thr Cys
   1 5 10 15
   Thr Val Ser Gly Gly Ser Ile Ser Ser Gly Gly His Tyr Trp Ser Trp
   20 25 30
   Ile Arg Gln His Pro Gly Lys Gly Leu Glu Trp Ile Gly Tyr Ile Tyr
   35 40 45
   Tyr Ile Gly Asn Thr Tyr Tyr Asn Pro Ser Leu Lys Ser Arg Val Thr
   50 55 60
   Ile Ser Val Asp Thr Ser Lys Asn Gln Phe Ser Leu Lys Leu Ser Ser
   65 70 75 80
   Val Thr Ala Ala Asp Thr Ala Val Tyr Tyr Cys Ala Arg Asp Ser Gly
   85 90 95
   Asp Tyr Tyr Gly Ile Asp Val Trp Gly Gln Gly Thr Thr Val Thr Val
   100 105 110
   Ser Ser Ala Ser Thr Lys Gly Pro Ser Val Phe Pro Leu Ala Pro Cys
           115                 120                 125
   Ser Arg Ser Thr Ser Glu Ser Thr Ala Ala Leu Gly Cys Leu Val Lys
       130                 135                 140
   Asp Tyr Phe Pro Glu Pro Val Thr Val Ser Trp Asn Ser Gly Ala Leu
   145                 150                 155                 160
   Thr Ser Gly Val His Thr Phe Pro Ala Val Leu Gln
                   165                 170
<210> 87
   <211> 96
   <212> PRT
   <213> Homo sapiens
<400> 87
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
<210> 88
   <211> 141
   <212> PRT
   <213> Homo sapiens
<400> 88
   Gln Ser Pro Gly Thr Leu Ser Leu Ser Pro Gly Glu Arg Ala Thr Leu
   1               5                   10                  15
   Ser Cys Arg Ala Ser Gln Ser Ile Ser Ser Ser Phe Leu Ala Trp Tyr
               20                  25                  30
   Gln Gln Arg Pro Gly Gln Ala Pro Arg Leu Leu Ile Tyr Gly Ala Ser
           35                  40                  45
   Ser Arg Ala Thr Gly Ile Pro Asp Arg Phe Ser Gly Ser Gly Ser Gly
       50                  55                  60
   Thr Asp Phe Thr Leu Thr Ile Ser Arg Leu Glu Pro Glu Asp Phe Ala
   65 70 75 80
   Val Tyr Tyr Cys Gln Gln Tyr Gly Thr Ser Pro Trp Thr Phe Gly Gln
   85 90 95
   Gly Thr Lys Val Glu Ile Lys Arg Thr Val Ala Ala Pro Ser Val Phe
   100 105 110
   Ile Phe Pro Pro Ser Asp Glu Gln Leu Lys Ser Gly Thr Ala Ser Val
   115 120 125
   Val Cys Leu Leu Asn Asn Phe Tyr Pro Arg Glu Ala Lys
   130 135 140
<210> 89
   <211> 141
   <212> PRT
   <213> Homo sapiens
<400> 89
   Gln Ser Pro Gly Thr Leu Ser Leu Ser Pro Gly Glu Arg Ala Thr Leu
   1 5 10 15
   Ser Cys Arg Thr Ser Val Ser Ser Ser Tyr Leu Ala Trp Tyr Gln Gln
   20 25 30
   Lys Pro Gly Gln Ala Pro Arg Leu Leu Ile Tyr Gly Ala Ser Ser Arg
   35 40 45
   Ala Thr Gly Ile Pro Asp Arg Phe Ser Gly Ser Gly Ser Gly Thr Asp
   50 55 60
   Phe Thr Leu Thr Ile Ser Arg Leu Glu Pro Glu Asp Phe Ala Val Tyr
   65 70 75 80
   Tyr Cys Gln Gln Tyr Gly Ile Ser Pro Phe Thr Phe Gly Gly Gly Thr
   85 90 95
   Lys Val Glu Ile Lys Arg Thr Val Ala Ala Pro Ser Val Phe Ile Phe
   100 105 110
   Pro Pro Ser Asp Glu Gln Leu Lys Ser Gly Thr Ala Ser Val Val Cys
   115 120 125
   Leu Leu Asn Asn Phe Tyr Pro Arg Glu Ala Lys Val Gln
   130 135 140
<210> 90
   <211> 139
   <212> PRT
   <213> Homo sapiens
<400> 90
   Gly Thr Leu Ser Leu Ser Pro Gly Glu Arg Ala Thr Leu Ser Cys Arg
   1 5 10 15
   Ala Ser Gln Ser Val Ser Ser Tyr Leu Ala Trp Tyr Gln Gln Lys Pro
               20                  25                  30
   Gly Gln Ala Pro Arg Leu Leu Ile Tyr Gly Ala Ser Ser Arg Ala Thr
           35                  40                  45
   Gly Ile Pro Asp Arg Phe Ser Gly Ser Gly Ser Gly Thr Asp Phe Thr
       50                  55                  60
   Leu Thr Ile Ser Arg Leu Glu Pro Glu Asp Phe Ala Val Tyr Tyr Cys
   65                  70                  75                  80
   Gln Gln Tyr Gly Arg Ser Pro Phe Thr Phe Gly Pro Gly Thr Lys Val
                   85                  90                  95
   Asp Ile Lys Arg Thr Val Ala Ala Pro Ser Val Phe Ile Phe Pro Pro
               100                 105                 110
   Ser Asp Glu Gln Leu Lys Ser Gly Thr Ala Ser Val Val Cys Leu Leu
           115                 120                 125
   Asn Asn Phe Tyr Pro Arg Glu Ala Lys Val Gln
       130                 135
<210> 91
   <211> 142
   <212> PRT
   <213> Homo sapiens
<400> 91
   Gln Ser Pro Gly Thr Leu Ser Leu Ser Pro Gly Glu Arg Ala Thr Leu
   1               5                   10                  15
   Ser Cys Arg Ala Ser Gln Ser Val Ser Ser Tyr Leu Ala Trp Tyr Gln
               20                  25                  30
   Gln Lys Pro Gly Gln Ala Pro Arg Pro Leu Ile Tyr Gly Val Ser Ser
           35                  40                  45
   Arg Ala Thr Gly Ile Pro Asp Arg Phe Ser Gly Ser Gly Ser Gly Thr
       50                  55                  60
   Asp Phe Thr Leu Thr Ile Ser Arg Leu Glu Pro Glu Asp Phe Ala Val
   65                  70                  75                  80
   Tyr Tyr Cys Gln Gln Tyr Gly Ile Ser Pro Phe Thr Phe Gly Pro Gly
                   85                  90                  95
   Thr Lys Val Asp Ile Lys Arg Thr Val Ala Ala Pro Ser Val Phe Ile
               100                 105                 110
   Phe Pro Pro Ser Asp Glu Gln Leu Lys Ser Gly Thr Ala Ser Val Val
           115                 120                 125
   Cys Leu Leu Asn Asn Phe Tyr Pro Arg Glu Ala Lys Val Gln
       130                 135                 140
<210> 92
   <211> 142
   <212> PRT
   <213> Homo sapiens
<400> 92
   Ser Pro Gly Thr Leu Ser Leu Ser Pro Gly Glu Arg Ala Thr Leu Ser
    1               5                  10                  15
   Cys Arg Ala Ser Gln Ser Ile Ser Ser Asn Phe Leu Ala Trp Tyr Gln
               20                  25                  30
   Gln Lys Pro Gly Gln Ala Pro Arg Leu Leu Ile Tyr Arg Pro Ser Ser
           35                  40                  45
   Arg Ala Thr Gly Ile Pro Asp Ser Phe Ser Gly Ser Gly Ser Gly Thr
       50                  55                  60
   Asp Phe Thr Leu Thr Ile Ser Arg Leu Glu Pro Glu Asp Phe Ala Leu
   65                  70                  75                  80
   Tyr Tyr Cys Gln Gln Tyr Gly Thr Ser Pro Phe Thr Phe Gly Pro Gly
                   85                  90                  95
   Thr Lys Val Asp Ile Lys Arg Thr Val Ala Ala Pro Ser Val Phe Ile
               100                 105                 110
   Phe Pro Pro Ser Asp Glu Gln Leu Lys Ser Gly Thr Ala Ser Val Val
           115                 120                 125
   Cys Leu Leu Asn Asn Phe Tyr Pro Arg Glu Ala Lys Val Gln
       130                 135                 140
<210> 93
   <211> 146
   <212> PRT
   <213> Homo sapiens
<400> 93
   Gln Ser Pro Gly Thr Leu Ser Leu Ser Pro Gly Glu Arg Ala Thr Leu
    1               5                  10                  15
   Ser Cys Arg Ala Ser Gln Ser Val Ser Ser Tyr Leu Ala Trp Tyr Gln
               20                  25                  30
   Gln Lys Pro Gly Gln Ala Pro Arg Leu Leu Ile Tyr Gly Ala Ser Ser
           35                  40                  45
   Arg Ala Thr Gly Ile Pro Asp Arg Phe Ser Gly Ser Gly Ser Gly Thr
       50                  55                  60
   Asp Phe Thr Leu Thr Ile Ser Arg Leu Glu Pro Glu Asp Phe Ala Val
   65                  70                  75                  80
   Tyr Tyr Cys Gln Gln Tyr Gly Arg Ser Pro Phe Thr Phe Gly Pro Gly
                   85                  90                  95
   Thr Lys Val Asp Ile Lys Arg Thr Val Ala Ala Pro Ser Val Phe Ile
   100 105 110
   Phe Pro Pro Ser Asp Glu Gln Leu Lys Ser Gly Thr Ala Ser Val Val
   115 120 125
   Cys Leu Leu Asn Asn Phe Tyr Pro Arg Glu Ala Lys Val Gln Trp Lys
   130 135 140
   Gly Gly
   145
<210> 94
   <211> 95
   <212> PRT
   <213> Homo sapiens
<400> 94
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Tyr Ser Thr Pro
   85 90 95
<210> 95
   <211> 152
   <212> PRT
   <213> Homo sapiens
<400> 95
   Gln Ser Pro Ser Ser Leu Ser Ala Ser Val Gly Asp Arg Val Thr Ile
   1 5 10 15
   Thr Cys Arg Ala Ser Gln Ser Ile Asn Thr Tyr Leu Ile Trp Tyr Gln
   20 25 30
   Gln Lys Pro Gly Lys Ala Pro Asn Phe Leu Ile Ser Ala Thr Ser Ile
   35 40 45
   Leu Gln Ser Gly Val Pro Ser Arg Phe Arg Gly Ser Gly Ser Gly Thr
   50 55 60
   Asn Phe Thr Leu Thr Ile Asn Ser Leu His Pro Glu Asp Phe Ala Thr
   65 70 75 80
   Tyr Tyr Cys Gln Gln Ser Tyr Ser Thr Pro Phe Thr Phe Gly Pro Gly
   85 90 95
   Thr Lys Val Asp Ile Lys Arg Thr Val Ala Ala Pro Ser Val Phe Ile
   100 105 110
   Phe Pro Pro Ser Asp Glu Gln Leu Lys Ser Gly Thr Ala Ser Val Val
   115 120 125
   Cys Leu Leu Asn Asn Phe Tyr Pro Arg Glu Ala Lys Val Gln Trp Lys
   130 135 140
   Val Asp Asn Ala Leu Gln Ser Gly
   145 150
<210> 96
   <211> 139
   <212> PRT
   <213> Homo sapiens
<400> 96
   Pro Ser Ser Leu Ser Ala Ser Val Gly Asp Arg Val Thr Ile Thr Cys
   1 5 10 15
   Arg Ala Ser Gln Ser Ile Asn Ser Tyr Leu Asp Trp Tyr Gln Gln Lys
   20 25 30
   Pro Gly Lys Ala Pro Lys Leu Leu Ile Tyr Ala Ala Ser Ser Leu Gln
   35 40 45
   Ser Gly Val Pro Ser Arg Phe Ser Gly Ser Gly Ser Gly Thr Asp Phe
   50 55 60
   Thr Leu Thr Ile Ser Ser Leu Gln Pro Glu Asp Phe Ala Thr Tyr Tyr
   65 70 75 80
   Cys Gln Gln Tyr Tyr Ser Thr Pro Phe Thr Phe Gly Pro Gly Thr Lys
   85 90 95
   Val Glu Ile Lys Arg Thr Val Ala Ala Pro Ser Val Phe Ile Phe Pro
   100 105 110
   Pro Ser Asp Glu Gln Leu Lys Ser Gly Thr Ala Ser Val Val Cys Leu
   115 120 125
   Leu Asn Asn Phe Tyr Pro Arg Glu Ala Lys Val
   130 135
<210> 97
   <211> 134
   <212> PRT
   <213> Homo sapiens
<400> 97
   Thr Gln Ser Pro Ser Ser Leu Ser Ala Ser Val Gly Asp Arg Val Thr
    1               5                  10                  15
   Ile Thr Cys Arg Ala Ser Gln Asn Ile Ser Arg Tyr Leu Asn Trp Tyr
               20                  25                  30
   Gln Gln Lys Pro Gly Lys Ala Pro Lys Phe Leu Ile Tyr Val Ala Ser
           35                  40                  45
   Ile Leu Gln Ser Gly Val Pro Ser Gly Phe Ser Ala Ser Gly Ser Gly
       50                  55                  60
   Pro Asp Phe Thr Leu Thr Ile Ser Ser Leu Gln Pro Glu Asp Phe Ala
   65                  70                  75                  80
   Thr Tyr Tyr Cys Gln Gln Ser Tyr Ser Thr Pro Phe Thr Phe Gly Pro
                   85                  90                  95
   Gly Thr Lys Val Asp Ile Lys Arg Thr Val Ala Ala Pro Ser Val Phe
               100                 105                 110
   Ile Phe Pro Pro Ser Asp Glu Gln Leu Lys Ser Gly Thr Ala Ser Val
           115                 120                 125
   Val Cys Leu Leu Asn Asn
       130
<210> 98
   <211> 150
   <212> PRT
   <213> Homo sapiens
<400> 98
   Thr Gln Ser Pro Ser Ser Leu Ser Ala Ser Val Gly Asp Arg Val Thr
    1               5                  10                  15
   Ile Thr Cys Arg Ala Ser Gln Ser Ile Cys Asn Tyr Leu Asn Trp Tyr
               20                  25                  30
   Gln Gln Lys Pro Gly Lys Ala Pro Arg Val Leu Ile Tyr Ala Ala Ser
           35                  40                  45
   Ser Leu Gln Gly Gly Val Pro Ser Arg Phe Ser Gly Ser Gly Ser Gly
       50                  55                  60
   Ile Asp Cys Thr Leu Thr Ile Ser Ser Leu Gln Pro Glu Asp Phe Ala
   65                  70                  75                  80
   Thr Tyr Tyr Cys Gln Gln Ser Tyr Ile Thr Pro Phe Thr Phe Gly Pro
                   85                  90                  95
   Gly Thr Arg Val Asp Ile Glu Arg Thr Val Ala Ala Pro Ser Val Phe
               100                 105                 110
   Ile Phe Pro Pro Ser Asp Glu Gln Leu Lys Ser Gly Thr Ala Ser Val
           115                 120                 125
   Val Cys Leu Leu Asn Asn Phe Tyr Pro Arg Glu Ala Lys Val Gln Trp
       130                 135                 140
   Lys Val Asp Asn Ala Tyr
   145                 150
<210> 99
   <211> 96
   <212> PRT
   <213> Homo sapiens
<400> 99
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Gln Lys Pro Asp Gln Ser Pro Lys Leu Leu Ile
           35                  40                  45
   Lys Tyr Ala Ser Gln Ser Phe Ser Gly Val Pro Ser Arg Phe Ser Gly
       50                  55                  60
   Ser Gly Ser Gly Thr Asp Phe Thr Leu Thr Ile Asn Ser Leu Glu Ala
   65                  70                  75                  80
   Glu Asp Ala Ala Thr Tyr Tyr Cys His Gln Ser Ser Ser Leu Pro Gln
                   85                  90                  95
<210> 100
   <211> 364
   <212> PRT
   <213> Homo sapiens
<400> 100
   Met Gly Val Leu Leu Thr Gln Arg Thr Leu Leu Ser Leu Val Leu Ala
   1               5                   10                  15
   Leu Leu Phe Pro Ser Met Ala Ser Met Ala Met His Val Ala Gln Pro
               20                  25                  30
   Ala Val Val Leu Ala Ser Ser Arg Gly Ile Ala Ser Phe Val Cys Glu
           35                  40                  45
   Tyr Ala Ser Pro Gly Lys Ala Thr Glu Val Arg Val Thr Val Leu Arg
       50                  55                  60
   Gln Ala Asp Ser Gln Val Thr Glu Val Cys Ala Ala Thr Tyr Met Met
   65                  70                  75                  80
   Gly Asn Glu Leu Thr Phe Leu Asp Asp Ser Ile Cys Thr Gly Thr Ser
                   85                  90                  95
   Ser Gly Asn Gln Val Asn Leu Thr Ile Gln Gly Leu Arg Ala Met Asp
               100                 105                 110
   Thr Gly Leu Tyr Ile Cys Lys Val Glu Leu Met Tyr Pro Pro Pro Tyr
   115 120 125
   Tyr Leu Gly Ile Gly Asn Gly Thr Gln Ile Tyr Val Ile Asp Pro Glu
   130 135 140
   Pro Cys Pro Asp Ser Asp Leu Glu Gly Ala Pro Ser Val Phe Leu Phe
   145 150 155 160
   Pro Pro Lys Pro Lys Asp Thr Leu Met Ile Ser Arg Thr Pro Glu Val
   165 170 175
   Thr Cys Val Val Val Asp Val Ser His Glu Asp Pro Glu Val Lys Phe
   180 185 190
   Asn Trp Tyr Val Asp Gly Val Glu Val His Asn Ala Lys Thr Lys Pro
   195 200 205
   Arg Glu Glu Gln Tyr Asn Ser Thr Tyr Arg Val Val Ser Val Leu Thr
   210 215 220
   Val Leu His Gln Asp Trp Leu Asn Gly Lys Glu Tyr Lys Cys Lys Val
   225 230 235 240
   Ser Asn Lys Ala Leu Pro Thr Pro Ile Glu Lys Thr Ile Ser Lys Ala
   245 250 255
   Lys Gly Gln Pro Arg Glu Pro Gln Val Tyr Thr Leu Pro Pro Ser Arg
   260 265 270
   Asp Glu Leu Thr Lys Asn Gln Val Ser Leu Thr Cys Leu Val Lys Gly
   275 280 285
   Phe Tyr Pro Ser Asp Ile Ala Val Glu Trp Glu Ser Asn Gly Gln Pro
   290 295 300
   Glu Asn Asn Tyr Lys Thr Thr Pro Pro Val Leu Asp Ser Asp Gly Ser
   305 310 315 320
   Phe Phe Leu Tyr Ser Lys Leu Thr Val Asp Lys Ser Arg Trp Gln Gln
   325 330 335
   Gly Asn Val Phe Ser Cys Ser Val Met His Glu Ala Leu His Asn His
   340 345 350
   Tyr Thr Gln Lys Ser Leu Ser Leu Ser Pro Gly Lys
   355 360
<210> 101
   <211> 12
   <212> PRT
   <213> Homo sapiens
<400> 101
   Met His Val Ala Gln Pro Ala Val Val Leu Ala Ser
   1 5 10
<210> 102
   <211> 120
   <212> PRT
   <213> Homo sapiens
<400> 102
   Met His Val Ala Gln Pro Ala Val Val Leu Ala Ser Ser Arg Gly Ile
    1               5                  10                  15
   Ala Ser Phe Val Cys Glu Tyr Ala Ser Pro Gly Lys Ala Thr Glu Val
               20                  25                  30
   Arg Val Thr Val Leu Arg Gln Ala Asp Ser Gln Val Thr Glu Val Cys
           35                  40                  45
   Ala Ala Thr Tyr Met Met Gly Asn Glu Leu Thr Phe Leu Asp Asp Ser
       50                  55                  60
   Ile Cys Thr Gly Thr Ser Ser Gly Asn Gln Val Asn Leu Thr Ile Gln
   65                  70                  75                  80
   Gly Leu Arg Ala Met Asp Thr Gly Leu Tyr Ile Cys Lys Val Glu Leu
                   85                  90                  95
   Met Tyr Pro Pro Pro Tyr Tyr Leu Gly Ile Gly Asn Gly Thr Gln Ile
               100                 105                 110
   Tyr Val Ile Asp Pro Glu Pro Cys
           115                 120
<210> 103
   <211> 11
   <212> PRT
   <213> Homo sapiens
<400> 103
   Met His Val Ala Gln Pro Ala Val Val Leu Ala
    1               5                  10
<210> 104
   <211> 23
   <212> DNA
   <213> Artificial Sequence
<220>
   <223> Description of Artificial Sequence: Primer
<220>
   <221> modified_base
   <222> (21)
   <223> i
<400> 104
   caggtgcagc tggagcagtc ngg 23
<210> 105
   <211> 24
   <212> DNA
   <213> Artificial Sequence
<220>
   <223> Description of Artificial Sequence: Primer
<400> 105
   gctgagggag tagagtcctg agga 24
<210> 106
   <211> 49
   <212> DNA
   <213> Artificial Sequence
<220>
   <223> Description of Artificial Sequence: Primer
<400> 106
   tatctaagct tctagactcg accgccacca tggagtttgg gctgagctg 49
<210> 107
   <211> 46
   <212> DNA
   <213> Artificial Sequence
<220>
   <223> Description of Artificial Sequence: Primer
<400> 107
   ttctctgatc agaattccta tcatttaccc ggagacaggg agagct 46
<210> 108
   <211> 9
   <212> DNA
   <213> Artificial Sequence
<220>
   <223> Description of Artificial Sequence: Optimal Kozak sequence
<400> 108
   accgccacc 9
<210> 109
   <211> 45
   <212> DNA
   <213> Homo sapiens
<400> 109
   tcttcaagct tgcccgggcc cgccaccatg gaaaccccag cgcag 45
<210> 110
   <211> 43
   <212> DNA
   <213> Artificial Sequence
<220>
   <223> Description of Artificial Sequence: Primer
<400> 110
   ttctttgatc agaattctca ctaacactct cccctgttga agc 43
<210> 111
   <211> 48
   <212> DNA
   <213> Artificial Sequence
<220>
   <223> Description of Artificial Sequence: Primer
<400> 111
   tcttcaagct tgcccgggcc cgccaccatg gacatgaggg tccccgct 48
<210> 112
   <211> 155
   <212> PRT
   <213> Homo sapiens
<400> 112
   Ser Pro Asp Phe Gln Ser Val Thr Pro Lys Glu Lys Val Thr Ile Thr
   1               5                   10                  15
   Cys Arg Ala Ser Gln Ser Ile Gly Ser Ser Leu His Trp Tyr Gln Gln
               20                  25                  30
   Lys Pro Asp Gln Ser Pro Lys Leu Leu Ile Lys Tyr Ala Ser Gln Ser
           35                  40                  45
   Phe Ser Gly Val Pro Ser Arg Phe Ser Gly Ser Gly Ser Gly Thr Asp
       50                  55                  60
   Phe Thr Leu Thr Ile Asn Ser Leu Glu Ala Glu Asp Ala Ala Thr Tyr
   65                  70                  75                  80
   Tyr Cys His Gln Ser Ser Ser Leu Pro Leu Thr Phe Gly Gly Gly Thr
                   85                  90                  95
   Lys Val Glu Ile Lys Arg Thr Val Ala Ala Pro Ser Val Phe Ile Phe
               100                 105                 110
   Pro Pro Ser Asp Glu Gln Leu Lys Ser Gly Thr Ala Ser Val Val Cys
           115                 120                 125
   Leu Leu Asn Asn Phe Tyr Pro Arg Glu Ala Lys Val Gln Trp Lys Val
       130                 135                 140
   Asp Asn Ala Leu Gln Ser Gly Asn Ser Gln Glu
   145                 150                 155
<210> 113
   <211> 100
   <212> PRT
   <213> Homo sapiens
<400> 113
   Asp Val Val Met Thr Gln Ser Pro Leu Ser Leu Pro Val Thr Leu Gly
   1 5 10 15
   Gln Pro Ala Ser Ile Ser Cys Arg Ser Ser Gln Ser Leu Val Tyr Ser
   20 25 30
   Asp Gly Asn Thr Tyr Leu Asn Trp Phe Gln Gln Arg Pro Gly Gln Ser
   35 40 45
   Pro Arg Arg Leu Ile Tyr Lys Val Ser Asn Arg Asp Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Gly
   85 90 95
   Thr His Trp Pro
   100
<210> 114
   <211> 139
   <212> PRT
   <213> Homo sapiens
<400> 114
   Pro Leu Ser Leu Pro Val Thr Leu Gly Gln Pro Ala Ser Ile Ser Cys
   1 5 10 15
   Arg Ser Ser Gln Ser Leu Val Tyr Ser Asp Gly Asn Thr Tyr Leu Asn
   20 25 30
   Trp Phe Gln Gln Arg Pro Gly Gln Ser Pro Arg Arg Leu Ile Tyr Lys
   35 40 45
   Val Ser Asn Trp Asp Ser Gly Val Pro Asp Arg Phe Ser Gly Ser Gly
   50 55 60
   Ser Gly Thr Asp Phe Thr Leu Lys Ile Ser Arg Val Glu Ala Glu Asp
   65 70 75 80
   Val Gly Val Tyr Tyr Cys Met Gln Gly Ser His Trp Pro Pro Thr Phe
   85 90 95
   Gly Gln Gly Thr Lys Val Glu Ile Lys Arg Thr Val Ala Ala Pro Ser
   100 105 110
   Val Phe Ile Phe Pro Pro Ser Asp Glu Gln Leu Lys Ser Gly Thr Ala
   115 120 125
   Ser Val Val Cys Leu Leu Asn Asn Phe Tyr Pro
   130 135
<210> 115
   <211> 100
   <212> PRT
   <213> Homo sapiens
<400> 115
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Pro
   100
<210> 116
   <211> 133
   <212> PRT
   <213> Homo sapiens
<400> 116
   Pro Gly Glu Pro Ala Ser Ile Ser Cys Arg Ser Ser Gln Ser Leu Leu
   1 5 10 15
   His Ser Asn Gly Tyr Asn Tyr Leu Asp Trp Tyr Leu Gln Lys Pro Gly
   20 25 30
   Gln Ser Pro Gln Leu Leu Ile Tyr Leu Gly Ser Asn Arg Ala Ser Gly
   35 40 45
   Val Pro Asp Arg Phe Ser Gly Ser Gly Ser Gly Thr Asp Phe Thr Leu
   50 55 60
   Lys Leu Ser Arg Val Glu Ala Glu Asp Val Gly Val Tyr Tyr Cys Met
   65 70 75 80
   Gln Ala Leu Gln Thr Pro Leu Thr Phe Gly Gly Gly Thr Lys Val Glu
   85 90 95
   Ile Lys Arg Thr Val Ala Ala Pro Ser Val Phe Ile Phe Pro Pro Ser
   100 105 110
   Asp Glu Gln Leu Lys Ser Gly Thr Ala Ser Val Val Cys Leu Leu Asn
   115 120 125
   Asn Phe Tyr Pro Arg
   130
<210> 117
   <211> 20
   <212> PRT
   <213> Homo sapiens
<400> 117
   Asp Ile Gln Met Thr Gln Ser Pro Ser Ser Leu Ser Ala Ser Val Gly
    1              5                   10                  15
   Asp Arg Val Thr
               20
<210> 118
   <211> 20
   <212> PRT
   <213> Homo sapiens
<400> 118
   Glu Ile Val Leu Thr Gln Ser Pro Gly Thr Leu Ser Leu Ser Pro Gly
    1              5                   10                  15
   Glu Arg Ala Thr
               20
<210> 119
   <211> 20
   <212> PRT
   <213> Homo sapiens
<400> 119
   Glu Ile Val Leu Thr Gln Ser Pro Gly Thr Leu Ser Leu Ser Pro Gly
    1              5                   10                  15
   Glu Arg Ala Thr
               20
<210> 120
   <211> 20
   <212> PRT
   <213> Homo sapiens
<400> 120
   Asp Ile Gln Met Thr Gln Ser Pro Ser Ser Val Ser Ala Ser Val Gly
    1              5                   10                  15
   Asp Arg Val Thr
               20
<210> 121
   <211> 20
   <212> PRT
   <213> Homo sapiens
<400> 121
   Thr Gly Glu Phe Val Leu Thr Gln Ser Pro Gly Thr Leu Ser Leu Ser
   1 5 10 15
   Pro Gly Glu Arg
   20
<210> 122
   <211> 20
   <212> PRT
   <213> Homo sapiens
<400> 122
   Glu Phe Val Leu Thr Gln Ser Pro Gly Thr Leu Ser Leu Ser Pro Gly
   1 5 10 15
   Glu Arg Ala Thr
   20
<210> 123
   <211> 20
   <212> PRT
   <213> Homo sapiens
<400> 123
   Glu Ile Val Leu Thr Gln Ser Pro Gly Thr Leu Ser Leu Ser Pro Gly
   1 5 10 15
   Glu Arg Ala Thr
   20
<210> 124
   <211> 20
   <212> PRT
   <213> Homo sapiens
<400> 124
   Glu Ile Val Leu Thr Gln Ser Pro Gly Thr Leu Ser Leu Ser Pro Gly
   1 5 10 15
   Glu Arg Ala Thr
   20
<210> 125
   <211> 20
   <212> PRT
   <213> Homo sapiens
<400> 125
   Glu Ile Val Leu Thr Gln Ser Pro Gly Thr Leu Ser Leu Ser Pro Gly
   1 5 10 15
   Glu Arg Ala Thr
   20
<210> 126
   <211> 21
   <212> PRT
   <213> Homo sapiens
<400> 126
   Gln Val Gln Leu Val Glu Ser Gly Gly Gly Val Val Gln Pro Gly Arg
   1 5 10 15
   Ser Leu Arg Leu Ser
   20
<210> 127
   <211> 5
   <212> PRT
   <213> Homo sapiens
<400> 127
   Pro Glu Val Gln Phe
   1 5
<210> 128
   <211> 21
   <212> PRT
   <213> Homo sapiens
<400> 128
   Gln Val Gln Leu Val Glu Ser Gly Gly Gly Val Val Gln Pro Gly Arg
   1 5 10 15
   Ser Leu Arg Leu Ser
   20
<210> 129
   <211> 10
   <212> PRT
   <213> Homo sapiens
<400> 129
   Pro Glu Val Gln Phe Asn Trp Tyr Val Asp
   1 5 10
<210> 130
   <211> 18
   <212> PRT
   <213> Homo sapiens
<400> 130
   Gln Val Gln Leu Val Glu Ser Gly Gly Gly Val Val Gln Pro Gly Arg
    1               5                  10                  15
   Ser Leu
<210> 131
   <211> 8
   <212> PRT
   <213> Homo sapiens
<400> 131
   Pro Glu Val Gln Phe Asn Trp Tyr
    1               5
<210> 132
   <211> 20
   <212> PRT
   <213> Homo sapiens
<400> 132
   Glu Val Gln Leu Leu Glu Ser Gly Gly Gly Leu Val Gln Pro Gly Gly
    1               5                  10                  15
   Ser Leu Arg Leu
               20
<210> 133
   <211> 21
   <212> PRT
   <213> Homo sapiens
<400> 133
   Gln Val Gln Leu Val Glu Ser Gly Gly Gly Val Val Gln Pro Gly Arg
    1               5                  10                  15
   Ser Leu Arg Leu Ser
               20
<210> 134
   <211> 10
   <212> PRT
   <213> Homo sapiens
<400> 134
   Pro Glu Val Gln Phe Asn Trp Tyr Val Asp
    1               5                  10
<210> 135
   <211> 21
   <212> PRT
   <213> Homo sapiens
<400> 135
   Gln Val Gln Leu Val Glu Ser Gly Gly Gly Val Val Gln Pro Gly Arg
    1               5                  10                  15
   Ser Leu Arg Leu Ser
               20
<210> 136
   <211> 9
   <212> PRT
   <213> Homo sapiens
<400> 136
   Pro Glu Val Gln Phe Asn Trp Tyr Val
    1               5
<210> 137
   <211> 21
   <212> PRT
   <213> Homo sapiens
<400> 137
   Gln Val Gln Leu Val Glu Ser Gly Gly Gly Val Val Gln Pro Gly Arg
    1               5                  10                  15
   Ser Leu Arg Leu Ser
               20
<210> 138
   <211> 6
   <212> PRT
   <213> Homo sapiens
<400> 138
   Pro Glu Val Gln Phe Asn
    1               5
<210> 139
   <211> 21
   <212> PRT
   <213> Homo sapiens
<400> 139
   Gln Val Gln Leu Val Glu Ser Gly Gly Gly Val Val Glu Pro Gly Arg
    1               5                  10                  15
   Ser Leu Arg Leu Ser
               20
<210> 140
   <211> 10
   <212> PRT
   <213> Homo sapiens
<400> 140
   Pro Glu Val Gln Phe Asn Trp Tyr Val Asp
    1               5                   10
<210> 141
   <211> 8
   <212> PRT
   <213> Homo sapiens
<400> 141
   Asp Ile Gln Met Thr Gln Ser Pro
    1               5
<210> 142
   <211> 8
   <212> PRT
   <213> Homo sapiens
<400> 142
   Glu Ile Val Leu Thr Gln Ser Pro
    1               5
<210> 143
   <211> 8
   <212> PRT
   <213> Homo sapiens
<400> 143
   Glu Ile Val Leu Thr Gln Ser Pro
    1               5
<210> 144
   <211> 10
   <212> PRT
   <213> Homo sapiens
<400> 144
   Thr Gly Glu Phe Val Leu Thr Gln Ser Pro
    1               5                   10
<210> 145
   <211> 8
   <212> PRT
   <213> Homo sapiens
<400> 145
   Glu Phe Val Leu Thr Gln Ser Pro
   1 5
<210> 146
   <211> 8
   <212> PRT
   <213> Homo sapiens
<400> 146
   Glu Ile Val Leu Thr Gln Ser Pro
   1 5
<210> 147
   <211> 8
   <212> PRT
   <213> Homo sapiens
<400> 147
   Glu Ile Val Leu Thr Gln Ser Pro
   1 5

## Description

### BACKGROUND OF THE INVENTION

### 2. Summary of the Invention

In accordance with the present invention, there are provided fully human monoclonal antibodies against human cytotoxic T-lymphocyte antigen 4 (CTLA-4). Nucleotide sequences encoding and amino acid sequences comprising heavy and light chain immunoglobulin molecules, particularly contiguous heavy and light chain sequences spanning the complementarity determining regions (CDRs), specifically from within FR1 and/or CDR1 through CDR3 and/or within FR4, are provided. Further provided are antibodies having similar binding properties and antibodies (or other antagonists) having similar functionality as antibodies disclosed herein.

### 3. Background of the Technology

Regulation of immune response in patients would provide a desirable treatment of many human diseases that could lead to a specificity of action that is rarely found through the use of conventional drugs. Both up-regulation and down-regulation of responses of the immune system would be possible. The roles of T cells and B cells have been extensively studied and characterized in connection with the regulation of immune response. From these studies, the role of T cells appear, in many cases, to be particularly important in disease prevention and treatment.

T cells possess very complex systems for controlling their interactions. Interactions between T cells utilize numerous receptors and soluble factors for the process. Thus, what effect any particular signal may have on the immune response generally varies and depends on the particular factors, receptors and counter-receptors that are involved in the pathway. The pathways for down-regulating responses are as important as those required for activation. Thymic education leading to T-cell tolerance is one mechanism for preventing an immune response to a particular antigen. Other mechanisms, such as secretion of suppressive cytokines, are also known.

Activation of T cells requires not only stimulation through the antigen receptor (T cell receptor (TCR)), but additional signaling through co-stimulatory surface molecules such as CD28. The ligands for CD28 are the B7-1 (CD80) and B7-2 (CD86) proteins, which are expressed on antigen-presenting cells such as dendritic cells, activated B-cells or monocytes that interact with T-cell CD28 or CTLA-4 to deliver a costimulatory signal. The role of costimulatory signaling was studied in experimental allergic encephalomyelitis (EAE) by Perrin et al. Immunol Res 14:189-99 (1995). EAE is an autoimmune disorder, induced by Th1 cells directed against myelin antigens that provides an *in vivo* model for studying the role of B7-mediated costimulation in the induction of a pathological immune response. Using a soluble fusion protein ligand for the B7 receptors, as well as monoclonal antibodies specific for either CD80 or CD86, Perrin et al. demonstrated that B7 costimulation plays a prominent role in determining clinical disease outcome in EAE.

The interaction between B7 and CD28 is one of several co-stimulatory signaling pathways that appear to be sufficient to trigger the maturation and proliferation of antigen specific T-cells. Lack of co-stimulation, and the concomitant inadequacy of IL-2 production, prevent subsequent proliferation of the T cell and induce a state of non-reactivity termed "anergy". A variety of viruses and tumors may block T cell activation and proliferation, leading to insufficient activity or non-reactivity of the host's immune system to the infected or transformed cells. Among a number of possible T-cell disturbances, anergy may be at least partly responsible for the failure of the host to clear the pathogenic or tumorgenic cells.

The use of the B7 protein to mediate anti-tumor immunity has been described in Chen et al. Cell 71:1093-1102 (1992) and Townsend and Allison Science 259:368 (1993). Schwartz Cell 71:1065 (1992) reviews the role of CD28, CTLA-4, and B7 in IL-2 production and immunotherapy. Harding et al. Nature 356:607-609 (1994) demonstrates that CD28 mediated signaling co-stimulates murine T cells and prevents the induction of anergy in T cell clones. *See also* U.S. Patent Nos. 5,434,131, 5,770,197, and 5,773,253, and International Patent Application Nos. WO 93/00431, WO 95/01994, WO 95/03408, WO 95/24217, and WO 95/33770. In particular, WO 95/33770 provides legends for induction of antigen specific apoptosis in cells.

From the foregoing, it was clear that T-cells required two types of signals from the antigen presenting cell (APC) for activation and subsequent differentiation to effector function. First, there is an antigen specific signal generated by interactions between the TCR on the T-cell and MHC molecules presenting peptides on the APC. Second, there is an antigen-independent signal that is mediated by the interaction of CD28 with members of the B7 family (B7-1 (CD80) or B7-2 (CD86)). Exactly where CTLA-4 fit into the milieu of immune responsiveness was initially evasive. Murine CTLA-4 was first identified and cloned by Brunet et al. Nature 328:267-270 (1987), as part of a quest for molecules that are preferentially expressed on cytotoxic T lymphocytes. Human CTLA-4 was identified and cloned shortly thereafter by Dariavach et al. Eur. J. Immunol. 18:1901-1905 (1988). The murine and human CTLA-4 molecules possess approximately 76% overall sequence homology and approach complete sequence identity in their cytoplasmic domains (Dariavach et al. Eur. J. Immunol. 18:1901-1905 (1988)). CTLA-4 is a member of the immunoglobulin (Ig) superfamily of proteins. The Ig superfamily is a group of proteins that share key structural features of either a variable (V) or constant (C) domain of Ig molecules. Members of the Ig superfamily include, but are not limited to, the immunoglobulins themselves, major histocompatibility complex (MHC) class molecules (i.e., MHC class I and II), and TCR molecules.

In 1991, Linsley et al. J. Exp. Med. 174:561-569 (1991), proposed that CTLA-4 was a second receptor for B7. Similarly, Harper et al. J Immunol 147:1037-44 (1991) demonstrated that the CTLA-4 and CD28 molecules are closely related in both mouse and human as to sequence, message expression, gene structure, and chromosomal location. *See also* Balzano et al. Int J Cancer Suppl 7:28-32 (1992). Further evidence of this role arose through functional studies. For example, Lenschow et al. Science 257:789-792 (1992) demonstrated that CTLA-4-Ig induced long term survival of pancreatic islet grafts. Freeman et al. Science 262:907-909 (1993) examined the role of CTLA-4 in B7 deficient mice. Examination of the ligands for CTLA-4 are described in Lenschow et al. P.N.A.S. 90:11054-11058 (1993). Linsley et al. Science 257:792-795 (1992) describes immunosuppression *in vivo* by a soluble form of CTLA-4. Linsley et al. J Exp Med 176:1595-604 (1992) prepared antibodies that bound CTLA-4 and that were not cross-reactive with CD28 and concluded that CTLA-4 is coexpressed with CD28 on activated T lymphocytes and cooperatively regulates T cell adhesion and activation by B7. Kuchroo et al. Cell 80:707-18 (1995) demonstrated that the B7-1 and B7-2 costimulatory molecules differentially activated the Th1/Th2 developmental pathways. Yi-qun et al. Int Immunol 8:37-44 (1996) demonstrated that there are differential requirements for co-stimulatory signals from B7 family members by resting versus recently activated memory T cells towards soluble recall antigens. *See also* de Boer et al. Eur J Immunol 23:3120-5 (1993).

Several groups proposed alternative or distinct receptor/ligand interactions for CTLA-4 as compared to CD28 and even proposed a third B-7 complex that was recognized by a BB1 antibody. *See, for example,* Hathcock et al. Science 262:905-7 (1993), Freeman et al. Science 262:907-9 (1993), Freeman et al. J Exp Med 178:2185-92 (1993), Lenschow et al. Proc Natl Acad Sci U S A 90:11054-8 (1993), Razi-Wolf et al. Proc Natl Acad Sci U S A 90:11182-6 (1993), and Boussiotis et al. Proc Natl Acad Sci U S A 90:11059-63 (1993). *But, see,* Freeman et al. J Immunol 161:2708-15 (1998) who discuss finding that BB1 antibody binds a molecule that is identical to the cell surface form of CD74 and, therefore, the BB1 mAb binds to a protein distinct from B7-1, and this epitope is also present on the B7-1 protein. Thus, this observation required the field to reconsider studies using BB1 mAb in the analysis of CD80 expression and function.

Beginning in 1993 and culminating in 1995, investigators began to further delineate the role of CTLA-4 in T-cell stimulation. First, through the use of monoclonal antibodies against CTLA-4, Walunas et al. *Immunity* **1**:405-13 (1994) provided evidence that CTLA-4 can function as a negative regulator of T cell activation. Thereafter, Waterhouse et al. *Science* **270**:985-988 (1995) demonstrated that mice deficient for CTLA-4 accumulated T cell blasts with up-regulated activation markers in their lymph nodes and spleens. The blast cells also infiltrated liver, heart, lung, and pancreas tissue, and amounts of serum immunoglobulin were elevated and their T cells proliferated spontaneously and strongly when stimulated through the T cell receptor, however, they were sensitive to cell death induced by cross-linking of the Fas receptor and by gamma irradiation. Waterhouse et al. concluded that CTLA-4 acts as a negative regulator of T cell activation and is vital for the control of lymphocyte homeostasis. In a comment in the same issue, Allison and Krummel *Science* **270**:932-933 (1995), discussed the work of Waterhouse et al. as demonstrative that CTLA-4 acts to down regulate T-cell responsiveness or has an inhibitory signaling role in T-cell activation and development. Tivol et al. *Immunity* **3**:541-7 (1995) also generated CTLA-4-deficient mice and demonstrated that such mice rapidly develop lymphoproliferative disease with multiorgan lymphocytic infiltration and tissue destruction, with particularly severe myocarditis and pancreatitis. They concluded that CTLA-4 plays a key role in down-regulating T cell activation and maintaining immunologic homeostasis. Also, Krummel and Allison J Exp Med 182:459-65 (1995) further clarified that CD28 and CTLA-4 have opposing effects on the response of T cells to stimulation. They generated an antibody to CTLA-4 and investigated the effects of its binding to CTLA-4 in a system using highly purified T cells. In their report, they showed that the presence of low levels of B7-2 on freshly explanted T cells can partially inhibit T cell proliferation, and this inhibition was mediated by interactions with CTLA-4. Cross-linking of CTLA-4 together with the TCR and CD28 strongly inhibits proliferation and IL-2 secretion by T cells. Finally, the results showed that CD28 and CTLA-4 deliver opposing signals that appear to be integrated by the T cell in determining the response to antigen. Thus, they concluded that the outcome of T cell antigen receptor stimulation is regulated by CD28 costimulatory signals, as well as inhibitory signals derived from CTLA-4. *See also* Krummel et al. Int Immunol 8:519-23 (1996) and U.S. Patent No. 5,811,097 and International Patent Application No. WO 97/20574.

A variety of additional experiments have been conducted further elucidating the above function of CTLA-4. For example, Walunas et al. J Exp Med 183:2541-50 (1996), through the use of anti-CTLA-4 antibodies, suggested that CTLA-4 signaling does not regulate cell survival or responsiveness to IL-2, but does inhibit CD28-dependent IL-2 production. Also, Perrin et al. J Immunol 157:1333-6 (1996), demonstrated that anti-CTLA-4 antibodies in experimental allergic encephalomyelitis (EAE), exacerbated the disease and enhanced mortality. Disease exacerbation was associated with enhanced production of the encephalitogenic cytokines TNF-alpha, IFN-gamma and IL-2. Thus, they concluded that CTLA-4 regulates the intensity of the autoimmune response in EAE, attenuating inflammatory cytokine production and clinical disease manifestations. *See also* Hurwitz et al. J Neuroimmunol 73:57-62 (1997) and Cepero et al. J Exp Med 188:199-204 (1998) (an anti-CTLA-4 hairpin ribozyme that specifically abrogates CTLA-4 expression after gene transfer into a murine T-cell model).

In addition, Blair et al. J Immunol 160:12-5 (1998) assessed the functional effects of a panel of CTLA-4 monoclonal antibodies (mAbs) on resting human CD4+ T cells. Their results demonstrated that some CTLA-4 mAbs could inhibit proliferative responses of resting CD4+ cells and cell cycle transition from G0 to G1. The inhibitory effects of CTLA-4 were evident within 4 h, at a time when cell surface CTLA-4 expression remained undetectable. Other CTLA-4 mAbs, however, had no detectable inhibitory effects, indicating that binding of mAbs to CTLA-4 alone was not sufficient to mediate down-regulation of T cell responses. Interestingly, while IL-2 production was shut off, inhibitory anti-CTLA-4 mAbs permitted induction and expression of the cell survival gene bcl-X(L). Consistent with this observation, cells remained viable and apoptosis was not detected after CTLA-4 ligation. WO 98/42752 provides peptides that bind to CTLA-4 and co-stimulate the proliferation of T-cells by inhibiting the binding of B7 to CTLA-4.

In connection with anergy, Perez et al. Immunity 6:411-7 (1997) demonstrated that the induction of T cell anergy was prevented by blocking CTLA-4 and concluded that the outcome of antigen recognition by T cells is determined by the interaction of CD28 or CTLA-4 on the T cells with B7 molecules. Also, Van Parijs et al., J Exp Med 186:1119-28 (1997) examined the role of interleukin 12 and costimulators in T cell anergy *in vivo* and found that through inhibiting CTLA-4 engagement during anergy induction, T cell proliferation was blocked, and full Th1 differentiation was not promoted. However, T cells exposed to tolerogenic antigen in the presence of both IL-12 and anti-CTLA-4 antibody were not anergized, and behaved identically to T cells which have encountered immunogenic antigen. These results suggested that two processes contribute to the induction of anergy *in vivo:* CTLA-4 engagement, which leads to a block in the ability of T cells to proliferate, and the absence of a prototypic inflammatory cytokine, IL-12, which prevents the differentiation of T cells into Th1 effector cells. The combination of IL-12 and anti-CTLA-4 antibody was sufficient to convert a normally tolerogenic stimulus to an immunogenic one.

In connection with infections, McCoy et al. J Exp Med 186:183-7 (1997) demonstrated that anti-CTLA-4 antibodies greatly enhanced and accelerated the T cell immune response to *Nippostrongylus brasiliensis,* resulting in a profound reduction in adult worm numbers and early termination of parasite egg production. *See also* Murphy et al. J. Immunol. 161:4153-4160 (1998) (*Leishmania donovani*)*.*

In connection with cancer, Kwon et al. PNAS USA 94:8099-103 (1997) established a syngeneic murine prostate cancer model and examined two distinct manipulations intended to elicit an antiprostate cancer response through enhanced T cell costimulation: (i) provision of direct costimulation by prostate cancer cells transduced to express the B7.1 ligand and (ii) *in vivo* antibody-mediated blockade of T cell CTLA-4, which prevents T cell down-regulation. It was demonstrated that *in vivo* antibody-mediated blockade of CTLA-4 enhanced antiprostate cancer immune responses. Also, Yang et al. Cancer Res 57:4036-41 (1997) investigated whether the blockade of the CTLA-4 function leads to enhancement of antitumor T cell responses at various stages of tumor growth. Based on *in vitro* and *in vivo* results they found that CTLA-4 blockade in tumor-bearing individuals enhanced the capacity to generate antitumor T-cell responses, but the expression of such an enhancing effect was restricted to early stages of tumor growth in their model. Further, Hurwitz et al. Proc Natl Acad Sci U S A 95:10067-71 (1998) investigated the generation of a T cell-mediated antitumor response depends on T cell receptor engagement by major histocompatibility complex/antigen as well as CD28 ligation by B7. Certain tumors, such as the SM1 mammary carcinoma, were refractory to anti-CTLA-4 immunotherapy. Thus, through use of a combination of CTLA-4 blockade and a vaccine consisting of granulocyte-macrophage colony-stimulating factor-expressing SM1 cells, regression of parental SM1 tumors was observed, despite the ineffectiveness of either treatment alone. This combination therapy resulted in long-lasting immunity to SM1 and depended on both CD4(+) and CD8(+) T cells. The findings suggested that CTLA-4 blockade acts at the level of a host-derived antigen-presenting cell.

In connection with diabetes, Luhder et al. J Exp Med 187:427-32 (1998) injected an anti-CTLA-4 mAb into a TCR transgenic mouse model of diabetes at different stages of disease. They found that engagement of CTLA-4 at the time when potentially diabetogenic T cells are first activated is a pivotal event; if engagement is permitted, invasion of the islets occurs, but remains quite innocuous for months. If not, insulitis is much more aggressive, and diabetes quickly ensues.

In connection with vaccine immunization, Horspool et al. J Immunol 160:2706-14 (1998) found that intact anti-CTLA-4 mAb but not Fab fragments suppressed the primary humoral response to pCIA/beta gal without affecting recall responses, indicating CTLA-4 activation inhibited Ab production but not T cell priming. Blockade of the ligands for CD28 and CTLA-4, CD80 (B7-1) and CD86 (B7-2), revealed distinct and nonoverlapping function. Blockade of CD80 at initial immunization completely abrogated primary and secondary Ab responses, whereas blockade of CD86 suppressed primary but not secondary responses. Simultaneous blockade of CD80 + CD86 was less effective at suppressing Ab responses than either alone. Enhancement of costimulation via coinjection of B7-expressing plasmids augmented CTL responses but not Ab responses, and without evidence of Th1 to Th2 skewing. These findings suggest complex and distinct roles for CD28, CTLA-4, CD80, and CD86 in T cell costimulation following nucleic acid vaccination.

In connection with allograft rejection, Markees et al. J Clin Invest 101:2446-55 (1998) found in a mouse model of skin allograft rejection that acceptance initially depended on the presence of IFN-gamma, CTLA-4, and CD4(+) T cells. Addition of anti-CTLA-4 or anti-IFN-gamma mAb to the protocol was associated with prompt graft rejection, whereas anti-IL-4 mAb had no effect.

In connection with the role of CTLA-4 in relation to CD28, Fallarino et al. J Exp Med 188:205-10 (1998) generated TCR transgenic/recombinase activating gene 2-deficient/CD28-wild-type or CD28-deficient mice which were immunized with an antigen-expressing tumor. Primed T cells from both types of mice produced cytokines and proliferated in response to stimulator cells lacking B7 expression. However, whereas the response of CD28+/+ T cells was augmented by costimulation with B7-1, the response of the CD28-/- T cells was strongly inhibited. This inhibition was reversed by monoclonal antibody against B7-1 or CTLA-4. Thus, CTLA-4 can potently inhibit T cell activation in the absence of CD28, indicating that antagonism of a TCR-mediated signal is sufficient to explain the inhibitory effect of CTLA-4. Also, Lin et al. J Exp Med 188:199-204 (1998) studied rejection of heart allografts in CD28-deficient mice. H-2(q) hearts were transplanted into allogeneic wild-type or CD28-deficient mice (H-2(b)). Graft rejection was delayed in CD28-deficient compared with wild-type mice. Treatment of wild-type recipients with CTLA-4-immunoglobulin (Ig), or with anti-B7-1 plus anti-B7-2 mAbs significantly prolonged allograft survival. In contrast, treatment of CD28-deficient mice with CTLA-4-Ig, anti-B7-1 plus anti-B7-2 mAbs, or a blocking anti-CTLA-4 mAb induced acceleration of allograft rejection. This increased rate of graft rejection was associated with more severe mononuclear cell infiltration and enhanced levels of IFN-gamma and IL-6 transcripts in donor hearts of untreated wild-type and CTLA-4-Ig- or anti-CTLA-4 mAb-treated CD28-deficient mice. Thus, the negative regulatory role of CTLA-4 extends beyond its potential ability to prevent CD28 activation through ligand competition. Even in the absence of CD28, CTLA-4 plays an inhibitory role in the regulation of allograft rejection.

Also, further characterization of the expression of CTLA-4 has been investigated. For example, Alegre et al. J Immunol 157:4762-70 (1996) proposed that surface CTLA-4 is rapidly internalized, which may explain the low levels of expression generally detected on the cell surface. They concluded that both CD28 and IL-2 play important roles in the up-regulation of CTLA-4 expression. In addition, the cell surface accumulation of CTLA-4 appeared to be primarily regulated by its rapid endocytosis. Also, Castan et al. Immunology 90:265-71 (1997) based on *in situ* immunohistological analyses of the expression of CTLA-4, suggested that germinal center T cells, which were CTLA-4 positive, could be important to immune regulation.

Accordingly, in view of the broad and pivotal role that CTLA-4 appears to possess in immune responsiveness, it would be desirable to generate antibodies to CTLA-4 that can be utilized effectively in immunotherapy. Moreover, it would be desirable to generate antibodies against CTLA-4 that can be utilized in chronic diseases in which repeat administrations of the antibodies are required.

### BRIEF DESCRIPTION OF THE DRAWING FIGURES

Figure 1 provides a series of nucleotide and an amino acid sequences of heavy chain and kappa light chain immunoglobulin molecules in accordance with the invention: 4.1.1 (Figure 1A), 4.8.1 (Figure 1B), 4.14.3 (Figure 1C), 6.1.1 (Figure 1D), 3.1.1 (Figure IE), 4.10.2 (Figure 1F), 2.1.3 (Figure 1G), 4.13.1 (Figure 1H), 11.2.1 (Figure 1I), 11.6.1 (Figure 1J), 11.7.1 (Figure 1K), 12.3.1.1 (Figure 1L), and 12.9.1.1 (Figure 1M).
Figure 2 provides a sequence alignment between the predicted heavy chain amino acid sequences from the clones 4.1.1, 4.8.1, 4.14.3, 6.1.1, 3.1.1, 4.10.2, 4.13.1, 11.2.1, 11.6.1, 11.7.1, 12.3.1.1, and 12.9.1.1 and the germline DP-50 (3-33) amino acid sequence. Differences between the DP-50 germline sequence and that of the sequence in the clones are indicated in bold. The Figure also shows the positions of the CDR1, CDR2, and CDR3 sequences of the antibodies as shaded.
Figure 3 provides a sequence alignment between the predicted heavy chain amino acid sequence of the clone 2.1.3 and the germline DP-65 (4-31) amino acid sequence. Differences between the DP-65 germline sequence and that of the sequence in the clone are indicated in bold. The Figure also shows the positions of the CDR1, CDR2, and CDR3 sequences of the antibody as underlined.
Figure 4 provides a sequence alignment between the predicted kappa light chain amino acid sequence of the clones 4.1.1, 4.8.1, 4.14.3, 6.1.1, 4.10.2, and 4.13.1 and the germline A27 amino acid sequence. Differences between the A27 germline sequence and that of the sequence in the clone are indicated in bold. The Figure also shows the positions of the CDR1, CDR2, and CDR3 sequences of the antibody as underlined. Apparent deletions in the CDRls of clones 4.8.1, 4.14.3, and 6.1.1 are indicated with "0s".
Figure 5 provides a sequence alignment between the predicted kappa light chain amino acid sequence of the clones 3.1.1, 11.2.1, 11.6.1, and 11.7.1 and the germline 012 amino acid sequence. Differences between the 012 germline sequence and that of the sequence in the clone are indicated in bold. The Figure also shows the positions of the CDR1, CDR2, and CDR3 sequences of the antibody as underlined.
Figure 6 provides a sequence alignment between the predicted kappa light chain amino acid sequence of the clone 2.1.3 and the germline A10/A26 amino acid sequence. Differences between the A10/A26 germline sequence and that of the sequence in the clone are indicated in bold. The Figure also shows the positions of the CDR1, CDR2, and CDR3 sequences of the antibody as underlined.
Figure 7 provides a sequence alignment between the predicted kappa light chain amino acid sequence of the clone 12.3.1 and the germline A17 amino acid sequence. Differences between the A17 germline sequence and that of the sequence in the clone are indicated in bold. The Figure also shows the positions of the CDR1, CDR2, and CDR3 sequences of the antibody as underlined.
Figure 8 provides a sequence alignment between the predicted kappa light chain amino acid sequence of the clone 12.9.1 and the germline A3/A19 amino acid sequence. Differences between the A3/A19 germline sequence and that of the sequence in the clone are indicated in bold. The Figure also shows the positions of the CDR1, CDR2, and CDR3 sequences of the antibody as underlined.
Figure 9 provides a summary of N-terminal amino acid sequences generated through direct protein sequencing of the heavy and light chains of the antibodies.

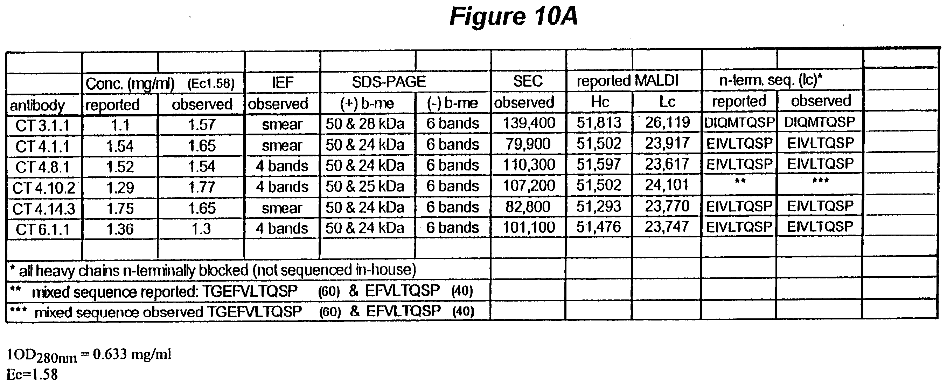

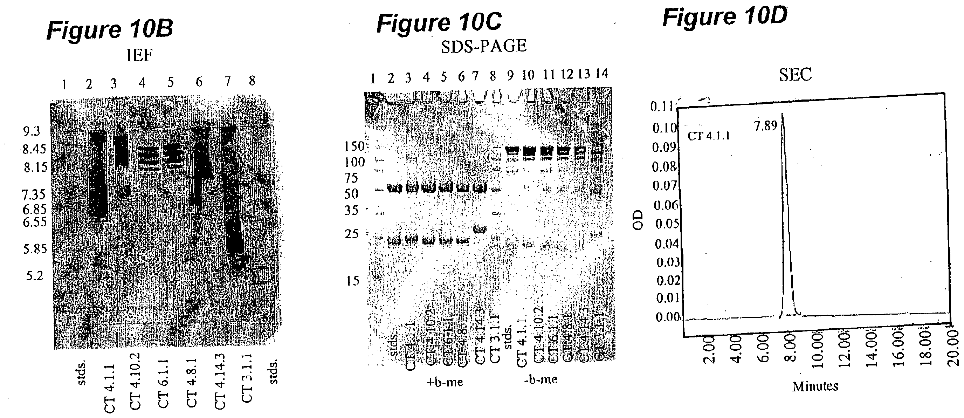

Figure 10 provides certain additional characterizing information about certain of the antibodies in accordance with the invention. In Figure 10A, data related to clones 3.1.1, 4.1.1, 4.8.1, 4.10.2, 4.14.3, and 6.1.1 is summarized. Data related to concentration, isoelectric focusing (IEF), SDS-PAGE, size exclusion chromatography, liquid chromatography/mass spectroscopy (LCMS), mass spectroscopy (MALDI), light chain N-terminal sequences is provided. Additional detailed information related to IEF is provided in Figure 10B; related to SDS-PAGE is provided in 10C; and SEC of the 4.1.1 antibody in 10D.
Figure 11 shows the expression of B7-1 and B7-2 on Raji cells using anti-CD80-PE and anti-CD86-PE mAbs.
Figure 12 shows the concentration dependent enhancement of IL-2 production in the T cell blast/Raji assay induced by anti-CTLA-4 blocking antibodies (BNI3, 4.1.1, 4.8.1, and 6.1.1).
Figure 13 shows the concentration dependent enhancement of IFN-γ production in the T cell blast/Raji assay induced by anti-CTLA-4 blocking antibodies (BNI3, 4.1.1, 4.8.1, and 6.1.1)(same donor T cells).
Figure 14 shows the mean enhancement of IL-2 production in T cells from 6 donors induced by anti-CTLA-4 blocking antibodies in the T cell blast/Raji assay.
Figure 15 shows the mean enhancement of IFN-γ production in T cells from 6 donors induced by anti-CTLA-4 blocking antibodies in the T cell blast/Raji assay.
Figure 16 shows the enhancement of IL-2 production in hPBMC from 5 donors induced by anti-CTLA-4 blocking mAbs as measured at 72 hours after stimulation with SEA.
Figure 17 shows the enhancement of IL-2 production in whole blood from 3 donors induced by anti-CTLA-4 blocking mAbs as measured at 72 and 96 hours after stimulation with SEA.
Figure 18 shows the inhibition of tumor growth with an anti-murine CTLA-4 antibody in a murine fibrosarcoma tumor model.
Figure 19 shows enhancement of IL-2 production induced by anti-CTLA4 antibodies (4.1.1 and 11.2.1) of the invention in a 72 hour T blast/Raji and Superantigen (whole blood and peripheral blood mononuclear cells from 6 donors) assays.
Figure 20 shows dose dependent enhancement of IL-2 production induced by anti-CTLA4 antibodies (4.1.1 and 11.2.1) of the invention in a 72 hour T blast/Raji assay.
Figure 21 shows dose dependent enhancement of IL-2 production induced by anti-CTLA4 antibodies (4.1.1 and 11.2.1) of the invention in a 72 hour Superantigen whole blood assay stimulated with 100 ng/ml superantigen.

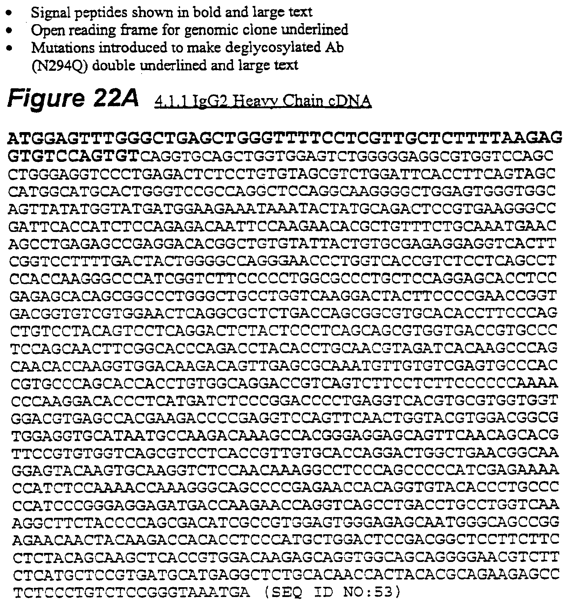

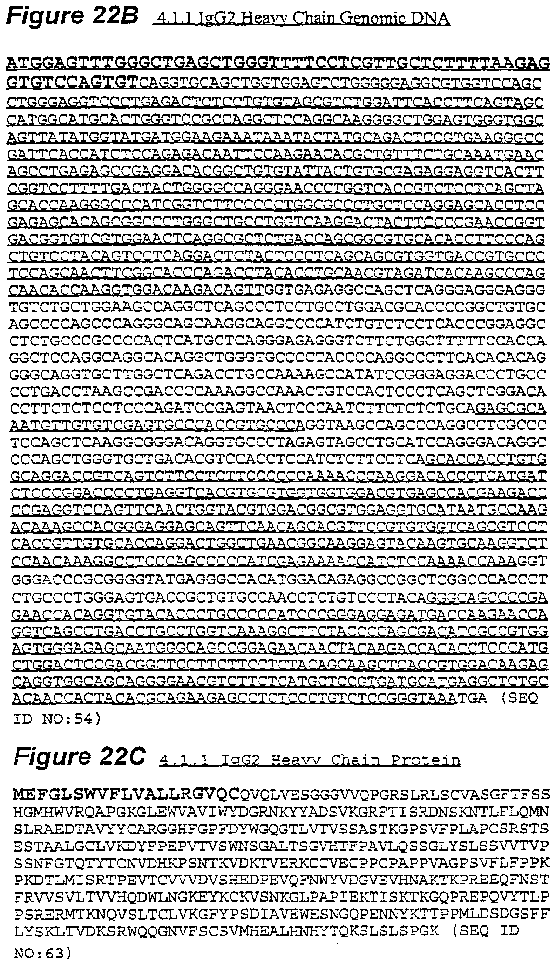

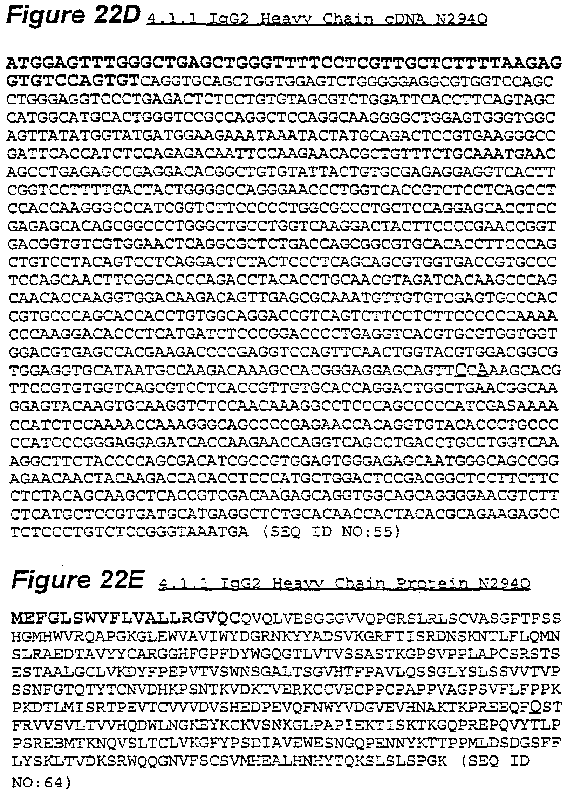

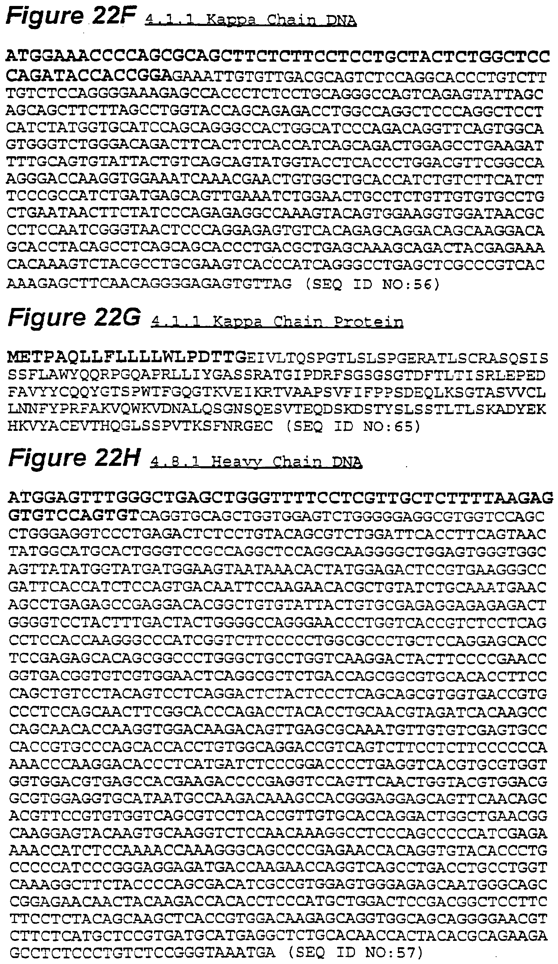

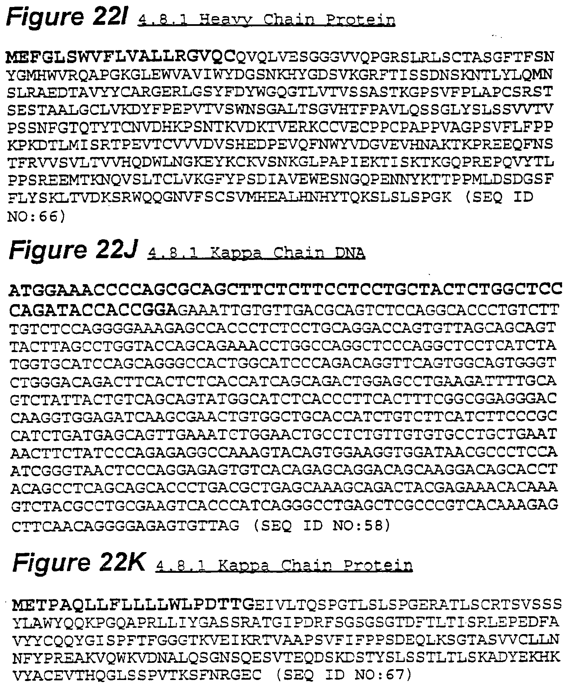

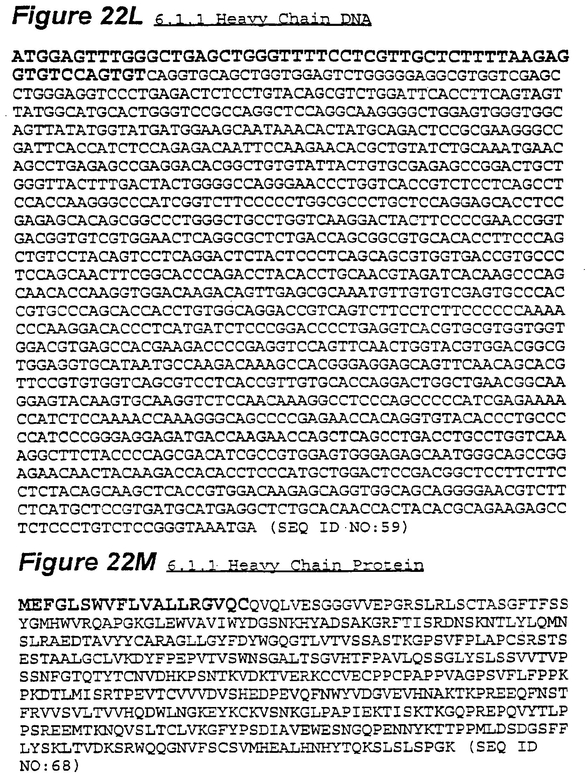

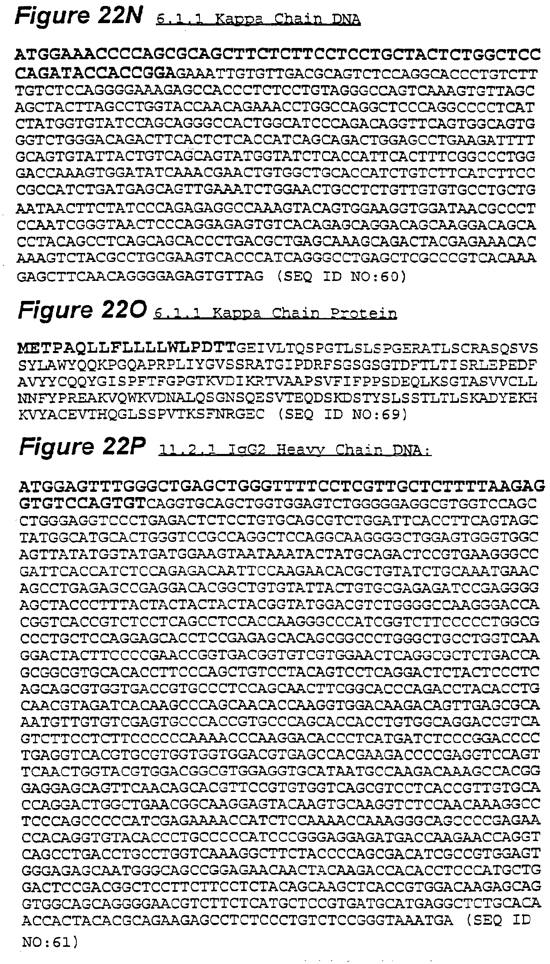

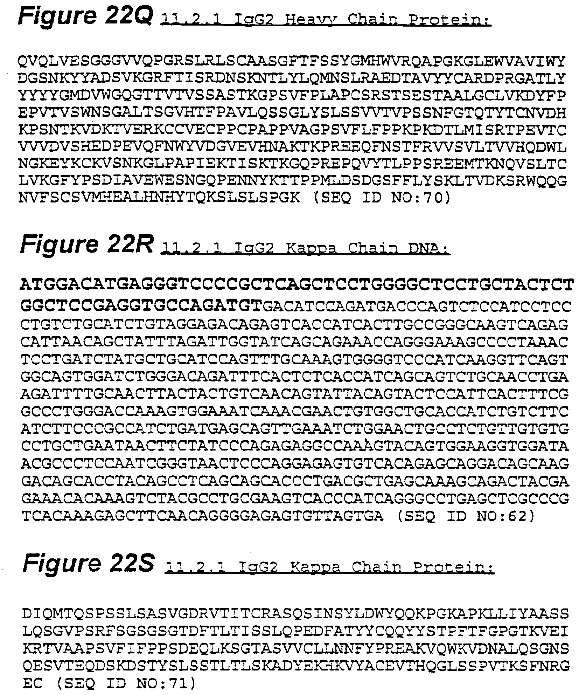

Figure 22 provides a series of additional nucleotide and amino acid sequences of the following anti-CTLA-4 antibody chains: full length 4.1.1 heavy chain (cDNA 22(a), genomic 22(b), and amino acid 22(c)), full length aglycosylated 4.1.1 heavy chain (cDNA 22(d) and amino acid 22(e)), 4.1.1 light chain (cDNA 22(f) and amino acid 22(g)), full length 4.8.1 heavy chain (cDNA 22(h) and amino acid 22(i)), 4.8.1 light chain (cDNA 22(j) and amino acid 22(k)), full length 6.1.1 heavy chain (cDNA 22(1) and amino acid 22(m)), 6.1.1 light chain (cDNA 22(n) and amino acid 22(o)), full length 11.2.1 heavy chain (cDNA 22(p) and amino acid 22(q)), and 11.2.1 light chain (cDNA 22 (r) and amino acid 22(s)).

### SUMMARY OF THE INVENTION

The invention relates to the embodiments as defined in the claims. Thus, it relates to a human monoclonal antibody that binds to CTLA-4, or an antigen-binding fragment thereof, wherein said antibody or fragment competes for binding to CTLA-4 with a reference antibody comprising a heavy chain that comprises the amino acid sequence of SEQ ID NO. 9 or an amino acid sequence at least 90% identical thereto and a light chain that comprises the amino acid sequence of SEQ ID NO. 22 or an amino acid sequence at least 90% identical thereto, wherein the antibody or antigen-binding fragment thereof has the following properties: a) a binding affinity for CTLA-4 of 10⁻⁹ M or greater as determined by BIAcore; b) inhibits binding between CTLA-4 and B7-1 with an IC₅₀ of 100 nM or lower; and c) inhibits binding between CTLA-4 and B7-2 with an IC₅₀ of 100 nM or lower. In addition, the invention relates to a pharmaceutical composition comprising the antibody or antigen-binding fragment of the invention and a pharmaceutically acceptable carrier, in particular for treating cancer. Moreover, provided is a nucleic acid that encodes the light and heavy chain amino acid sequences of the antibody or antigen-binding fragment of the invention. Furthermore, the invention provides for a method for production of the antibody or antigen-binding fragment of the invention comprising expressing said antibody or antigen binding fragment in a host cell line and recovering said antibody or antigen-binding fragment.

The invention relates to the embodiments as defined in the claims. Thus, it relates to a human monoclonal antibody that binds to CTLA-4, or an antigen-binding fragment thereof, wherein said antibody or fragment competes for binding to CTLA-4 with a reference antibody, wherein the antibody or antigen-binding fragment thereof has the following properties a) a binding affinity for CTLA-4 of 10⁻⁹ M or greater as determined by BIAcore; b) inhibits binding between CTLA-4 and B7-1 with an IC₅₀ of 100 nM or lower; and c) inhibits binding between CTLA-4 and B7-2 with an IC₅₀ of 100 nM or lower, wherein the reference antibody is selected from the group consisting of i) an antibody comprising a heavy chain that comprises the amino acid sequence of SEQ ID NO: 9 and a light chain that comprises the amino acid sequence of SEQ ID NO: 22; ii) an antibody comprising a heavy chain that comprises the amino acid sequence of SEQ ID NO: 5 and a light chain that comprises the amino acid sequence of SEQ ID NO: 18; iii) an antibody comprising a heavy chain that comprises the amino acid sequence of SEQ ID NO: 1 and a light chain that comprises the amino acid sequence of SEQ ID NO: 14; iv) an antibody comprising a heavy chain that comprises the amino acid sequence of SEQ ID NO: 2 and a light chain that comprises the amino acid sequence of SEQ ID NO: 15; v) an antibody comprising a heavy chain that comprises the amino acid sequence of SEQ ID NO: 8 and a light chain that comprises the amino acid sequence of SEQ ID NO: 21; vi) an antibody comprising a heavy chain that comprises the amino acid sequence of SEQ ID NO: 3 and a light chain that comprises the amino acid sequence of SEQ ID NO: 16; vii) an antibody comprising a heavy chain that comprises the amino acid sequence of SEQ ID NO: 4 and a light chain that comprises the amino acid sequence of SEQ ID NO: 17; viii) an antibody comprising a heavy chain that comprises the amino acid sequence of SEQ ID NO: 10 and a light chain that comprises the amino acid sequence of SEQ ID NO: 23; and ix) an antibody comprising a heavy chain that comprises the amino acid sequence of SEQ ID NO: 11 and a light chain that comprises the amino acid sequence of SEQ ID NO: 24. In addition, the invention relates to a pharmaceutical composition comprising the antibody or antigen-binding fragment of the invention and a pharmaceutically acceptable carrier, in particular for treating cancer. Moreover, provided is a nucleic acid that encodes the light and heavy chain amino acid sequences of the antibody or antigen-binding fragment of the invention. Furthermore, the invention provides for a method for production of the antibody or antigen-binding fragment of the invention comprising expressing said antibody or antigen binding fragment in a host cell line and recovering said antibody or antigen-binding fragment.

In accordance with a fifth aspect of the present disclosure, there is provided an isolated human monoclonal antibody that is capable of binding to CTLA-4. In a preferred embodiment, antibody is capable of competing for binding with CTLA-4 with an antibody selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1. In another preferred embodiment, the antibody possesses a substantially similar binding specificity to CTLA-4 as an antibody selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1. In another preferred embodiment, the antibody is selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1. In another preferred embodiment, the antibody is not cross reactive with CTLA-4 from lower mammalian species, preferably the lower mammalian species comprises mouse, rat, and rabbit and more preferably mouse and rat. In another preferred embodiment, the antibody is cross reactive with CTLA-4 from primates, preferably the primates comprise cynomolgous and rhesus monkeys. In another preferred embodiment, the antibody possesses a selectivity for CTLA-4 over CD28, B7-2, CD44, and hIgG1 of greater than about 100:1 and preferably about 500:1 or greater. In another preferred embodiment, the binding affinity of the antibody is about 10⁻⁹ M or greater and preferably about 10⁻¹⁰ M or greater. In another preferred embodiment, the antibody inhibits binding between CTLA-4 and B7-2 with an IC₅₀ of lower than about 100 nM and preferably lower than about 0.38 nM. In another preferred embodiment, the antibody inhibits binding between CTLA-4 and B7-1 with an IC₅₀ of lower than about 100 nM or greater and preferably lower than about 0.50 nM. In another preferred embodiment, the antibody enhances IL-2 production in a T cell blast/Raji assay by about 500 pg/ml or greater and preferably by about 3846 pg/ml or greater. In another preferred embodiment, the antibody enhances IFN-γ production in a T cell blast/Raji assay by about 500 pg/ml or greater and preferably by about 1233 pg/ml or greater. In another preferred embodiment, the antibody enhances IL-2 production in a hPBMC or whole blood superantigen assay by about 500 pg/ml or greater. In another preferred embodiment, the antibody enhances IL-2 production in a hPBMC or whole blood superantigen assay by about 500 pg/ml or preferably 1500 pg/ml or greater or by greater than about 30% or preferably 50% relative to control.

In accordance with a sixth aspect of the present disclosure, there is provided a humanized antibody that possesses a substantially similar binding specificity to CTLA-4 as an antibody selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1. In a preferred embodiment, the antibody is not cross reactive with CTLA-4 from lower mammalian species, preferably the lower mammalian species comprises mouse, rat, and rabbit and preferably mouse and rat. In another preferred embodiment, the antibody is cross reactive with CTLA-4 from primates, preferably the primates comprise cynomolgous and rhesus monkeys. In another preferred embodiment, the antibody possesses a selectivity for CTLA-4 over CD28, B7-2, CD44, and hIgG1 of greater than about 100:1 and preferably about 500:1 or greater. In another preferred embodiment, the binding affinity of the antibody is about 10⁻⁹ M or greater and preferably about 10⁻¹⁰ M or greater. In another preferred embodiment, the antibody inhibits binding between CTLA-4 and B7-2 with an IC₅₀ of lower than about 100 nM and preferably lower than about 0.38 nM. In another preferred embodiment, the antibody inhibits binding between CTLA-4 and B7-1 with an IC₅₀ of lower than about 100 nM or greater and preferably lower than about 0.50 nM. In another preferred embodiment, the antibody enhances IL-2 production in a T cell blast/Raji assay by about 500 pg/ml or greater and preferably by about 3846 pg/ml or greater. In another preferred embodiment, the antibody enhances IFN-γ production in a T cell blast/Raji assay by about 500 pg/ml or greater and preferably by about 1233 pg/ml or greater. In another preferred embodiment, the antibody induces IL-2 production in a hPBMC or whole blood superantigen assay by about 500 pg/ml or greater. In another preferred embodiment, the antibody enhances IL-2 production in a hPBMC or whole blood superantigen assay by about 500 pg/ml or preferably 1500 pg/ml or greater or by greater than about 30% or preferably 50% relative to control.

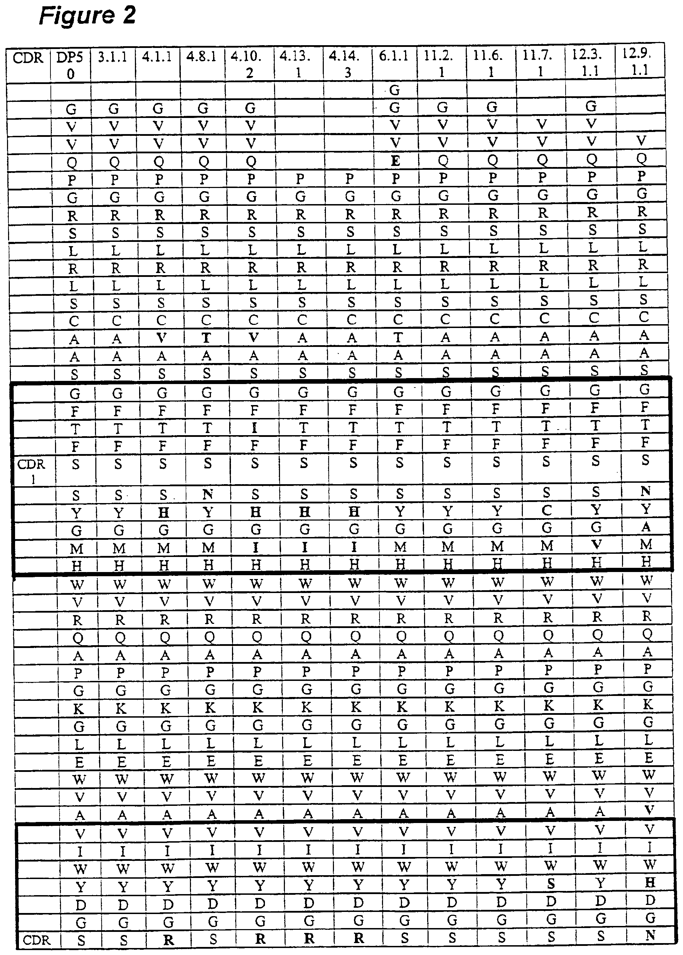

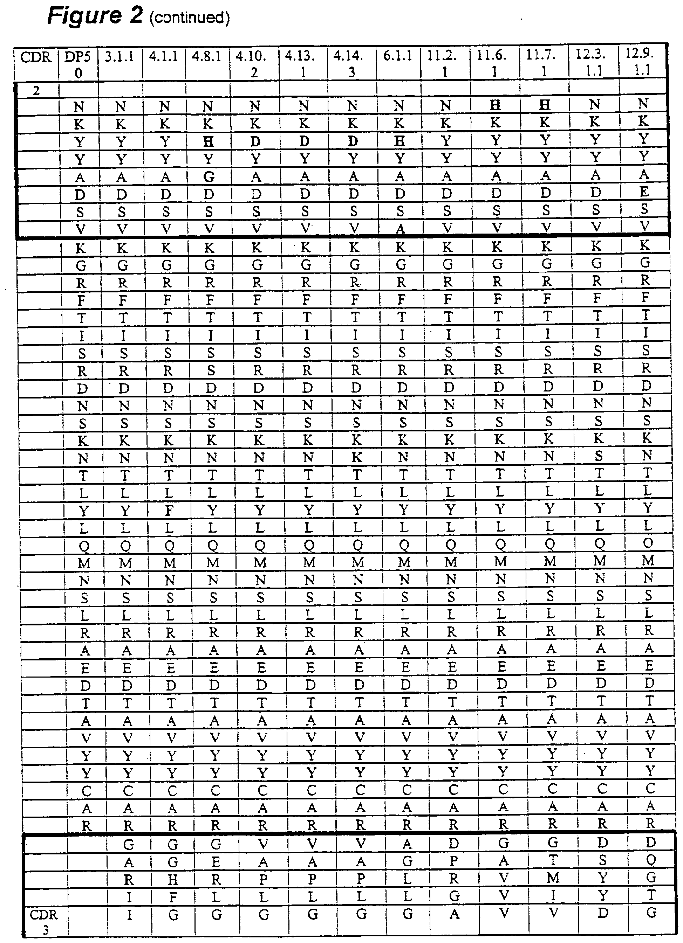

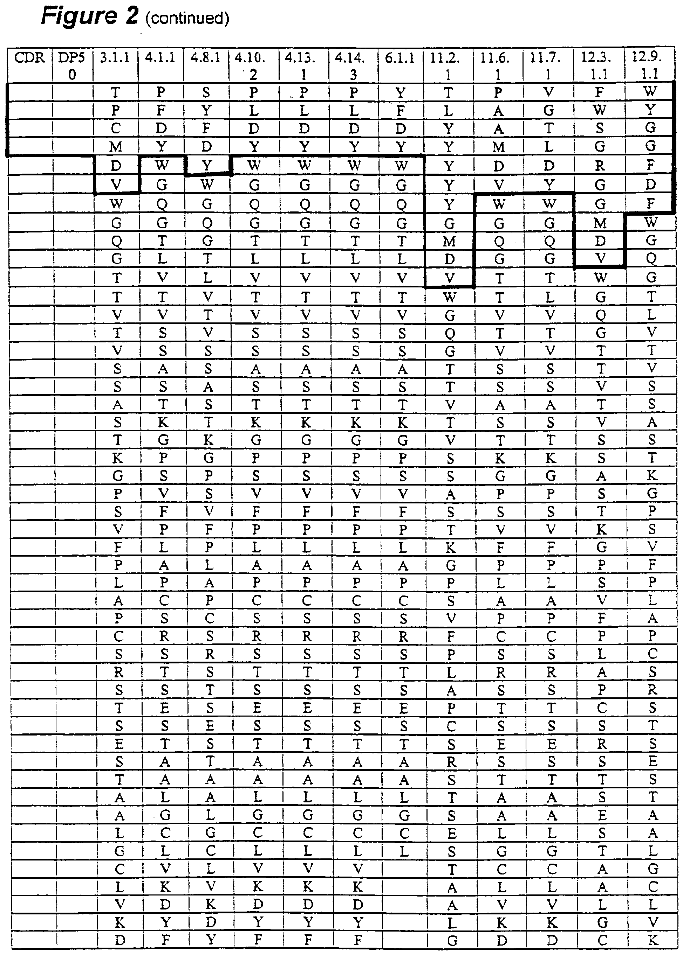

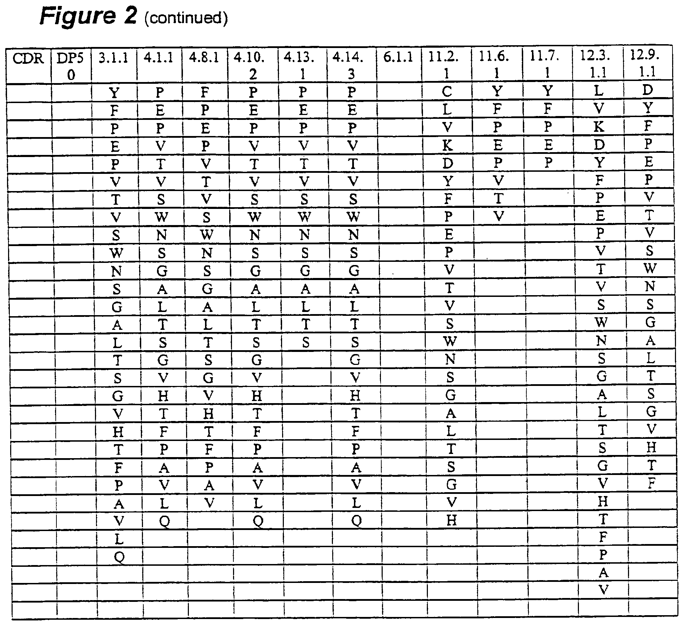

In accordance with a seventh aspect of the present disclosure, there is provided an antibody that binds to CTLA-4, comprising a heavy chain amino acid sequence comprising human FR1, FR2, and FR3 sequences encoded by a human V_{H} 3-33 gene family operably linked in frame with a CDR1, a CDR2, and a CDR3 sequence, the CDR1, CDR2, and CDR3 sequences being independently selected from the CDR1, CDR2, and CDR3 sequences illustrated in Figure 2. In a preferred embodiment, the antibody of Claim 32, further comprising any of the somatic mutations to the FR1, FR2, and FR3 sequences as illustrated in Figure 2.

In accordance with an eighth aspect of the present disclosure, there is provided an antibody that binds to CTLA-4, comprising a heavy chain amino acid sequence comprising human FR1, FR2, and FR3 sequences encoded by a human V_{H} 3-33 gene family operably linked in frame with a CDR1, a CDR2, and a CDR3 sequence, which antibody has the following properties: a binding affinity for CTLA-4 of about 10⁻⁹ or greater, inhibits binding between CTLA-4 and B7-1 with an IC₅₀ of about 100 nM or lower; inhibits binding between CTLA-4 and B7-2 with an IC₅₀ of about 100 nM or lower, and enhances cytokine production in an assay of human T cells by 500 pg/ml or greater.

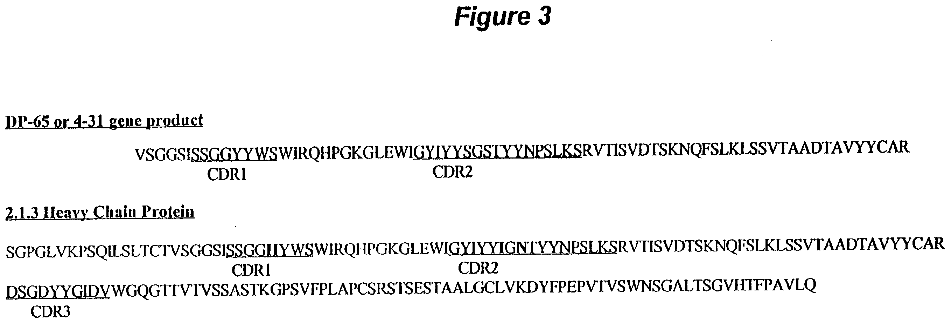

In accordance with a ninth aspect of the present disclosure, there is provided an antibody that binds to CTLA-4, comprising a heavy chain amino acid sequence comprising FR1, FR2, and FR3 sequences operably linked in frame with a CDR1, a CDR2, and a CDR3 sequence independently selected from the CDR1, CDR2, and CDR3 sequences illustrated in Figures 2 and 3, which antibody has the following properties: a binding affinity for CTLA-4 of about 10⁻⁹ or greater; inhibits binding between CTLA-4 and B7-1 with an IC₅₀ of about 100 nM or lower; inhibits binding between CTLA-4 and B7-2 with an IC₅₀ of about 100 nM or lower; and enhances cytokine production in an assay of human T cells by 500 pg/ml or greater.

In accordance with a tenth aspect of the present disclosure, there is provided a cell culture system for assaying T cell stimulation, comprising a culture of human T cell blasts co-cultured with a Raji cell line. In a preferred embodiment, the T cell blasts are washed prior to culture with the Raji cell line.

In accordance with an eleventh aspect of the present disclosure, there is provided an assay for measuring T cell stimulation, comprising: providing a culture of human T cell blasts and a Raji cell line; contacting the culture with an agent; and measuring cytokine production by the culture.

In accordance with an twelfth aspect of the present disclosure, there is provided a functional assay for screening a moiety for T cell stimulatory function, comprising: providing a culture of human T cell blasts and a Raji cell line; contacting the culture with the moiety; and assessing cytokine production by the culture.

In accordance with a thirteenth aspect of the present disclosure, there is provided a T cell stimulatory assay for CTLA-4 inhibitory function, comprising contacting a culture comprising human T cell blasts and a Raji cell line with an agent and assessing cytokine production by the culture.

In accordance with a fourteenth aspect of the present disclosure, there is provided a method for screening an agent for T cell stimulatory activity, comprising: contacting the agent with a cell culture comprising human T cell blasts and a Raji cell line; and assessing cytokine production by the culture.

In each of the tenth through the fourteenth aspects of the present disclosure, in a preferred embodiment, the T cell blasts are washed prior to culture with the Raji cell line. In another preferred embodiment, the cytokine is IL-2 or IFN-γ. In a preferred embodiment, cytokine production is measured in supernatant isolated from the culture. In a preferred embodiment, the agent is an antibody and preferably binds to CTLA-4.

In accordance with a fifteenth aspect of the present disclosure, there is provided an assay for measuring T cell stimulation, comprising: providing a population of human peripheral blood mononuclear cells or human whole blood stimulated with staphylococcus enterotoxin A; contacting the culture with an agent; and measuring cytokine production by the cell population.

In accordance with a sixteenth aspect of the present disclosure, there is provided a functional assay for screening a moiety for T cell stimulatory function, comprising: providing a population of human peripheral blood mononuclear cells or human whole blood stimulated with staphylococcus enterotoxin A; contacting the culture with the moiety; and assessing cytokine production by the cell population.

In accordance with a seventeenth aspect of the present disclosure, there is provided a T cell stimulatory assay for CTLA-4 inhibitory function, comprising contacting a population of human peripheral blood mononuclear cells or human whole blood stimulated with staphylococcus enterotoxin A with an agent and assessing cytokine production by the cell population.

In accordance with an eighteenth aspect of the present disclosure, there is provided a method for screening an agent for T cell stimulatory activity, comprising: contacting the agent with a population of human peripheral blood mononuclear cells or human whole blood stimulated with staphylococcus enterotoxin A; and assessing cytokine production by the cell population.

In each of the fifteenth through the eighteenth aspects of the present disclosure, in a preferred embodiment, the cytokine is IL-2. In another preferred embodiment, cytokine production is measured in supernatant isolated from the culture. In a preferred embodiment, the agent is an antibody and preferably binds to CTLA-4.

### DETAILED DESCRIPTION OF THE PREFERRED EMBODIMENTS

In accordance with the present invention, there are provided fully human monoclonal antibodies against human CTLA-4. Nucleotide sequences encoding and amino acid sequences comprising heavy and light chain immunoglobulin molecules, particularly sequences corresponding to a contiguous heavy and light chain sequences from FR1 and CDR1 through CDR3 and FR4, are provided. Further provided are antibodies having similar binding properties and antibodies (or other antagonists) having similar functionality as antibodies disclosed herein. Hybridomas expressing such immunoglobulin molecules and monoclonal antibodies are also provided.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al. Molecular Cloning: A Laboratory Manual (2d ed., Cold Spring Harbor Laboratory Press, Cold Spring Harbor, N.Y. (1989)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The term "isolated polynucleotide" as used herein shall mean a polynucleotide of genomic, cDNA, or synthetic origin or some combination thereof, which by virtue of its origin the "isolated polynucleotide" (1) is not associated with all or a portion of a polynucleotide in which the "isolated polynucleotide" is found in nature, (2) is operably linked to a polynucleotide which it is not linked to in nature, or (3) does not occur in nature as part of a larger sequence.

The term "isolated protein" referred to herein means a protein of cDNA, recombinant RNA, or synthetic origin or some combination thereof, which by virtue of its origin, or source of derivation, the "isolated protein" (1) is not associated with proteins found in nature, (2) is free of other proteins from the same source, e.g. free of murine proteins, (3) is expressed by a cell from a different species, or (4) does not occur in nature.

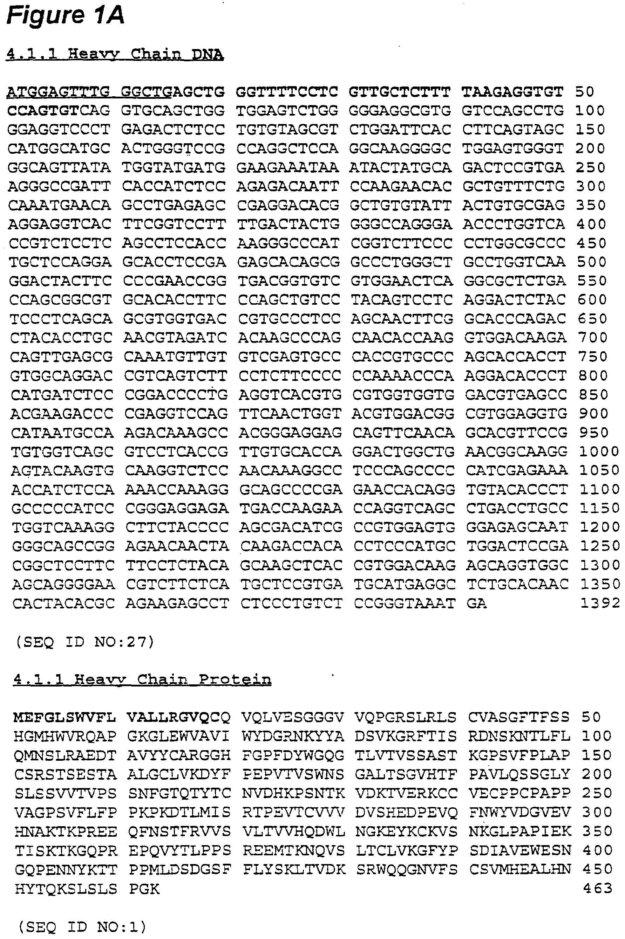

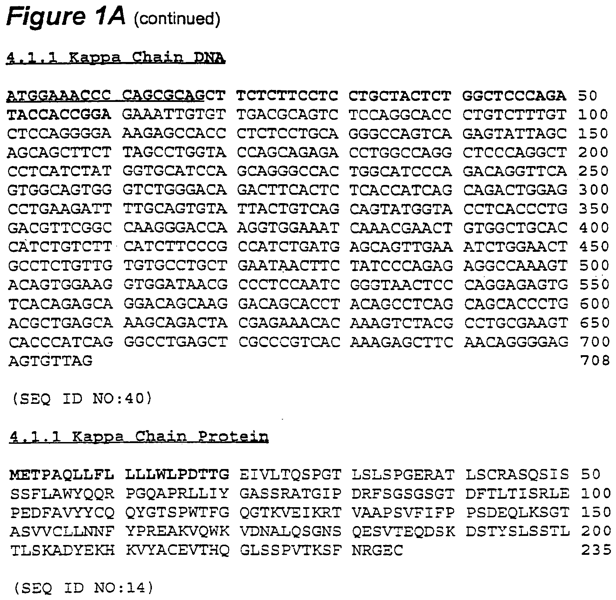

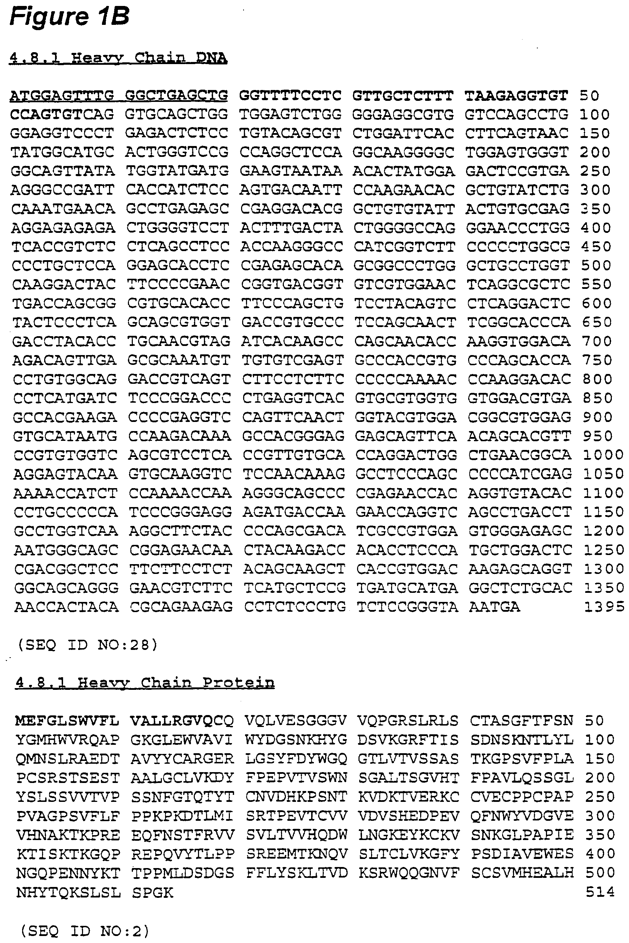

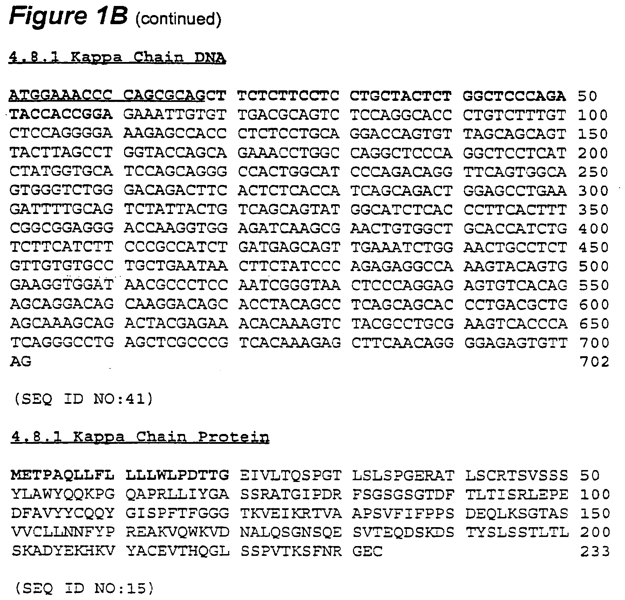

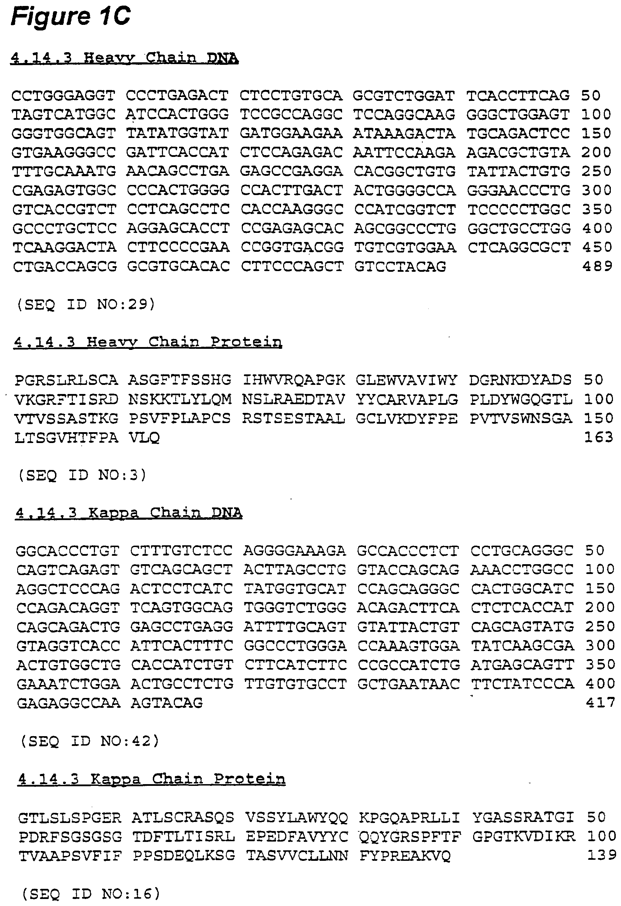

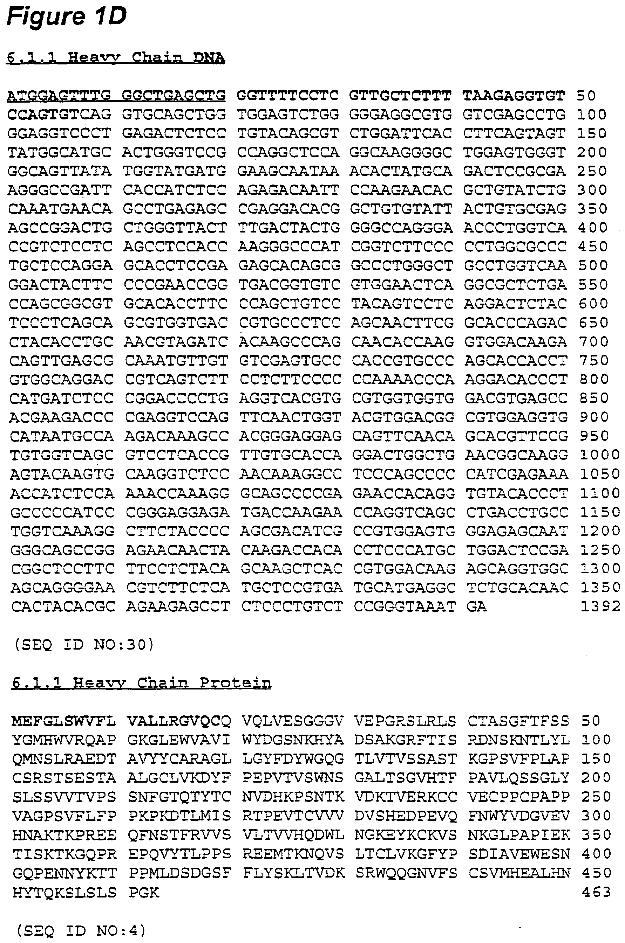

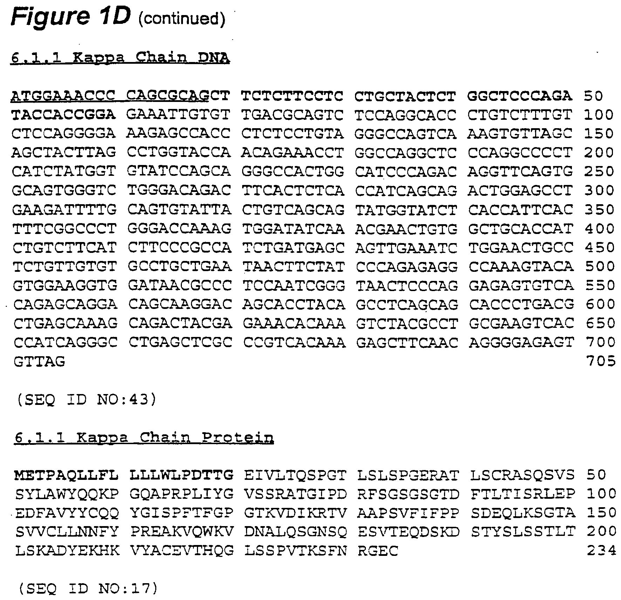

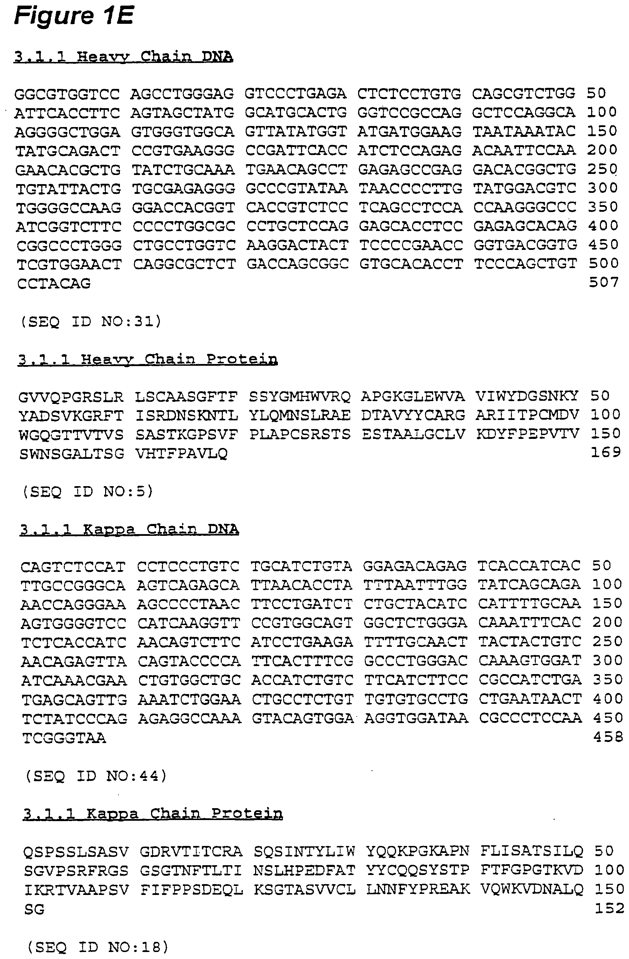

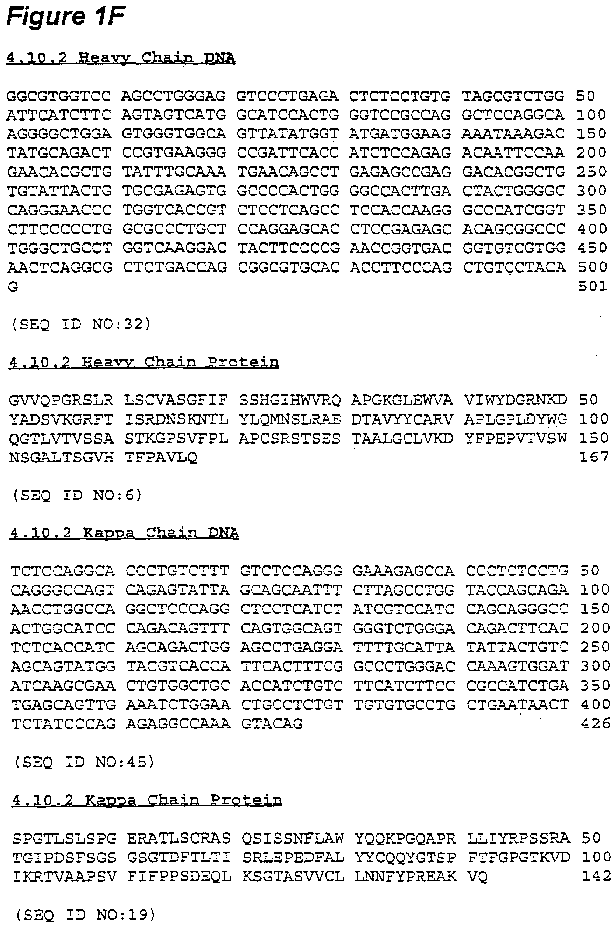

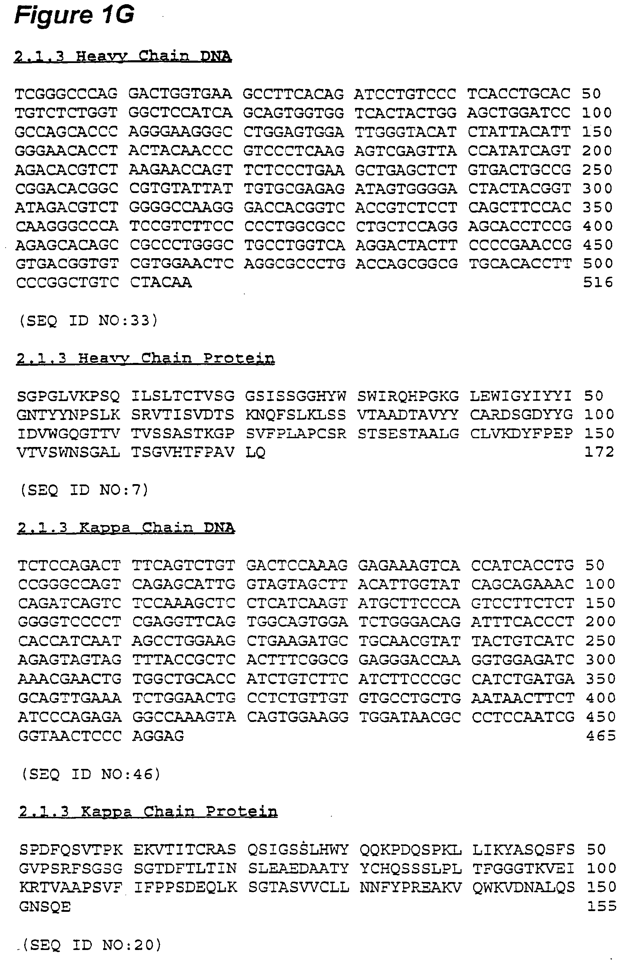

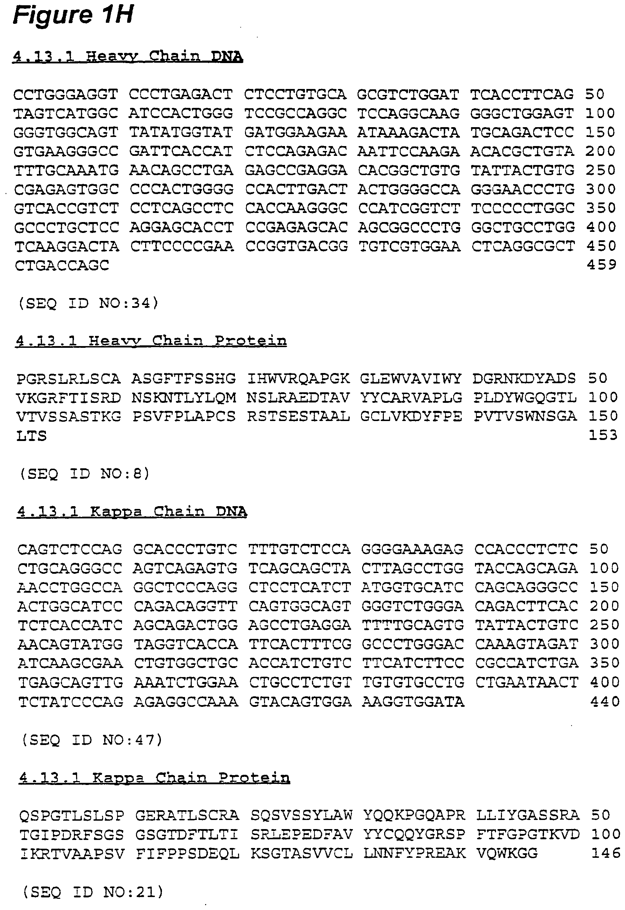

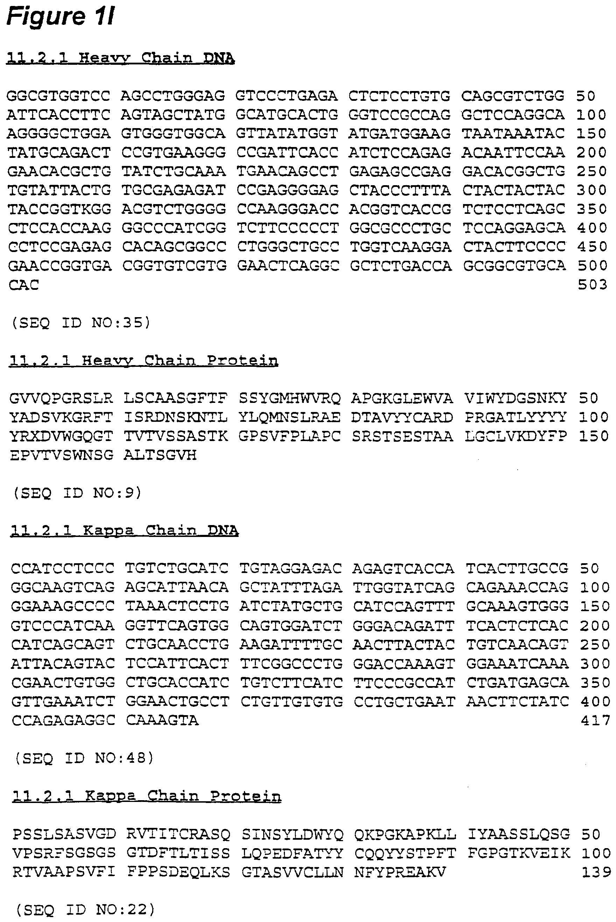

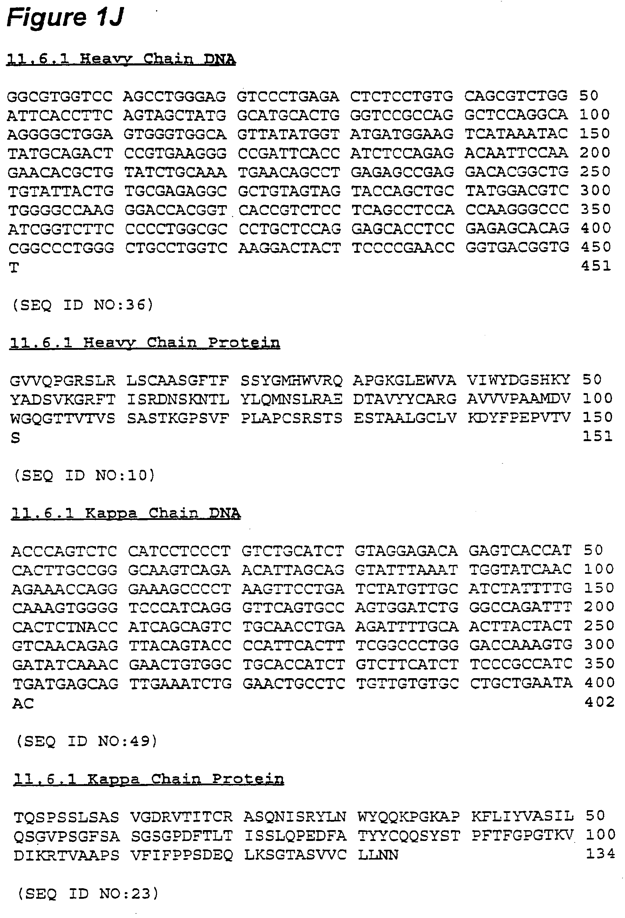

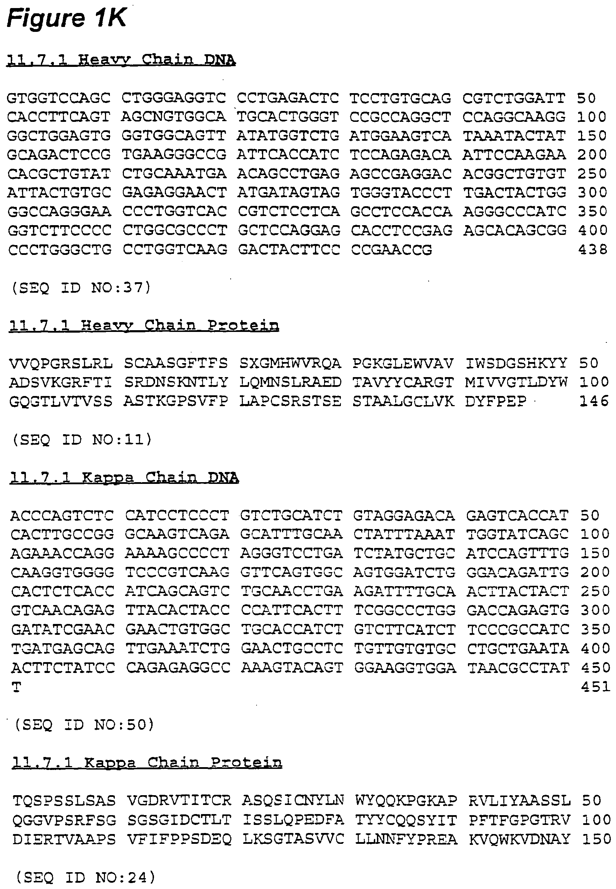

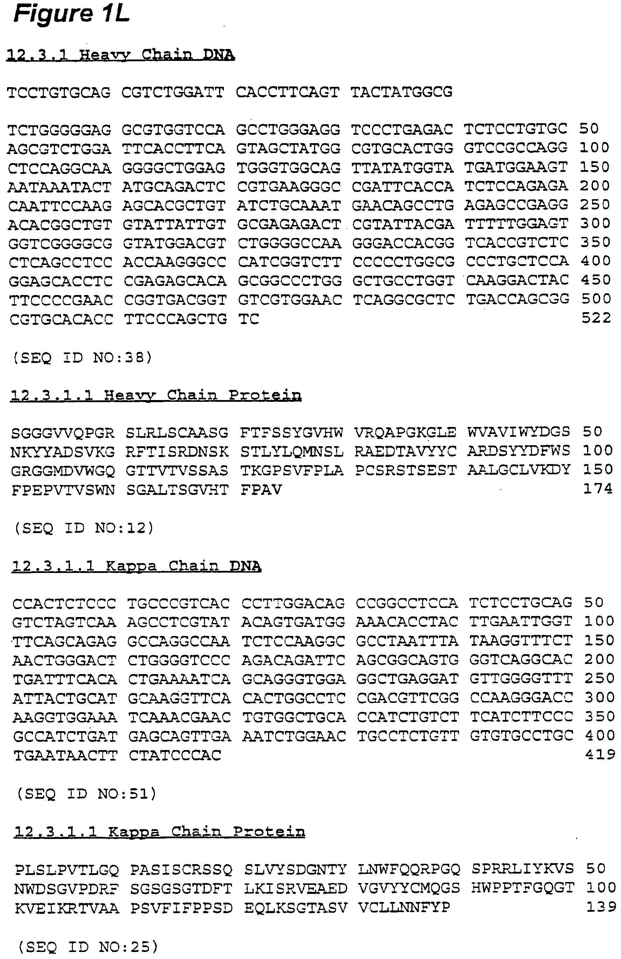

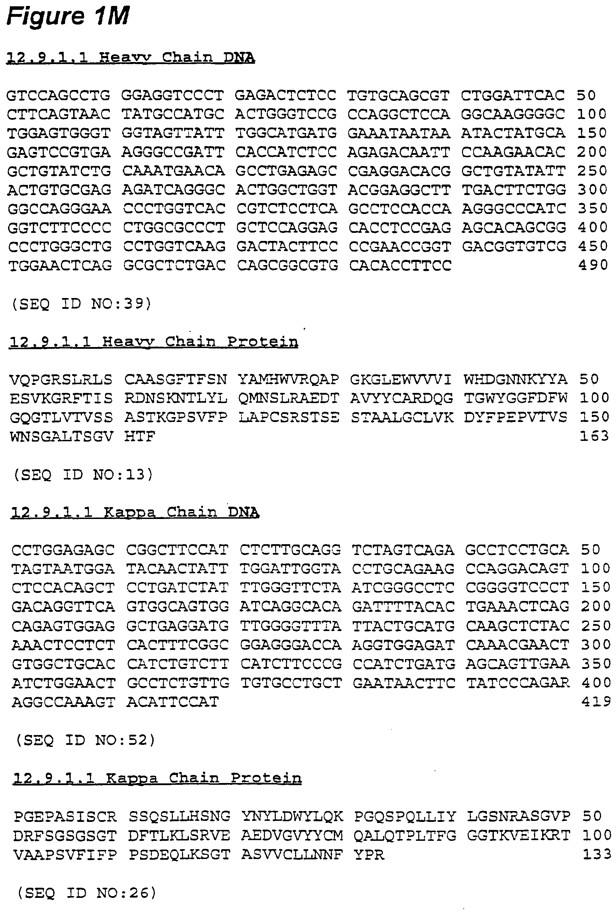

The term "polypeptide" as used herein as a generic term to refer to native protein, fragments, or analogs of a polypeptide sequence. Hence, native protein, fragments, and analogs are species of the polypeptide genus. Preferred polypeptides in accordance with the invention comprise the human heavy chain immunoglobulin molecules and the human kappa light chain immunoglobulin molecules represented in Figure 1, as well as antibody molecules formed by combinations comprising the heavy chain immunoglobulin molecules with light chain immunoglobulin molecules, such as the kappa light chain immunoglobulin molecules, and vice versa, as well as fragments and analogs thereof.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "operably linked" as used herein refers to positions of components so described are in a relationship permitting them to function in their intended manner. A control sequence "operably linked" to a coding sequence is ligated in such a way that expression of the coding sequence is achieved under conditions compatible with the control sequences.

The term "control sequence" as used herein refers to polynucleotide sequences which are necessary to effect the expression and processing of coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is essential for expression and processing, and can also include additional components whose presence is advantageous, for example, leader sequences and fusion partner sequences.

The term "polynucleotide" as referred to herein means a polymeric form of nucleotides of at least 10 bases in length, either ribonucleotides or deoxynucleotides or a modified form of either type of nucleotide. The term includes single and double stranded forms of DNA.

The term "oligonucleotide" referred to herein includes naturally occurring, and modified nucleotides linked together by naturally occurring, and non-naturally occurring oligonucleotide linkages. Oligonucleotides are a polynucleotide subset generally comprising a length of 200 bases or fewer. Preferably oligonucleotides are 10 to 60 bases in length and most preferably 12, 13, 14, 15, 16, 17, 18, 19, or 20 to 40 bases in length. Oligonucleotides are usually single stranded, e.g. for probes; although oligonucleotides may be double stranded, e.g. for use in the construction of a gene mutant. Oligonucleotides of the disclosure can be either sense or antisense oligonucleotides.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phosphoroselenoate, phosphorodiselenoate, phosphoroanilothioate, phoshoraniladate, phosphoroamidate, and the like. *See e.g.,* LaPlanche et al. Nucl. Acids Res. 14:9081 (1986); Stec et al. J. Am. Chem. Soc. 106:6077 (1984); Stein et al. Nucl. Acids Res. 16:3209 (1988); Zon et al. Anti-Cancer Drug Design 6:539 (1991); Zon et al. Oligonucleotides and Analogues: A Practical Approach, pp. 87-108 (F. Eckstein, Ed., Oxford University Press, Oxford England (1991)); Stec et al. U.S. Patent No. 5,151,510; Uhlmann and Peyman Chemical Reviews 90:543 (1990). An oligonucleotide can include a label for detection, if desired.

The term "selectively hybridize" referred to herein means to detectably and specifically bind. Polynucleotides, oligonucleotides and fragments thereof in accordance with the invention selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments of the invention and a nucleic acid sequence of interest will be at least 80%, and more typically with preferably increasing homologies of at least 85%, 90%, 95%, 99%, and 100%. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. *See* Dayhoff, M.O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program. The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotide or amino acid sequences: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence. Generally, a reference sequence is at least 18 nucleotides or 6 amino acids in length, frequently at least 24 nucleotides or 8 amino acids in length, and often at least 48 nucleotides or 16 amino acids in length. Since two polynucleotides or amino acid sequences may each (1) comprise a sequence (i.e., a portion of the complete polynucleotide or amino acid sequence) that is similar between the two molecules, and (2) may further comprise a sequence that is divergent between the two polynucleotides or amino acid sequences, sequence comparisons between two (or more) molecules are typically performed by comparing sequences of the two molecules over a "comparison window" to identify and compare local regions of sequence similarity. A "comparison window", as used herein, refers to a conceptual segment of at least 18 contiguous nucleotide positions or 6 amino acids wherein a polynucleotide sequence or amino acid sequence may be compared to a reference sequence of at least 18 contiguous nucleotides or 6 amino acid sequences and wherein the portion of the polynucleotide sequence in the comparison window may comprise additions, deletions, substitutions, and the like (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman Adv. Appl. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85:2444 (1988), by computerized implementations of these algorithms (GAP, BESTFIT, FASTA, and TFASTA in the Wisconsin Genetics Software Package Release 7.0, (Genetics Computer Group, 575 Science Dr., Madison, Wis.), Geneworks, or MacVector software packages), or by inspection, and the best alignment (i.e., resulting in the highest percentage of homology over the comparison window) generated by the various methods is selected.

The term "sequence identity" means that two polynucleotide or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See* Immunology - A Synthesis (2nd Edition, E.S. Golub and D.R. Gren, Eds., Sinauer Associates, Sunderland, Mass. (1991)), Stereoisomers (e.g., D-amino acids) of the twenty conventional amino acids, unnatural amino acids such as α-, α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the lefthand direction is the amino terminal direction and the righthand direction is the carboxy-terminal direction, in accordance with standard usage and convention.

Similarly, unless specified otherwise, the lefthand end of single-stranded polynucleotide sequences is the 5' end; the lefthand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity, and most preferably at least 99 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

As discussed herein, minor variations in the amino acid sequences of antibodies or immunoglobulin molecules are contemplated as being encompassed by the present invention, providing that the variations in the amino acid sequence maintain at least 75%, more preferably at least 80%, 90%, 95%, and most preferably 99%. In particular, conservative amino acid replacements are contemplated. Conservative replacements are those that take place within a family of amino acids that are related in their side chains. Genetically encoded amino acids are generally divided into families: (1) acidic=aspartate, glutamate; (2) basic=lysine, arginine, histidine; (3) nonpolar=alanine, valine, leucine, isoleucine, proline, phenylalanine, methionine, tryptophan; and (4) uncharged polar=glycine, asparagine, glutamine, cysteine, serine, threonine, tyrosine. More preferred families are: serine and threonine are aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid within a framework site. Whether an amino acid change results in a functional peptide can readily be determined by assaying the specific activity of the polypeptide derivative. Assays are described in detail herein. Fragments or analogs of antibodies or immunoglobulin molecules can be readily prepared by those of ordinary skill in the art. Preferred amino- and carboxy-termini of fragments or analogs occur near boundaries of functional domains. Structural and functional domains can be identified by comparison of the nucleotide and/or amino acid sequence data to public or proprietary sequence databases. Preferably, computerized comparison methods are used to identify sequence motifs or predicted protein conformation domains that occur in other proteins of known structure and/or function. Methods to identify protein sequences that fold into a known three-dimensional structure are known. Bowie et al. Science 253:164 (1991). Thus, the foregoing examples demonstrate that those of skill in the art can recognize sequence motifs and structural conformations that may be used to define structural and functional domains in accordance with the invention.

Preferred amino acid substitutions are those which: (1) reduce susceptibility to proteolysis, (2) reduce susceptibility to oxidation, (3) alter binding affinity for forming protein complexes, (4) alter binding affinities, and (4) confer or modify other physicochemical or functional properties of such analogs. Analogs can include various muteins of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts. A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et at. Nature 354:105 (1991).

The term "polypeptide fragment" as used herein refers to a polypeptide that has an amino-terminal and/or carboxy-terminal deletion, but where the remaining amino acid sequence is identical to the corresponding positions in the naturally-occurring sequence deduced, for example, from a full-length cDNA sequence. Fragments typically are at least 5, 6, 8 or 10 amino acids long, preferably at least 14 amino acids long, more preferably at least 20 amino acids long, usually at least 50 amino acids long, and even more preferably at least 70 amino acids long. The term "analog" as used herein refers to polypeptides which are comprised of a segment of at least 25 amino acids that has substantial identity to a portion of a deduced amino acid sequence and which has at least one of the following properties: (1) specific binding to CTLA-4, under suitable binding conditions, (2) ability to block CTLA-4 binding with its receptors, or (3) ability to inhibit CTLA-4 expressing cell growth *in vitro* or *in vivo.* Typically, polypeptide analogs comprise a conservative amino acid substitution (or addition or deletion) with respect to the naturally-occurring sequence. Analogs typically are at least 20 amino acids long, preferably at least 50 amino acids long or longer, and can often be as long as a full-length naturally-occurring polypeptide.

Peptide analogs are commonly used in the pharmaceutical industry as non-peptide drus with properties analogous to those of the template peptide. These types of non-peptide compound are termed "peptide mimetics" or "peptidomimetics". Fauchere, J. Adv. Drug Res. 15:29 (1986); Veber and Freidinger TINS p.392 (1985); and Evans et al. J. Med. Chem. 30:1229 (1987). Such compounds are often developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biochemical property or pharmacological activity), such as human antibody, but have one or more peptide linkages optionally replaced by a linkage selected from the group consisting of: --CH₂NH--, --CH₂S--,-CH₂-CH₂--, --CH=CH--(cis and trans), --COCH₂--, --CH(OH)CH₂--, and-CH₂SO--, by methods well known in the art. Systematic substitution of one or more amino acids of a consensus sequence with a D-amino acid of the same type (e.g., D-lysine in place of L-lysine) may be used to generate more stable peptides. In addition, constrained peptides comprising a consensus sequence or a substantially identical consensus sequence variation may be generated by methods known in the art (Rizo and Gierasch Ann. Rev. Biochem. 61:387 (1992),); for example, by adding internal cysteine residues capable of forming intramolecular disulfide bridges which cyclize the peptide.

"Antibody" or "antibody peptide(s)" refer to an intact antibody, or a binding fragment thereof that competes with the intact antibody for specific binding. Binding fragments are produced by recombinant DNA techniques, or by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody substantially inhibits adhesion of a receptor to a counterreceptor when an excess of antibody reduces the quantity of receptor bound to counterreceptor by at least about 20%, 40%, 60% or 80%, and more usually greater than about 85% (as measured in an *in vitro* competitive binding assay).

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is ≤1 µM, preferably ≤ 100 nM and most preferably ≤ 10 nM.

The term "agent" is used herein to denote a chemical compound, a mixture of chemical compounds, a biological macromolecule, or an extract made from biological materials.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). In certain situations, the label or marker can also be therapeutic. Various methods of labeling polypeptides and glycoproteins are known in the art and may be used. Examples of labels for polypeptides include, but are not limited to, the following: radioisotopes or radionuclides (*e.g*., ³H, ¹⁴C, ¹⁵N, ³⁵S, ⁹⁰Y, ⁹⁹Tc, ¹¹¹In, ¹²⁵I, ¹³¹I), fluorescent labels (*e.g.,* FITC, rhodamine, lanthanide phosphors), enzymatic labels (*e.g*., horseradish peroxidase, β-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)),).

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

The term patient includes human and veterinary subjects.

### Antibody Structure

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. *See generally,* Fundamental Immunology Ch. 7 (Paul, W., ed., 2nd ed. Raven Press, N.Y. (1989)).
The variable regions of each light/heavy chain pair form the antibody binding site.

Thus, an intact IgG antibody has two binding sites. Except in bifunctional or bispecific antibodies, the two binding sites are the same.

The chains all exhibit the same general structure of relatively conserved framework regions (FR) joined by three hyper variable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of Kabat Sequences of Proteins of Immunological Interest (National Institutes of Health, Bethesda, Md. (1987 and 1991)), or Chothia & Lesk J. Mol. Biol. 196:901-917 (1987); Chothia et al. Nature 342:878-883 (1989).

A bispecific or bifunctional antibody is an artificial hybrid antibody having two different heavy/light chain pairs and two different binding sites. Bispecific antibodies can be produced by a variety of methods including fusion of hybridomas or linking of Fab' fragments. *See, e.g.,* Songsivilai & Lachmann Clin. Exp. Immunol. 79: 315-321 (1990), Kostelny et al. J. Immunol. 148:1547-1553 (1992). In addition, bispecific antibodies may be formed as "diabodies" (Holliger et al. "'Diabodies': small bivalent and bispecific antibody fragments" PNAS USA 90:6444-6448 (1993)) or "Janusins" (Traunecker et al. "Bispecific single chain molecules (Janusins) target cytotoxic lymphocytes on HIV infected cells" EMBO J 10:3655-3659 (1991) and Traunecker et al. "Janusin: new molecular design for bispecific reagents" Int J Cancer Suppl 7:51-52 (1992)). Production of bispecific antibodies can be a relatively labor intensive process compared with production of conventional antibodies and yields and degree of purity are generally lower for bispecific antibodies. Bispecific antibodies do not exist in the form of fragments having a single binding site (e.g., Fab, Fab', and Fv).

### Human Antibodies and Humanization of Antibodies

Human antibodies avoid certain of the problems associated with antibodies that possess murine or rat variable and/or constant regions. The presence of such murine or rat derived proteins can lead to the rapid clearance of the antibodies or can lead to the generation of an immune response against the antibody by a patient. In order to avoid the utilization of murine or rat derived antibodies, it has been postulated that one can develop humanized antibodies or generate fully human antibodies through the introduction of human antibody function into a rodent so that the rodent would produce antibodies having fully human sequences.

### Human Antibodies

The ability to clone and reconstruct megabase-sized human loci in YACs and to introduce them into the mouse germline provides a powerful approach to elucidating the functional components of very large or crudely mapped loci as well as generating useful models of human disease. Furthermore, the utilization of such technology for substitution of mouse loci with their human equivalents could provide unique insights into the expression and regulation of human gene products during development, their communication with other systems, and their involvement in disease induction and progression.

An important practical application of such a strategy is the "humanization" of the mouse humoral immune system. Introduction of human immunoglobulin (Ig) loci into mice in which the endogenous Ig genes have been inactivated offers the opportunity to study the mechanisms underlying programmed expression and assembly of antibodies as well as their role in B-cell development. Furthermore, such a strategy could provide an ideal source for production of fully human monoclonal antibodies (Mabs) an important milestone towards fulfilling the promise of antibody therapy in human disease. Fully human antibodies are expected to minimize the immunogenic and allergic responses intrinsic to mouse or mouse-derivatized Mabs and thus to increase the efficacy and safety of the administered antibodies. The use of fully human antibodies can be expected to provide a substantial advantage in the treatment of chronic and recurring human diseases, such as inflammation, autoimmunity, and cancer, which require repeated antibody administrations.

One approach towards this goal was to engineer mouse strains deficient in mouse antibody production with large fragments of the human Ig loci in anticipation that such mice would produce a large repertoire of human antibodies in the absence of mouse antibodies. Large human Ig fragments would preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains should yield high affinity antibodies against any antigen of interest, including human antigens. Using the hybridoma technology, antigen-specific human Mabs with the desired specificity could be readily produced and selected.

This general strategy was demonstrated in connection with our generation of the first XenoMouse™ strains as published in 1994. *See* Green et al. Nature Genetics 7:13-21 (1994). The XenoMouse™ strains were engineered with yeast artificial chromosomes (YACs) containing 245 kb and 190 kb-sized germline configuration fragments of the human heavy chain locus and kappa light chain locus, respectively, which contained core variable and constant region sequences. *Id.* The human Ig containing YACs proved to be compatible with the mouse system for both rearrangement and expression of antibodies and were capable of substituting for the inactivated mouse Ig genes. This was demonstrated by their ability to induce B-cell development, to produce an adult-like human repertoire of fully human antibodies, and to generate antigen-specific human Mabs. These results also suggested that introduction of larger portions of the human Ig loci containing greater numbers of V genes, additional regulatory elements, and human Ig constant regions might recapitulate substantially the full repertoire that is characteristic of the human humoral response to infection and immunization. The work of Green et al. was recently extended to the introduction of greater than approximately 80% of the human antibody repertoire through introduction of megabase sized, germline configuration YAC fragments of the human heavy chain loci and kappa light chain loci, respectively, to produce XenoMouse™ mice. *See* Mendez et al. Nature Genetics 15:146-156 (1997), Green and Jakobovits J. Exp. Med. 188:483-495 (1998), and U.S. Patent Application Serial No. 08/759,620, filed December 3, 1996.

Such approach is further discussed and delineated in Mendez et al. Nature Genetics 15:146-156 (1997) and Green and Jakobovits J. Exp. Med. 188:483-495 (1998). *See also* European Patent No., EP 0 463 151 B1, grant published June 12, 1996, International Patent Application No., WO 94/02602, published February 3, 1994, International Patent Application No., WO 96/34096, published October 31, 1996, and WO 98/24893, published June 11, 1998.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more V_{H} genes, one or more D_{H} genes, one or more J_{H} genes, a mu constant region, and a second constant region (preferably a gamma constant region) are formed into a construct for insertion into an animal. This approach is described in U.S. Patent No. 5,545,807 to Surani et al. and U.S. Patent Nos. 5,545,806, 5,625,825, 5,625,126, 5,633,425, 5,661,016, 5,770,429, 5,789,650, and 5,814,318 each to Lonberg and Kay, U.S. Patent No. 5,591,669 to Krimpenfort and Berns, U.S. Patent Nos. 5,612,205, 5,721,367, 5,789,215 to Berns et al., and U.S. Patent No. 5,643,763 to Choi and Dunn, 546 073 B1, International Patent Application Nos. WO 92/03918, WO 92/22645, WO 92/22647, WO 92122670, WO 93/12227, WO 94/00569, WO 94/25585, WO 96/14436, WO 97/13852, and WO 98/24884, the disclosures of which are hereby incorporated by reference in their entirety. *See further* Taylor et al., 1992, Chen et al., 1993, Tuaillon et al., 1993, Choi et al., 1993, Lonberg et al., (1994), Taylor et al., (1994), and Tuaillon et al., (1995), Fishwild et al., (1996).

An advantage of the minilocus approach is the rapidity with which constructs including portions of the Ig locus can be generated and introduced into animals. Commensurately, however, a significant disadvantage of the minilocus approach is that, in theory, insufficient diversity is introduced through the inclusion of small numbers of V, D, and J genes. Indeed, the published work appears to support this concern. B-cell development and antibody production of animals produced through use of the minilocus approach appear stunted. Therefore, research surrounding the present invention has consistently been directed towards the introduction of large portions of the Ig locus in order to achieve greater diversity and in an effort to reconstitute the immune repertoire of the animals.

Human anti-mouse antibody (HAMA) responses have led the industry to prepare chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. Thus, it would be desirable to provide fully human antibodies against CTLA-4 in order to vitiate concerns and/or effects of HAMA or HACA response.

### Humanization and Display Technologies

As was discussed above in connection with human antibody generation, there are advantages to producing antibodies with reduced immunogenicity. To a degree, this can be accomplished in connection with techniques of humanization and display techniques using appropriate libraries. It will be appreciated that murine antibodies or antibodies from other species can be humanized or primatized using techniques well known in the art. *See e.g.,* Winter and Harris Immunol Today 14:43-46 (1993) and Wright et al. Crit. Reviews in Immunol. 12125-168 (1992). The antibody of interest may be engineered by recombinant DNA techniques to substitute the CH1, CH2, CH3, hinge domains, and/or the framework domain with the corresponding human sequence (*see* WO 92/02190 and U.S. Patent Nos. 5,530,101, 5,585,089, 5,693,761, 5,693,792, 5,714,350, and 5,777,085). Also, the use of Ig cDNA for construction of chimeric immunoglobulin genes is known in the art (Liu et al. P.N.A.S. 84:3439 (1987) and J.Immunol.139:3521 (1987)). mRNA is isolated from a hybridoma or other cell producing the antibody and used to produce cDNA. The cDNA of interest may be amplified by the polymerase chain reaction using specific primers (U.S. Pat. Nos. 4,683,195 and 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Preferred isotypes are IgG1, IgG2, IgG3 and IgG4. Particularly preferred isotypes for antibodies of the invention are IgG2 and IgG4. Either of the human light chain constant regions, kappa or lambda, may be used. The chimeric, humanized antibody is then expressed by conventional methods.

Antibody fragments, such as Fv, F(ab')₂ and Fab may be prepared by cleavage of the intact protein, e.g. by protease or chemical cleavage. Alternatively, a truncated gene is designed. For example, a chimeric gene encoding a portion of the F(ab')₂ fragment would include DNA sequences encoding the CH1 domain and hinge region of the H chain, followed by a translational stop codon to yield the truncated molecule.

In one approach, consensus sequences encoding the heavy and light chain J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, cosmids, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, including retroviral LTRs, e.g. SV-40 early promoter, (Okayama et al. Mol. Cell. Bio. 3:280 (1983)), Rous sarcoma virus LTR (Gorman et al. P.N.A.S. 79:6777 (1982)), and moloney murine leukemia virus LTR (Grosschedl et al. Cell 41:885 (1985)); native 1g promoters, etc.

Further, human antibodies or antibodies from other species can be generated through display-type technologies, including, without limitation, phage display, retroviral display, ribosomal display, and other techniques, using techniques well known in the art and the resulting molecules can be subjected to additional maturation, such as affinity maturation, as such techniques are well known in the art. Wright and Harris, *supra.,* Hanes and Plucthau PNAS USA 94:4937-4942 (1997) (ribosomal display), Parmley and Smith Gene 73:305-318 (1988) (phage display), Scott TIBS 17:241-245 (1992), Cwirla et al. PNAS USA 87:6378-6382 (1990), Russel et al. Nucl. Acids Research 21:1081-1085 (1993), Hoganboom et al. Immunol. Reviews 130:43-68 (1992), Chiswell and McCafferty TIBTECH 10:80-84 (1992), and U.S. Patent No. 5,733,743. If display technologies are utilized to produce antibodies that are not human, such antibodies can be humanized as described above.

Using these techniques, antibodies can be generated to CTLA-4 expressing cells, CTLA-4 itself, forms of CTLA-4, epitopes or peptides thereof, and expression libraries thereto (*see e.g.* U.S. Patent No. 5,703,057) which can thereafter be screened as described above for the activities described above.

### Additional Criteria for Antibody Therapeutics

As will be appreciated, it is generally not desirable to kill CTLA-4 expressing cells. Rather, one generally desires to simply inhibit CTLA-4 binding with its ligands to mitigate T cell down regulation. One of the major mechanisms through which antibodies kill cells is through fixation of complement and participation in CDC. The constant region of an antibody plays an important role in connection with an antibody's ability to fix complement and participate in CDC. Thus, generally one selects the isotype of an antibody to either provide the ability of complement fixation, or not. In the case of the present invention, generally, as mentioned above, it is generally not preferred to utilize an antibody that kills the cells. There are a number of isotypes of antibodies that are capable of complement fixation and CDC, including, without limitation, the following: murine IgM, murine IgG2a, murine IgG2b, murine IgG3, human IgM, human IgG1, and human IgG3. Those isotypes that do not include, without limitation, human IgG2 and human IgG4.

It will be appreciated that antibodies that are generated need not initially possess a particular desired isotype but, rather, the antibody as generated can possess any isotype and the antibody can be isotype switched thereafter using conventional techniques that are well known in the art. Such techniques include the use of direct recombinant techniques (*see e.g.,* U.S. Patent No. 4,816,397), cell-cell fusion techniques (*see e.g.,* U.S. Patent Application No. 08/730,639, filed October 11, 1996), among others.

In the cell-cell fusion technique, a myeloma or other cell line is prepared that possesses a heavy chain with any desired isotype and another myeloma or other cell line is prepared that possesses the light chain. Such cells can, thereafter, be fused and a cell line expressing an intact antibody can be isolated.

By way of example, the majority of the CTLA-4 antibodies discussed herein are human anti-CTLA-4 IgG2 antibody. Since such antibodies possess desired binding to the CTLA-4 molecule, any one of such antibodies can be readily isotype switched to generate a human IgG4 isotype, for example, while still possessing the same variable region (which defines the antibody's specificity and some of its affinity).

Accordingly, as antibody candidates are generated that meet desired "structural" attributes as discussed above, they can generally be provided with at least certain additional "functional" attributes that are desired through isotype switching.

### Design and Generation of Other Therapeutics

In accordance with the present invention and based on the activity of the antibodies that are produced and characterized herein with respect to CTLA-4, the design of other therapeutic modalities including other antibodies, other antagonists, or chemical moieties other than antibodies is facilitated. Such modalities include, without limitation, antibodies having similar binding activity or functionality, advanced antibody therapeutics, such as bispecific antibodies, immunotoxins, and radiolabeled therapeutics, generation of peptide therapeutics, gene therapies, particularly intrabodies, antisense therapeutics, and small molecules. Furthermore, as discussed above, the effector function of the antibodies of the invention may be changed by isotype switching to an IgG1, IgG2, IgG3, IgG4, IgD, IgA, IgE, or IgM for various therapeutic uses.

In connection with the generation of advanced antibody therapeutics, where complement fixation is a desirable attribute, it may be possible to sidestep the dependence on complement for cell killing through the use of bispecifics, immunotoxins, or radiolabels, for example.

In connection with bispecific antibodies, bispecific antibodies can be generated that comprise (i) two antibodies one with a specificity to CTLA-4 and another to a second molecule that are conjugated together, (ii) a single antibody that has one chain specific to CTLA-4 and a second chain specific to a second molecule, or (iii) a single chain antibody that has specificity to CTLA-4 and the other molecule. Such bispecific antibodies can be generated using techniques that are well known for example, in connection with (i) and (ii) *see e.g.,* Fanger et al. Immunol Methods 4:72-81 (1994) and Wright and Harris, *supra.* and in connection with (iii) *see e.g.,* Traunecker et al. Int. J. Cancer (Suppl.) 7:51-52 (1992).

In addition, "Kappabodies" (Ill et al. "Design and construction of a hybrid immunoglobulin domain with properties of both heavy and light chain variable regions" Protein Eng 10:949-57 (1997)), "Minibodies" (Martin et al. "The affinity-selection of a minibody polypeptide inhibitor of human interleukin-6" EMBO J 13:5303-9 (1994)), "Diabodies" (Holliger et al. "'Diabodies': small bivalent and bispecific antibody fragments" PNAS USA 90:6444-6448 (1993)), or "Janusins" (Traunecker et al. "Bispecific single chain molecules (Janusins) target cytotoxic lymphocytes on HIV infected cells" EMBO J 10:3655-3659 (1991) and Traunecker et al. "Janusin: new molecular design for bispecific reagents" Int J Cancer Suppl 7:51-52 (1992)) may also be prepared.

In connection with immunotoxins, antibodies can be modified to act as immunotoxins utilizing techniques that are well known in the art. *See e.g.,* Vitetta Immunol Today 14:252 (1993). *See also* U.S. Patent No. 5,194,594. In connection with the preparation of radiolabeled antibodies, such modified antibodies can also be readily prepared utilizing techniques that are well known in the art. *See e.g.,* Junghans et al. in Cancer Chemotherapy and Biotherapy 655-686 (2d edition, Chafner and Longo, eds., Lippincott Raven (1996)). *See also* U.S. Patent Nos. 4,681,581, 4,735,210, 5,101,827, 5,102,990 (RE 35,500), 5,648,471, and 5,697,902. Each of immunotoxins and radiolabeled molecules would be likely to kill cells expressing CTLA-4, and particularly those cells in which the antibodies of the invention are effective.

In connection with the generation of therapeutic peptides, through the utilization of structural information related to CTLA-4 and antibodies thereto, such as the antibodies of the invention (as discussed below in connection with small molecules) or screening of peptide libraries, therapeutic peptides can be generated that are directed against CTLA-4. Design and screening of peptide therapeutics is discussed in connection with Houghten et al. Biotechniques 13:412-421 (1992), Houghten PNAS USA 82:5131-5135 (1985), Pinalla et al. Biotechniques 13:901-905 (1992), Blake and Litzi-Davis BioConjugate Chem. 3:510-513 (1992). Immunotoxins and radiolabeled molecules can also be prepared, and in a similar manner, in connection with peptidic moieties as discussed above in connection with antibodies.

Important information related to the binding of an antibody to an antigen can be gleaned through phage display experimentation. Such experiments are generally accomplished through panning a phage library expressing random peptides for binding with the antibodies of the invention to determine if peptides can be isolated that bind. If successful, certain epitope information can be gleaned from the peptides that bind.

In general, phage libraries expressing random peptides can be purchased from New England Biolabs (7-mer and 12-mer libraries, Ph.D.-7 Peptide 7-mer Library Kit and Ph.D.-12 Peptide 12-mer Library Kit, respectively) based on a bacteriophage M13 system. The 7-mer library represents a diversity of approximately 2.0 x 10⁹ independent clones, which represents most, if not all, of the 20⁷ = 1.28 x 10⁹ possible 7-mer sequences. The 12-mer library contains approximately 1.9 x 10⁹ independent clones and represents only a very small sampling of the potential sequence space of 20¹² = 4.1 x 10¹⁵ 12-mer sequences. Each of 7-mer and 12-mer libraries are panned or screened in accordance with the manufacturer's recommendations in which plates were coated with an antibody to capture the appropriate antibody (a goat anti-human IgG Fc for an IgG antibody for example) followed by washing. Bound phage are eluted with 0.2 M glycine-HCl, pH 2.2. After 3 rounds of selection/amplification at constant stringency (0.5% Tween), through use of DNA sequencing, one can characterize clones from the libraries that are reactive with one or more of the antibodies. Reactivity of the peptides can be determined by ELISA. For an additional discussion of epitope analysis of peptides *see also* Scott, J.K. and Smith, G.P. Science 249:386-390 (1990); Cwirla et al. PNAS USA 87:6378-6382 (1990); Felici et al. J. Mol. Biol. 222:301-310 (1991), and Kuwabara et al. Nature Biotechnology 15:74-78 (1997).

The design of gene and/or antisense therapeutics through conventional techniques is also facilitated through the present invention. Such modalities can be utilized for modulating the function of CTLA-4. In connection therewith the antibodies of the present invention facilitate design and use of functional assays related thereto. A design and strategy for antisense therapeutics is discussed in detail in International Patent Application No. WO 94/29444. Design and strategies for gene therapy are well known. However, in particular, the use of gene therapeutic techniques involving intrabodies could prove to be particularly advantageous. *See e.g.,* Chen et al. Human Gene Therapy 5:595-601 (1994) and Marasco Gene Therapy 4:11-15 (1997). General design of and considerations related to gene therapeutics is also discussed in International Patent Application No. WO 97/38137. Genetic materials encoding an antibody of the invention (such as the 4.1.1, 4.8.1, or 6.1.1, or others) may be included in a suitable expression system (whether viral, attenuated viral, non-viral, naked, or otherwise) and administered to a host for *in vivo* generation of the antibody in the host.

Small molecule therapeutics can also be envisioned in accordance with the present invention. Drugs can be designed to modulate the activity of CTLA-4 based upon the present invention. Knowledge gleaned from the structure of the CTLA-4 molecule and its interactions with other molecules in accordance with the present invention, such as the antibodies of the invention, CD28, B7, B7-1, B7-2, and others can be utilized to rationally design additional therapeutic modalities. In this regard, rational drug design techniques such as X-ray crystallography, computer-aided (or assisted) molecular modeling (CAMM), quantitative or qualitative structure-activity relationship (QSAR), and similar technologies can be utilized to focus drug discovery efforts. Rational design allows prediction of protein or synthetic structures which can interact with the molecule or specific forms thereof which can be used to modify or modulate the activity of CTLA-4. Such structures can be synthesized chemically or expressed in biological systems. This approach has been reviewed in Capsey et al. Genetically Engineered Human Therapeutic Drugs (Stockton Press, NY (1988)). Indeed, the rational design of molecules (either peptides, peptidomimetics, small molecules, or the like) based upon known, or delineated, structure-activity relationships with other molecules (such as antibodies in accordance with the invention) has become generally routine. *See, e.g.,* Fry et al. "Specific, irreversible inactivation of the epidermal growth factor receptor and erbB2, by a new class of tyrosine kinase inhibitor" Proc Natl Acad Sci U S A 95:12022-7 (1998); Hoffman et al. "A model of Cdc25 phosphatase catalytic domain and- Cdk-interaction surface based on the presence of a rhodanese homology domain" J Mol Biol 282:195-208 (1998); Ginalski et al. "Modelling of active forms of protein kinases: p38--a case study" Acta Biochim Pol 44:557-64 (1997); Jouko et al. "Identification of csk tyrosine phosphorylation sites and a tyrosine residue important for kinase domain structure" Biochem J 322:927-35 (1997); Singh et al. "Structure-based design of a potent, selective, and irreversible inhibitor of the catalytic domain of the erbB receptor subfamily of protein tyrosine kinases" J Med Chem 40:1130-5 (1997); Mandel et al. "ABGEN: a knowledge-based automated approach for antibody structure modeling" Nat Biotechnol 14:323-8 (1996); Monfardini et al. "Rational design, analysis, and potential utility of GM-CSF antagonists" Proc Assoc Am Physicians 108:420-31 (1996); Furet et al. "Modelling study of protein kinase inhibitors: binding mode of staurosporine and origin of the selectivity of CGP 52411" J Comput Aided Mol Des 9:465-72 (1995).

Further, combinatorial libraries can be designed and sythesized and used in screening programs, such as high throughput screening efforts.

### Therapeutic Administration and Formulations

It will be appreciated that administration of therapeutic entities in accordance with the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences (15th ed, Mack Publishing Company, Easton, PA (1975)), particularly Chapter 87 by Blaug, Seymour, therein. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies in accordance with the present invention, provided that the active ingredient in the formulation is not inactivated by the formulation and the formulation is physiologically compatible and tolerable with the route of administration. *See also* Powell et al. "Compendium of excipients for parenteral formulations" PDA J Pharm Sci Technol. 52:238-311 (1998) and the citations therein for additional information related to excipients and carriers well known to pharmaceutical chemists.

### Preparation of Antibodies

Antibodies in accordance with the invention are preferably prepared through the utilization of a transgenic mouse that has a substantial portion of the human antibody producing genome inserted but that is rendered deficient in the production of endogenous, murine, antibodies. Such mice, then, are capable of producing human immunoglobulin molecules and antibodies and are deficient in the production of murine immunoglobulin molecules and antibodies. Technologies utilized for achieving the same are disclosed in the patents, applications, and references disclosed in the Background, herein. In particular, however, a preferred embodiment of transgenic production of mice and antibodies therefrom- is disclosed in U.S. Patent Application Serial No. 08/759,620, filed December 3, 1996, the disclosure of which is hereby incorporated by reference. *See also* Mendez et al. Nature Genetics 15:146-156 (1997).

Through use of such technology, we have produced fully human monoclonal antibodies to a variety of antigens. Essentially, we immunize XenoMouse™ lines of mice with an antigen of interest, recover lymphatic cells (such as B-cells) from the mice that express antibodies, fuse such recovered cells with a myeloid-type cell line to prepare immortal hybridoma cell lines, and such hybridoma cell lines are screened and selected to identify hybridoma cell lines that produce antibodies specific to the antigen of interest. We utilized these techniques in accordance with the present invention for the preparation of antibodies specific to CTLA-4. Herein, we describe the production of multiple hybridoma cell lines that produce antibodies specific to CTLA-4. Further, we provide a characterization of the antibodies produced by such cell lines, including nucleotide and amino acid sequence analyses of the heavy and light chains of such antibodies.

The antibodies derived from hybridoma cell lines discussed herein are designated 3.1.1, 4.1.1, 4.8.1, 4.10.2, 4.13.1, 4.14.3, 6.1.1, 11.2.1, 11.6.1, 11.7.1, 12.3.1.1, and 12.9.1.1. Each of the antibodies produced by the aforementioned cell lines are either fully human IgG2 or IgG4 heavy chains with human kappa light chains. In general, antibodies in accordance with the invention possess very high affinities, typically possessing Kd's of from about 10⁻⁹ through about 10⁻¹¹ M, when measured by either solid phase or solution phase.

As will be appreciated, antibodies in accordance with the present invention can be expressed in cell lines other than hybridoma cell lines. Sequences encoding the cDNAs or genomic clones for the particular antibodies can be used for transformation of a suitable mammalian or nonmammalian host cells. Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Patent Nos. 4,399,216, 4,912,040, 4,740,461, and 4,959,455). The transformation procedure used depends upon the host to be transformed. Methods for introduction of heterologous polynucleotides into mammalian cells are well known in the art and include, but are not limited to, dextran-mediated transfection, calcium phosphate precipitation, polybrene mediated transfection, protoplast fusion, electroporation, particle bombardment, encapsulation of the polynucleotide(s) in liposomes, peptide conjugates, dendrimers, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, NSO₀, HeLa cells, baby hamster kidney (BHK) cells, monkey kidney cells (COS), human hepatocellular carcinoma cells (e.g., Hep G2), and a number of other cell lines. Non-mammalian cells including but not limited to bacterial, yeast, insect, and plants can also be used to express recombinant antibodies. Site directed mutagenesis of the antibody CH2 domain to eliminate glycosylation may be preferred in order to prevent changes in either the immunogenicity, pharmacokinetic, and/or effector functions resulting from non-human glycosylation. The expression methods are selected by determining which system generates the highest expression levels and produce antibodies with constitutive CTLA-4 binding properties.

Further, expression of antibodies of the invention (or other moieties therefrom) from production cell lines can be enhanced using a number of known techniques. For example, the glutamine sythetase and DHFR gene expression systems are common approaches for enhancing expression under certain conditions. High expressing cell clones can be identified using conventional techniques, such as limited dilution cloning and Microdrop technology. The GS system is discussed in whole or part in connection with European Patent Nos. 0 216 846, 0 256 055, and 0 323 997 and European Patent Application No. 89303964.4.

Antibodies of the invention can also be produced transgenically through the generation of a mammal or plant that is transgenic for the immunoglobulin heavy and light chain sequences of interest and production of the antibody in a recoverable form therefrom. In connection with the transgenic production in mammals, antibodies can be produced in, and recovered from, the milk of goats, cows, or other mammals. *See, e.g.,* U.S. Patent Nos. 5,827,690, 5,756,687, 5,750,172, and 5,741,957.

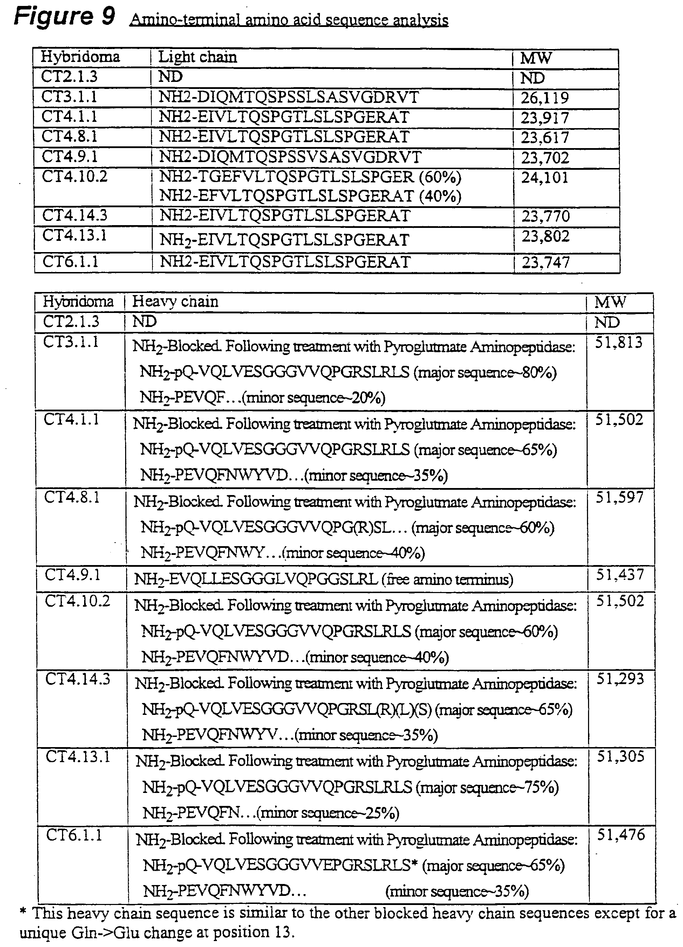

Antibodies in accordance with the present invention have been analyzed structurally and functionally. In connection with the structures of the antibodies, amino acid sequences of the heavy and kappa light chains have been predicted based on cDNA sequences obtained through RT-PCR of the hybridomas. *See* Examples 3 and 4 and Figures 1-8. N-terminal sequencing of the antibodies was also conducted in confirmation of the results discussed in Examples 3 and 4. *See* Example 5 and Figure 9. Kinetic analyses of the antibodies were conducted to determine affinities. *See* Example 2. Antibodies in accordance with the invention (and particularly the 4.1.1, 4.8.1, and 6.1.1 antibodies of the invention) have high affinities (4.1.1:1.63 X 10¹⁰ 1/M; 4.8.1:3.54 X 10¹⁰ 1/M; and 6.1.1:7.2 X 10⁹ 1/M). Further, antibodies were analyzed by isoelectric focusing (IEF), reducing gel electrophoresis (SDS-PAGE), size exclusion chromatography, liquid chromatography/mass spectroscopy, and mass spectroscopy and antibody production by the hybridomas was assessed. *See* Example 6 and Figure 10.

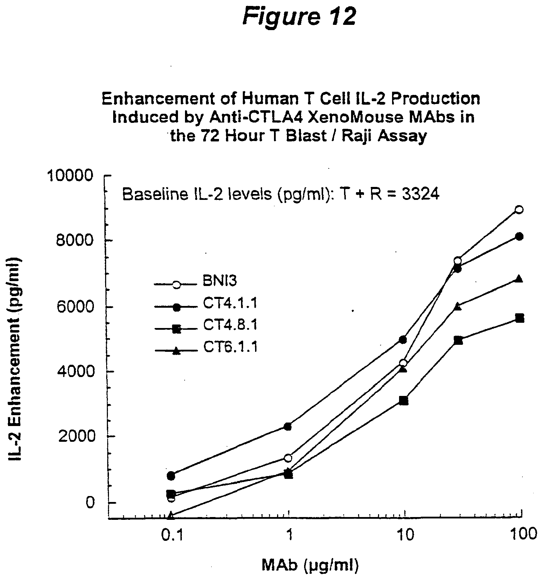

In connection with functional analysis of antibodies in accordance with the present invention, such antibodies proved to be potent inhibitors of CTLA-4 and its binding to its ligands of the B7 family of molecules. For example, antibodies in accordance with the present invention were demonstrated to block CTLA-4 binding to either B7-1 or B7-2. *See* Example 7. Indeed, many of the antibodies in accordance with the invention possess nanomolar and subnanomolar IC₅₀s with respect to inhibiting CTLA-4 binding to B7-1 and B7-2. Further, antibodies of the invention possess excellent selectivity for CTLA-4 as compared to CD28, CD44, B7-2, or hIgG1. *See* Example 8. Selectivity is a ratio that reflects the degree of preferential binding of a molecule with a first agent as compared to the molecules binding with a second, and optionally other molecules. Herein, selectivity refers to the degree of preferential binding of an antibody of the invention to CTLA-4 as compared to the antibody's binding to other molecules such as CD28, CD44, B7-2, or hIgG1. Selectivity values of antibodies of the invention greater than 500:1 are common. Antibodies of the invention have also been demonstrated to induce or enhance expression of certain cytokines (such as IL-2 and IFN-γ) by cultured T cells in a T cell blast model. *See* Examples 9 and 10 and Figures 12-17. Further, it is expected that antibodies of the invention will inhibit the growth of tumors in appropriate *in vivo* tumor models. The design of which models are discussed in Example 11 and 12.

The results demonstrated in accordance with the present invention indicate that antibodies of the present invention possess certain qualities that may make the present antibodies more efficacious than current therapeutic antibodies against CTLA-4.

In particular, the 4.1.1, 4.8.1, and 6.1.1 antibodies of the invention possess highly desirable properties. Their structural characteristics, functions, or activities provide criteria that facilitate the design or selection of additional antibodies or other molecules as discussed above. Such criteria include one or more of the following:
Ability to compete for binding to CTLA-4 with one or more of the antibodies of the invention;
Similar binding specificity to CTLA-4 as one or more of the antibodies of the invention;
A binding affinity for CTLA-4 of about 10⁻⁹ M or greater and preferably of about 10⁻¹⁰M or greater;
Does not cross react with lower mammalian CTLA-4, including, preferably, mouse, rat, or rabbit and preferably mouse or rat CTLA-4;
Cross reacts with primate CTLA-4, including, preferably, cynomolgous and rhesus CTLA-4;
A selectivity for CTLA-4 over CD28, B7-2, CD44, or hIgG1 of at least about 100:1 or greater and preferably of about 300, 400, or 500:1 or greater;
An IC₅₀ in blocking CTLA-4 binding to B7-2 of about 100 nM or lower and preferably 5, 4, 3, 2, 1, 0.5, or 0.38 nM or lower;
An IC₅₀ in blocking CTLA-4 binding to B7-1 of about of about 100 nM or lower and preferably 5, 4, 3, 2, 1, 0.5, or 0.50 nM or lower;
An enhancement of cytokine production in one or more *in vitro* assays, for example:
   An enhancement of IL-2 production in a T cell blast/Raji assay of about 500 pg/ml or greater and preferably 750, 1000, 1500, 2000, 3000, or 3846 pg/ml or greater,
   An enhancement of IFN-γ production in a T cell blast/Raji assay of about 500 pg/ml or greater and preferably 750, 1000, or 1233 pg/ml or greater; or
   An enhancement of IL-2 production in a hPBMC or whole blood superantigen assay of about 500 pg/ml or greater and preferably 750, 1000, 1200, or 1511 pg/ml or greater. Expressed another way, it is desirable that IL-2 production is enhanced by about 30, 35, 40, 45, 50 percent or more relative to control in the assay.

It is expected that antibodies (or molecules designed or synthesized therefrom) having one or more of these properties will possess similar efficacy to the antibodies described in the present invention.

The desirable functional properties discussed above can often result from binding to and inhibition of CTLA4 by a molecule (i.e., antibody, antibody fragment, peptide, or small molecule) in a similar manner as an antibody of the invention (i.e., binding to the same or similar epitope of the CTLA4 molecule). The molecule may either be administered directly (i.e., direct administration to a patient of such molecules). Or, alternatively, the molecule may be "administered" indirectly (i.e., a peptide or the like that produces an immune response in a patient (similar to a vaccine) wherein the immune response includes the generation of antibodies that bind to the same or similar epitope or an antibody or fragment that is produced *in situ* after administration of genetic materials that encode such antibodies or fragments thereof which bind to the same or similar epitope). Thus, it will be appreciated that the epitope on CTLA4 to which antibodies of the invention bind to can be useful in connection with the preparation and/or design of therapeutics in accordance with the invention. In drug design, negative information is often useful as well (i.e., the fact that an antibody which binds to CTLA4 does not appear to bind to an epitope that acts as an inhibitor of CTLA4 is useful). Thus, the epitope to which antibodies of the invention bind that do not lead to the desired functionality can also be very useful. Accordingly, also contemplated in accordance with the present disclosure are molecules (and particularly antibodies) that bind to the same or similar epitopes as antibodies of the invention.

In addition to the fact that antibodies of the disclosure and the epitopes to which they bind are contemplated in accordance with the disclosure, we have conducted some preliminary epitope mapping studies of certain antibodies in accordance with the disclosure and particularly the 4.1.1 and the 11.2.1 antibodies of the disclosure.

As a first step, we conducted BIAcore competition studies to generate a rough map of binding as between certain antibodies of the invention in connection with their ability to compete for binding to CTLA4. To this end, CTLA4 was bound to a BIAcore chip and a first antibody, under saturating conditions, was bound thereto and competition of subsequent secondary antibodies binding to CTLA4 was measured. This technique enabled generation of a rough map in to which families of antibodies can be classified.

Through this process, we determined that the certain antibodies in accordance with the invention could be categorized as falling into the following epitopic categories:

| Category | Antibodies | Competition for CTLA4 Binding |
|---|---|---|
| A | BO1M* | Freely cross-compete with one another; cross-compete with category B; some cross-competition with category D |
| | BO2M** | |
| B | 4.1.1 | Freely cross-compete with one another; cross-compete with category A, C and D. |
| | 4.13.1 | |
| C | 6.1.1 | Freely cross-compete with one another, cross-compete with category B and category D |
| | 3.1.1 | |
| | 4.8.1 | |
| | 11.2.1 | |
| | 11.6.1 | |
| | 11.7.1 | |
| D | 4.14.3 | Cross-compete with category C and B; some cross-competition with category A |
| E | 4.9.1 | BNI3 blocks 4.9.1 binding to CTLA4 but not the reverse |
| | BNI3*** | |

| | | |
|---|---|---|
| (*) (**) Available from Biostride. (***) Available from Pharmingen. | | |

As a next step, we endeavored to determine if the antibodies of the invention recognized a linear epitope on CTLA4 under reducing and non-reducing conditions on Western blots. We observed that none of the 4.1.1, 3.1.1, 11.7.1, 11.6.1, or 11.2.1 antibodies of the invention appeared to recognize a reduced form of CTLA4 on Western blot. Accordingly, it appeared likely that the epitope to which each of these antibodies bound was not a linear epitope but more likely was a conformational epitope the structure of which may have been abrogated under reducing conditions.

Therefore, we sought to determine whether we could learn about residues within the CTLA4 molecule that are important for binding of antibodies of the invention. One manner that we utilized was to conduct kinetic assessments of off-rates as between human CTLA4 and two highly conserved primate CTLA4 molecules (cynomologous and marmoset CTLA4). BIAcore studies demonstrated that the 4.1.1 antibody of the invention bound to human, cynomologous, and marmoset CTLA4 at the same rate. However, with respect to off-rates (affinity), the 4.1.1 antibody had the highest affinity (slowest off-rate) for human, a faster off-rate with cynomologous, and a much faster off-rate for marmoset. The 11.2.1 antibody of the invention, on the other hand, binds to human, cynomologous, and marmoset CTLA4 at the about the same rate and has about the same relative off-rate for each of the three. This information further indicates that the 4.1.1 and 11.2.1 antibodies of the invention bind to different epitopes on CTLA4.

To further study the epitope to which the category B and C antibodies of the invention bind, we conducted certain site directed mutagenesis studies. Marmoset CTLA4 possesses two important changes at residues 105 and 106 relative to human CTLA4. Such differences are a leucine to methionine change at residue 105 and a glycine to serine change at residue 106. Accordingly, we mutated cDNA encoding human CTLA4 to encode a mutated CTLA4 having the L105M and G106S changes. The homologue replacement mutant CTLA4 did not effect binding of a B7.2-IgGl fusion protein. Further, binding with the 11.2.1 antibody of the invention was not effected. However, such molecule was significantly inhibited in its ability to bind with the 4.1.1 antibody of the invention (similar to marmoset). Next, we mutated a cDNA encoding marmoset CTLA4 to create a mutant marmoset CTLA4 having a S106G change. Such change resulted in restoration of stable binding between the 4.1.1 antibody and the marmoset CTLA4 mutant. In addition, we mutated a cDNA encoding marmoset CTLA4 to create a mutant marmoset CTLA4 having a M105L change. Such change partially restored binding between the 4.1.1 antibody and the mutant CTLA4.

Each of the category B through D antibodies of the invention appear to possess similar functional properties and appear to have the potential to act as strong anti-CTLA4 therapeutic agents. Further, each of the molecules certain cross-competition in their binding for CTLA4. However, as will be observed from the above discussion, each of the molecules in the different categories appear to bind to separate conformational epitopes on CTLA4.

From the foregoing, it will be appreciated that the epitope information discussed above indicates that antibodies (or other molecules, as discussed above) that cross-compete with antibodies of the invention will likely have certain therapeutic potential in accordance with the present invention. Further, it is expected that antibodies (or other molecules, as discussed above) that cross-compete with antibodies of the invention (i.e., cross-compete with category B, C and/or D antibodies) will likely have certain additional therapeutic potential in accordance with the present invention. Additionally, it is expected that antibodies (or other molecules, as discussed above) that cross-compete with antibodies of the invention (i.e., cross-compete with category B, C and/or D antibodies) and that (i) are not reduced in their binding to marmoset CTLA4 (similar to the 11.2.1 antibody) or (ii) are reduced in their binding to marmoset CTLA4 (similar to the 4.1.1 antibody) will likely have certain additional therapeutic potential in accordance with the present invention. Antibodies (or other molecules, as discussed above) that compete with categories A and E may also have certain therapeutic potential.

### EXAMPLES

The following examples, including the experiments conducted and results achieved are provided for illustrative purposes only and are not to be construed as limiting upon the present invention.

### EXAMPLE 1

### Generation of Anti-CTLA-4-Antibody Producing Hybridomas

Antibodies of the invention were prepared, selected, and assayed in accordance with the present Example.

***Antigen Preparation:*** Three distinct immunogens were prepared for immunization of the XenoMouse™ mice: (i) a CTLA-4-IgG fusion protein, (ii) a CTLA-4 peptide, and (iii) 300.19 murine lymphoma cells transfected with a mutant of CTLA-4 (Y201V) that is constitutively expressed on the cell surface.

### (i) CTLA-4-IgG1 Fusion Protein:

### Expression Vector Construction:

The cDNA encoding the mature extracellular domain of CTLA-4 was PCR amplified from human thymus cDNA library (Clontech) using primers designed to published sequence (Eur. J Immunol 18:1901-1905 (1988)). The fragment was directionally subcloned into pSR5, a Sindbis virus expression plasmid (InVitrogen), between the human oncostatin M signal peptide and human IgG gamma 1 (IgG1) CH1/CH2/CH3 domains. The fusion protein does not contain a hinge domain but contains cysteine 120 in the extracellular domain of CTLA-4 to form a covalent dimer. The resulting vector was called CTLA-4-IgG1/pSR5. The complete CTLA-4-IgG1 cDNA in the vector was sequence confirmed in both strands. The amino acid sequence the CTLA4-Ig protein is shown below. The mature extracellular domain for CD44 was PCR amplified from human lymphocyte library (Clontech) and subcloned into pSinRep5 to generate a control protein with the identical IgG1 tail.

### OM-CTLA4-IgG1 Fusion Protein:

Underlined: signal peptide
Bold: CTLA4 extracellular domain

The cDNAs for mature extracellular domain of CD28 were PCR amplified from human lymphocyte library (Clontech) and then subcloned into pCDM8 (J. Immunol. 151: 5261-71 (1993)) to produce a human IgG1 fusion protein containing both thrombin cleavage and hinge regions. Marmoset, Cynomologous, and Rhesus CTLA4 were cloned from mRNA isolated from PHA stimulated PBMCs using standard techniques of degenerate PCR. Sequencing demonstrated that rhesus and cynomologous amino acid sequence were identical with three differences from mature human CTLA4 extracellular domain (S13N, I17T and L105M). Marmoset demonstrated ten amino acid differences from the mature human CTLA4 extracellular domain (V21A, V33I, A41T, A51G, 54I, S71F, Q75K, T88M, L105M and G106S). Site directed mutagenesis was used to make single point mutations of all amino acids different in marmoset CTLA4 to map amino acids important for interation of the antibodies with human CTLA4-IgG. Mutations of human and marmoset CTLA-IgG for epitope mapping were generated by matchmaker site-directed mutagenesis (Promega). The IgG fusion proteins were produced by transient transfection of Cos7 cells and purified using standard Protein A techniques. Mutant CTLA4-IgG proteins were evaluated for binding to antibodies by immunoblotting and using BIAcore analyses.

### Recombinant Protein Expression/Purification:

Recombinant sindbis virus was generated by electroporating (Gibco) Baby Hamster Kidney cells with SP6 *in vitro* transcribed CTLA-4-IgG/pSR5 mRNA and DH-26S helper mRNA as described by InVitrogen. Forty eight hours later recombinant virus was harvested and titered for optimal protein expression in Chinese hamster ovary cells (CHO-K1). CHO-K1 cells were cultured in suspension in DMEM/F12 (Gibco) containing 10% heat-inactivated fetal bovine serum (Gibco), non-essential amino acids (Gibco), 4mM glutamine (Gibco), penicillin/streptomycin (Gibco), 10mM Hepes pH 7.5 (Gibco). To produce CTLA-4-IgG, the CHO-K1 cells were resuspended at 1x10⁷ cells/ml in DMEM/F12 and incubated with sindbis virus for one hour at room temperature. Cells were then diluted to 1x10⁶/ml in DMLM/F12 containing 1% fetal bovine serum depleted of bovine IgG using protein A sepharose (Pharmacia), non-essential amino acids, 4mM glutamine, 12.5mM Hepes pH 7.5, and penicillin/streptomycin. Forty eight hours post-infection cells were pelleted and conditioned media was harvested and supplemented with complete protease inhibitor tablets (Boehringer Mannheim), pH adjusted to 7.5, and filtered 0.2µ (Nalgene). FPLC (Pharmacia) was used to affinity purify the fusion protein using a 5ml protein A HiTrap column (Pharmacia) at a 10ml/min flow rate. The column was washed with 30 bed volumes of PBS and eluted with 0.1M glycine/HCl pH 2.8 at 1ml/min. Fractions (1ml) were immediately neutralized to pH 7.5 with Tris pH 9. The fractions containing CTLA-4-IgG1 were identified by SDS-PAGE and then concentrated using centriplus 50 (Amicon) before applying to sepharose 200 column (Pharmacia) at 1ml/min using PBS as the solvent. Fractions containing CTLA-4-IgG1 were pooled, sterile filtered 0.2µ (Millipore), aliquoted and frozen at -80°C. CD44-IgG1 was expressed and purified using the same methods. CD28-IgG was purified from conditioned media from transiently transfected Cos7 cells.

### Characterization CTLA-4-IgG1:

The purified CTLA-4-IgG1 migrated as a single band on SDS-PAGE using colloidal coomassie staining (Novex). Under non-reducing conditions CTLA-4-IgG1 was a dimer (100kDa), that reduced to a 50kDa monomer when treated with 50mM DTT. Amino acid sequencing of the purified CTLA-4-IgG1 in solution confirmed the N-terminus of CTLA-4 (MHVAQPAVVLAS), and that the oncostatin-M signal peptide was cleaved from the mature fusion protein

The CTLA-4-IgG1 bound to immobilized B7.1-IgG in a concentration dependent manner and the binding was blocked by a hamster-anti-human anti-CTLA-4 antibody (BNI3: PharMingen). The sterile CTLA-4-IgG was endotoxin free and quantitated by OD280 using 1.4 as the extinction coefficient. The yield of purified CTLA-4-IgG ranged between 0.5-3mgs/liter of CHO-K1 cells.

### (ii) CTLA-4 Peptide:

The following CTLA-4 peptide was prepared as described below:
NH₂:MHVAQPAVVLASSRGIASFVCEYASPGKATEVRVTVLRQADSQVT EVCAATYMMGNELTFLDDSICTGTSSGNQVNLTIQGLRAMDTGLYICK VELMYPPPYYLGIGNGTQIYVIDPEPC-CONH₂

### Abbreviations/Materials:

NMP, N-Methylpyrrolidinone; TFE, 2,2,2-Trifluoroethanol; DCM, Dichloromethane; FMOC, Fluorenyl Methoxycarbonyl. All reagents were supplied by Perkin Elmer, with the following exceptions: TFE, Aldrich Chemical, FMOC-PAL-PEG resin, Perseptive Biosystems. Fmoc-Arg(PMC)-OH, FMOC-Asn(Trt)-OH, FMOC-Asp(tBu)-OH, FMOC-Cys(Trt)-OH, FMOC-Glu(tBu)-OH, FMOC-Gln(Trt)-OH, FMOC-His(Boc)-OH, FMOC-Lys(BOC)-OH, FMOC-Ser(tBu)-OH, FMOC-Thr(tBu)-OH and FMOC-Tyr(tBu)-OH were used for those amino acids requiring side chain protecting groups

### Peptide Synthesis:

Peptide synthesis was performed on a Perkin-Elmer 431A, retrofitted with feedback monitoring via UV absorbance at 301nm (Perkin-Elmer Model 759A detector). The peptide sequence was assembled on a FMOC-PAL-PEG resin using conditional double coupling cycles. Forced double couplings were performed at cycles 10,11,18,19,20 and 28 through 33. The resin was washed with a 50% mixture of DCM and TFE at the completion of each acylation cycle, followed by capping of unreacted amino groups with acetic anhydride in NMP. Resin was removed from the reactor after completing cycle 49 and the remainder continued to completion. Peptide cleavage from the resin was performed using Reagent K (King et al. International Journal of Protein and Peptide Research 36:255-266 (1990)) for 6 hours on 415mg of resin affording 186mg crude CTLA-4 peptide.

### Peptide Characterization:

25mg aliquots of the crude CTLA-4 peptide were dissolved in 5ml 6M Guanidine HCl/100mM K₂PO₃ at pH6.4 and eluted over a Pharmacia Hi Load Superdex 75 16/60 column (16mm x 600mm, 120ml bed volume) with 2M Guanidine.HCl / 100mM K₂PO₃ at pH6.4 at 2 ml / min for 180 minutes collecting 5 ml fractions. The fractions were analyzed by loading 1.7µl of fractions onto a NuPAGE Laemeli gel running with MES running buffer and visualizing via Daichii silver stain protocol. Those fractions exhibiting a molecular weight of 12 KDa, as judged versus molecular weight standards, were pooled together and stored at 4°C. The combined fractions were analyzed by UV and gel electrophoresis. Amino acid sequencing was performed by absorbing a 100 microliter sample in a ProSorb cartridge (absorbed onto a PVDF membrane) and washing to remove the buffer salts. Sequencing was performed on an Applied Biosystems 420. The expected N-terminal sequence (M H V A Q P A V V L A) was observed. Immunoblotting demonstrated that the peptide was recognized by the BNI3 anti-human CTLA-4 (PharMingen). To desalt, an aliquot containing 648µg of material was placed in 3500 Da MWCO dialysis tubing and dialyzed against 0.1% TFA / H2O at 4°C for 9 days with stirring. The entire contents of the dialysis bag was lyophilyzed to a powder.

### (iii) 300.19 cells transfected with CTLA-4 (Y201V)

The full length CTLA-4 cDNA was PCR amplified from human thymus cDNA library (Stratagene) and subcloned into pIRESneo (Clontech). A mutation of CTLA-4 that results in constitutive cell surface expression was introduced using MatchMaker Mutagenesis System (Promega). Mutation of tyrosine, Y201 to valine inhibits binding of the adaptin protein AP50 that is responsible for the rapid internalization of CTLA-4 (Chuang et al. J. Immunol. 159:144-151 (1997)). Mycoplasma-free 300.19 murine lymphoma cells were cultured in RPMI-1640 containing 10% fetal calf serum, non-essential amino acids, penicillin/streptomycin, 2mM glutamine, 12.5mM Hepes pH 7.5, and 25uM beta-mercaptoethanol. Cells were electroporated (3x10⁶/0.4ml serum free RPMI) in a 1ml chamber with 20ug CTLA-4-Y201V/pIRESneo using 200V/1180uF (Gibco CellPorator). Cells were rested for 10 minutes and then 8mls of prewarmed complete RPMI media. At 48 hours cells were diluted to 0.5 x10⁶/ml in complete RPMI media containing 1mg/ml G418 (Gibco). Resistant cells were expanded and shown to express CTLA-4 on the cell surface using the BNI3 antibody conjugated with phycoerythrin (PharMingen). High level expressing cells were isolated by sterile sorting.

***Immunization and hybridoma generation:*** XenoMouse mice (8 to 10 weeks old) were immunized (i) subcutaneously at the base of tails with 1x10⁷ 300.19 cells that were transfected to express CTLA-4 as described above, resuspended in phosphate buffered saline (PBS) with complete Freund's adjuvant, or (ii) subcutaneously at the base of tail with (a) 10 µg the CTLA-4 fusion protein or (b) 10 µg CTLA-4 peptide, emulsified with complete Freund's adjuvant. In each case, the dose was repeated three or four times in incomplete Freund's adjuvant. Four days before fusion, the mice received a final injection of the immunogen or cells in PBS. Spleen and/or lymph node lymphocytes from immunized mice were fused with the [murine non-secretory myeloma P3 cell line] and were subjected to HAT selection as previously described (Galfre, G. and Milstein, C., "Preparation of monoclonal antibodies: strategies and procedures." Methods Enzymol. 73:3-46 (1981)). A large panel of hybridomas all secreting CTLA-4 specific human IgG₂κ or IgG₄κ (as detected below) antibodies were recovered.

***ELISA assay:*** ELISA for determination of antigen-specific antibodies in mouse serum and in hybridoma supernatants was carried out as described (Coligan et al., Unit 2.1, "Enzyme-linked immunosorbent assays," in Current protocols in immunology (1994)) using CTLA-4-Ig fusion protein to capture the antibodies. For animals that are immunized with the CTLA-4-Ig fusion protein, we additionally screen for non-specific reactivity against the human Ig portion of the fusion protein. This is accomplished using ELISA plates coated with human IgG1 as a negative control for specificity.

In a preferred ELISA assay, the following techniques are used:
ELISA plates are coated with 100 µl/well of the antigen in plate coating buffer (0.1 M Carbonate Buffer, pH 9.6 and NaHCO₃ (MW 84) 8.4g/L). Plates are then incubated at 4°C overnight. After incubation, coating buffer is removed and the plate is blocked with 200 µl/well blocking buffer (0.5% BSA, 0.1% Tween 20, 0.01% Thimerosal in 1x PBS) and incubated at room temperature for 1 hour. Alternatively, the plates are stored in refrigerator with blocking buffer and plate sealers. Blocking buffer is removed and 50 µl/well of hybridoma supernatant, serum or other hybridoma supernatant (positive control) and HAT media or blocking buffer (negative control) is added. The plates are incubated at room temperature for 2 hours. After incubation, the plate is washed with washing buffer (1x PBS). The detecting antibody (i.e., mouse anti-human IgG2-HRP (SB, #9070-05) for IgG2 antibodies or mouse anti-human IgG4-HRP (SB #9200-05) for IgG4 antibodies) is added at 100µl/well (mouse anti-human IgG2-HRP @ 1:2000 or mouse anti-human IgG4-HRP @ 1:1000 (each diluted in blocking buffer)). The plates are incubated at room temperature for 1 hour and then washed with washing buffer. Thereafter, 100 µl/well of freshly prepared developing solution (10 ml Substrate buffer, 5 mg OPD (o-phenylenediamine, Sigma Cat No. P-7288), and 10 µl 30% H₂O₂ (Sigma)) is added to the wells. The plates are allowed to develop 10-20 minutes, until negative control wells barely start to show color. Thereafter, 100 µl/well of stop solution (2 M H₂SO₄) is added and the plates are read on an ELISA plate reader at wavelength 490 nm.

***Determination of affinity constants of fully human Mabs by BIAcore:*** Affinity measurement of purified human monoclonal antibodies, Fab fragments, or hybridoma supernatants by plasmon resonance was carried out using the BIAcore 2000 instrument, using general procedures outlined by the manufacturers.

Kinetic analysis of the antibodies was carried out using antigens immobilized onto the sensor surface at a low density. Three surfaces of the BIAcore sensorchip were immobililized with the CTLA-4-Ig fusion protein at a density ranging from approximately 390-900 using CTLA-4-Ig fusion protein at 20 or 50 µg/ml in 10 mM sodium acetate at pH 5.0 using the amine coupling kit supplied by the manufacturer (BIAcore, Inc.). The fourth surface of the BIAcore sensorchip was immobilized with IgG1 (900 RU) and was used as a negative control surface for non-specific binding. Kinetic analysis was performed at a flow rate of 25 or 50 microliters per minute and dissociation (kd or k_{off}) and association (ka or kₒₙ) rates were determined using the software provided by the manufacturer (BIA evaluation 3.0) that allows for global fitting calculations.

### EXAMPLE 2

### Affinity Measurement of Anti-CTLA-4-Antibodies

In the following Table, affinity measurements for certain of the antibodies selected in this manner are provided:

**TABLE I**

| | **Solid Phase** | | | | |
|---|---|---|---|---|---|
| | **(by BIAcore)** | | | | |
| **Hybridoma** | **On-rates Kₐ (M⁻¹S⁻¹x10⁶)** | **Off-rates K_{d} (S⁻¹x10⁻⁴)** | **Association Constant KA (1/M)= kₐ/k_{d}x10¹⁰** | **Dissociation Constant KD(M)= K_{d}/kₐx10⁻¹⁰** | **Surface Density [RU]** |
| **Moab01** | 0.68 | 1.01 | 0.67 | 1.48 | 878.7 |
| | 0.70 | 4.66 | 0.15 | 6.68 | 504.5 |
| | 0.77 | 6.49 | 0.19 | 8.41 | 457.2 |
| | 0.60 | 3.08 | 0.20 | 5.11 | 397.8 |
| **4.1.1** | 1.85 | 0.72 | 2.58 | 0.39 | 878.7 |
| | 1.88 | 1.21 | 1.55 | 0.64 | 504.5 |
| | 1.73 | 1.54 | 1.13 | 0.88 | 457.2 |
| | 1.86 | 1.47 | 1.26 | 0.79 | 397.8 |
| **4.8.1** | 0.32 | 0.07 | 4.46 | 0.22 | 878.7 |
| | 0.31 | 0.23 | 1.33 | 0.75 | 504.5 |
| | 0.28 | 0.06 | 4.82 | 0.21 | 397.8 |
| **4.14.3** | 2.81 | 3.04 | 0.92 | 1.08 | 878.7 |
| | 2.88 | 3.97 | 0.73 | 1.38 | 504.5 |
| | 2.84 | 6.66 | 0.43 | 2.35 | 457.2 |
| | 3.17 | 5.03 | 0.63 | 1.58 | 397.8 |
| **6.1.1** | 0.43 | 0.35 | 1.21 | 0.83 | 878.7 |
| | 0.46 | 0.90 | 0.51 | 1.98 | 504.5 |
| | 0.31 | 0.51 | 0.61 | 1.63 | 457.2 |
| | 0.45 | 0.79 | 0.57 | 1.76 | 397.8 |
| **3.1.1** | 1.04 | 0.96 | 1.07 | 0.93 | 878.7 |
| | 0.95 | 1.72 | 0.55 | 1.82 | 504.5 |
| | 0.73 | 1.65 | 0.44 | 2.27 | 457.2 |
| | 0.91 | 2.07 | 0.44 | 2.28 | 397.8 |
| **4.9.1** | 1.55 | 13.80 | 0.11 | 8.94 | 878.7 |
| | 1.43 | 19.00 | 0.08 | 13.20 | 504.5 |
| | 1.35 | 20.50 | 0.07 | 15.20 | 397.8 |
| **4.10.2** | 1.00 | 2.53 | 0.39 | 2.54 | 878.7 |
| | 0.94 | 4.30 | 0.22 | 4.55 | 504.5 |
| | 0.70 | 5.05 | 0.14 | 7.21 | 457.2 |
| | 1.00 | 5.24 | 0.19 | 5.25 | 397.8 |
| **2.13** | 1.24 | 9.59 | 0.13 | 7.72 | 878.7 |
| | 1.17 | 13.10 | 0.09 | 11.20 | 504.5 |
| | 1.11 | 13.00 | 0.09 | 11.70 | 397.8 |
| **4.13.1** | 1.22 | 5.83 | 0.21 | 4.78 | 878.7 |
| | 1.29 | 6.65 | 0.19 | 5.17 | 504.5 |
| | 1.23 | 7.25 | 0.17 | 5.88 | 397.8 |

As will be observed, antibodies prepared in accordance with the invention possess high affinities and binding constants.

### EXAMPLE 3

### Structures of Anti-CTLA-4-Antibodies Prepared in Accordance with the Invention

In the following discussion, structural information related to antibodies prepared in accordance with the invention is provided.

In order to analyze structures of antibodies produced in accordance with the invention, we cloned genes encoding the heavy and light chain fragments out of the particular hybridoma. Gene cloning and sequencing was accomplished as follows:
Poly(A)⁺ mRNA was isolated from approximately 2 X 10⁵ hybridoma cells derived from immunized XenoMouse mice using a Fast-Track kit (Invitrogen). The generation of random primed cDNA was followed by PCR. Human V_{H} or human V_{κ} family specific variable region primers (Marks et al., "Oligonucleotide primers for polymerase chain reaction amplification of human immunoglobulin variable genes and design of family-specific oligonucleotide probes." Eur. J. Immunol. 21:985-991 (1991)) or a universal human V_{H} primer, MG-30 (CAGGTGCAGCTGGAGCAGTCIGG) was used in conjunction with primers specific for the human Cγ2 constant region (MG-40d; 5'-GCTGAGGGAGTAGAGTCCTGAGGA-3') or C_{κ} constant region (hκP2; as previously described in Green et al., 1994). Sequences of human Mabs-derived heavy and kappa chain transcripts from hybridomas were - obtained by direct sequencing of PCR products generated from poly(A⁺) RNA using the primers described above. PCR products were also cloned into pCRII using a TA cloning kit (Invitrogen) and both strands were sequenced using Prism dye-terminator sequencing kits and an ABI 377 sequencing machine. All sequences were analyzed by alignments to the "V BASE sequence directory" (Tomlinson et al., MRC Centre for Protein Engineering, Cambridge, UK) using MacVector and Geneworks software programs.

Further, each of the antibodies 4.1.1, 4.8.1, 11.2.1, and 6.1.1 were subjected to full length DNA sequences. For such sequencing, Poly(A)⁺ mRNA was isolated from approximately 4 X 10⁶ hybridoma cells using mRNA Direct kit (Dynal). The mRNA was reverse transcribed using oligo-dT(18) and the Advantage RT/PCR kit (Clonetech). The Variable region database (V Base) was used to design amplification primers beginning at the ATG start site of the heavy chain DP50 gene (5'-TATCTAAGCTTCTAGACTCGACCGCCACCATGGAGTTTGGGCTGAGC TG-3') and to the stop codon of the IgG2 constant region (5'-TTCTCTGATCAGAATTCCTATCATTTACCCGGAGACAGGGAGAGCT-3'). An optimal Kozak sequence (ACCGCCACC) was added 5' to the ATG start site. The same method was used to design a primer to the ATG start site of the kappa chain A27 gene (5'-TCTTCAAGCTTGCCCGGGCCCGCCACCATGGAAACCCCAGCGCAG -3') and the stop codon of the kappa constant region (5'-TTCTTTGATCAGAATTCTCACTAACACTCTCCCCTGTTGAAGC-3'). The 012 cDNA was cloned by using a primer to the ATG start site (5'-TCTTCAAGCTTGCCCGGGCCCGCCACCATGGACATGAGGGTCCCCGC T-3) and the kappa constant region stop codon primer above. The heavy chain cDNAs were also cloned as genomic constructs by site directed mutagenesis to add an NheI site at the end of the variable J domain and subcloning an NheI-fragment containing the genomic IgG2 CH1/Hinge/CH2/CH3 regions. The point mutation to generate NheI site does not alter the amino acid sequence from germline. The primer pairs were used to amplify the cDNAs using Advantage High Fidelity PCR Kit (Clonetech). Sequence of the PCR was obtained by direct sequencing using dye-terminator sequencing kits and an ABI sequencing machine. The PCR product was cloned into pEE glutamine synthetase mammalian expression vectors (Lonza) and three clones were sequenced to confirm somatic mutations. For each clone, the sequence was verified on both strands in at least three reactions. An aglycosylated 4.1.1 antibody was generated by site directed mutagenesis of N294Q in the CH2 domain. Recombinant antibodies were produced by transient trasnfection of Cos7 cells in IgG depleted FCS and purified using standard Protein A sepharose techniques. Stable transfectants were generated by electroporation of murine NSO cells and selection in glutamine free media. Recombinant 4.1.1 with or without glycosylation exhibited identical specificity and affinity for CTLA4 in the *in vitro* ELISA and BIAcore assays.

### Gene Utilization Analysis

The following Table sets forth the gene utilization evidenced by selected hybridoma clones of antibodies in accordance with the invention:

**TABLE II**

| **Heavy and Light Chain Gene Utilization** | | | | | | |
|---|---|---|---|---|---|---|
| **Clone** | **Heavy Chain** | | | | **Kappa Light Chain** | |
| | **VH** | **D** | **JH** | | **VK** | **JK** |
| **4.1.1** | DP-50 | DIR4 or DIR3 | JH4 | | A27 | JK1 |
| **4.8.1** | DP-50 | 7-27 | JH4 | | A27 | JK4 |
| **4.14.3** | DP-50 | 7-27 | JH4 | | A27 | JK3 |
| **6.1.1** | DP-50 | DIR5 or DIR5rc | JH4 | | A27 | JK3 |
| **3.1.1** | DP-50 | 3-3 | JH6 | | 012 | JK3 |
| **4.10.2** | DP-50 | 7-27 | JH4 | | A27 | JK3 |
| **2.1.3** | DP-65 | 1-26 | JH6 | | A10/A26 | JK4 |
| **4.13.1** | DP-50 | 7-27 | JH4 | | A27 | JK3 |
| **11.2.1** | DP-50 | D1-26 | JH6 | | 012 | JK3 |
| **11.6.1** | DP-50 | D2-2 or D4 | JH6 | | 012 | JK3 |
| **11.7.1** | DP-50 | D3-22 or D21-9 | JH4 | | 012 | JK3 |
| **12.3.1.1** | DP-50 | D3-3 or DXP4 | JH6 | | A17 | JK1 |
| **12.9.1.1** | DP-50 | D6-19 | JH4 | | A3/A19 | JK4 |
| **4.9.1** | DP-47 | 5-24 and/or 6-19 | JH4 | | L5 | JK1 |

As will be observed, antibodies in accordance with the present invention were generated with a strong bias towards the utilization of the DP-50 heavy chain variable region. The DP-50 gene is also referred to as a V_{H} 3-33 family gene. Only one antibody that was selected on the basis of CTLA-4 binding and preliminary *in vitro* functional assays showed a heavy chain gene utilization other than DP-50. That clone, 2.1.3, utilizes a DP-65 heavy chain variable region and is an IgG4 isotype. The DP-65 gene is also referred to as a V_{H} 4-31 family gene. On the other hand, the clone, 4.9.1, which possesses a DP-47 heavy chain variable region binds to CTLA-4 but does not inhibit binding to B7-1 or B7-2. In XenoMouse mice, there are more than 30 distinct functional heavy chain variable genes with which to generate antibodies. Bias, therefore, is indicative of a preferred binding motif of the antibody-antigen interaction with respect to the combined properties of binding to the antigen and functional activity.

### Mutation Analysis

As will be appreciated, gene utilization analysis provides only a limited overview of antibody structure. As the B-cells in XenoMouse animals stocastically generate V-D-J heavy or V-J kappa light chain transcripts, there are a number of secondary processes that occur, including, without limitation, somatic hypermutation, n-additions, and CDR3 extensions. *See, for example,* Mendez et al. Nature Genetics 15:146-156 (1997) and U.S. Patent Application Serial No. 08/759,620, filed December 3, 1996. Accordingly, to further examine antibody structure predicted amino acid sequences of the antibodies were generated from the cDNAs obtained from the clones. In addition, N-terminal amino acid sequences were obtained through protein sequencing.

Figure 1 provides nucleotide and predicted amino acid sequences of the heavy and kappa light chains of the clones 4.1.1 (Figure 1A), 4.8.1 (Figure 1B), 4.14.3 (Figure 1C), 6.1.1 (Figure 1D), 3.1.1 (Figure IE), 4.10.2 (Figure IF), 2.1.3 (Figure 1G), 4.13.1 (Figure 1H), 11.2.1 (Figure 1I), 11.6.1 (Figure 1J), 11.7.1 (Figure 1K), 12.3.1.1 (Figure 1L), and 12.9.1.1 (Figure 1M). In Figures 1A, 1B, and 1D, extended sequences of the antibodies 4.1.1, 4.8.1, and 6.1.1 were obtained by full length cloning of the cDNAs as described above. In such Figures, the signal peptide sequence (or the bases encoding the same) are indicated in bold and sequences utilized for the 5' PCR reaction are underlined.

Figure 2 provides a sequence alignment between the predicted heavy chain amino acid sequences from the clones 4.1.1, 4.8.1, 4.14.3, 6.1.1, 3.1.1, 4.10.2, 4.13.1, 11.2.1, 11.6.1, 11.7.1, 12.3.1.1, and 12.9.1.1 and the germline DP-50 (3-33) amino acid sequence. Differences between the DP-50 germline sequence and that of the sequence in the clones are indicated in bold. The Figure also shows the positions of the CDR1, CDR2, and CDR3 sequences of the antibodies as shaded.

Figure 3 provides a sequence alignment between the predicted heavy chain amino acid sequence of the clone 2.1.3 and the germline DP-65 (4-31) amino acid sequence. Differences between the DP-65 germline sequence and that of the sequence in the clone are indicated in bold. The Figure also shows the positions of the CDR1, CDR2, and CDR3 sequences of the antibody as underlined.

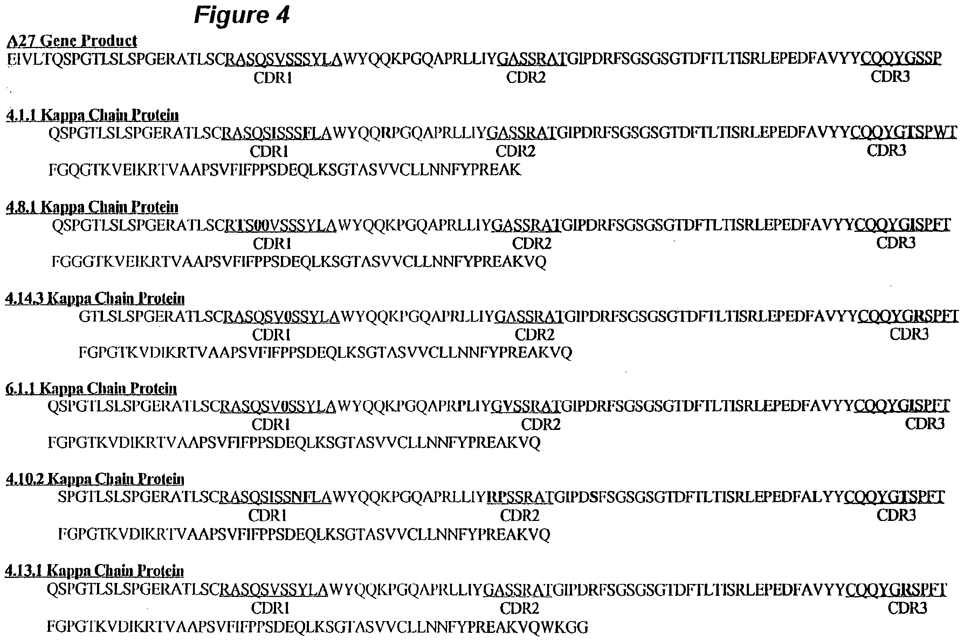

Figure 4 provides a sequence alignment between the predicted kappa light chain amino acid sequence of the clones 4.1.1, 4.8.1, 4.14.3, 6.1.1, 4.10.2, and 4.13.1 and the germline A27 amino acid sequence. Differences between the A27 germline sequence and that of the sequence in the clone are indicated in bold. The Figure also shows the positions of the CDR1, CDR2, and CDR3 sequences of the antibody as underlined. Apparent deletions in the CDR1s of clones 4.8.1, 4.14.3, and 6.1.1 are indicated with "0s".

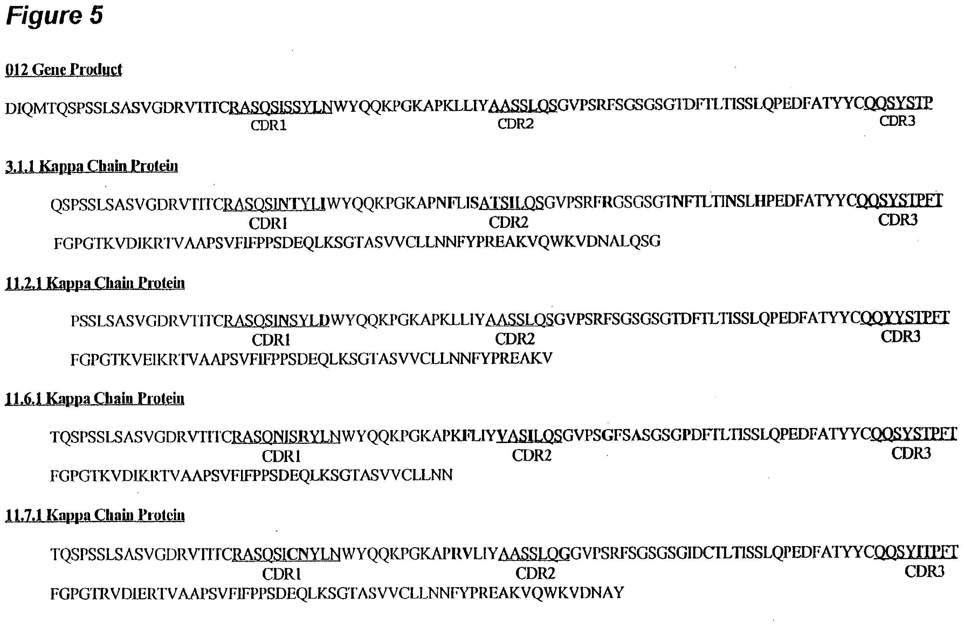

Figure 5 provides a sequence alignment between the predicted kappa light chain amino acid sequence of the clones 3.1.1, 11.2.1, 11.6.1, and 11.7.1 and the germline 012 amino acid sequence. Differences between the 012 germline sequence and that of the sequence in the clone are indicated in bold. The Figure also shows the positions of the CDR1, CDR2, and CDR3 sequences of the antibody as underlined.

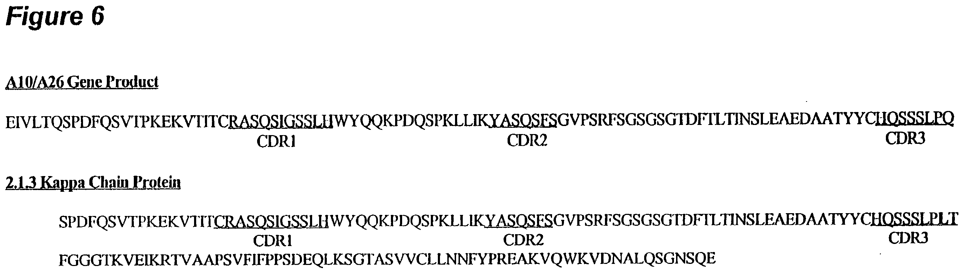

Figure 6 provides a sequence alignment between the predicted kappa light chain amino acid sequence of the clone 2.1.3 and the germline A10/A26 amino acid sequence. Differences between the A10/A26 germline sequence and that of the sequence in the clone are indicated in bold. The Figure also shows the positions of the CDR1, CDR2, and CDR3 sequences of the antibody as underlined.

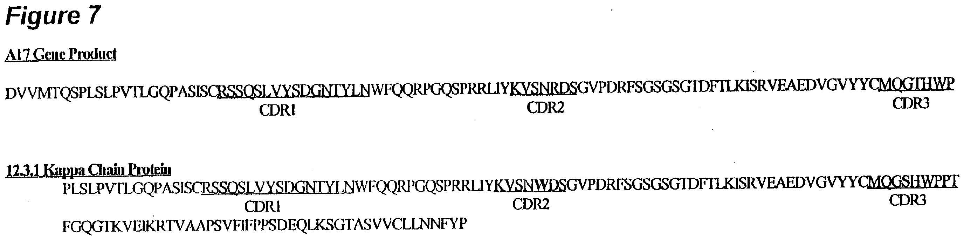

Figure 7 provides a sequence alignment between the predicted kappa light chain amino acid sequence of the clone 12.3.1 and the germline A17 amino acid sequence. Differences between the A17 germline sequence and that of the sequence in the clone are indicated in bold. The Figure also shows the positions of the CDR1, CDR2, and CDR3 sequences of the antibody as underlined.

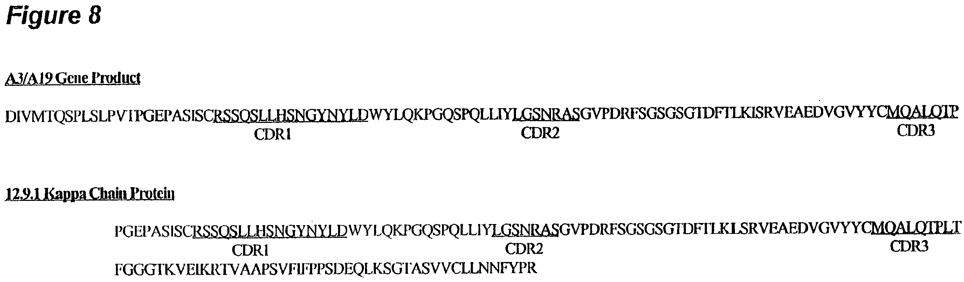

Figure 8 provides a sequence alignment between the predicted kappa light chain amino acid sequence of the clone 12.9.1 and the germline A3/A19 amino acid sequence. Differences between the A3/A19 germline sequence and that of the sequence in the clone are indicated in bold. The Figure also shows the positions of the CDR1, CDR2, and CDR3 sequences of the antibody as underlined.

Figure 22 provides a series of additional nucleotide and amino acid sequences of the following anti-CTLA-4 antibody chains:
***4.1.1*:**
   full length 4.1.1 heavy chain (cDNA 22(a), genomic 22(b), and amino acid 22(c));
   full length aglycosylated 4.1.1 heavy chain (cDNA 22(d) and amino acid 22(e));
   4.1.1 light chain (cDNA 22(f) and amino acid 22(g));
***4.8.1*:**
   full length 4.8.1 heavy chain (cDNA 22(h) and amino acid 22(i));
   4.8.1 light chain (cDNA 22(j) and amino acid 22(k));
***6.1.1*:**
   full length 6.1.1 heavy chain (cDNA 22(1) and amino acid 22(m));
   6.1.1 light chain (cDNA 22(n) and amino acid 22(o));
***11.2.1*:**
   full length 11.2.1 heavy chain (cDNA 22(p) and amino acid 22(q)); and
   11.2.1 light chain (cDNA 22 (r) and amino acid 22(s)).

Signal peptide sequences are shown in bold and large text. The open reading frames in the full length 4.1.1 genomic DNA sequence (Fig. 22(b)) are underlined. And, the mutations introduced to make the aglycosylated 4.1.1 heavy chain and the resulting change (N294Q) are shown in doble underline and bold text (cDNA (Fig. 22(b) and amino acid (Fig. 22(c)).

### EXAMPLE 4

### Analysis of Heavy and Light Chain Amino Acid Substitutions

In Figure 2, which provides a sequence alignment between the predicted heavy chain amino acid sequences from the clones 4.1.1, 4.8.1, 4.14.3, 6.1.1, 3.1.1, 4.10.2, 4.13.1, 11.2.1, 11.6.1, 11.7.1, 12.3.1.1, and 12.9.1.1 and the germline DP-50 (3-33) amino acid sequence, an interesting pattern emerges. In addition to the fact of the bias for heavy chain DP-50 in the majority of the clones, there is relatively limited hypermutation in the antibodies relative to the germline DP-50 gene. For example, clones 3.1.1 and 11.2.1 have no mutations. Moreover, the mutations in the other clones are generally conservative changes, involving substitutions of amino acids with similar properties to the amino acids in the germline. Mutations within many of the CDR1 and CRD2 sequences are particularly conservative in nature. Three of the heavy chains represented in Figure 2, 4.10.2, 4.13.1, and 4.14.3, are clearly derived from a single recombination event (i.e., derive from an identical germinal center) and are nearly identical in sequence. If these three are considered as a single sequence, then, among the 10 different antibodies containing the DP50 heavy chain, in CDR1 and CDR2 there are 3 positions in which a nonpolar residue is replaced by another nonpolar residue, 12 in which a polar uncharged residue is replaced by another polar uncharged residue, and 1 in which a polar charged residue is replaced by another polar charged residue. Further, there are two positions in which two residues which are very similar structurally, glycine and alanine, are substituted for one another. The only mutations not strictly conservative involve 3 substitutions of a polar charged residue for a polar uncharged residue and one substitution of a nonpolar residue for a polar residue.

The light chains of these antibodies are derived from 5 different Vk genes. The A27 gene is the most heavily represented and is the source of 6 different light chains. Comparison of these 6 sequences reveals two noteworthy features. First, in three of them, 4.8.1, 4.14.3, and 6.1.1, contain deletions of one or two residues in CDR1, a rare event. Second, there is a strong prejudice against the germline serine at position six in CDR3 in that the serine has been replaced in every sequence. This suggests that a serine at this position is incompatible with CTLA4 binding.

It will be appreciated that many of the above-identified amino acid substitutions exist in close proximity to or within a CDR. Such substitutions would appear to bear some effect upon the binding of the antibody to the CTLA-4 molecule. Further, such substitutions could have significant effect upon the affinity of the antibodies.

### EXAMPLE 5

### N-Terminal Amino Acid Sequence Analysis of Antibodies in Accordance with the Invention

In order to further verify the composition and structure of the antibodies in accordance with the invention identified above, we sequenced certain of the antibodies using a Perkin-Elmer sequencer. Both heavy and kappa light chains of the antibodies were isolated and purified through use of preparative gel electrophoresis and electroblotting techniques and thereafter directly sequenced as described in Example 6. A majority of the heavy the heavy chain sequences were blocked on their amino terminus. Therefore, such antibodies were first treated with pyroglutamate aminopeptidase and thereafter sequenced.

The results from this experiment are shown in Figure 9. Figure 9 also provides the molecular weight of the heavy and light chains as determined by mass spectroscopy (MALDI).

### EXAMPLE 6

### Additional Characterization of Antibodies in Accordance with the Invention

Figure 10 provides certain additional characterizing information about certain of the antibodies in accordance with the invention. In the Figure, data related to clones 3.1.1, 4.1.1, 4.8.1, 4.10.2, 4.14.3, and 6.1.1 is summarized. The following data is provided: Concentration, isoelectric focusing (IEF), SDS-PAGE, size exclusion chromatography, FACS, mass spectroscopy (MALDI), and light chain N-terminal sequences.

Generally, the data was generated as follows:

### Materials and Methods

Protein concentration was determined at 280 nm from a UV scan (200-350 nm), where 1.58 absorbance units at 280 nm equaled 1mg/ml.

SDS-PAGE was performed using the Novex NuPAGE electrophoresis system with a 10% NuPAGE gel and MES running buffer. Samples were prepared by diluting 3:1 with 4x NuPAGE sample buffer (+/-) beta-mercaptoethanol, heated and ∼ 5 ug of protein was loaded onto the gel. The gel was then stained with Brilliant Blue R staining solution (Sigma cat.# B-6529) and molecular size estimates were made by comparing stained bands to "Perfect Protein Markers" (Novagen cat# 69149-3).

For N-terminal sequencing, samples were run as above on NuPAGE gels, transferred to Pro Blot immobilization membrane (Applied Biosystems) then stained with Coomassie Blue R-250. The stained protein bands were excised and subjected to sequence analysis by automated Edman degradation on an Applied Biosystems 494 Procise HT Sequencer.

Isoelectric focusing (IEF) was performed using Pharmacia IEF 3-9 pHast gels (cat# 17-0543-01). Samples were diluted in 10% glycerol to -0.8 mg/ml and lul was loaded onto gel and then silver stained. The pI estimates were made by comparing stained bands to broad range (pH3-10) IEF standards (Pharmacia cat # 17-0471-01)

Size exclusion chromatography (SEC) was carried in phosphate buffered saline (PBS) on the Pharmacia SMART system using the Superdex 75 PC 3.2/30 column. Molecular size estimates were made by comparing peak retention time to the retention times of gel

For FACS studies, human peripheral T cells were prepared and stimulated for 48 hours. T cells were washed once, resuspended in FACS buffer at 1x10⁶ cells/100 ul and stained for CD3 surface expression with 10 ul of anti-CD3-FITC (Immunotech, Marseille, France) for 30 minutes at room temperature. Cells were washed twice, then fixed, permeabilized (Fix and Perm, Caltag), and stained for intracellular CTLA-4 expression with 10 ul anti-CD152-PE (Pharmingen). Flow cytometry was performed using a Becton Dickinson FACSort. Quadrants were set by analysis of relevant isotype control antibodies (Caltag).

As was discussed above, anti-CTLA-4 antibodies have been demonstrated to possess certain powerful immune modulation activities. The following experiments were carried out in order to determine if antibodies in accordance with the present invention possessed such activities. In general, the experiments were designed to assess ability of the antibodies to inhibit the interaction between CTLA-4 and B7 molecules, be selective as between CTLA-4 and B7 molecules and CD28, and promote T cell cytokine production, including, but not limited to IL-2 and/or IFN-γ expression. Further, examination of cross-reactivity of antibodies of the invention with certain human tissues and CTLA-4 molecules in other species (e.g., mouse and primate) was undertaken.

### EXAMPLE 7

### Competition ELISA: Inhibition of CTLA-4/B7-1 or B7-2 Interaction by Antibodies in Accordance with the Invention

An *in vitro* assay was conducted to determine if antibodies in accordance with the present invention were capable of inhibiting the binding of CTLA-4 with either B7-1 or B7-2. As will be appreciated, antibodies of the invention that are capable of inhibiting the binding of CTLA-4 with B7 molecules would be expected to be candidates for immune regulation through the CTLA-4 pathway. In the assay, the following materials and methods were utilized:

### Materials and Methods

3 nM B7.1-Ig(G1) or B7.2-Ig(G1) (Repligen, Inc. Needham, MA) in Dulbecco's PBS was coated on 96-well MaxiSorp plates (Nunc, Denmark, #439454) and incubated at 4°C overnight. On day 2, B7-Ig was removed and plates were blocked with 1% BSA plus 0.05% Tween-20 in D-PBS for two hours. Plates were washed 3X with wash buffer (0.05% Tween-20 in D-PBS). Antibody at appropriate test concentrations and CTLA-4-Ig(G4) (0.3 nM final conc.) (Repligen, Inc. Needham, MA) were pre-mixed for 15 minutes and then added to the B7-Ig coated plate (60 ul total volume) and incubated at RT for 1.5 hours. Plates were washed 3X and 50 µl of a 1 to 1000 dilution of HRP-labeled mouse anti-human IgG4 antibody (Zymed, San Francisco, CA, #05-3820) was added and incubated at RT for 1 hour. Plates were washed 3X and 50 µl TMB Microwell peroxidase substrate (Kirkegaard & Perry, Gaithersburg, MD, #50-76-04) was added and incubated at RT for 20 minutes, and then 50 µl 1N H₂SO₄ was added to the plate. Plates were read at 450 nm using a Molecular Devices plate reader (Sunnyvale, CA). All samples were tested in duplicate. Maximal signal was defined as CTLA-4-Ig binding in the absence of test antibody. Non-specific binding was defined as absorbance in the absence of CTLA-4-Ig and test antibody.

The results from the assay are provided in Table IIIA and IIIB. In Table IIIA, results are shown for a variety of antibodies in accordance with the invention. In Table IIIB, results are shown comparing the 4.1.1 antibody of the invention with the 11.2.1 antibody of the invention from a separate experiment.

**TABLE IIIA**

| Clone CTLA-4-Ig | Isotype | CTLA4/B7.2 Comp. ELISA IC50(nM) | CTLA4/B7.1 Comp. ELISA IC₅₀ (nM) |
|---|---|---|---|
| CT3.1.1 | IgG2 | 0.45 ± 0.07 (n=3) | 0.63 ± 0.10 (n=2) |
| CT4.1.1 | IgG2 | 0.38 ± 0.06 (n=5) | 0.50 ± 0.05 (n=2) |
| CT4.8.1 | Ig-G2 | 0.57 ± 0.03 (n=3) | 0.17 ± 0.28 (n=2) |
| CT4.9.1 | IgG2 | Non-competitive (n=3) | non-competitive (n=2) |
| CT4.10.2 | IgG2 | 1.50 ± 0.37 (n=3) | 3.39 ± 0.31 (n=2) |
| CT4.13.1 | IgG2 | 0.49 ± 0.05 (n=3) | 0.98 ± 0.11 (n=2) |
| CT4.14.3 | IgG2 | 0.69 ± 0.11 (n=3) | 1.04 ± 0.15 (n=2) |
| CT6.1.1 | IpG2 | 0.39 ± 0.06 (n=3) | 0.67 ± 0.07 (n=2) |

**TABLE IIIB**

| Clone CTLA-4-Ig | Isotype | CTLA4/B7.2 Comp. ELISA IC50 (nM) | CTLA4/B7.1 Comp. ELISA IC50 (nM) |
|---|---|---|---|
| CT4.1.1 | IgG2 | 0.55 ± 0.08 (n=4) | 0.87 ± 0.14 (n=2) |
| CT11.2.1 | IgG2 | 0.56 ± 0.05 (n=4) | 0.81 ± 0.24 (n=2) |

### EXAMPLE 8

### Selectivity Ratios of Antibodies of the Invention with Respect to CTLA-4 Versus Either CD28 or B7-2

Another *in vitro* assay was conducted to determine the selectivity of antibodies of the invention with respect to CTLA-4 versus either CD28 or B7-2. The following materials and methods were utilized in connection with the experiments:

### CTLA-4 Selectivity ELISA: Materials and Methods

A 96-well FluroNUNC plate (Nunc Cat No.475515) was platecoated with four antigens: CTLA-4/Ig, CD44/Ig, CD28/Ig, and B7.2/Ig (antigens generated in-house). The antigens were platecoated overnight at +4°C at 1ug/ml 100ul/well in 0.1M sodium bicarbonate buffer, pH 9.6. The plate was then washed with PBST (PBS + 0.1% Tween-20) three times using a NUNC plate washer. The plate was blocked with PBST+0.5%BSA at 150 ul/well. The plate was incubated at RT for 1 hour then washed with PBST three times. Next the anti-CTLA-4 antibodies of the invention were diluted in block at 1 µg/ml and were added to the plate. The plate was incubated at RT for 1 hour then washed with PBST three times. The wells that contained the antibodies of the invention were then treated with 100 µl/well anti-human IgG2-HRP (Southern Biotech Cat No.9070-05) at a 1:4000 dilution in block. Also, one row was treated with anti-human IgG (Jackson Cat No. 209-035-088) to normalize for platecoating. This antibody was diluted to 1:5000 in block and added at 100 ul/well. Also, one row was treated with anti-human CTLA-4-HRP (Pharmingen Cat No. 345815/Custom HRP conjugated) as a positive control. This antibody was used at 0.05 ug/ml diluted in block. The plate was incubated at RT for 1 hour then washed with PBST three times. LBA chemiluminescent substrate (Pierce) was added at 100 µl/well and the plate was incubated on a plateshaker for 5 min. The plate was then read using a lumi-imager for a 2 min. exposure.

### IGEN CTLA-4-Ig Selectivity Binding Assay: Materials and Methods

M-450 Dynabeads (Dynal A.S, Oslo, Norway #140.02) were washed 3X with Na phosphate buffer, pH 7.4 and resuspended in Na phosphate buffer. 1.0 µg CTLA-4-Ig(G1), 1.0 µg CD28-Ig(G1) or 1.0 to 3.0 µg B7.2-Ig(G1) (Repligen, Inc. Needham, MA) were added to 100 µl of beads and incubated overnight on a rotator at 4°C. On day 2 the beads were washed 3X in 1% BSA plus 0.05% Tween-20 in Dulbecco's PBS and blocked for 30 minutes. Beads were diluted 1 to 10 with blocking buffer and 25 µl of the coated beads were added to 12x75 mm polypropylene tubes. All samples were tested in duplicate. 50 µl test antibody (1 µg/ml final concentration) or blocking buffer was added to the tubes and incubated for 30 minutes on the Origen 1.5 Analyzer carousel (IGEN International, Inc., Gaithersburg, MD) at RT, vortexing at 100 rpm. 25 µl of ruthenylated murine anti-human IgG1, IgG2 or IgG4 (Zymed, Inc. San Francisco, CA #05-3300, 05-3500 and 05-3800) (final concentration of 3 µg/ml in 100 µl total volume) was added to the tubes. Tubes were incubated for 30 minutes at RT on the carousel vortexing at 100 rpm. 200 µl of Origen assay buffer (IGEN International, Inc., Gaithersburg, MD #402-050-03) per tube was added and briefly vortexed and then the tubes were counted in the Origen Analyzer and ECL (electrochemiluminescence) units were determined for each tube. Normalization factors were determined to correct for differences in binding of fusion proteins to Dynabeads, and ECL units were corrected for non-specific binding before calculating selectivity ratios.

The results from the assays are provided in Tables IVA and IVB.

**TABLE IVA**

| Clone | Isotype | CTLA4/CD28 ELISA | CTLA4/B7.2 ELISA | CTLA4/CD44 ELISA | CTLA4/CD28 IGEN | CTLA4/B7.2 IGEN |
|---|---|---|---|---|---|---|
| 3.1.1 | IgG2 | >500:1 (n=3) | >500:1 (n=3) | >500:1 (n=3) | >500:1 (n=2) | >500:1 (n=1) |
| | | | | | | 195:1 (n=1) |
| 4.1.1 | IgG2 | >500:1 (n=3) | >500:1 (n=2) | >500:1 (n=3) | >500:1 (n=1) | >500:1 (n=1) |
| | | | 485:1 (n=1) | | 261:1 (n=1) | 107:1 (n=1) |
| 4.8.1 | IgG2 | >500:1 (n=3) | >500:1 (n=2) | >500:1 (n=3) | >500:1 (n=2) | >500:1 (n=2) |
| | | | 190:1 (n=1) | | | |
| 4.9.1 | IgG2 | >500:1 (n=2) | >500:1 (n=2) | >500:1 (n=3) | >500:1 (n=1) | >500:1 (n=1) |
| | | 244:1 (n=1) | 33:1 (n=1) | | | |
| 4.10.2 | IgG2 | >500:1 (n=3) | >500:1 (n=3) | >500:1 (n=3) | >500:1 (n=1) | >500:1 (n=1) |
| 4.13.1 | IgG2 | >500:1 (n=2) | >500:1 (n=3) | >500:1 (n=3) | >500:1 (n=1) | >500:1 (n=2) |
| | | 46:1 (n=1) | | | 329:1 (n=1) | |
| 4.14.3 | IgG2 | >500:1 (n=2) | >500:1 (n=2) | >500:1 (n=2) | >413:1 (n=1) | >234:1 (n=1) |
| | | 80:1 (n=1) | 10:1 (n=1) | 126:1 (n=1) | | |
| 6.1.1 | IgG2 | >500:1 (n=2) | >500:1 (n=3) | >500:1 (n=3) | >500:1 (n=2) | >500:1 (n=2) |
| | | 52:1 (n=1) | | | | |

**TABLE IVB**

| Clone | isotype | CTLA4/CD28 ELISA | CTLA4/B7.2 ELISA | CTLA4/hIgG ELISA |
|---|---|---|---|---|
| 4.1.1 | IgG2 | >500:1 (n=3) | >500:1 (n=2) | >500:1 (n=3) |
| 11.2.1 | IgG2 | >500:1 (n=3) | >500:1 (n=3) | >500:1 (n=3) |

### EXAMPLE 9

### Human T-Cell Signal Model

In order to further define the activity of antibodies in accordance with the invention to act as immune regulators, we developed certain T-cell assays in order to quantify the enhancement of T-cell IL-2 production upon blockade of CTLA-4 signal with the antibodies. The following materials and methods were utilized in connection with the experiments:

### Materials and Methods

Freshly isolated human T cells were prepared by using Histopaque (Sigma, St. Louis, MO #A-70543) and T-kwik (Lympho-Kwik, One Lambda, Canoga Park, CA, #LK-50-T), and stimulated with PHA (1 µg/ml) (Purified Phytohemagglutinin, Murex Diagnostics Ltd. Dartford, England, #HA 16) in medium (RPMI 1640 containing L-glutamine, MEM non-essential amino acids, penicillin, streptomycin, 25 mM Hepes and 10% FBS) at a concentration of 1x10⁶ cells/ml and incubated at 37°C for 2 days. The cells were washed and diluted in medium to 2x10⁶ cells/ml. Raji cells (Burkitt lymphoma, Human ATCC No.: CCL 86 Class II American Type Culture Collection Rockville, MD) were treated with mitomycin C (Sigma St. Louis, MO, # M-4287) (25 µg/ml) for one hour at 37°C. The Raji cells were washed 4X in PBS and resuspended at 2x10⁶ cells/ml. Human T cell blasts (5x10⁵/ml), Raji cells (5x10⁵/ml) and anti-CTLA-4 antibodies or an isotyped-matched control antibody at various concentrations were added to 96-well microtiter plates and the plates were incubated at 37°C for 72 hours. Total volume per well was 200 µl. Seventy-two hours post stimulation, the plates were spun down and supernatant removed and frozen for later determination of IL-2 (Quantikine IL-2 ELISA kit, R&D Systems, Minneapolis, MN, #D2050) and IFN-γ (Quantikine IFN-g ELISA kit, R&D Systems). Cytokine enhancement was defined as the difference between cytokine levels in cultures containing an anti-CTLA-4 blocking mAb versus an isotype-matched control antibody. For flow cytometry experiments, Raji cells were washed 1x with FACS buffer (PBS containing 2% heat inactivated FCS, 0.025% sodium azide). Cell pellets were resuspended in FACS buffer at 1x10⁶ cells/100 µl and incubated with 10 µl of anti-CD80-PE (Becton Dickinson, San Jose, CA) or anti-CD86-PE (Pharmingen, San Diego, CA) for 30 minutes at room temperature. Cells were washed twice and resuspended in 1 ml FACS buffer. Flow cytometry was performed using a Becton Dickinson FACSort. Histogram markers were set by analysis of relevant isotype control antibodies (Caltag, Burlingame, CA).

In general, we have developed an assay that can be used for rapid determination of T-cell IL-2 upregulation. As will be appreciated, stimulation of T cells is B7 and CD28 dependent. Further, washed T blasts do not make detectable IL-2 and Raji cells do not make detectable IL-2 even when stimulated with LPS or PWM. However, in combination, the T blasts co-cultured with Raji cells can model B7, CTLA-4, and CD28 signaling events and the effects of antibodies thereon can be assessed.

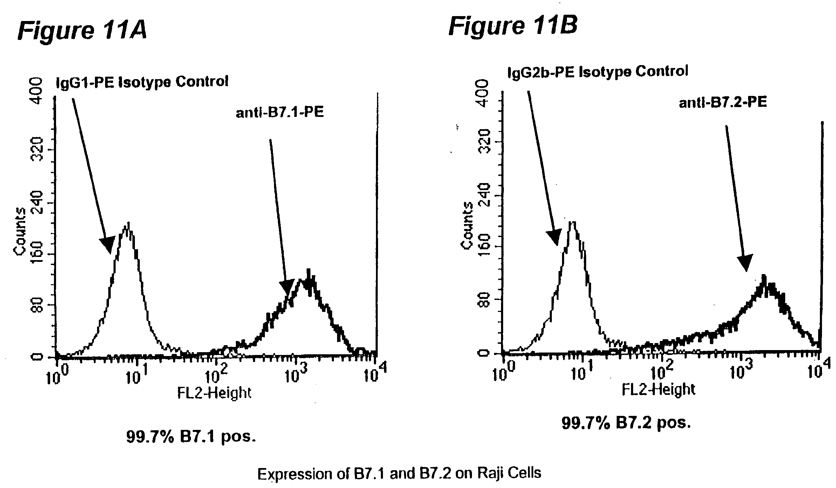

Figure 11 shows the expression of B7-1 and B7-2 on Raji cells using anti-CD80-PE and anti-CD86-PE mAbs using flow cytometry (FACs) as described in Example 6.

Figure 12 shows the concentration dependent enhancement of IL-2 production in the T cell blast/Raji assay induced by CTLA-4 blocking antibodies (BNI3 (PharMingen) and the 4.1.1, 4.8.1, and 6.1.1 antibodies of the invention).

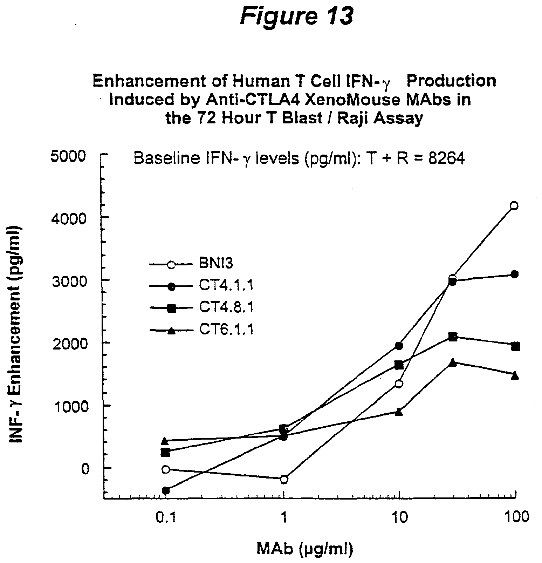

Figure 13 shows the concentration dependent enhancement of IFN-γ production in the T cell blast/Raji assay induced by CTLA-4 blocking antibodies (BNI3 (PharMingen) and the 4.1.1, 4.8.1, and 6.1.1 antibodies of the invention) (same donor T cells).

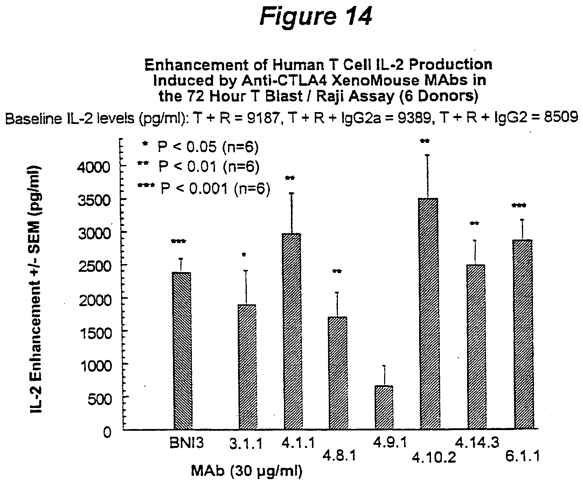

Figure 14 shows the mean enhancement of IL-2 production in T cells from 6 donors induced by CTLA-4 blocking antibodies in the T cell blast/Raji assay. It is interesting to consider that the mAb, CT4.9.1, binds to CTLA4 but does not block B7 binding. Thus, simply binding to CTLA-4 is insufficient by itself to provide a functional antibody of the invention.

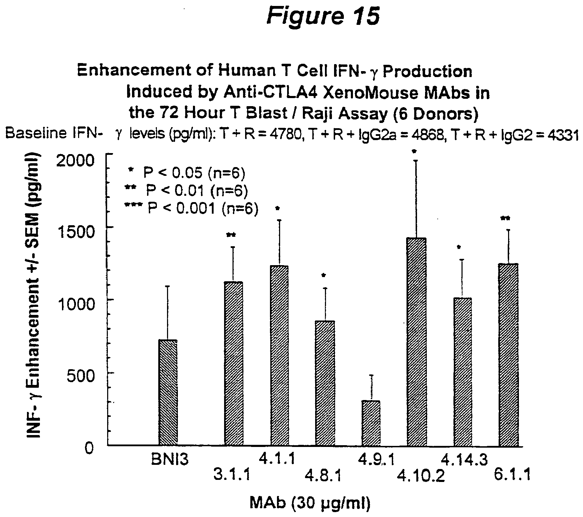

Figure 15 shows the mean enhancement of IFN-γ production in T cells from 6 donors induced by CTLA-4 blocking antibodies in the T cell blast/Raji assay.

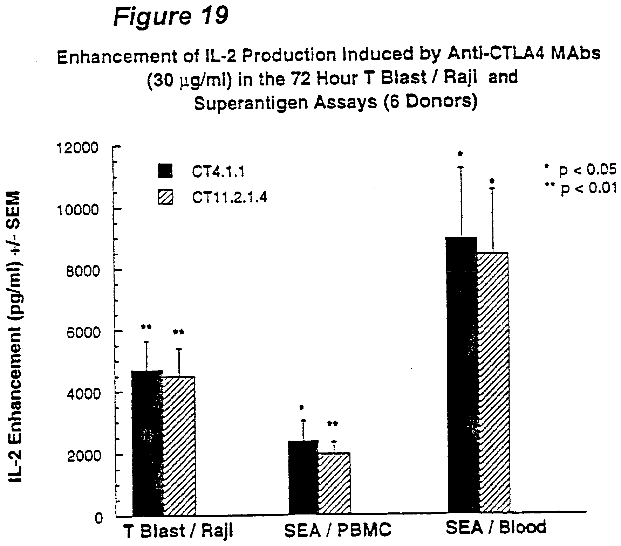

Figure 19 shows a comparison between the 4.1.1 and 11.2.1 antibodies of the invention at a concentration of 30µg/ml in the 72 hour T cell blast/Raji assay as described in this Example 9 and the Superantigen assay described in Example 10.

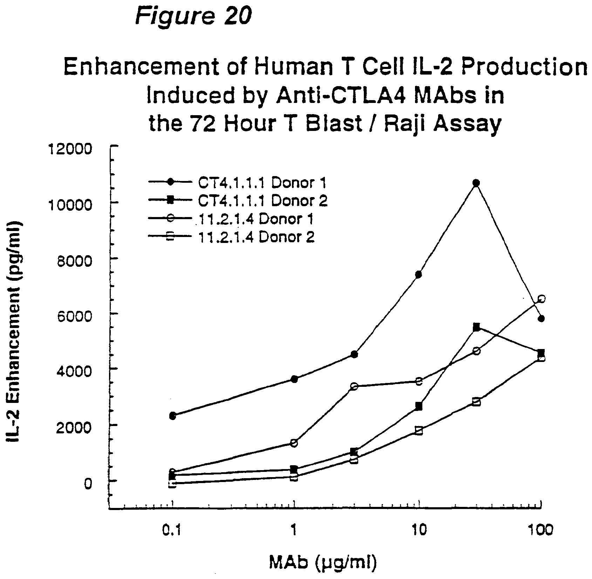

Figure 20 shows the concentration dependent enhancement of IL-2 production in the T cell blast/Raji assay induced by the 4.1.1 and 11.2.1 CTLA4 antibodies of the invention.

The following Table IVc provides information related to mean enhancement and range of enhancement of cytokine response in the Raji and SEA assays of the invention. Each of the experiments included in the results are based on antibody at a dose of 30 µg/ml and measured at 72 hours. Numbers of donors used in the experiments as well as responses are shown.

**TABLE IVC**

| Assay | mAb | Cytokine | Mean Enhancement pg/ml | SEM | Range Enhancement pg/ml | n | Donor Response |
|---|---|---|---|---|---|---|---|
| cell blast/Raji | 4.1.1 | IL-2 | 3329 | 408 | 0 to 8861 | 42 | 19 of 21 |
| T cell blast/Raji | 4.1.1 | IFN-γ | 3630 | 980 | 600 to 13939 | 17 | 13 of 13 |
| cell blast/Raji | 11.2.1 | IL-2 | 3509 | 488 | 369 to 6424 | 18 | 14 of 14 |
| SEA (PBMC) | 4.1.1 | IL-2 | 2800 | 312 | 330 to 6699 | 42 | 17 of 17 |
| SEA (PBMC) | 11.2.1 | IL-2 | 2438 | 366 | 147 to 8360 | 25 | 15 of 15 |
| SEA (Whole Blood) | 4.1.1 | IL-2 | 6089 | 665 | -168 to 18417 | 46 | 15 of 17 |
| SEA (Whole Blood) | 11.2.1 | IL-2 | 6935 | 700 | -111 to 11803 | 25 | 12 of 14 |

### EXAMPLE 10

### Human T-Cell Signal Model

We developed a second cellular assay in order to quantify the enhancement of T-cell IL-2 upregulation upon blockade of CTLA-4 signal with the antibodies. The following materials and methods were utilized in connection with the expeiments:

### Materials and Methods

Human PBMC were prepared using Accuspin. Microtiter plates were precoated with an anti-CD3 antibody (leu4, Becton Dickinson) (60 ng/ml) and incubated for 2 hours at 37°C. hPBMC were added to the wells at 200,000 cells per well. Staphylcoccus enterotoxin A (SEA) (Sigma) was added to the wells at 100 ng/ml. Antibodies were added to the wells, usually at 30 µg/ml. Cells were then stimulated for 48, 72 or 96 hours. Plates were centrifuged at the desired time-point and supernatants were removed from the wells. Thereafter, supernatants were checked for IL-2 production using ELISA (R&D Systems).

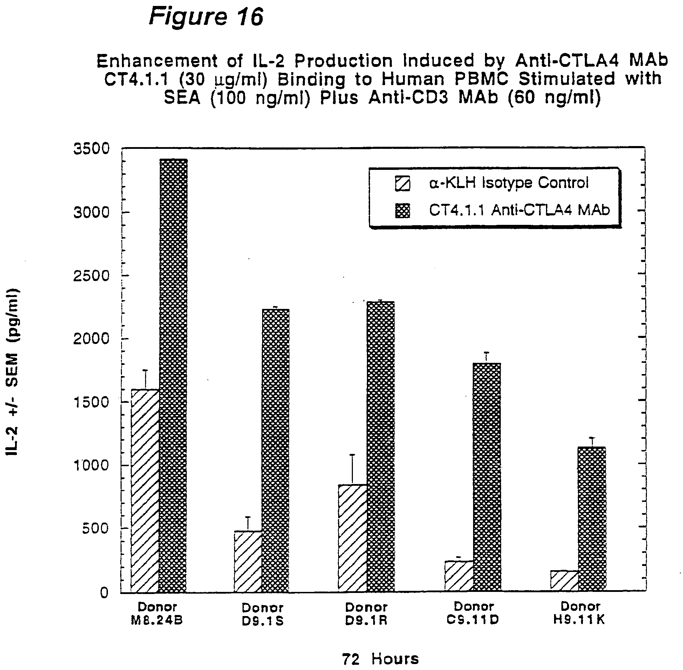

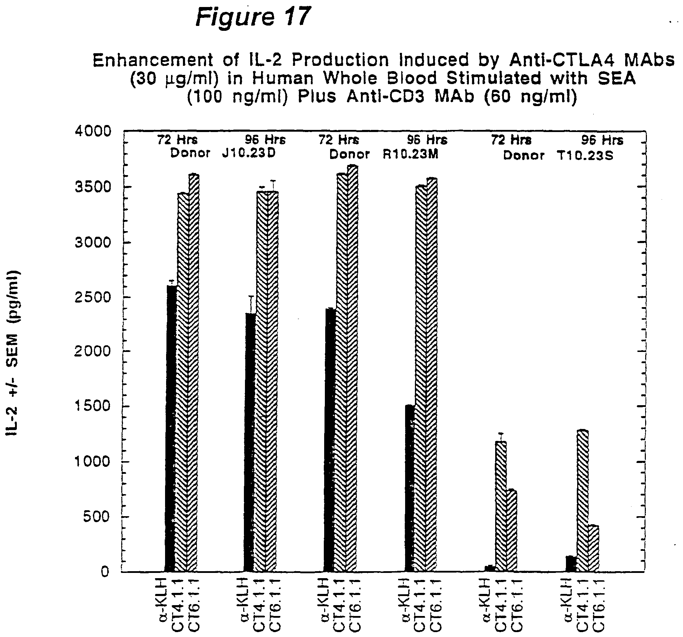

Results from these experiments are shown in Figures 16, 17, and 21. In Figure 16, induction of IL-2 production in hPBMC from 5 donors was measured 72 hours after stimulation. In Figure 17, results are shown from measurement of whole blood, analyzing the difference in induction of IL-2 production in the blood of 3 donors as measured at 72 and 96 hours after stimulation.

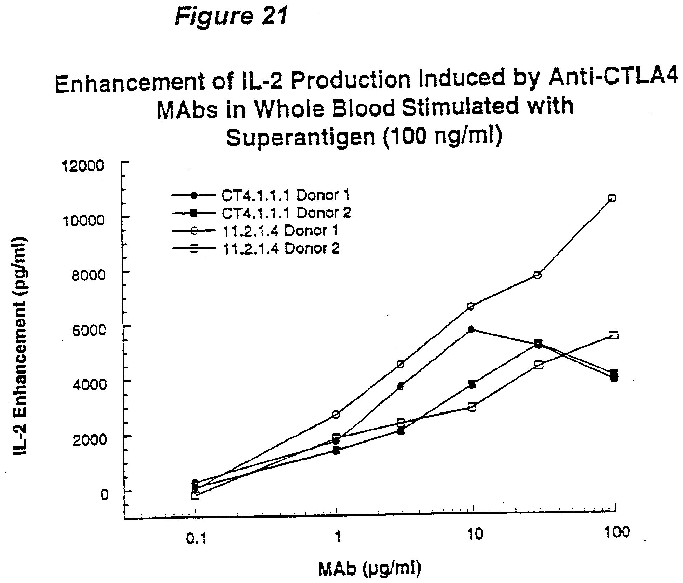

In Figure 21, the enhancement of IL-2 production in whole blood of 2 donors as measured at 72 hours after stimulation.

### EXAMPLE 11

### Tumor Animal Model

We have established an animal tumor model for the *in vivo* analysis of anti-murine-CTLA-4 antibodies in inhibiting tumor growth. In the model, a murine fibrosarcoma tumor is grown and the animals are treated with anti-murine-CTLA-4 antibodies. The materials and methods for establishment of the model are provided below:

### Materials and Methods

Female A/J mice (6-8 weeks old) were injected subcutaneously on the dorsal side of the neck with 0.2 ml of SalN tumor cells (1x10⁶) (Baskar 1995). Anti-murine CTLA-4 or an isotype matched control antibody (PharMingen, San Diego, CA, 200 ug/animal) were injected intraperitioneally on days 0, 4, 7 and 14 following the injection of tumor cells. Tumor measurements were taken during the course of the 3-4 week experiments using a Starrett SPC *Plus* electronic caliper (Athol, MA) and tumor size was expressed as the surface area covered by tumor growth (mm²).

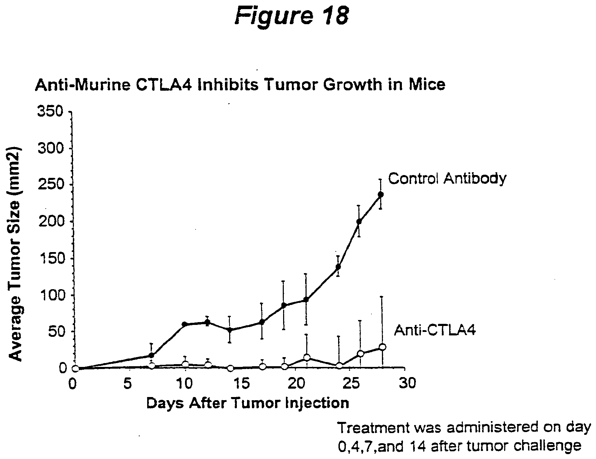

Figure 18 shows the inhibition of tumor growth with an anti-murine CTLA-4 antibody in a murine fibrosarcoma tumor model. As shown in Figure 18, animals treated with anti-CTLA-4 had a reduction in tumor growth as compared to animals treated with an isotype control antibody. Accordingly, anti-murine CTLA4 mAbs are capable of inhibiting growth of a fibrosarcoma in a mouse tumor model.

It is expected that antibodies that are cross-reactive with murine CTLA-4 would perform similarly in the model. However, of the antibodies of the invention that have been checked for cross-reactivity, none are cross-reactive with murine CTLA-4.

### EXAMPLE 12

### Tumor Animal Model

In order to further investigate the activity of antibodies in accordance with the invention, a xenograft SCID mouse model was designed to test the eradication of established tumors and their derived metastases. In the model, SCID mice are provided with grafted human T cells and are implanted with patient-derived non-small cell lung cell (NSCL) or colorectal carcinoma (CC) cells. Implantation is made into the gonadal fat pads of SCID mice. The tumors are allowed to grow, and thereafter removed. The mice develop human-like tumor and liver metastases. Such a model is described in Bumpers et al J. Surgical Res. 61:282-288 (1996).

It is expected that antibodies of the invention will inhibit growth of tumors formed in such mice.
Alegre et al. J Immunol 157:4762-70 (1996)
Allison and Krummel Science 270:932-933 (1995)
Balzano et al. Int J Cancer Suppl 7:28-32 (1992)
Blair et al. J Immunol 160:12-5 (1998)
Blake and Litzi-Davis BioConjugate Chem. 3:510-513 (1992)
Boussiotis et al. Proc Natl Acad Sci U S A 90:11059-63 (1993)
Bowie et al. Science 253:164 (1991)
Bruggeman et al. PNAS USA 86:6709-6713 (1989)
Bruggeman, M. and Neuberger, M.S. in Methods: A companion to Methods in Enzymology 2:159-165 (Lerner et al. eds. Academic Press (1991))
Bruggemann et al., "Human antibody production in transgenic mice: expression from 100 kb of the human IgH locus." Eur. J. Immunol. 21:1323-1326 (1991)
Bruggemann, M. and Neuberger, M.S. "Strategies for expressing human antibody repertoires in transgenic mice." Immunology Today 17:391-397 (1996)
Brunet et al. Nature 328:267-270 (1987)
Bumpers et al J. Surgical Res. 61:282-288 (1996)
Capsey et al. Genetically Engineered Human Therapeutic Drugs (Stockton Press, NY (1988))
Castan et al. Immunology 90:265-71 (1997)
Cepero et al. J Exp Med 188:199-204 (1998)
Chen et al. "Immunoglobulin gene rearrangement in B-cell deficient mice generated by targeted deletion of the JH locus" International Immunology 5:647-656 (1993)
Chen et al. Cell 71:1093-1102 (1992)
Chen et al. Human Gene Therapy 5:595-601 (1994)
Chiswell and McCafferty TIBTECH 10:80-84 (1992)
Choi et al. "Transgenic mice containing a human heavy chain immunoglobulin gene fragment cloned in a yeast artificial chromosome" Nature Genetics 4:117-123 (1993)
Chothia & Lesk J. Mol. Biol. 196:901-917 (1987)
Chothia et al. Nature 342:878-883 (1989)
Chuang et al. J. Immunol. 159:144-151(1997)
Coligan et al., Unit 2.1, "Enzyme-linked immunosorbent assays," in Current protocols in immunology (1994)
Cwirla et al. PNAS USA 87:6378-6382 (1990)
Dariavach et al. Eur. J. Immunol. 18:1901-1905 (1988)
Dayhoff, M.O., in Atlas of Protein Sequence and Structure, pp. 101-110 (Volume 5, National Biomedical Research Foundation (1972)) and Supplement 2 to this volume, pp. 1-10
de Boer et al. Eur J Immunol 23:3120-5 (1993)
Eckstein, Ed., Oxford University Press, Oxford England (1991))
Evans et al. J. Med. Chem. 30:1229 (1987)
Fallarino et al. J Exp Med 188:205-10 (1998)
Fanger et al. Immunol Methods 4:72-81 (1994)
Fauchere, J. Adv. Drug Res. 15:29 (1986)
Fishwild et al., "High-avidity human IgCrγ monoclonal antibodies from a novel strain of minilocus transgenic mice." Nature Biotech. 14:845-851 (1996).
Freeman et al. J Exp Med 178:2185-92 (1993)
Freeman et al. J Immunol 161:2708-15 (1998)
Freeman et al. Science 262:907-9 (1993)
Fundamental Immunology Ch. 7 (Paul, W., ed., 2nd ed. Raven Press, N.Y. (1989))
Galfre, G. and Milstein, C., "Preparation of monoclonal antibodies: strategies and procedures." Methods Enzymol. 73:3-46 (1981)
Gorman et al. P.N.A.S. 79:6777 (1982)
Green and Jakobovits J. Exp. Med. 188:483-495 (1998)
Green et al. Nature Genetics 7:13-21 (1994)
Grosschedl et al. Cell 41:885 (1985)
Hanes and Plucthau PNAS USA 94:4937-4942 (1997)
Harding et al. Nature 356:607-609 (1994)
Harper et al. J Immunol 147:1037-44 (1991)
Hathcock et al. Science 262:905-7 (1993)
Hoganboom et al. Immunol. Reviews 130:43-68 (1992)
Horspool et al. J Immunol 160:2706-14 (1998)
Houghten et al. Biotechniques 13:412-421 (1992)
Houghten PNAS USA 82:5131-5135 (1985)
Hurwitz et al. J Neuroimmunol 73:57-62 (1997)
Hurwitz et al. Proc Natl Acad Sci U S A 95:10067-71 (1998)
Immunology - A Synthesis (2nd Edition, E.S. Golub and D.R. Gren, Eds., Sinauer Associates, Sunderland, Mass. (1991))
Introduction to Protein Structure (C. Branden and J. Tooze, eds., Garland Publishing, New York, N.Y. (1991))
Jakobovits et al., "Germ-line transmission and expression of a human-derived yeast artificial-chromosome." Nature 362:255-258 (1993)
Jakobovits, A. et al., "Analysis of homozygous mutant chimeric mice: Deletion of the immunoglobulin heavy-chain joining region blocks B-cell development and antibody production." Proc. Natl. Acad. Sci. USA 90:2551-2555 (1993)
Jakobovits, A., "Humanizing the mouse genome." Current Biology 4:761-763 (1994)
Jakobovits, A., "Production of fully human antibodies by transgenic mice." Current Opinion in Biotechnology 6:561-566 (1995)
Junghans et al. in Cancer Chemotherapy and Biotherapy 655-686 (2d edition, Chafner and Longo, eds., Lippincott Raven (1996))
Kabat et al. (1991) Sequences of Proteins of Immunological Interest, N.I.H. publication no. 91-3242
Kabat Sequences of Proteins of Immunological Interest (National Institutes of Health, Bethesda, Md. (1987 and 1991))
Kostelny et al. J. Immunol. 148:1547-1553 (1992)
Krummel and Allison J Exp Med 182:459-65 (1995)
Krummel et al. Int Immunol 8:519-23 (1996)
Kuchroo et al. Cell 80:707-18 (1995)
Kwon et al. PNAS USA 94:8099-103 (1997)
LaPlanche et al. Nucl. Acids Res. 14:9081 (1986)
Lenschow et al. Proc Natl Acad Sci U S A 90:11054-8 (1993)
Lenschow et al. Science 257:789-792 (1992)
Lin et al. J Exp Med 188:199-204 (1998)
Linsley et al. J Exp Med 176:1595-604 (1992)
Linsley et al. J. Exp. Med. 174:561-569 (1991)
Linsley et al. Science 257:792-795 (1992)
Liu et al. J.Immunol.139:3521 (1987)
Liu et al. P.N.A.S. 84:3439 (1987)
Lonberg et al., "Antigen-specific human antibodies from mice comprising four distinct genetic modifications." Nature 368:856-859 (1994).
Luhder et al. J Exp Med 187:427-32 (1998)
Marasco Gene Therapy 4:11-15 (1997)
Markees et al. J Clin Invest 101:2446-55 (1998)
Marks et al., "Oligonucleotide primers for polymerase chain reaction amplification of human immunoglobulin variable genes and design of family-specific oligonucleotide probes." Eur. J. Immunol. 21:985-991 (1991)
McCoy et al. J Exp Med 186:183-7 (1997)
Mendez et al. Nature Genetics 15:146-156 (1997)
Needleman and Wunsch J. Mol. Biol. 48:443 (1970)
Okayama et al. Mol. Cell. Bio. 3:280 (1983)
Parmley and Smith Gene 73:305-318 (1988)
Pearson and Lipman Proc. Natl. Acad. Sci. (U.S.A.) 85:2444 (1988)
Perez et al. Immunity 6:411-7 (1997)
Perrin et al. Immunol Res 14:189-99 (1995)
Perrin et al. J Immunol 157:1333-6 (1996)
Pinalla et al. Biotechniques 13:901-905 (1992)
Proteins, Structures and Molecular Principles (Creighton, Ed., W. H. Freeman and Company, New York (1984))
Razi-Wolf et al. Proc Natl Acad Sci U S A 90:11182-6 (1993)
Remington's Pharmaceutical Sciences (15th ed, Mack Publishing Company, Easton, PA (1975)), particularly Chapter 87 by Blaug, Seymour
Rizo and Gierasch Ann. Rev. Biochem. 61:387 (1992)
Russel et al. Nucl. Acids Research 21:1081-1085 (1993)
Schwartz Cell 71:1065 (1992)
Scott TIBS 17:241-245 (1992)
Smith and Waterman Adv. Appl. Math. 2:482 (1981)
Songsivilai & Lachmann Clin. Exp. Immunol. 79: 315-321 (1990)
Stec et al. J. Am. Chem. Soc. 106:6077 (1984)
Stein et al. Nucl. Acids Res. 16:3209 (1988)
Taylor et al., "A transgenic mouse that expresses a diversity of human sequence heavy and light chain immunoglobulins." Nucleic Acids Research 20:6287-6295 (1992)
Taylor et al., "Human immunoglobulin transgenes undergo rearrangement, somatic mutation and class switching in mice that lack endogenous IgM." International Irnmunology 6:579-591 (1994)
The McGraw-Hill Dictionary of Chemical Terms (Parker, S., Ed., McGraw-Hill, San Francisco (1985))
Thornton et at. Nature 354:105 (1991)
Tivol et al. Immunity 3:541-7 (1995)
Townsend and Allison Science 259:368 (1993)
Traunecker et al. Int. J. Cancer (Suppl.) 7:51-52 (1992)
Tuaillon et al. "Analysis of direct and inverted DJH rearrangements in a human Ig heavy chain transgenic minilocus" J. Immunol. 154:6453-6465 (1995)
Tuaillon et al., "Human immunoglobulin heavy-chain minilocus recombination in transgenic mice: gene-segment use in µ and γ transcripts." Proc. Natl. Acad. Sci. USA 90:3720-3724 (1993)
Uhlmann and Peyman Chemical Reviews 90:543 (1990)
Van Parijs et al. J Exp Med 186:1119-28 (1997)
Veber and Freidinger TINS p.392 (1985)
Vitetta Immunol Today 14:252 (1993)
Walunas et al. Immunity 1:405-13 (1994)
Walunas et al. J Exp Med 183:2541-50 (1996)
Waterhouse et al. Science 270:985-988 (1995)
Winter and Harris Immunol Today 14:43-46 (1993)
Wright et al. Crit. Reviews in Immunol. 12125-168 (1992)
Yang et al. Cancer Res 57:4036-41 (1997)
Yi-qun et al. Int Immunol 8:37-44 (1996)
Zon et al. Anti-Cancer Drug Design 6:539 (1991)
Zon et al. Oligonucleotides and Analogues: A Practical Approach, pp. 87-108 (F. Eckstein, Ed., Oxford University Press, Oxford England (1991))
Fry et al. "Specific, irreversible inactivation of the epidermal growth factor receptor and erbB2, by a new class of tyrosine kinase inhibitor" Proc Natl Acad Sci U S A 95:12022-7 (1998)
Hoffman et al. "A model of Cdc25 phosphatase catalytic domain and Cdk-interaction surface based on the presence of a rhodanese homology domain" J Mol Biol 282:195-208 (1998)
Ginalski et al. "Modelling of active forms of protein kinases: p38-a case study" Acta Biochim Pol 44:557-64 (1997)
Jouko et al. "Identification of csk tyrosine phosphorylation sites and a tyrosine residue important for kinase domain structure" Biochem J 322:927-35 (1997)
Singh et al. "Structure-based design of a potent, selective, and irreversible inhibitor of the catalytic domain of the erbB receptor subfamily of protein tyrosine kinases" J Med Chem 40:1130-5 (1997)
Mandel et al. "ABGEN: a knowledge-based automated approach for antibody structure modeling" Nat Biotechnol 14:323-8 (1996)
Monfardini et al. "Rational design, analysis, and potential utility of GM-CSF antagonists" Proc Assoc Am Physicians 108:420-31 (1996)
Furet et al. "Modelling study of protein kinase inhibitors: binding mode of staurosporine and origin of the selectivity of CGP 52411" J Comput Aided Mol Des 9:465-72 (1995)
Ill et al. "Design and construction of a hybrid immunoglobulin domain with properties of both heavy and light chain variable regions" Protein Eng 10:949-57 (1997)
Martin et al. "The affinity-selection of a minibody polypeptide inhibitor of hurnan interleukin-6" EMBO J 13:5303-9 (1994)

U.S. Patent Application Serial No. 07/466,008, filed January 12, 1990
U.S. Patent Application Serial No. 07/574,748, filed August 29, 1990
U.S. Patent Application Serial No. 07/575,962, filed August 31, 1990
U.S. Patent Application Serial No. 07/610,515, filed November 8, 1990
U.S. Patent Application Serial No. 07/810,279, filed December 17, 1991
U.S. Patent Application Serial No. 07/853,408, filed March 18, 1992
U.S. Patent Application Serial No. 07/904,068, filed June 23, 1992
U.S. Patent Application Serial No. 07/919,297, filed July 24, 1992
U.S. Patent Application Serial No. 07/922,649, filed July 30, 1992
U.S. Patent Application Serial No. 07/990,860, filed December 16, 1992
U.S. Patent Application Serial No. 08/031,801, filed March 15,1993
U.S. Patent Application Serial No. 08/053,131, filed April 26, 1993
U.S. Patent Application Serial No. 08/096,762, filed July 22, 1993
U.S. Patent Application Serial No. 08/112,848, filed August 27, 1993
U.S. Patent Application Serial No. 08/155,301, filed November 18, 1993
U.S. Patent Application Serial No. 08/161,739, filed December 3, 1993
U.S. Patent Application Serial No. 08/165,699, filed December 10, 1993
U.S. Patent Application Serial No. 08/209,741, filed March 9, 1994
U.S. Patent Application Serial No. 08/234,145, filed April 28, 1994
U.S. Patent Application Serial No. 08/724,752, filed October 2, 1996
U.S. Patent Application Serial No. 08/730,639, filed October 11, 1996
U.S. Patent Application Serial No. 08/759,620, filed December 3, 1996
U.S. Patent Application Serial No. 08/759,620, filed December 3, 1996

U.S. Patent No. 4,399,216
U.S. Patent No. 4,681,581
U.S. Patent No. 4,683,195
U.S. Patent No. 4,683,202
U.S. Patent No. 4,735,210
U.S. Patent No. 4,740,461
U.S. Patent No. 4,816,397
U.S. Patent No. 4,912,040
U.S. Patent No. 4,959,455
U.S. Patent No. 5,101,827
U.S. Patent No. 5,102,990 (RE 35,500)
U.S. Patent No. 5,151,510
U.S. Patent No. 5,194,594
U.S. Patent No. 5,434,131
U.S. Patent No. 5,530,101
U.S. Patent No. 5,545,806
U.S. Patent No. 5,545,807
U.S. Patent No. 5,585,089
U.S. Patent No. 5,591,669
U.S. Patent No. 5,612,205
U.S. Patent No. 5,625,126
U.S. Patent No. 5,625,825
U.S. Patent No. 5,633,425
U.S. Patent No. 5,643,763
U.S. Patent No. 5,648,471
U.S. Patent No. 5,661,016
U.S. Patent No. 5,693,761
U.S. Patent No. 5,693,792
U.S. Patent No. 5,697,902
U.S. Patent No. 5,703,057
U.S. Patent No. 5,714,350
U.S. Patent No. 5,721,367
U.S. Patent No. 5,733,743
U.S. Patent No. 5,770,197
U.S. Patent No. 5,770,429
U.S. Patent No. 5,773,253
U.S. Patent No. 5,777,085
U.S. Patent No. 5,789,215
U.S. Patent No. 5,789,650
U.S. Patent No. 5,811,097

European Patent No. EP 0 546 073 B1
European Patent No. EP 0 463 151 B1, grant published June 12, 1996

International Patent Application No. WO 92/02190
International Patent Application No. WO 92/03918
International Patent Application No. WO 92/22645
International Patent Application No. WO 92/22647
International Patent Application No. WO 92/22670
International Patent Application No. WO 93/00431
International Patent Application No. WO 93/12227
International Patent Application No. WO 94/00569
International Patent Application No. WO 94/02602, published February 3, 1994
International Patent Application No. WO 94/25585
International Patent Application No. WO 94/29444
International Patent Application No. WO 95/01994
International Patent Application No. WO 95/03408
International Patent Application No. WO 95/24217
International Patent Application No. WO 95/33770
International Patent Application No. WO 96/14436
International Patent Application No. WO 96/34096, published October 31, 1996
International Patent Application No. WO 97/13852
International Patent Application No. WO 97/20574
International Patent Application No. WO 97/38137
International Patent Application No. WO 98/24884
International Patent Application No. WO 98/24893, published June 11, 1998

### EQUIVALENTS

The foregoing description and Examples detail certain preferred embodiments of the invention and describes the best mode contemplated by the inventors.

THE PRESENT DISCLOSURE CAN ALSO BE CHARACTERIZED BY THE FOLLOWING ITEMS:
1. An antibody that is capable of binding CTLA-4, comprising a heavy chain variable region amino acid sequence that comprises a contiguous amino acid sequence from within an FR1 sequence through an FR3 sequence that is encoded by a human V_{H}3-33 family gene and that comprises at least one of the amino acid substitutions in the CDR1 sequences, CDR2 sequences, or framework sequences shown in Figure 2.
2. The antibody of Item 1, wherein the amino acid sequence comprises a sequence selected from the group consisting of SEQ ID NO:1, SEQ ID NO:2, SEQ ID NO:3, SEQ ID NO:4, SEQ ID NO:5, SEQ ID NO:6, SEQ ID NO:8, SEQ ID NO:9, SEQ ID NO:10, SEQ ID NO:11, SEQ ID NO:12, SEQ ID NO:13, SEQ ID NO:63, SEQ ID NO:64, SEQ ID NO:66, SEQ ID NO:68, and SEQ ID NO:70.
3. The antibody of Item 1, further comprising a light chain variable region amino acid sequence comprising a sequence selected from the group consisting of a sequence comprising SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:65, SEQ ID NO:67, SEQ ID NO:69, and SEQ ID NO:71.
4. The antibody of Item 2, further comprising a light chain variable region amino acid sequence comprising a sequence selected from the group consisting of a sequence comprising SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:65, SEQ ID NO:67, SEQ ID NO:69, and SEQ ID NO:71.
5. An antibody comprising a heavy chain amino acid sequence comprising SEQ ID NO:1 and a light chain variable amino acid sequence comprising SEQ ID NO:14.
6. An antibody comprising a heavy chain amino acid sequence comprising SEQ ID NO:2 and a light chain variable amino acid sequence comprising SEQ ID NO:15.
7. An antibody comprising a heavy chain amino acid sequence comprising SEQ ID NO:4 and a light chain variable amino acid sequence comprising SEQ ID NO:17.
8. A isolated human monoclonal antibody that is capable of binding to CTLA-4.
9. The antibody of Item 8, wherein the antibody is capable of competing for binding with CTLA-4 with an antibody selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1.
10. The antibody of Item 8, wherein the antibody possesses a substantially similar binding specificity to CTLA-4 as an antibody selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1.
11. The antibody of Item 8, wherein the antibody is selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1.
12. The antibody of Item 8, wherein the antibody is not cross reactive with CTLA-4 from lower mammalian species.
13. The antibody of Item 12, wherein the lower mammalian species comprises mouse, rat, and rabbit.
14. The antibody of Item 8, wherein the antibody is cross reactive with CTLA-4 from primates.
15. The antibody of Item 14, wherein the primates comprise cynomolgous and rhesus monkeys.
16. The antibody of Item 8, wherein the antibody possesses a selectivity for CTLA-4 over CD28, B7-2, CD44, and hIgG1 of greater than about 100:1.
17. The antibody of Item 16, wherein the selectivity is about 500:1 or greater.
18. The antibody of Item 8, wherein a binding affinity of the antibody is about 10⁻⁹ M or greater.
19. The antibody of Item 18, wherein a binding affinity of the antibody is about 10⁻¹⁰ M or greater.
20. The antibody of Item 8, wherein the antibody inhibits binding between CTLA-4 and B7-2 with an IC₅₀ of lower than about 100 nM.
21. The antibody of Item 20, wherein the antibody inhibits binding between CTLA-4 and B7-2 with an IC₅₀ of lower than about 0.38 nM.
22. The antibody of Item 8, wherein the antibody inhibits binding between CTLA-4 and B7-1 with an IC₅₀ of lower than about 100 nM or greater.
23. The antibody of Item 22, wherein the antibody inhibits binding between CTLA-4 and B7-1 with an IC₅₀ of lower than about 0.50 nM.
24. The antibody of Item 8, wherein the antibody enhances IL-2 production in a T cell blast/Raji assay by about 500 pg/ml or greater.
25. The antibody of Item 24, wherein the antibody enhances IL-2 production in a T cell blast/Raji assay by about 3846 pg/ml or greater.
26. The antibody of Item 8, wherein the antibody enhances IFN-γ production in a T cell blast/Raji assay by about 500 pg/ml or greater.
27. The antibody of Item 26, wherein the antibody enhances IFN-γ production in a T cell blast/Raji assay by about 1233 pg/ml or greater.
28. The antibody of Item 8, wherein the antibody induces IL-2 production in a hPBMC or whole blood superantigen assay by about 500 pg/ml or greater.
29. The antibody of Item 26, wherein the antibody induces IL-2 production in a hPBMC or whole blood superantigen assay by about 1500 pg/ml or greater
30. The antibody of Item 26, wherein the antibody induces IL-2 production in a hPBMC or whole blood superantigen assay by greater than about 30% relative to control.
31. The antibody of Item 26, wherein the antibody induces IL-2 production in a hPBMC or whole blood superantigen assay by greater than about 50% relative to control.
32. A humanized antibody that possesses a substantially similar binding specificity to CTLA-4 as an antibody selected from the group consisting of 3.1.1, 4.1.1, 4.8.1, 4.10.2, 4.13.1, 4.14.3, 6.1.1, 11.2.1, 11.6.1, 11.7.1, 12.3.1.1, and 12.9.1.1.
33. The antibody of Item 32, wherein the antibody is not cross reactive with CTLA-4 from lower mammalian species.
34. The antibody of Item 33, wherein the lower mammalian species comprises mouse, rat, and rabbit.
35. The antibody of Item . 32, wherein the antibody is cross reactive with CTLA-4 from primates.
36. The antibody of Item 35, wherein the primates comprise cynomolgous and rhesus monkeys.
37. The antibody of Item 32, wherein the antibody possesses a selectivity for CTLA-4 over CD28, B7-2, CD44, and hIgG1 of greater than about 100:1.
38. The antibody of Item 37, wherein the selectivity is about 500:1 or greater.
39. The antibody of Item 32, wherein a binding affinity of the antibody is about 10⁻⁹ M or greater.
40. The antibody of Item 39, wherein a binding affinity of the antibody is about 10⁻¹⁰ M or greater.
41. The antibody of Item 32, wherein the antibody inhibits binding between CTLA-4 and B7-2 with an IC₅₀ of lower than about 100 nM.
42. The antibody of Item 41, wherein the antibody inhibits binding between CTLA-4 and B7-2 with an IC₅₀ of lower than about 0.38 nM.
43. The antibody of Item 32, wherein the antibody inhibits binding between CTLA-4 and B7-1 with an IC₅₀ of lower than about 100 nM or greater.
44. The antibody of Item 43, wherein the antibody inhibits binding between CTLA-4 and B7-1 with an IC₅₀ of lower than about 0.50 nM.
45. The antibody of Item 32, wherein the antibody enhances IL-2 production in a T cell blast/Raji assay by about 500 pg/ml or greater.
46. The antibody of Item 45, wherein the antibody enhances IL-2 production in a T cell blast/Raji assay by about 3846 pg/ml or greater.
47. The antibody of Item 32, wherein the antibody enhances IFN-γ production in a T cell blast/Raji assay by about 500 pg/ml or greater.
48. The antibody of Item 47, wherein the antibody enhances IFN-γ production in a T cell blast/Raji assay by about 1233 pg/ml or greater.
49. The antibody of Item 32, wherein the antibody enhances IL-2 production in a hPBMC or whole blood superantigen assay by about 500 pg/ml or greater.
50. The antibody of Item 49, wherein the antibody enhances IL-2 production in a hPBMC or whole blood superantigen assay by about 1500 pg/ml or greater
51. The antibody of Item 32, wherein the antibody enhances IL-2 production in a hPBMC or whole blood superantigen assay by greater than about 30% relative to control.
52. The antibody of Item 50, wherein the antibody enhances IL-2 production in a hPBMC or whole blood superantigen assay by greater than about 50% relative to control.
53. An antibody that binds to CTLA-4, comprising a heavy chain amino acid sequence comprising human FR1, FR2, and FR3 sequences encoded by a human V_{H} 3-33 gene family operably linked in frame with a CDR1, a CDR2, and a CDR3 sequence, the CDR1, CDR2, and CDR3 sequences being independently selected from the CDR1, CDR2, and CDR3 sequences illustrated in Figure 2.
54. The antibody of Item 32, further comprising any of the somatic mutations to the FR1, FR2, and FR3 sequences as illustrated in Figure 2.
55. An antibody that binds to CTLA-4, comprising a heavy chain amino acid sequence comprising human FR1, FR2, and FR3 sequences encoded by a human V_{H} 3-33 gene family operably linked in frame with a CDR1, a CDR2, and a CDR3 sequence, which antibody has the following properties:
   a binding affinity for CTLA-4 of about 10⁻⁹ or greater;
   inhibits binding between CTLA-4 and B7-1 with an IC₅₀ of about 100 nM or lower;
   inhibits binding between CTLA-4 and B7-2 with an IC₅₀ of about 100 nM or lower; and
   enhances cytokine production in an assay of human T cells by 500 pg/ml or greater.
56. An antibody that binds to CTLA-4, comprising a heavy chain amino acid sequence comprising FR1, FR2, and FR3 sequences operably linked in frame with a CDR1, a CDR2, and a CDR3 sequence independently selected from the CDR1, CDR2, and CDR3 sequences illustrated in Figures 2 and 3, which antibody has the following properties:
   a binding affinity for CTLA-4 of about 10⁻⁹ or greater;
   inhibits binding between CTLA-4 and B7-1 with an IC₅₀ of about 100 nM or lower;
   inhibits binding between CTLA-4 and B7-2 with an IC₅₀ of about 100 nM or lower; and
   enhances cytokine production in an assay of human T cells by 500 pg/ml or greater.
57. A cell culture system for assaying T cell stimulation, comprising a culture of human T cell blasts co-cultured with a Raji cell line.
58. The cell culture system of Item 57, wherein the T cell blasts are washed prior to culture with the Raji cell line.
59. An assay for measuring T cell stimulation, comprising:
   providing a culture of human T cell blasts and a Raji cell line; contacting the culture with an agent; and
   measuring cytokine production by the culture.
60. The assay of Item 59, wherein the T cell blasts are washed prior to culture with the Raji cell line.
61. The assay of Item 59, wherein the cytokine is IL-2.
62. The assay of Item 59, wherein the cytokine is IFN-γ.
63. The assay of Item 59, wherein cytokine production is measured in supernatant isolated from the culture.
64. The assay of Item 59, wherein the agent is an antibody.
65. The assay of Item 59, wherein the antibody binds to CTLA-4.
66. A functional assay for screening a moiety for T cell stimulatory function, comprising:
   providing a culture of human T cell blasts and a Raji cell line;
   contacting the culture with the moiety; and
   assessing cytokine production by the culture.
67. The assay of Item 66, wherein the T cell blasts are washed prior to culture with the Raji cell line.
68. The assay of Item 66, wherein the cytokine is IL-2.
69. The assay of Item 66, wherein the cytokine is IFN-γ.
70. The assay of Item 66, wherein cytokine production is assessed in supernatant isolated from the culture.
71. The assay of Item 66, wherein the moiety is an antibody.
72. The assay of Item 66, wherein the antibody binds to CTLA-4.
73. A T cell stimulatory assay for CTLA-4 inhibitory function, comprising contacting a culture comprising human T cell blasts and a Raji cell line with an agent and assessing cytokine production by the culture.
74. The assay of Item 73, wherein the T cell blasts are washed prior to culture with the Raji cell line.
75. The assay of Item 73, wherein the cytokine is IL-2.
76. The assay of Item 73, wherein the cytokine is IFN-γ.
77. The assay of Item 73, wherein cytokine production is assessed in supernatant isolated from the culture.
78. The assay of Item 73, wherein the agent is an antibody.
79. The assay of Item 73, wherein the antibody binds to CTLA-4.
80. A method for screening an agent for T cell stimulatory activity, comprising:
   contacting the agent with a cell culture comprising human T cell blasts and a Raji cell line; and
   assessing cytokine production by the culture.
81. The method of Item 80, wherein the T cell blasts are washed prior to culture with the Raji cell line.
82. The method of Item 80, wherein the cytokine is IL-2.
83. The method of Item 80, wherein the cytokine is IFN-γ.
84. The method of Item 80, wherein cytokine production is assessed in supernatant isolated from the culture.
85. The method of Item 80, wherein the agent is an antibody that binds to CTLA-4.
86. An assay for measuring T cell stimulation, comprising:
   providing a population of human peripheral blood mononuclear cells or human whole blood stimulated with staphylococcus enterotoxin A;
   contacting the culture with an agent; and
   measuring cytokine production by the cell population.
87. The assay of Item 86, wherein the cytokine is IL-2.
88. The assay of Item 86, wherein cytokine production is measured in supernatant isolated from the cell population.
89. The assay of Item 86, wherein the agent is an antibody.
90. The assay of Item 86, wherein the antibody binds to CTLA-4.
91. A functional assay for screening a moiety for T cell stimulatory function, comprising:
   providing a population of human peripheral blood mononuclear cells or human whole blood stimulated with staphylococcus enterotoxin A;
   contacting the culture with the moiety; and
   assessing cytokine production by the cell population.
92. The assay of Item 91, wherein the cytokine is IL-2.
93. The assay of Item 91, wherein cytokine production is assessed in supernatant isolated from the cell population.
94. The assay of Item 91, wherein the moiety is an antibody .
95. The assay of Item 91, wherein the antibody binds to CTLA-4.
96. A T cell stimulatory assay for CTLA-4 inhibitory function, comprising contacting a population of human peripheral blood mononuclear cells or human whole blood stimulated with staphylococcus enterotoxin A with an agent and assessing cytokine production by the cell population.
97. The assay of Item 96, wherein the cytokine is IL-2.
98. The assay of Item 96, wherein cytokine production is assessed in supernatant isolated from the cell population.
99. The assay of Item 96, wherein the agent is an antibody.
100. The assay of Item 96, wherein the antibody binds to CTLA-4.
101. A method for screening an agent for T cell stimulatory activity, comprising:
   contacting the agent with a population of human peripheral blood mononuclear cells or human whole blood stimulated with staphylococcus enterotoxin A; and
   assessing cytokine production by the cell population.
102. The method of Item 101, wherein the cytokine is IL-2.
103. The method of Item 101, wherein cytokine production is assessed in supernatant isolated from the cell population.
104. The method of Item 101, wherein the agent is an antibody that binds to CTLA-4.

## Claims

1. A human monoclonal antibody that binds to CTLA-4, or an antigen-binding fragment thereof, wherein said antibody or fragment competes for binding to CTLA-4 with a reference antibody, wherein the antibody or antigen-binding fragment thereof has the following properties:
a) a binding affinity for CTLA-4 of 10⁻⁹ M or greater as determined by BIAcore;
b) inhibits binding between CTLA-4 and B7-1 with an IC₅₀ of 100 nM or lower; and
c) inhibits binding between CTLA-4 and B7-2 with an IC₅₀ of 100 nM or lower,
wherein the reference antibody is selected from the group consisting of:
i) an antibody comprising a heavy chain that comprises the amino acid sequence of SEQ ID NO: 9 and a light chain that comprises the amino acid sequence of SEQ ID NO: 22;
ii) an antibody comprising a heavy chain that comprises the amino acid sequence of SEQ ID NO: 5 and a light chain that comprises the amino acid sequence of SEQ ID NO: 18;
iii) an antibody comprising a heavy chain that comprises the amino acid sequence of SEQ ID NO: 1 and a light chain that comprises the amino acid sequence of SEQ ID NO: 14;
iv) an antibody comprising a heavy chain that comprises the amino acid sequence of SEQ ID NO: 2 and a light chain that comprises the amino acid sequence of SEQ ID NO: 15;
v) an antibody comprising a heavy chain that comprises the amino acid sequence of SEQ ID NO: 8 and a light chain that comprises the amino acid sequence of SEQ ID NO: 21;
vi) an antibody comprising a heavy chain that comprises the amino acid sequence of SEQ ID NO: 3 and a light chain that comprises the amino acid sequence of SEQ ID NO: 16;
vii) an antibody comprising a heavy chain that comprises the amino acid sequence of SEQ ID NO: 4 and a light chain that comprises the amino acid sequence of SEQ ID NO: 17;
viii) an antibody comprising a heavy chain that comprises the amino acid sequence of SEQ ID NO: 10 and a light chain that comprises the amino acid sequence of SEQ ID NO: 23; and
ix) an antibody comprising a heavy chain that comprises the amino acid sequence of SEQ ID NO: 11 and a light chain that comprises the amino acid sequence of SEQ ID NO: 24.

2. The antibody or antigen-binding fragment according to claim 1, wherein said antibody comprises a heavy chain comprising the CDR1, CDR2 and CDR3 amino acid sequences in SEQ ID NO: 70.

3. The antibody or antigen-binding fragment according to claim 1, wherein said antibody comprises a light chain comprising the CDR1, CDR2 and CDR3 amino acid sequences in SEQ ID NO: 22.

4. The antibody or antigen-binding fragment according to claim 1, wherein said antibody comprises a heavy chain that comprises the amino acid sequence of SEQ ID NO: 70.

5. The antibody or antigen-binding fragment according to claim 1, wherein said antibody comprises a light chain that comprises the amino acid sequence of SEQ ID NO. 22.

6. A pharmaceutical composition comprising an antibody or antigen-binding fragment according to any one of claims 1 to 5 and a pharmaceutically acceptable carrier.

7. A pharmaceutical composition, comprising an antibody or antigen-binding fragment according to any one of claims 1 to 5 and a pharmaceutically acceptable carrier, for treating cancer.

8. A cell line that produces an antibody or antigen-binding fragment according to any one of claims 1 to 5.

9. The cell line of claim 8 that is a mammalian cell line.

10. The cell line of claim 9 that is a CHO cell line or an NSO cell line.

11. A nucleic acid that encodes the light and heavy chain amino acid sequences of an antibody or antigen-binding fragment as defined in any one of claims 1 to 5.

12. A method for production of an antibody or antigen-binding fragment according to any one of claims 1 to 5 comprising expressing said antibody or antigen binding fragment in a host cell line and recovering said antibody or antigen-binding fragment.

13. The method according to claim 12, wherein said host cell line is a mammalian cell line.

14. The method according to claim 13, wherein said mammalian cell line is a CHO cell line or an NSO cell line.

15. An antibody or antigen-binding fragment according to any one of claims 1 to 5 for use in treating cancer.

16. Use of an antibody or antigen-binding fragment according to any one of claims 1 to 5 in the manufacture of a medicament for treating cancer.

## Patentansprüche

1. Humaner monoclonaler Antikörper, der an CTLA-4 bindet, oder Antigenbindendes Fragment davon, wobei der Antikörper oder das Fragment um die Bindung an CTLA-4 mit einem Referenzantikörper konkurriert, wobei der Antikörper oder das Antigen-bindende Fragment davon die folgenden Eigenschaften aufweist:
a) eine Bindungsaffinität für CTLA-4 von 10⁻⁹ M oder größer, wie durch BIAcore bestimmt;
b) inhibiert die Bindung zwischen CTLA-4 und B7-1 mit einem IC₅₀ von 100 nM oder niedriger; und
c) inhibiert die Bindung zwischen CTLA-4 und B7-2 mit einem IC₅₀ von 100 nM oder niedriger,
wobei der Referenzantikörper ausgewählt ist aus der Gruppe bestehend aus:
i) einem Antikörper, umfassend eine schwere Kette, die die Aminosäuresequenz von SEQ ID NO:9 umfasst, und eine leichte Kette, die die Aminosäuresequenz von SEQ ID NO:22 umfasst;
ii) einem Antikörper, umfassend eine schwere Kette, die die Aminosäuresequenz von SEQ ID NO:5 umfasst, und eine leichte Kette, die die Aminosäuresequenz von SEQ ID NO:18 umfasst;
iii) einem Antikörper, umfassend eine schwere Kette, die die Aminosäuresequenz von SEQ ID NO:1 umfasst, und eine leichte Kette, die die Aminosäuresequenz von SEQ ID NO:14 umfasst;
iv) einem Antikörper, umfassend eine schwere Kette, die die Aminosäuresequenz von SEQ ID NO:2 umfasst, und eine leichte Kette, die die Aminosäuresequenz von SEQ ID NO:15 umfasst;
v) einem Antikörper, umfassend eine schwere Kette, die die Aminosäuresequenz von SEQ ID NO:8 umfasst, und eine leichte Kette, die die Aminosäuresequenz von SEQ ID NO:21 umfasst;
vi) einem Antikörper, umfassend eine schwere Kette, die die Aminosäuresequenz von SEQ ID NO:3 umfasst, und eine leichte Kette, die die Aminosäuresequenz von SEQ ID NO:16 umfasst;
vii) einem Antikörper, umfassend eine schwere Kette, die die Aminosäuresequenz von SEQ ID NO:4 umfasst, und eine leichte Kette, die die Aminosäuresequenz von SEQ ID NO:17 umfasst;
viii) einem Antikörper, umfassend eine schwere Kette, die die Aminosäuresequenz von SEQ ID NO:10 umfasst, und eine leichte Kette, die die Aminosäuresequenz von SEQ ID NO:23 umfasst; und
ix) einem Antikörper, umfassend eine schwere Kette, die die Aminosäuresequenz von SEQ ID NO:11 umfasst, und eine leichte Kette, die die Aminosäuresequenz von SEQ ID NO:24 umfasst.

2. Antikörper oder Antigen-bindendes Fragment nach Anspruch 1, wobei der Antikörper eine schwere Kette umfasst, die die CDR1-, CDR2- und CDR3-Aminosäuresequenzen in SEQ ID NO:70 umfasst.

3. Antikörper oder Antigen-bindendes Fragment nach Anspruch 1, wobei der Antikörper eine leichte Kette umfasst, die die CDR1-, CDR2- und CDR3-Aminosäuresequenzen in SEQ ID NO:22 umfasst.

4. Antikörper oder Antigen-bindendes Fragment nach Anspruch 1, wobei der Antikörper eine schwere Kette umfasst, die die Aminosäuresequenz von SEQ ID NO:70 umfasst.

5. Antikörper oder Antigen-bindendes Fragment nach Anspruch 1, wobei der Antikörper eine leichte Kette umfasst, die die Aminosäuresequenz von SEQ ID NO:22 umfasst.

6. Arzneimittel, das einen Antikörper oder ein Antigen-bindendes Fragment nach einem der Ansprüche 1 bis 5 und einen pharmazeutisch verträglichen Träger umfasst.

7. Arzneimittel, das einen Antikörper oder ein Antigen-bindendes Fragment nach einem der Ansprüche 1 bis 5 und einen pharmazeutisch verträglichen Träger umfasst, zur Behandlung von Krebs.

8. Zelllinie, die einen Antikörper oder ein Antigen-bindendes Fragment nach einem der Ansprüche 1 bis 5 herstellt.

9. Zelllinie nach Anspruch 8, die eine Säugerzelllinie ist.

10. Zelllinie nach Anspruch 9, die eine CHO-Zelllinie oder eine NSO-Zelllinie ist.

11. Nucleinsäure, die die Aminosäuresequenzen der leichten und schweren Kette eines Antikörpers oder eines Antigen-bindenden Fragments, wie in einem der Ansprüche 1 bis 5 definiert, codiert.

12. Verfahren zur Herstellung eines Antikörpers oder eines Antigen-bindenden Fragments nach einem der Ansprüche 1 bis 5, umfassend das Exprimieren des Antikörpers oder des Antigen-bindenden Fragments in einer Wirtszelllinie und das Gewinnen des Antikörpers oder des Antigen-bindenden Fragments.

13. Verfahren nach Anspruch 12, wobei die Wirtszelllinie eine Säugerzelllinie ist.

14. Verfahren nach Anspruch 13, wobei die Säugerzelllinie eine CHO-Zelllinie oder eine NSO-Zelllinie ist.

15. Antikörper oder Antigen-bindendes Fragment nach einem der Ansprüche 1 bis 5 zur Verwendung bei der Behandlung von Krebs.

16. Verwendung eines Antikörpers oder Antigen-bindenden Fragments nach einem der Ansprüche 1 bis 5 bei der Herstellung eines Medikaments zur Behandlung von Krebs.

## Revendications

1. Anticorps monoclonal humain qui se lie à CTLA-4, ou fragment de liaison d'antigène de celui-ci, où ledit anticorps ou fragment entre en compétition pour une liaison à CTLA-4 avec un anticorps de référence, où l'anticorps ou fragment de liaison d'antigène de celui-ci possède les propriétés suivantes :
a) une affinité de liaison pour CTLA-4 de 10⁻⁹ M ou plus grande telle que déterminée par BIAcore ;
b) empêche la liaison entre CTLA-4 et B7-1 avec un IC₅₀ de 100 nM ou plus bas ; et
c) empêche la liaison entre CTLA-4 et B7-2 avec un IC₅₀ de 100 nM ou plus bas,
où l'anticorps de référence est sélectionné dans le groupe consistant en :
i) un anticorps comprenant une chaîne lourde qui comprend la séquence d'acides aminés de SEQ ID NO: 9 et une chaîne légère qui comprend la séquence d'acides aminés de SEQ ID NO: 22 ;
ii) un anticorps comprenant une chaîne lourde qui comprend la séquence d'acides aminés de SEQ ID NO: 5 et une chaîne légère qui comprend la séquence d'acides aminés de SEQ ID NO: 18 ;
iii) un anticorps comprenant une chaîne lourde qui comprend la séquence d'acides aminés de SEQ ID NO: 1 et une chaîne légère qui comprend la séquence d'acides aminés de SEQ ID NO: 14 ;
iv) un anticorps comprenant une chaîne lourde qui comprend la séquence d'acides aminés de SEQ ID NO: 2 et une chaîne légère qui comprend la séquence d'acides aminés de SEQ ID NO: 15 ;
v) un anticorps comprenant une chaîne lourde qui comprend la séquence d'acides aminés de SEQ ID NO: 8 et une chaîne légère qui comprend la séquence d'acides aminés de SEQ ID NO: 21 ;
vi) un anticorps comprenant une chaîne lourde qui comprend la séquence d'acides aminés de SEQ ID NO: 3 et une chaîne légère qui comprend la séquence d'acides aminés de SEQ ID NO: 16 ;
vii) un anticorps comprenant une chaîne lourde qui comprend la séquence d'acides aminés de SEQ ID NO: 4 et une chaîne légère qui comprend la séquence d'acides aminés de SEQ ID NO: 17 ;
viii) un anticorps comprenant une chaîne lourde qui comprend la séquence d'acides aminés de SEQ ID NO: 10 et une chaîne légère qui comprend la séquence d'acides aminés de SEQ ID NO: 23 ; et
ix) un anticorps comprenant une chaîne lourde qui comprend la séquence d'acides aminés de SEQ ID NO: 11 et une chaîne légère qui comprend la séquence d'acides aminés de SEQ ID NO: 24.

2. Anticorps ou fragment de liaison d'antigène selon la revendication 1, où ledit anticorps comprend une chaîne lourde comprenant les séquences d'acides aminés CDR1, CDR2 et CDR3 dans SEQ ID NO: 70.

3. Anticorps ou fragment de liaison d'antigène selon la revendication 1, où ledit anticorps comprend une chaîne légère comprenant les séquences d'acides aminés CDR1, CDR2 et CDR3 dans SEQ ID NO: 22.

4. Anticorps ou fragment de liaison d'antigène selon la revendication 1, où ledit anticorps comprend une chaîne lourde qui comprend la séquence d'acides aminés de SEQ ID NO: 70.

5. Anticorps ou fragment de liaison d'antigène selon la revendication 1, où ledit anticorps comprend une chaîne légère qui comprend la séquence d'acides aminés de SEQ ID NO: 22.

6. Composition pharmaceutique comprenant un anticorps ou un fragment de liaison d'antigène selon l'une quelconque des revendications 1 à 5 et un support pharmaceutiquement acceptable.

7. Composition pharmaceutique, comprenant un anticorps ou un fragment de liaison d'antigène selon l'une quelconque des revendications 1 à 5 et un support pharmaceutiquement acceptable, pour traiter le cancer.

8. Ligne de cellules qui produit un anticorps ou un fragment de liaison d'antigène selon l'une quelconque des revendications 1 à 5.

9. Ligne de cellules selon la revendication 8, qui est une ligne de cellules de mammifère.

10. Ligne de cellules selon la revendication 9, qui est une ligne de cellules CHO ou une ligne de cellule NSO.

11. Acide nucléique qui code pour les séquences d'acides aminés de chaîne légère et lourde d'un anticorps ou fragment de liaison d'antigène tel que défini dans l'une quelconque des revendications 1 à 5.

12. Procédé de production d'un anticorps ou fragment de liaison d'antigène selon l'une quelconque des revendications 1 à 5, comprenant l'expression dudit anticorps ou fragment de liaison d'antigène dans une ligne de cellules hôtes et la récupération dudit anticorps ou fragment de liaison d'antigène.

13. Procédé selon la revendication 12, dans lequel ladite ligne de cellules hôtes est une ligne de cellules de mammifère.

14. Procédé selon la revendication 13, où ladite ligne de cellules de mammifère est une ligne de cellules CHO ou une ligne de cellules NSO.

15. Anticorps ou fragment de liaison d'antigène selon l'une quelconque des revendications 1 à 5 destiné à être utilisé pour traiter le cancer.

16. Utilisation d'un anticorps ou fragment de liaison d'antigène selon l'une quelconque des revendications 1 à 5 dans la fabrication d'un médicament pour traiter le cancer.
